(19) Europäisches Patentamt / European Patent Office / Office européen des brevets

(11) EP 4 768 489 A1

(12) EUROPEAN PATENT APPLICATION
published in accordance with Art. 153(4) EPC

(43) Date of publication:
**01.07.2026 Bulletin 2026/27**

(21) Application number: **24855869.4**

(22) Date of filing: **22.08.2024**

(51) International Patent Classification (IPC):
***C07D 498/04*** *(2006.01)* ***C07D 493/00*** *(2006.01)*
***C07D 491/00*** *(2006.01)* ***A61K 31/553*** *(2006.01)*
***A61K 31/423*** *(2006.01)* ***A61P 35/00*** *(2006.01)*

(52) Cooperative Patent Classification (CPC):
**A61K 31/423; A61K 31/553; A61P 35/00; C07D 491/00; C07D 493/00; C07D 498/04**

(86) International application number:
**PCT/CN2024/113870**

(87) International publication number:
**WO 2025/040147 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.08.2023 CN 202311073391**
**30.04.2024 CN 202410544791**
**15.08.2024 CN 202411125992**

(71) Applicant: **Chia Tai Tianqing Pharmaceutical Group Co., Ltd.**
**Lianyungang, Jiangsu 222062 (CN)**

(72) Inventors:
- **REN, Jing**
  **Lianyungang, Jiangsu 222062 (CN)**
- **XU, Sheng**
  **Lianyungang, Jiangsu 222062 (CN)**
- **HUANG, Yongkang**
  **Lianyungang, Jiangsu 222062 (CN)**
- **HU, Kongquan**
  **Lianyungang, Jiangsu 222062 (CN)**
- **WEI, Xiaochao**
  **Lianyungang, Jiangsu 222062 (CN)**
- **WEI, Jian**
  **Lianyungang, Jiangsu 222062 (CN)**

(74) Representative: **Lorenz Seidler Gossel Part. mbB**
**Widenmayerstr. 23**
**80538 München (DE)**

# (54) CYCLOBUTYL-CONTAINING COMPOUNDS

(57) The present application relates to the field of medicinal chemistry, relates to cyclobutyl-containing compounds, and particularly relates to compounds of formula I, a preparation method therefor, a pharmaceutical composition containing the compounds, and the use thereof in treating diseases such as cancers.

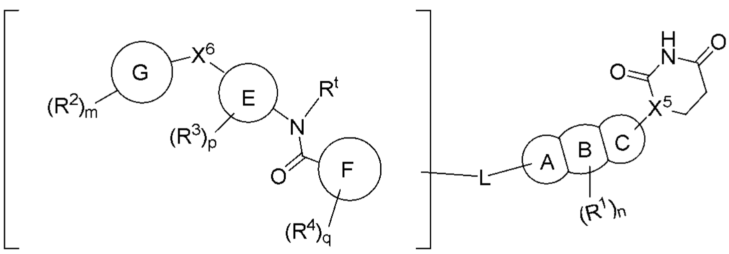

I

EP 4 768 489 A1

## Description

### CROSS-REFERENCE TO RELATED APPLICATIONS

The present application claims priority and benefit to the Chinese Patent Application No. 202311073391.5 filed with China National Intellectual Property Administration on August 23, 2023, the Chinese Patent Application No. 202410544791.8 filed with China National Intellectual Property Administration on April 30, 2024, and the Chinese Patent Application No. 202411125992.0 filed with China National Intellectual Property Administration on August 15, 2024, which are incorporated herein by reference in their entirety.

### TECHNICAL FIELD

The present application relates to a cyclobutyl-containing compound, a preparation method therefor, a pharmaceutical composition containing the compound, and use thereof for treating a related disease (e.g., cancer).

### BACKGROUND

The androgen receptor (AR) is a steroid receptor in the nuclear receptor superfamily. When bound to androgens (such as testosterone and dihydrotestosterone), AR is released from the complex formed by heat shock proteins for a phosphorylation reaction to form a dimer. The dimer is transferred into the nucleus and binds to a DNA fragment associated therewith, thereby stimulating transcription of its target gene. The transcriptional activity of androgen receptors activated by ligand binding is coordinated by proteins known as co-activators. The primary function of AR antagonists is to directly prevent testosterone or dihydrotestosterone from binding to androgen receptors, thereby blocking the effect of androgens on cells. This exerts anti-androgenic and cell growth inhibitory effects, ultimately resulting in cell apoptosis and achieving the important role in treating prostate cancer.

The proteolysis targeting chimera (PROTAC) molecule is a bifunctional compound capable of binding to a target protein and E3 ubiquitin ligase simultaneously. Such compounds can induce the target protein to be recognized by proteasomes of cells, cause the degradation of the target protein, and effectively reduce the content of the target protein in the cells. The introduction of ligands capable of binding to different target proteins into PROTAC molecules has made it possible to apply the PROTAC technology to the treatment of various diseases, and this technology has received much attention in recent years.

### SUMMARY

In one aspect, the present application relates to a compound of formula I, a stereoisomer thereof, or a pharmaceutically acceptable salt thereof,

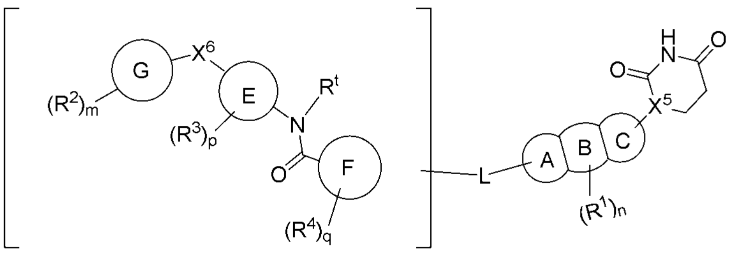

I

wherein

ring A is absent or selected from the group consisting of $C_{5-15}$ cycloalkenyl, 5- to 15-membered heterocycloalkenyl, phenyl, and 5- to 6-membered heteroaryl;
ring B is selected from the group consisting of phenyl and 5- to 6-membered heteroaryl;
ring C is selected from 5- to 6-membered heteroaryl;
each $R^1$ is independently selected from the group consisting of halogen, -OH, $-NH_2$, -CN, $C_{1-10}$ alkyl, $C_{1-10}$ alkoxy, and halogenated $C_{1-10}$ alkyl, wherein the -OH, $-NH_2$, $C_{1-10}$ alkyl, $C_{1-10}$ alkoxy, or halogenated $C_{1-10}$ alkyl is optionally substituted with one or more substituents;
n is selected from the group consisting of 0, 1, 2, and 3;
L is selected from a connecting group;

$X^5$ is selected from the group consisting of $C(R^f)$ and N;

$R^f$ is selected from the group consisting of H, halogen, deuterium, and $C_{1-6}$ alkyl optionally substituted with one or more substituents (e.g., $R^f$ is selected from the group consisting of halogen, deuterium, and $C_{1-6}$ alkyl optionally substituted with one or more substituents);

$X^6$ is selected from the group consisting of -O-, -NH-, and -N($C_{1-6}$ alkyl)-, wherein the -NH- or -N($C_{1-6}$ alkyl)- is optionally substituted with one or more substituents;

each $R^2$, each $R^3$, and each $R^4$ are independently selected from the group consisting of halogen, -OH, $-NH_2$, -CN, $C_{1-10}$ alkyl, $C_{1-10}$ alkoxy, and halogenated $C_{1-10}$ alkyl, wherein the -OH, $-NH_2$, $C_{1-10}$ alkyl, $C_{1-10}$ alkoxy, or halogenated $C_{1-10}$ alkyl is optionally substituted with one or more substituents;

m, p, and q are each independently selected from the group consisting of 0, 1, 2, 3, 4, 5, and 6;

ring G is selected from the group consisting of $C_{6-10}$ aryl and 5- to 10-membered heteroaryl;

ring E is selected from the group consisting of $C_{3-10}$ cycloalkyl and 3- to 10-membered heterocycloalkyl;

ring F is selected from the group consisting of $C_{6-10}$ aryl and 5- to 10-membered heteroaryl;

$R^t$ is selected from the group consisting of hydrogen, -OH, $C_{1-6}$ alkyl, $C_{3-10}$ cycloalkyl, and 3- to 10-membered heterocycloalkyl, wherein the $C_{1-6}$ alkyl, $C_{3-10}$ cycloalkyl, or 3- to 10-membered heterocycloalkyl is optionally substituted with one or more substituents.

In some embodiments, ring A is absent or selected from the group consisting of $C_{5-10}$ cycloalkenyl, 5- to 10-membered heterocycloalkenyl, phenyl, and 5- to 6-membered heteroaryl.

In some embodiments, ring A is absent or selected from the group consisting of $C_{5-9}$ cycloalkenyl, 5- to 9-membered heterocycloalkenyl, phenyl, and 5- to 6-membered heteroaryl.

In some embodiments, ring A is absent or selected from the group consisting of $C_{5-7}$ cycloalkenyl, 5- to 9-membered heterocycloalkenyl, phenyl, and 5- to 6-membered heteroaryl.

In some embodiments, ring A is absent or selected from the group consisting of $C_{5-6}$ cycloalkenyl, 5- to 10-membered heterocycloalkenyl, phenyl, and 5- to 6-membered heteroaryl.

In some embodiments, ring A is absent or selected from the group consisting of $C_{5-6}$ cycloalkenyl, 5- to 9-membered heterocycloalkenyl, phenyl, and 5- to 6-membered heteroaryl.

In some embodiments, ring A is absent or selected from the group consisting of $C_{5-6}$ cycloalkenyl, 5- to 9-membered heterocycloalkenyl, phenyl, and 5-membered heteroaryl.

In some embodiments, ring A is absent or selected from the group consisting of $C_{5-6}$ cycloalkenyl, 5- to 9-membered heterocycloalkenyl, phenyl, pyrrolyl, pyrazolyl, furanyl, and oxazolyl.

In some embodiments, ring A is absent or selected from the group consisting of $C_5$ cycloalkenyl, $C_6$ cycloalkenyl, 5-membered heterocycloalkenyl, 6-membered heterocycloalkenyl, 7-membered heterocycloalkenyl, 8-membered heterocycloalkenyl, 9-membered heterocycloalkenyl, phenyl, pyrrolyl, pyrazolyl, furanyl, and oxazolyl.

In some embodiments, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl.

In some specific embodiments, ring A is selected from the group consisting of $C_{5-10}$ cycloalkenyl, 5- to 10-membered heterocycloalkenyl, and 5- to 6-membered heteroaryl.

In some specific embodiments, ring A is selected from the group consisting of $C_{5-9}$ cycloalkenyl, 5- to 9-membered heterocycloalkenyl, and 5- to 6-membered heteroaryl.

In some specific embodiments, ring A is selected from the group consisting of $C_{5-7}$ cycloalkenyl, 5- to 9-membered heterocycloalkenyl, and 5- to 6-membered heteroaryl.

In some specific embodiments, ring A is selected from the group consisting of $C_{5-6}$ cycloalkenyl, 5- to 10-membered heterocycloalkenyl, and 5- to 6-membered heteroaryl.

In some specific embodiments, ring A is selected from the group consisting of $C_{5-6}$ cycloalkenyl, 5- to 9-membered heterocycloalkenyl, and 5- to 6-membered heteroaryl.

In some specific embodiments, ring A is selected from the group consisting of $C_{5-6}$ cycloalkenyl, 5- to 9-membered heterocycloalkenyl, and 5-membered heteroaryl.

In some specific embodiments, ring A is selected from the group consisting of $C_{5-10}$ cycloalkenyl and 5- to 10-membered heterocycloalkenyl. In some specific embodiments, ring A is selected from the group consisting of $C_{5-9}$ cycloalkenyl and 5- to 9-membered heterocycloalkenyl. In some specific embodiments, ring A is selected from $C_{5-8}$ cycloalkenyl. In some specific embodiments, ring A is selected from $C_{5-6}$ cycloalkenyl. In some specific embodiments, ring A is selected from 5- to 9-membered heterocycloalkenyl. In some embodiments, ring A is selected from 5- to 9-membered heterocycloalkenyl containing 1-2 heteroatoms selected from the group consisting of N and O. In some embodiments, ring A is selected from 5- to 8-membered heterocycloalkenyl containing 1-2 heteroatoms selected from the group consisting of N and O. In some embodiments, ring A is selected from 5- to 7-membered heterocycloalkenyl containing 1-2 heteroatoms selected from the group consisting of N and O. In some embodiments, ring A is selected from 5- to 7-membered

heterocycloalkenyl containing 1-2 N atoms.

In some specific embodiments, ring A is selected from the group consisting of cyclopentenyl, monocyclohexenyl, bicyclohexenyl, dihydropyrrolyl, tetrahydropyridinyl, tetrahydroazepinyl, azaspirononenyl, azaspirooctenyl, and dihydrooxazinyl.

In some embodiments, ring A is cyclopentenyl. In some embodiments, ring A is monocyclohexenyl. In some embodiments, ring A is bicyclohexenyl. In some embodiments, ring A is dihydropyrrolyl. In some embodiments, ring A is tetrahydropyridinyl. In some embodiments, ring A is tetrahydroazepinyl. In some embodiments, ring A is azaspirononenyl. In some embodiments, ring A is azaspirooctenyl. In some embodiments, ring A is dihydrooxazinyl. In some more specific embodiments, ring A is selected from the group consisting of phenyl, pyrrolyl, pyrazolyl, furanyl, and oxazolyl.

In some more specific embodiments, ring A is selected from the group consisting of phenyl, pyrrolyl, and pyrazolyl. In some embodiments, ring B is selected from the group consisting of phenyl and 6-membered heteroaryl (e.g., 6-membered heteroaryl containing 1-3 or 1-2 N atoms). In some embodiments, ring B is phenyl.

In some embodiments, ring C is selected from 5-membered heteroaryl. In some embodiments, ring C is selected from the group consisting of isoxazolyl and furanyl.

In some specific embodiments, ring C is isoxazolyl. In some specific embodiments, ring C is furanyl.

In some specific embodiments, ring A is present, and when ring A and ring B are both phenyl, ring C is not oxazolyl. In some embodiments, the substitution position for $R^1$ is selected from the group consisting of ring A and ring B. In some embodiments, the substitution position for $R^1$ is selected from ring A. In some embodiments, the substitution position for $R^1$ is selected from ring B. In some embodiments, the substitution position for $R^1$ is selected from ring C.

In some embodiments, ring F is linked to L. In some embodiments, ring A is linked to L. In some embodiments, ring B is linked to L. In some embodiments, ring C is linked to L. In some embodiments, ring A is linked to fragment

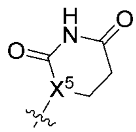

.

In some embodiments, ring B is linked to fragment

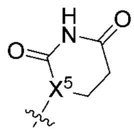

.

In some embodiments, ring C is linked to fragment

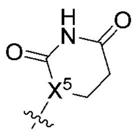

.

In some embodiments, ring F and ring A are each linked to L, and ring C is linked to fragment

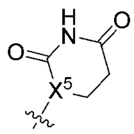

.

In some embodiments, the structural fragment

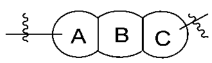

is selected from the group consisting of

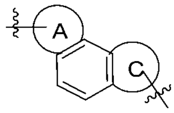 and 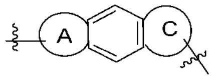.

In some embodiments, the structural fragment

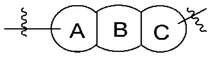

is selected from the group consisting of

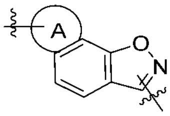, 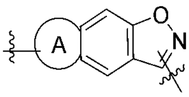, 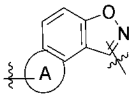, 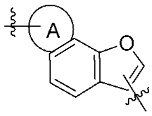,

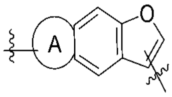, and 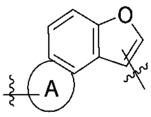.

In some embodiments, the structural fragment

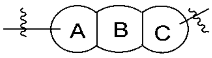

is selected from the group consisting of

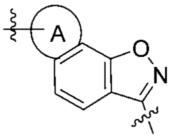, 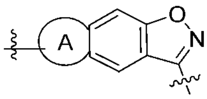, 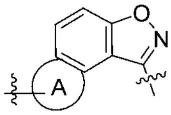, 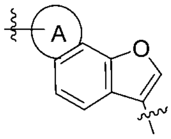,

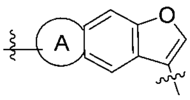, and 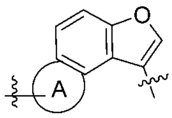.

In some embodiments, the structural fragment

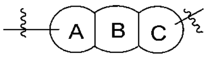

is selected from

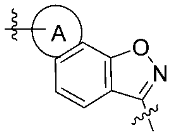.

In some embodiments, the structural fragment

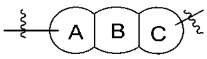

is selected from

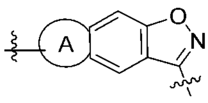.

In some embodiments, the structural fragment

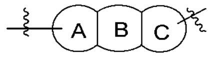

is selected from

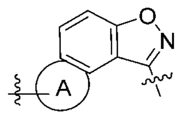.

In some embodiments, the structural fragment

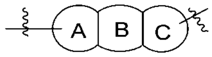

is selected from the group consisting of

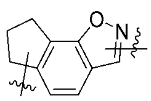, 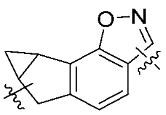, 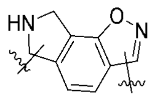, 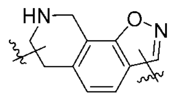, 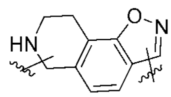, 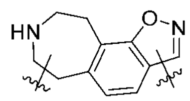,

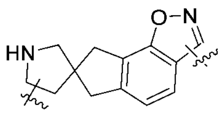, 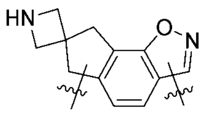, 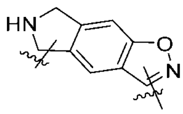, 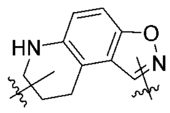, 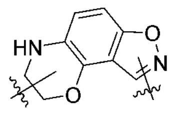, 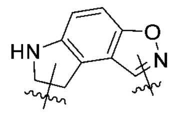,

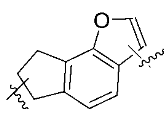, 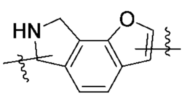, 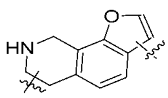, 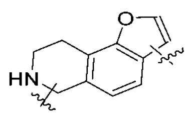, 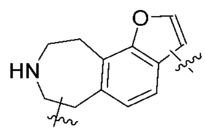, 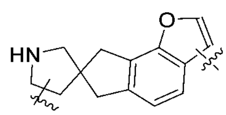,

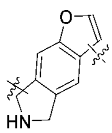, 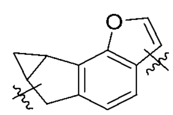, 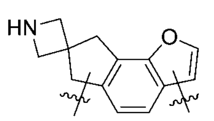, 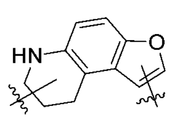, 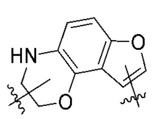, 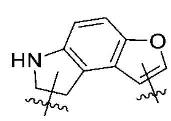,

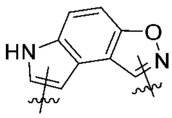, 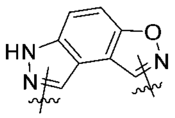, 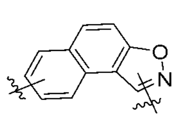, 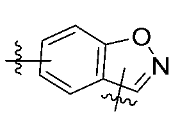, 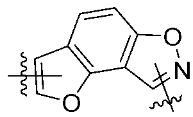, 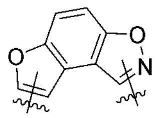,

and

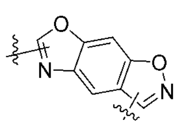.

In some embodiments, the structural fragment

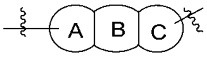

is not selected from

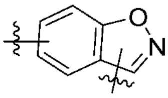
.

In some embodiments, the structural fragment

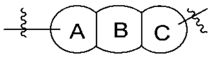

is not selected from the group consisting of

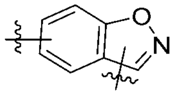 and 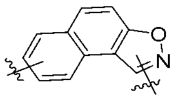.

In some embodiments, the structural fragment

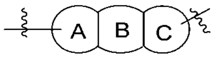

is selected from the group consisting of

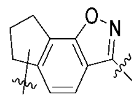, 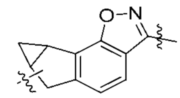, 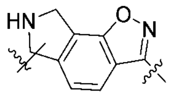, 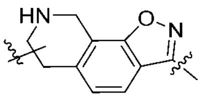, 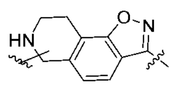, 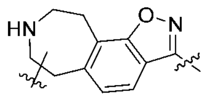,

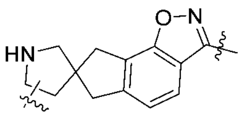, 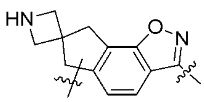, 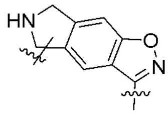, 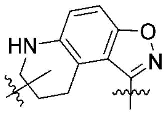, 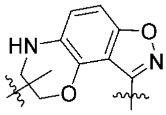, 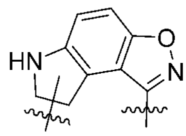,

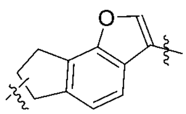, 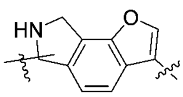, 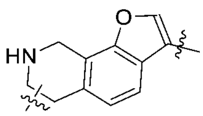, 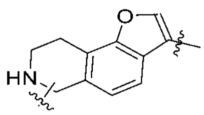, 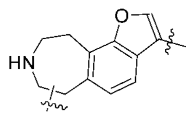,

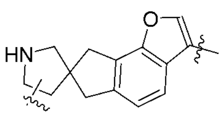, 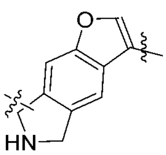, 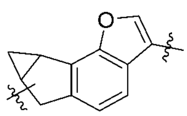, 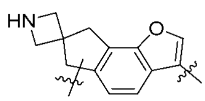, 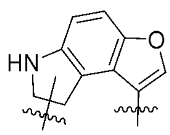, 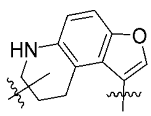,

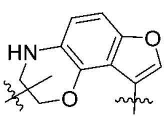, 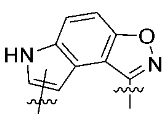, 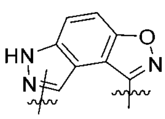, 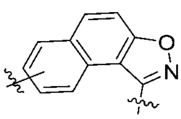, 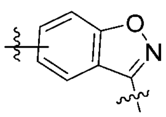, 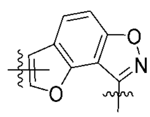,

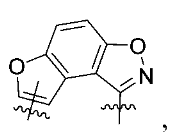, and 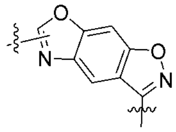.

In some embodiments, the structural fragment

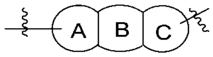

is not selected from

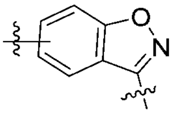.

In some embodiments, the structural fragment

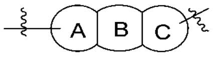

is not selected from the group consisting of

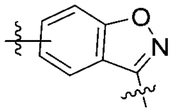 and 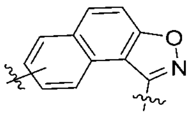.

In some embodiments, the structural fragment

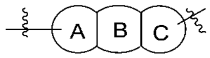

is selected from the group consisting of

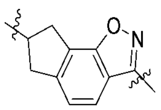, 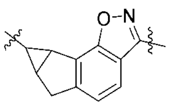, 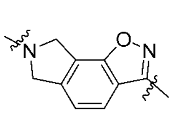, 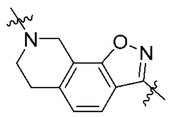, 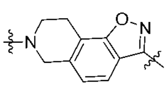, 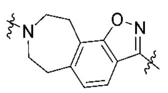,

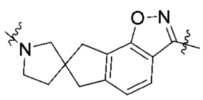, 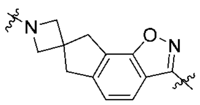, 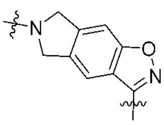, 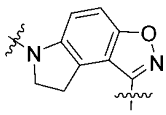, 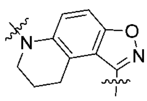, 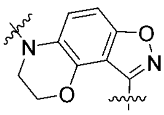,

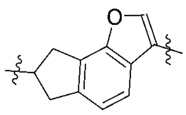, 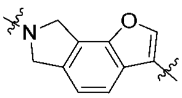, 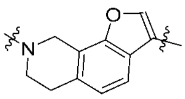, 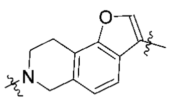, 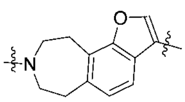, 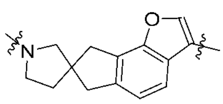,

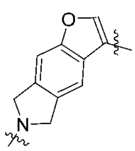, 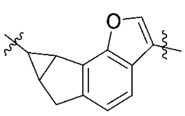, 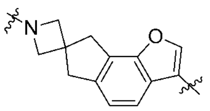, 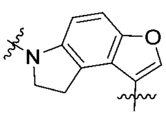, 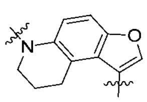, 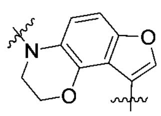,

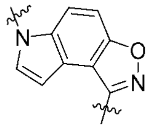, 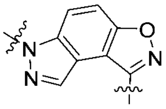, 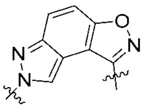, 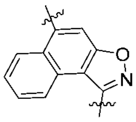, 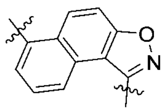, 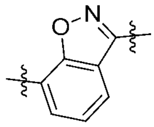, 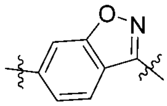,

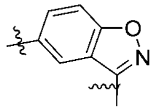, 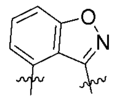, 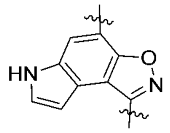, 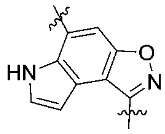, 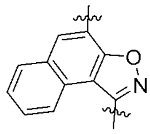, 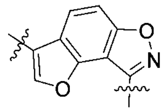, 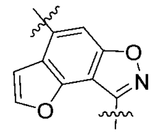,

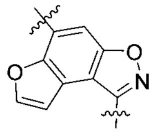, and 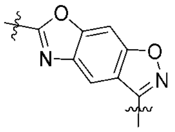.

In some embodiments, the structural fragment

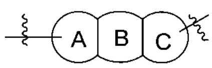 is 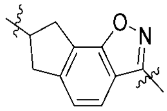.

In some embodiments, the structural fragment

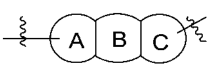 is 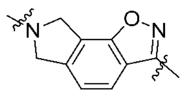.

In some embodiments, the structural fragment

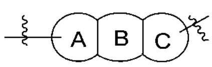 is 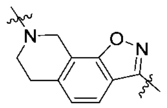.

In some embodiments, the structural fragment

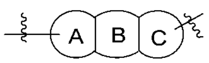

is

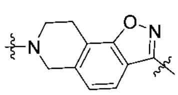.

In some embodiments, the structural fragment

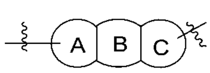 is 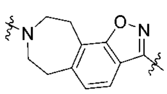.

**In** some embodiments, the structural fragment

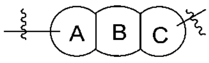 is 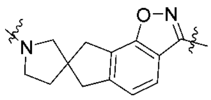.

In some embodiments, the structural fragment 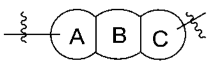 is 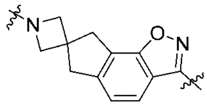.

In some embodiments, the structural fragment 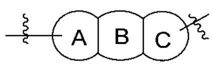 is 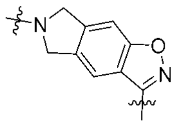.

In some embodiments, the structural fragment

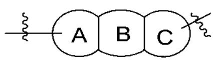

is

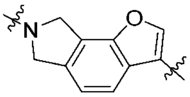.

In some embodiments, the structural fragment 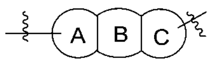 is 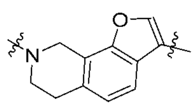.

**In** some embodiments, the structural fragment 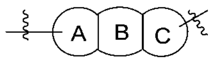 is 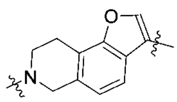.

In some embodiments, the structural fragment 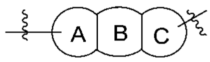 is 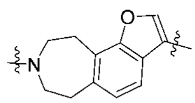.

In some embodiments, each $R^1$ is independently selected from the group consisting of halogen, -OH, $-NH_2$, -CN, $C_{1-6}$ alkyl, $C_{1-6}$ alkoxy, and halogenated $C_{1-6}$ alkyl, wherein the -OH, $-NH_2$, $C_{1-6}$ alkyl, $C_{1-6}$ alkoxy, or halogenated $C_{1-6}$ alkyl is optionally substituted with one or more substituents. In some embodiments, each $R^1$ is independently selected from the group consisting of halogen, -OH, $-NH_2$, -CN, $C_{1-4}$ alkyl, $C_{1-4}$ alkoxy, and halogenated $C_{1-4}$ alkyl. In some embodiments, each $R^1$ is independently selected from the group consisting of halogen, -OH, $-NH_2$, -CN, $C_{1-3}$ alkyl, $C_{1-3}$ alkoxy, and halogenated $C_{1-3}$ alkyl. In some embodiments, each $R^1$ is independently selected from the group consisting of fluorine, chlorine, bromine, -OH, $-NH_2$, and -CN. In some embodiments, each $R^1$ is independently selected from the group consisting of fluorine, chlorine, and bromine. In some embodiments, each $R^1$ is independently fluorine.

In some embodiments, n is selected from the group consisting of 0, 1, and 2. In some embodiments, n is selected

from the group consisting of 0 and 1. In some specific embodiments, n is 0.

In some embodiments, the structural fragment

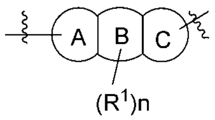

is selected from the group consisting of

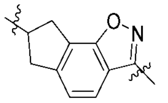, 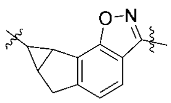, 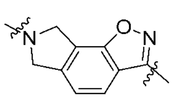, 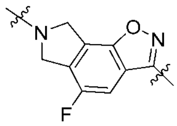, 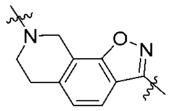, 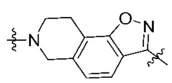,

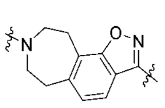, 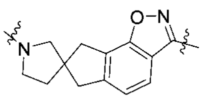, 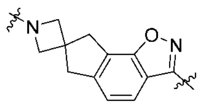, 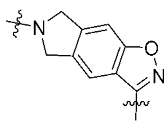, 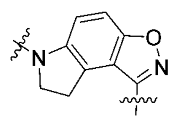, 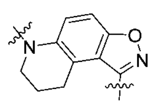,

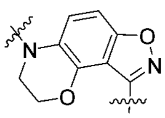, 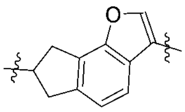, 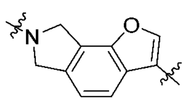, 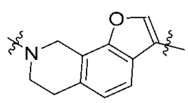, 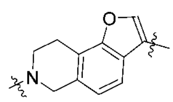, 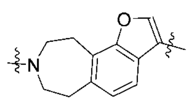,

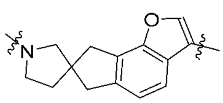, 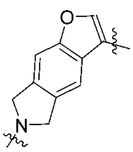, 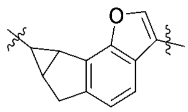, 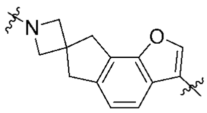, 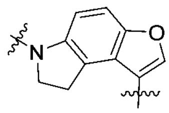, 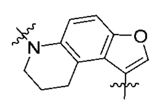,

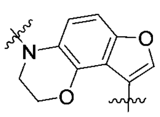, 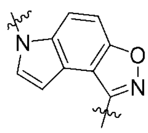, 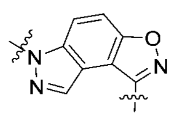, 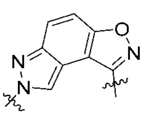, 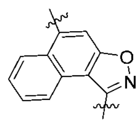, 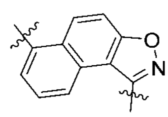, 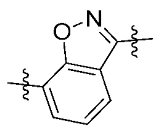,

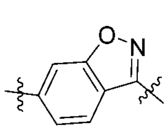, 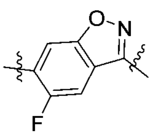, 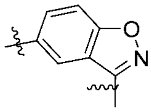, 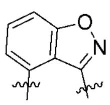, 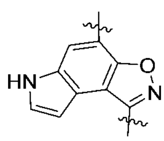, 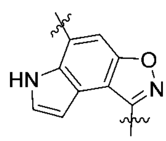, 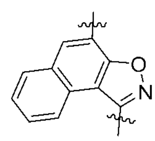,

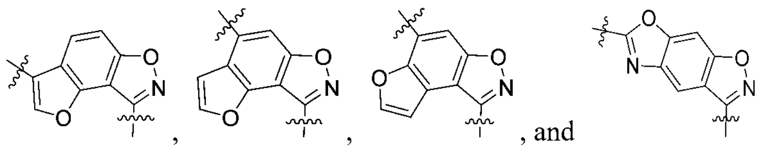

, , , and .

In some embodiments, L is selected from the group consisting of the following groups optionally substituted with one or more substituents: $C_{1-50}$ alkylene, $C_{2-50}$ alkenylene, and $C_{2-50}$ alkynylene, wherein 1 or more $-CH_2-$ of the $C_{1-50}$ alkylene, $C_{2-50}$ alkenylene, or $C_{2-50}$ alkynylene are optionally replaced by -O-, $C_{3-15}$ cycloalkyl, 3- to 15-membered heterocycloalkyl, 4- to 15-membered heterocycloalkenyl, $C_{6-15}$ aryl, 5- to 15-membered heteroaryl, -NH-, -N($C_{1-6}$ alkyl)-, or -S-.

In some embodiments, L is selected from the group consisting of the following groups optionally substituted with one or more substituents: $C_{1-30}$ alkylene, $C_{2-30}$ alkenylene, and $C_{2-30}$ alkynylene, wherein 1 or more $-CH_2-$ of the $C_{1-30}$ alkylene, $C_{2-30}$ alkenylene, or $C_{2-30}$ alkynylene are optionally replaced by -O-, $C_{3-12}$ cycloalkyl, 3- to 12-membered

heterocycloalkyl, 4- to 12-membered heterocycloalkenyl, $C_{6-12}$ aryl, 5- to 12-membered heteroaryl, -NH-, -N($C_{1-6}$ alkyl)-, or -S-.

In some embodiments, L is selected from the group consisting of the following groups optionally substituted with one or more substituents: $C_{1-20}$ alkylene, $C_{2-20}$ alkenylene, and $C_{2-20}$ alkynylene, wherein 1 or more $-CH_2-$ of the $C_{1-20}$ alkylene, $C_{2-20}$ alkenylene, or $C_{2-20}$ alkynylene are optionally replaced by -O-, $C_{3-10}$ cycloalkyl, 3- to 11-membered heterocycloalkyl, 4- to 10-membered heterocycloalkenyl, $C_{6-10}$ aryl, 5- to 10-membered heteroaryl, -NH-, -N($C_{1-6}$ alkyl)-, or -S-.

In some embodiments, L is selected from the group consisting of the following groups optionally substituted with one or more substituents: $C_{1-15}$ alkylene, $C_{2-15}$ alkenylene, and $C_{2-15}$ alkynylene, wherein 1 or more $-CH_2-$ of the $C_{1-15}$ alkylene, $C_{2-15}$ alkenylene, or $C_{2-15}$ alkynylene are optionally replaced by -O-, $C_{3-9}$ cycloalkyl, 3- to 11-membered heterocycloalkyl, 4- to 8-membered heterocycloalkenyl, $C_{6-8}$ aryl, 5- to 8-membered heteroaryl, -NH-, -N($C_{1-4}$ alkyl)-, or -S-.

In some embodiments, L is selected from the group consisting of the following groups optionally substituted with one or more substituents: $C_{1-10}$ alkylene, $C_{2-10}$ alkenylene, and $C_{2-10}$ alkynylene, wherein 1 or more $-CH_2-$ of the $C_{1-10}$ alkylene, $C_{2-10}$ alkenylene, or $C_{2-10}$ alkynylene are optionally replaced by -O-, $C_{3-9}$ cycloalkyl, 3- to 11-membered heterocycloalkyl, 4- to 6-membered heterocycloalkenyl, $C_6$ aryl, 5- to 6-membered heteroaryl, -NH-, -N($C_{1-3}$ alkyl)-, or -S-.

In some embodiments, L is selected from the group consisting of the following groups optionally substituted with one or more substituents: $C_{1-6}$ alkylene, $C_{2-6}$ alkenylene, and $C_{2-6}$ alkynylene, wherein 1 or more $-CH_2-$ of the $C_{1-6}$ alkylene, $C_{2-6}$ alkenylene, or $C_{2-6}$ alkynylene are optionally replaced by -O-, $C_{3-9}$ cycloalkyl, 3- to 11-membered heterocycloalkyl, 4- to 6-membered heterocycloalkenyl, $C_6$ aryl, 5- to 6-membered heteroaryl, -NH-, -N($C_{1-3}$ alkyl)-, or -S-.

In some embodiments, L is selected from the group consisting of the following groups optionally substituted with one or more substituents: $C_{1-4}$ alkylene, $C_{2-4}$ alkenylene, and $C_{2-4}$ alkynylene, wherein 1 or more (e.g., 1 or 2, 1 or 3, etc.) $-CH_2-$ of the $C_{1-4}$ alkylene, $C_{2-4}$ alkenylene, or $C_{2-4}$ alkynylene are optionally replaced by -O-, $C_{4-6}$ cycloalkyl, 4- to 6-membered heterocycloalkyl, 4- to 6-membered heterocycloalkenyl, $C_6$ aryl, 5- to 6-membered heteroaryl, - NH-, -N($C_{1-3}$ alkyl)-, or -S-. In some embodiments, L is selected from $C_{1-6}$ alkylene, wherein 1 or more $-CH_2-$ of the $C_{1-6}$ alkylene are optionally replaced by a group selected from the group consisting of -O-, $C_{3-10}$ cycloalkyl, 4- to 11-membered heterocycloalkyl, 4- to 10-membered heterocycloalkenyl, -NH-, -N($C_{1-3}$ alkyl)-, and -S-, and the $C_{1-6}$ alkylene is optionally substituted with one or more substituents.

In some embodiments, L is selected from $C_{1-6}$ alkylene, wherein 1 or more $-CH_2-$ of the $C_{1-6}$ alkylene are optionally replaced by a group selected from the group consisting of -O-, $C_{3-10}$ cycloalkyl, 4- to 11-membered heterocycloalkyl, 5- to 6-membered heterocycloalkenyl, -NH-, -N($C_{1-3}$ alkyl)-, and -S-, and the $C_{1-6}$ alkylene is optionally substituted with one or more substituents. In some embodiments, L is selected from the group consisting of -(5- to 8-membered heterocycloalkyl containing 1-2 N atoms or O atoms)-$C_{1-6}$ alkylene-, -($C_{5-8}$ cycloalkyl)-$C_{1-6}$ alkylene-, -$C_{1-6}$ alkylene-(5- to 8-membered heterocycloalkyl containing 1-2 N atoms or O atoms)-$C_{1-6}$ alkylene-, and -$C_{1-6}$ alkylene-($C_{5-8}$ cycloalkyl)-$C_{1-6}$ alkylene-.

In some embodiments, in the definition of L, the substituent is selected from the group consisting of halogen, =O, -OH, $-NH_2$, -CN, $C_{1-6}$ alkyl, $C_{1-6}$ alkoxy, halogenated $C_{1-6}$ alkyl, $C_{1-6}$ alkylamino, di-$C_{1-6}$ alkylamino, $C_{3-12}$ cycloalkyl, and 4- to 12-membered heterocycloalkyl. In some embodiments, in the definition of L, the substituent is selected from the group consisting of halogen, =O, -OH, $-NH_2$, -CN, $C_{1-4}$ alkyl, $C_{1-4}$ alkoxy, halogenated $C_{1-4}$ alkyl, $C_{1-4}$ alkylamino, di-$C_{1-4}$ alkylamino, $C_{3-10}$ cycloalkyl, and 4- to 10-membered heterocycloalkyl. In some embodiments, in the definition of L, the substituent is selected from the group consisting of halogen, =O, OH, $NH_2$, CN, $C_{1-6}$ alkyl, and $C_{1-6}$ alkoxy. In some embodiments, in the definition of L, the substituent is selected from the group consisting of =O, OH, $NH_2$, halogen, and CN. In some embodiments, in the definition of L, the substituent is =O.

In some embodiments, L is selected from $-LNK^1-Cy^1-LNK-Cy^2-LNK^2-Cy^3-$, wherein

$Cy^1$, $Cy^2$, or $Cy^3$ is each independently selected from the group consisting of a bond and the following groups optionally substituted with one or more $R^a$: $C_{3-12}$ cycloalkyl, 4- to 12-membered heterocycloalkyl, and 4- to 12-membered heterocycloalkenyl;

LNK, $LNK^1$, and $LNK^2$ are each independently selected from the group consisting of a bond, NH, O, S, and the following groups optionally substituted with one or more $R^b$: $C_{1-12}$ alkylene, $C_{2-12}$ alkenylene, $C_{2-12}$ alkynylene, and $C_{1-12}$ heteroalkylene;

each $R^a$ and each $R^b$ are independently selected from the group consisting of halogen, =O, -OH, $-NH_2$, -CN, $C_{1-6}$ alkyl, $C_{1-6}$ alkoxy, halogenated $C_{1-6}$ alkyl, $C_{1-6}$ alkylamino, di-$C_{1-6}$ alkylamino, $C_{3-12}$ cycloalkyl, and 4- to 12-membered heterocycloalkyl.

In some embodiments, $Cy^1$, $Cy^2$, and $Cy^3$ are not bonds at the same time. In some embodiments, $Cy^2$ and $Cy^3$ are bonds. In some embodiments, $LNK^1$ and $LNK^2$ are bonds.

In some embodiments, L is selected from the group consisting of $-Cy^1-$, $-Cy^2-$, $-LNK^1-$, $-Cy^1-LNK-$, $-Cy^1-Cy^2-$, -

$LNK^1$-$Cy^1$-LNK-, -LNK-$Cy^2$-$LNK^2$-, -$Cy^1$-$Cy^2$-$LNK^2$-, -$LNK^1$-$Cy^1$-$Cy^2$-, -$Cy^1$-LNK-$Cy^2$-, -$LNK^1$-$Cy^1$-$Cy^2$-$LNK^2$-, -$LNK^1$-$Cy^1$-LNK-$Cy^2$-, -$Cy^1$-LNK-$Cy^2$-$LNK^2$-, -$Cy^1$-$Cy^2$-$Cy^3$-, and -$Cy^1$-$Cy^1$-$LNK^2$-$Cy^3$-. In some embodiments, L is selected from -$Cy^1$-. In some embodiments, L is selected from -$LNK^1$-. In some embodiments, L is selected from -$Cy^1$-LNK-.

In some embodiments, LNK, $LNK^1$, and $LNK^2$ are each independently selected from the group consisting of a bond, NH, O, S, and the following groups optionally substituted with one or more $R^b$: $C_{1-10}$ alkylene, $C_{2-10}$ alkenylene, $C_{2-10}$ alkynylene, and $C_{1-10}$ heteroalkylene. In some embodiments, LNK, $LNK^1$, and $LNK^2$ are each independently selected from the group consisting of a bond, NH, O, S, and the following groups optionally substituted with one or more $R^b$: $C_{1-6}$ alkylene, $C_{2-6}$ alkenylene, $C_{2-6}$ alkynylene, and $C_{1-6}$ heteroalkylene. In some embodiments, LNK, $LNK^1$, and $LNK^2$ are each independently selected from the group consisting of a bond, NH, O, S, and the following groups optionally substituted with one or more $R^b$: $C_{1-4}$ alkylene, $C_{2-4}$ alkenylene, $C_{2-4}$ alkynylene, and $C_{1-4}$ heteroalkylene. In some embodiments, LNK, $LNK^1$, and $LNK^2$ are each independently selected from the group consisting of a bond, NH, O, S, and the following groups optionally substituted with one or more $R^b$: $C_{1-6}$ alkylene and $C_{1-6}$ heteroalkylene. In some embodiments, LNK, $LNK^1$, and $LNK^2$ are each independently selected from the group consisting of a bond, NH, O, S, and the following groups optionally substituted with one or more $R^b$: $C_{1-4}$ alkylene and $C_{1-4}$ heteroalkylene. In some embodiments, LNK, $LNK^1$, and $LNK^2$ are each independently selected from the group consisting of a bond, NH, O, and the following groups optionally substituted with one or more $R^b$: $C_{1-3}$ alkylene and $C_{1-3}$ heteroalkylene. In some embodiments, LNK, $LNK^1$, and $LNK^2$ are each independently selected from the group consisting of a bond, NH, O, and the following groups optionally substituted with one or more $R^b$: $C_{1-2}$ alkylene and $C_{1-2}$ heteroalkylene. In some embodiments, LNK, $LNK^1$, and $LNK^2$ are each independently selected from the group consisting of a bond, NH, O, $-NHCH_2-$, $-CH_2NHCH_2-$, $-CH_2-$, $-CH_2CH_2-$, -C(O)-, and $-C(O)CH_2-$. In some embodiments, LNK, $LNK^1$, and $LNK^2$ are each independently a bond. In some embodiments, LNK, $LNK^1$, and $LNK^2$ are each independently - $CH_2$-.

In some embodiments, $Cy^1$, $Cy^2$, or $Cy^3$ is each independently selected from the group consisting of a bond and the following groups optionally substituted with one or more $R^a$: $C_{3-11}$ cycloalkyl, 4- to 12-membered heterocycloalkyl, and 4- to 11-membered heterocycloalkenyl. In some embodiments, $Cy^1$, $Cy^2$, or $Cy^3$ is each independently selected from the group consisting of a bond and the following groups optionally substituted with one or more $R^a$: $C_{4-10}$ cycloalkyl, 4- to 11-membered heterocycloalkyl, and 5- to 6-membered heterocycloalkenyl. In some embodiments, $Cy^1$, $Cy^2$, or $Cy^3$ is each independently selected from the group consisting of a bond and the following groups optionally substituted with one or more $R^a$: $C_{4-9}$ cycloalkyl, 4- to 11-membered heterocycloalkyl, and 5- to 6-membered heterocycloalkenyl. In some embodiments, $Cy^1$, $Cy^2$, or $Cy^3$ is each independently selected from the group consisting of a bond and the following groups optionally substituted with one or more $R^a$: $C_{4-6}$ cycloalkyl, $C_9$ cycloalkyl, 4- to 11-membered heterocycloalkyl, and 6-membered heterocycloalkenyl.

In some embodiments, $Cy^1$, $Cy^2$, or $Cy^3$ is each independently selected from the group consisting of a bond and the following groups optionally substituted with one or more $R^a$: cyclobutyl, cyclopentyl, cyclohexyl, spirononanyl, azetidinyl, pyrrolidinyl, piperidinyl, tetrahydropyridinyl, piperazinyl, monoazaspiroheptyl, monoazaspirononanyl, diazaspirononanyl, monoazaspirodecyl, diazaspirodecyl, monoazaspiroundecanyl, diazaspiroundecanyl, octahydrocyclopentapyrrolyl, diazabicyclooctyl, and monoazabicyclononanyl.

In some specific embodiments, $Cy^1$, $Cy^2$, or $Cy^3$ is each independently selected from the group consisting of a bond and the following group optionally substituted with one or more $R^a$: 4- to 11-membered heterocycloalkyl. In some specific embodiments, $Cy^1$, $Cy^2$, or $Cy^3$ is each independently selected from the group consisting of a bond and the following group optionally substituted with one or more $R^a$: 7- to 11-membered heterocycloalkyl. In some specific embodiments, $Cy^1$, $Cy^2$, or $Cy^3$ is each independently selected from the group consisting of a bond and the following group optionally substituted with one or more $R^a$: 4- to 6-membered heterocycloalkyl. In some embodiments, $Cy^1$, $Cy^2$, or $Cy^3$ is each independently a bond. In some embodiments, $Cy^1$, $Cy^2$, or $Cy^3$ is each independently selected from 6-membered heterocycloalkyl optionally substituted with one or more $R^a$.

In some specific embodiments, $Cy^1$, $Cy^2$, or $Cy^3$ is each independently selected from the group consisting of a bond and the following groups optionally substituted with one or more $R^a$: cyclobutyl, cyclopentyl, cyclohexyl, spirononanyl, azetidinyl, pyrrolidinyl, piperidinyl, tetrahydropyridinyl, and piperazinyl. In some specific embodiments, $Cy^1$, $Cy^2$, or $Cy^3$ is each independently selected from the group consisting of a bond and the following groups optionally substituted with one or more $R^a$: monoazaspiroheptyl, monoazaspirononanyl, diazaspirononanyl, monoazaspirodecyl, diazaspirodecyl, monoazaspiroundecanyl, diazaspiroundecanyl, octahydrocyclopentapyrrolyl, diazabicyclooctyl, and monoazabicyclononanyl.

In some embodiments, $Cy^1$, $Cy^2$, or $Cy^3$ is each independently selected from the group consisting of a bond,

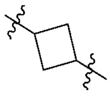, 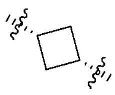, 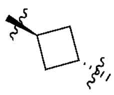, 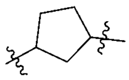, 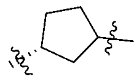, 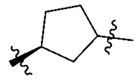, 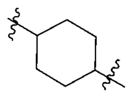, 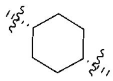,

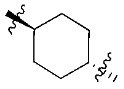, 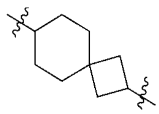, 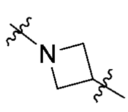, 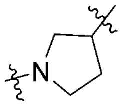, 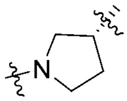, 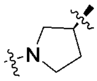, 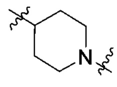, 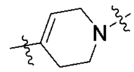,

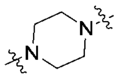, 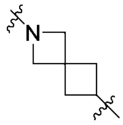, 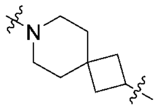, 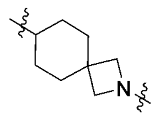, 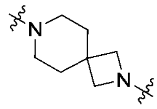, 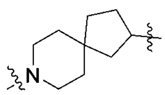, 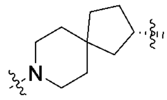,

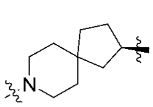, 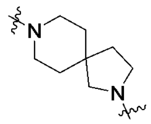, 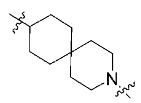, 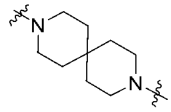, 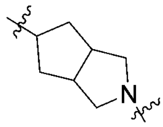, 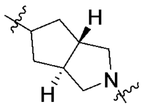, 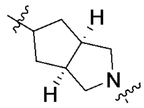,

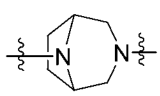, and 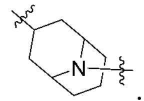.

In some embodiments, $Cy^1$, $Cy^2$, or $Cy^3$ is each independently

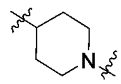.

**In** some embodiments, $Cy^1$, $Cy^2$, or $Cy^3$ is each independently

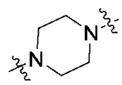.

In some embodiments, each $R^a$ and each $R^b$ are independently selected from the group consisting of halogen, =O, - OH, $-NH_2$, -CN, $C_{1-6}$ alkyl, $C_{1-6}$ alkoxy, halogenated $C_{1-6}$ alkyl, $C_{1-6}$ alkylamino, di-$C_{1-6}$ alkylamino, $C_{3-10}$ cycloalkyl, and 4- to 10-membered heterocycloalkyl. In some embodiments, each $R^a$ and each $R^b$ are independently selected from the group consisting of halogen, =O, OH, $NH_2$, CN, $C_{1-6}$ alkyl, $C_{1-6}$ alkoxy, halogenated $C_{1-6}$ alkyl, $C_{1-6}$ alkylamino, and di-$C_{1-6}$ alkylamino. In some embodiments, each $R^a$ and each $R^b$ are independently selected from the group consisting of halogen, =O, -OH, $-NH_2$, -CN, $C_{1-4}$ alkyl, $C_{1-4}$ alkoxy, halogenated $C_{1-4}$ alkyl, $C_{1-4}$ alkylamino, and di-$C_{1-4}$ alkylamino.

In some embodiments, each $R^a$ and each $R^b$ are independently selected from the group consisting of halogen, =O, - OH, $-NH_2$, -CN, and $C_{1-3}$ alkyl. In some embodiments, each $R^a$ and each $R^b$ are independently selected from the group consisting of halogen, =O, -OH, $-NH_2$, and -CN. In some embodiments, each $R^a$ and each $R^b$ are independently =O.

In some embodiments, L or $-LNK^1-$ is selected from the group consisting of a bond, $-NHCH_2-$, $-CH_2NHCH_2-$, $-CH_2-$, and $-C(O)CH_2-$.

In some embodiments, L or $-Cy^1-$ is selected from the group consisting of

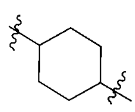, 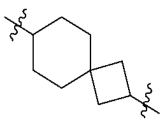, 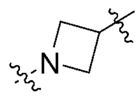,

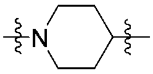, 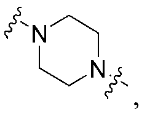, 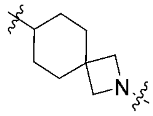, 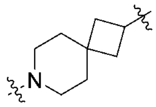, 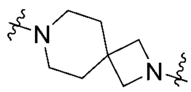, 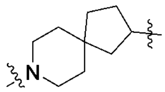, 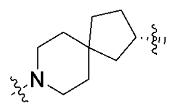,

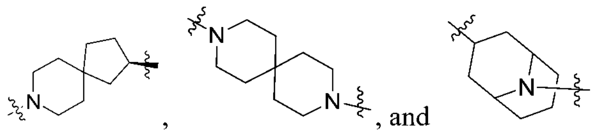
, , and .

In some embodiments, L or -$Cy^1$-LNK- is selected from the group consisting of

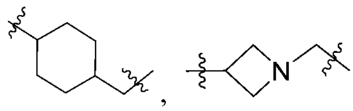
, ,

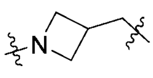, 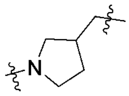, 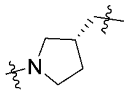, 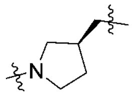, 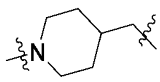, 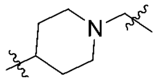, 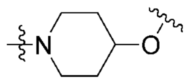,

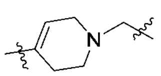, 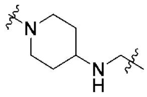, 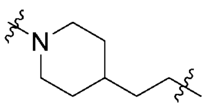, 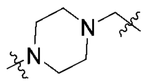, 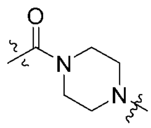, 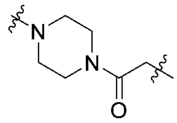,

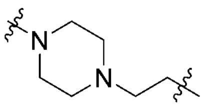, 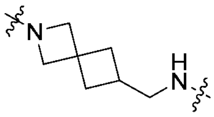, 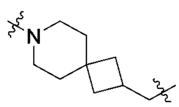, 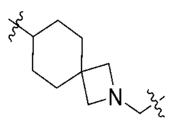, 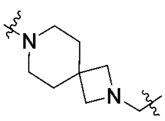, 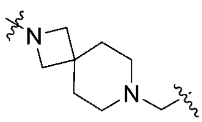,

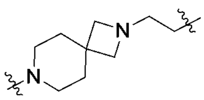, 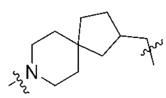, 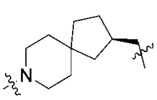, 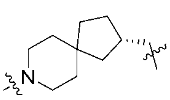, 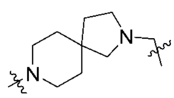, 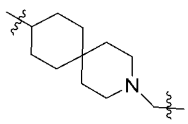,

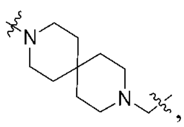, 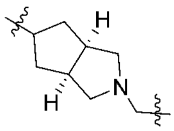, and 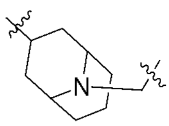.

In some embodiments, L or -$Cy^1$-$Cy^2$- is selected from the group consisting of

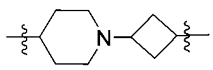,

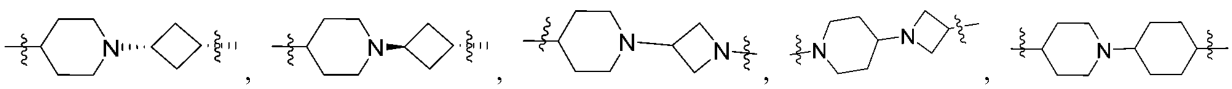
, , , , ,

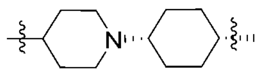 , 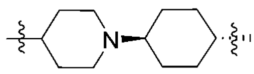 , 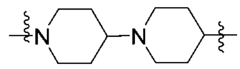 , 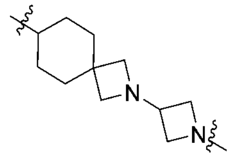 ,

and

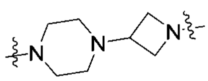 .

In some embodiments, L or -Cy[1]-Cy[2]-Cy[3]- is selected from the group consisting of

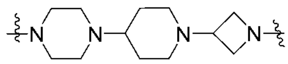 ,

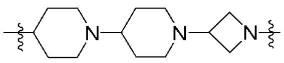 , 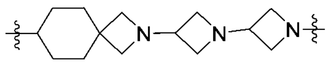 , 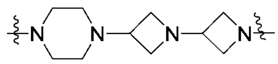 , 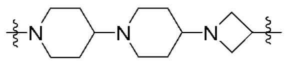 ,

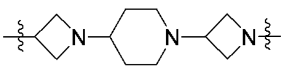 , 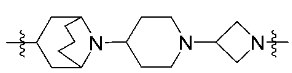 , 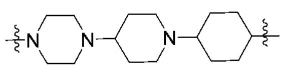 , 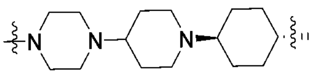 ,

and

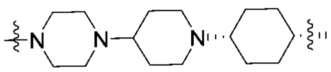 .

In some embodiments, L or -LNK[1]-Cy[1]-LNK- is selected from the group consisting of

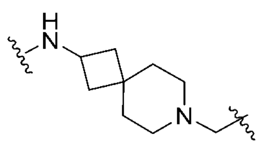 ,

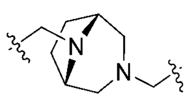 , 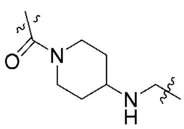 , 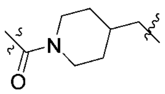 , 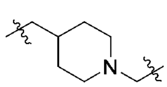 , 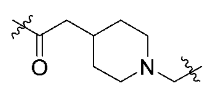 , 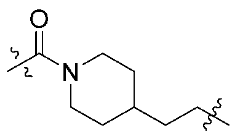 ,

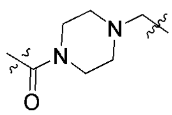 , 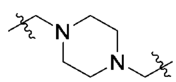 , and 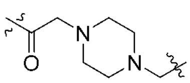 .

In some embodiments, L or -Cy[1]-Cy[2]-LNK[2]- is selected from the group consisting of

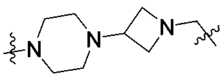 ,

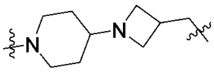, 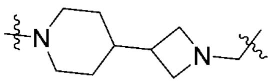, 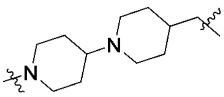, 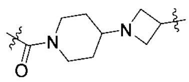, and 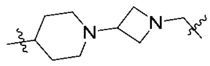.

In some embodiments, L or -$Cy^1$-LNK-$Cy^2$- is selected from the group consisting of

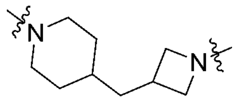,

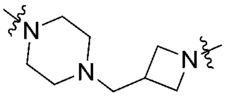, 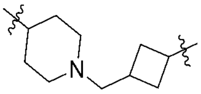, 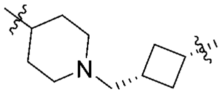, 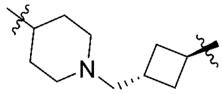, 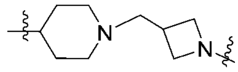,

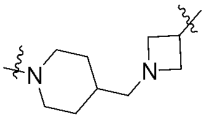, 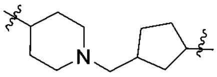, 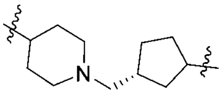, 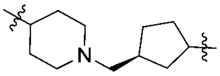, 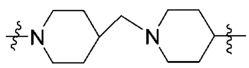,

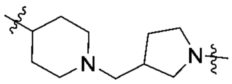, 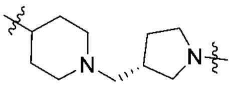, 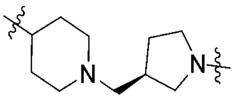, and 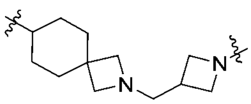.

In some embodiments, L or -$LNK^1$-$Cy^1$-$Cy^2$-$LNK^2$- is selected from the group consisting of

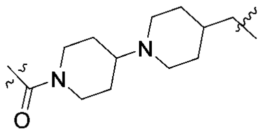,

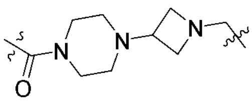 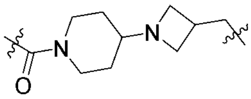, and 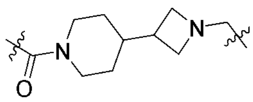.

In some embodiments, L or -$Cy^1$-LNK-$Cy^2$-$LNK^2$- is

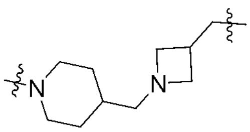.

In some embodiments, L is selected from the group consisting of a bond, -$NHCH_2$-, -$CH_2NHCH_2$-, -$CH_2$-, -C(O)$CH_2$-,

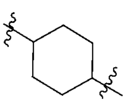, 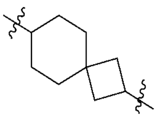, 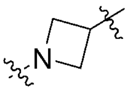, 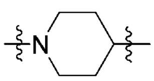, 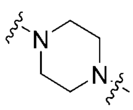, 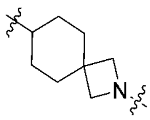, 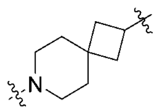,

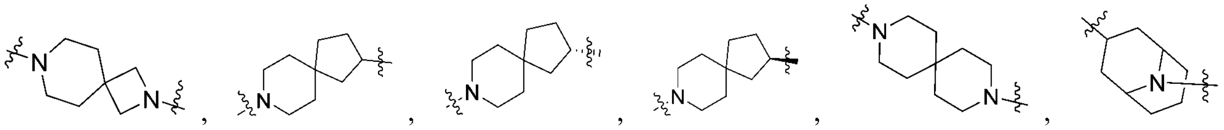

, , , , , ,

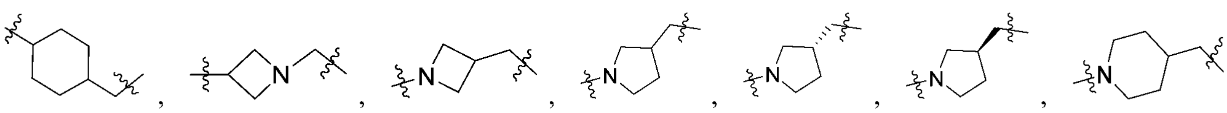

, , , , , , ,

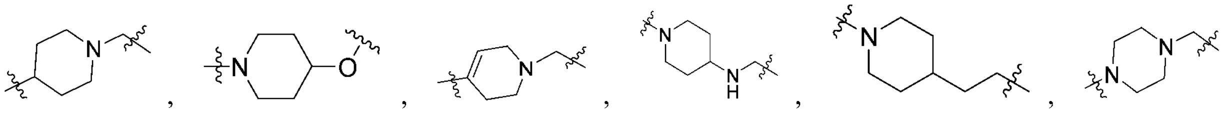

, , , , , ,

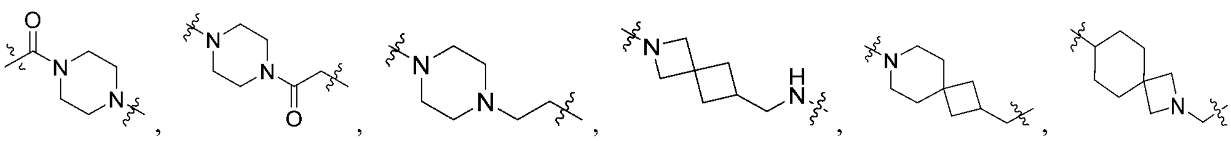

, , , , , ,

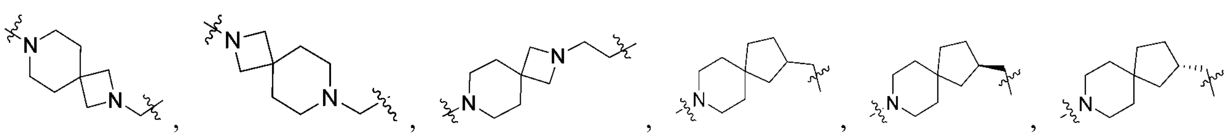

, , , , , ,

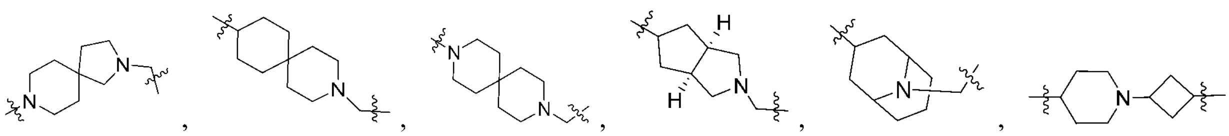

, , , , , ,

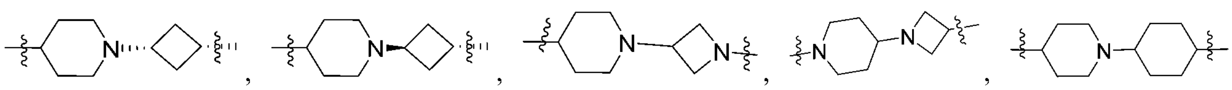

, , , , ,

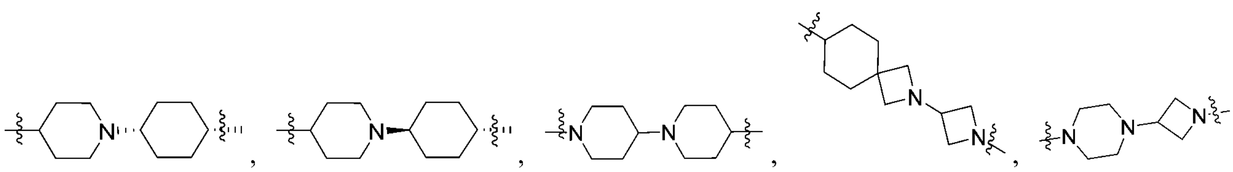

, , , , ,

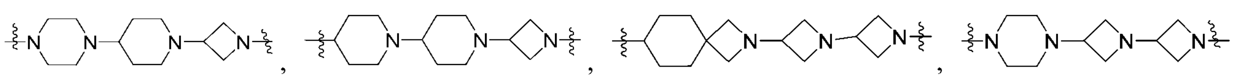

, , , ,

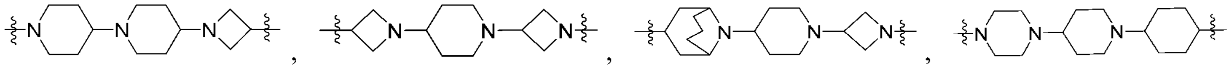

, , , ,

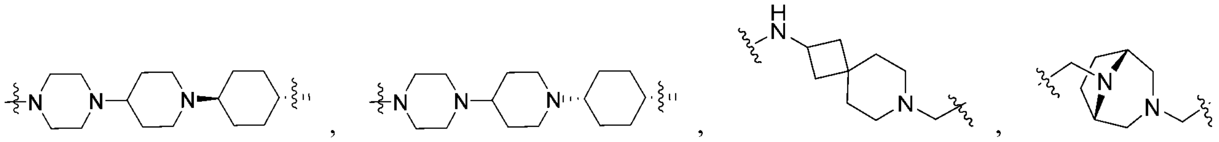

, , , ,

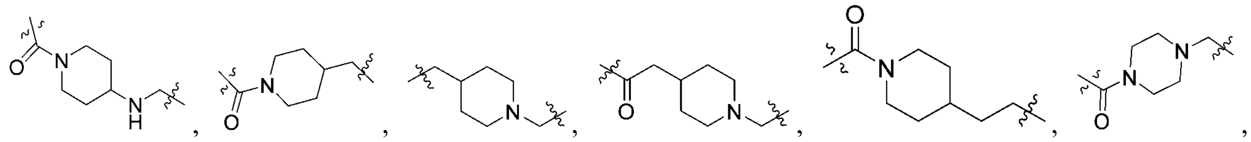

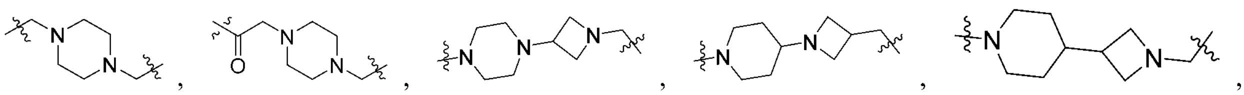

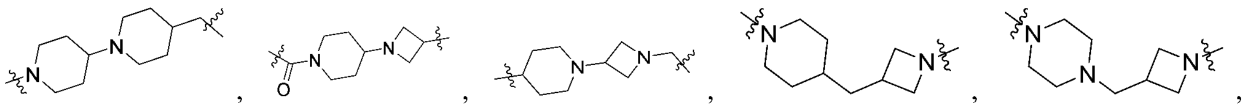

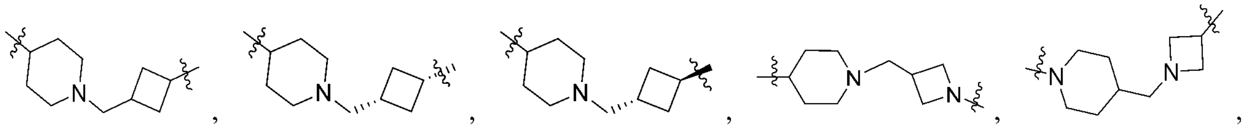

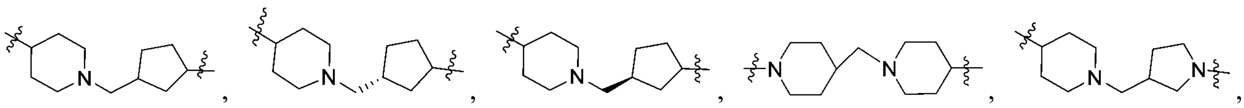

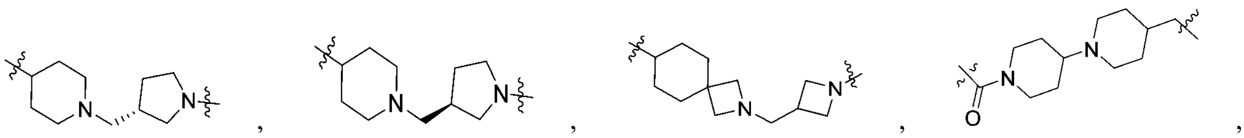

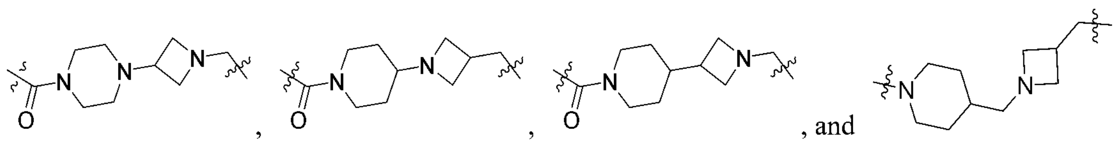

In some embodiments, L is selected from the group consisting of

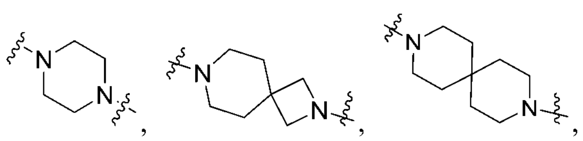

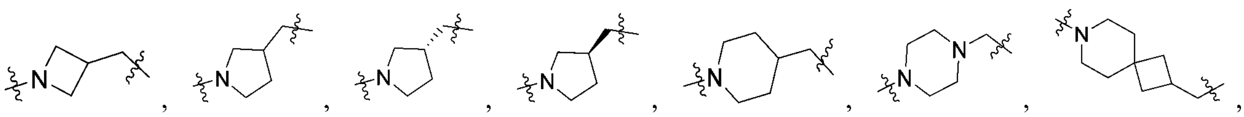

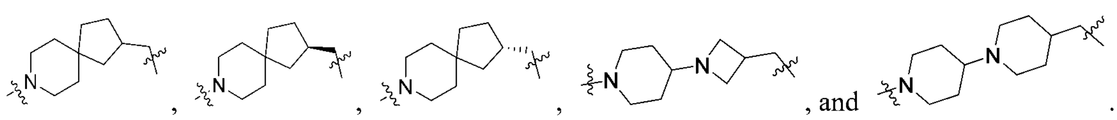

In some embodiments, L is

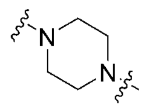.

In some embodiments, L is

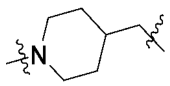.

In some embodiments, L is

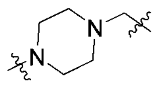.

In some embodiments, the structural fragment

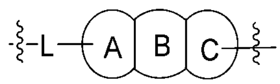

is selected from the group consisting of

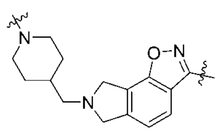, 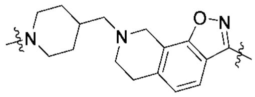, 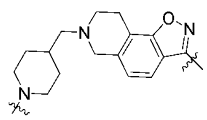, 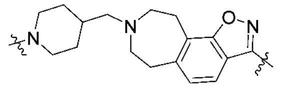,

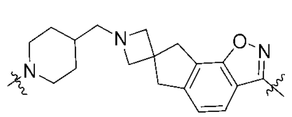, 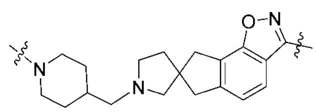, 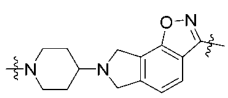, 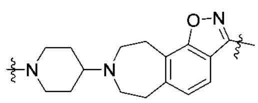,

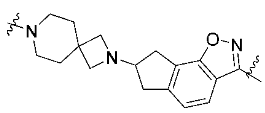, 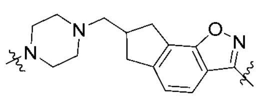, 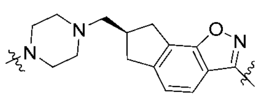, 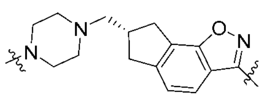,

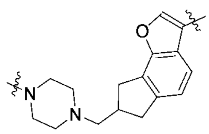, 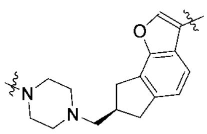, 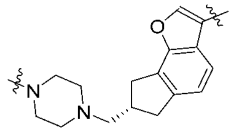, 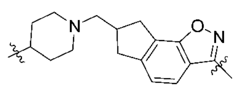, 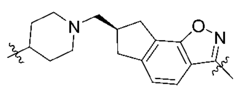,

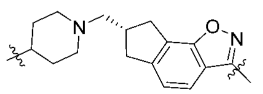, 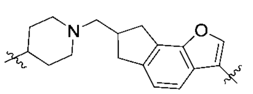, 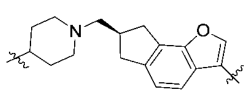, 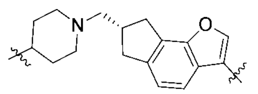,

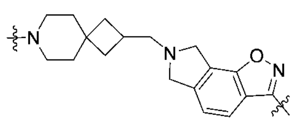, 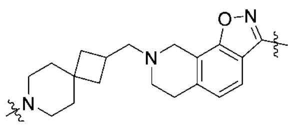, 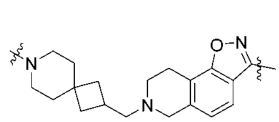, 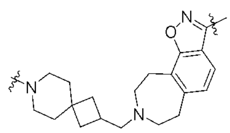,

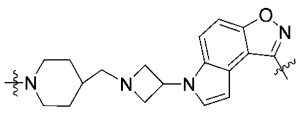 , 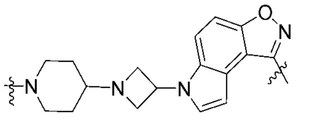 , 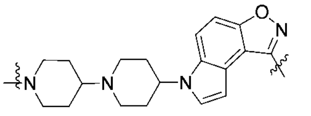 ,

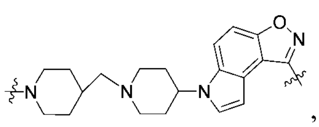 , 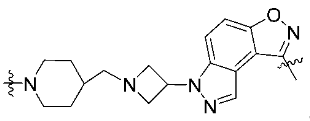 , 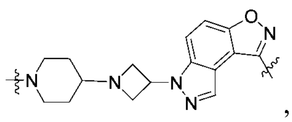 , 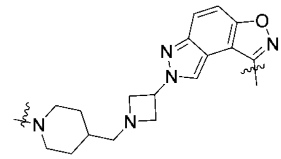 ,

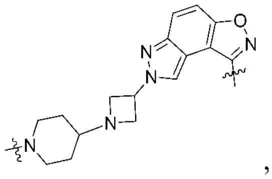 , 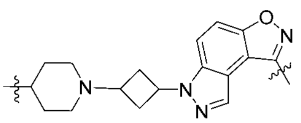 , 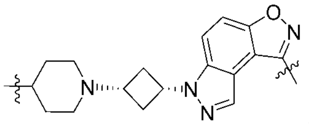 , 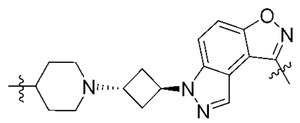 ,

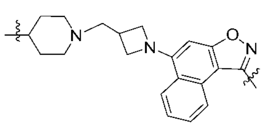 , 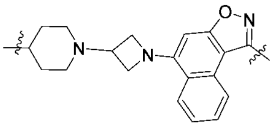 , 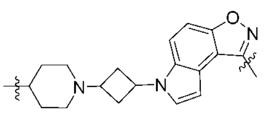 , 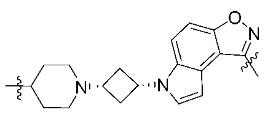 ,

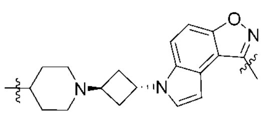 , 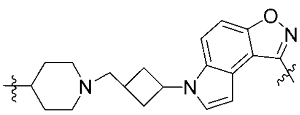 , 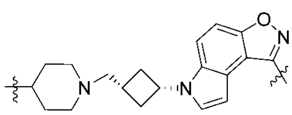 , 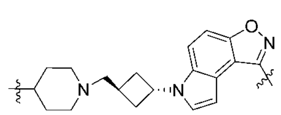 ,

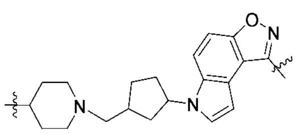 , 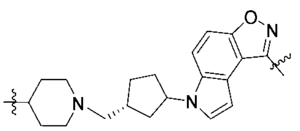 , 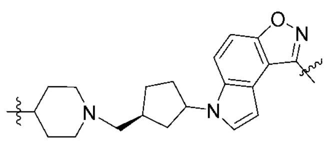 ,

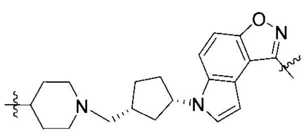 , 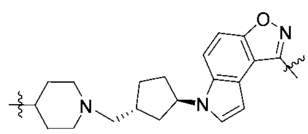 , 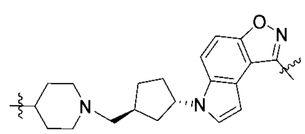 ,

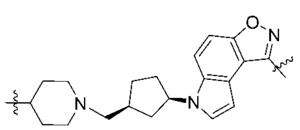 , 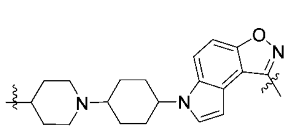 , 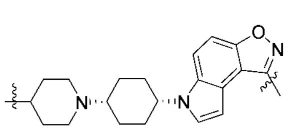 , 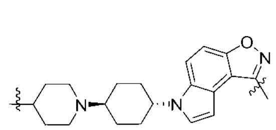 ,

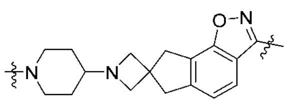 , 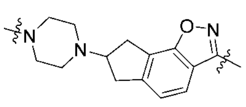 , 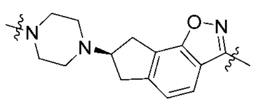 , 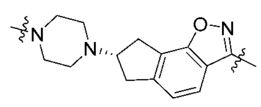 ,

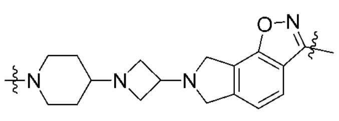 , 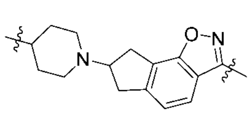 , 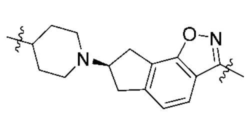 , 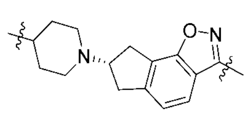 ,

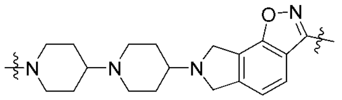, 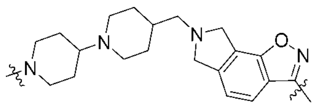, 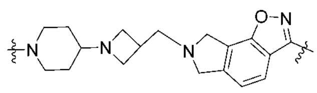,

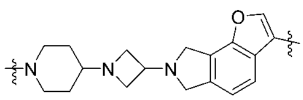, 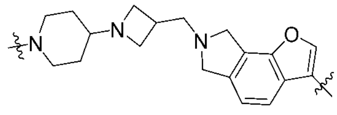, 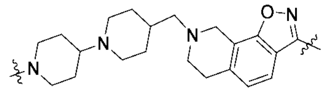,

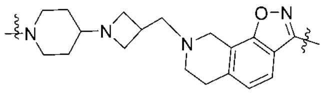, 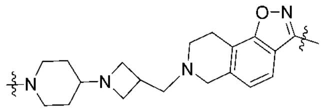, 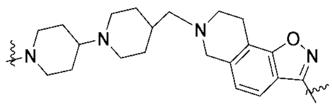,

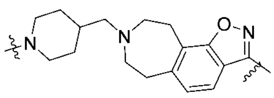, 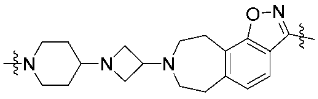, 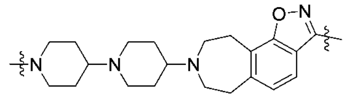,

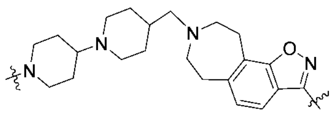, 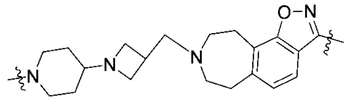, 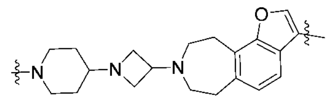,

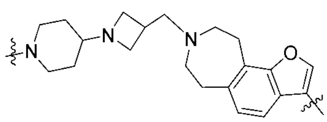, 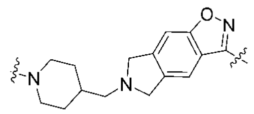, 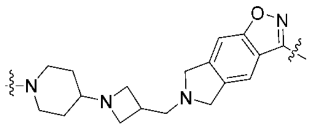,

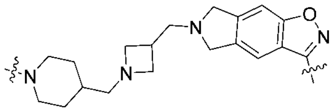, 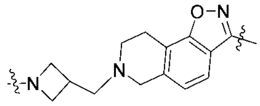, 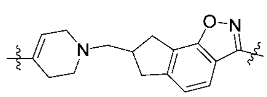, 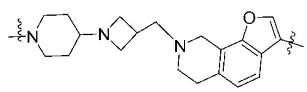,

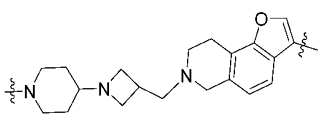, 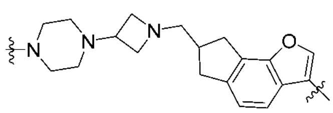, 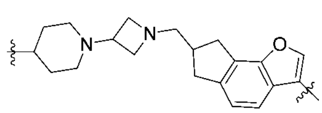,

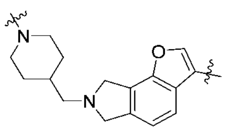, 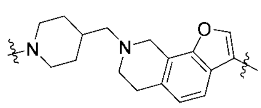, 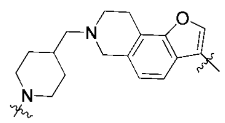, 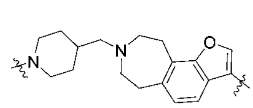,

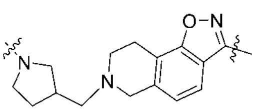, 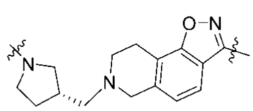, and 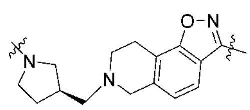.

In some embodiments, the structural fragment

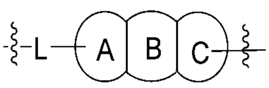

is selected from the group consisting of

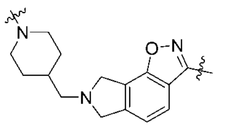 , 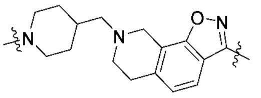 , 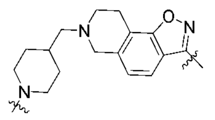 , 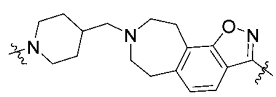 ,

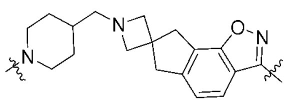 , 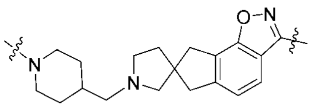 , 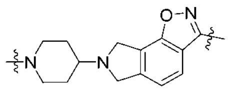 , 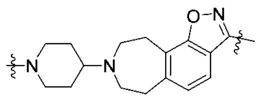 ,

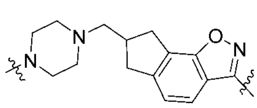 , 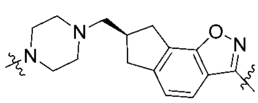 , 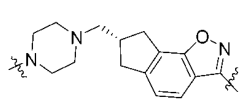 , 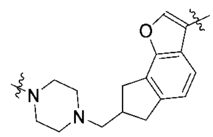 ,

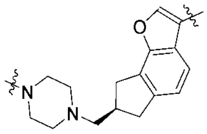 , 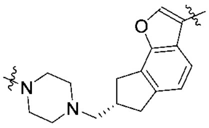 , 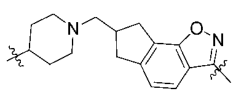 , 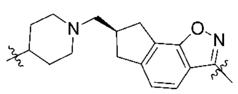 , 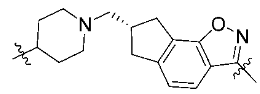 ,

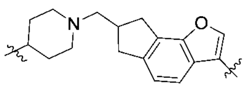 , 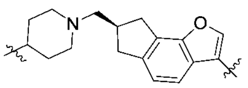 , 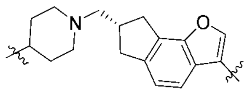 , 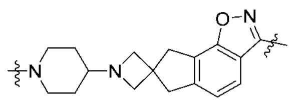 ,

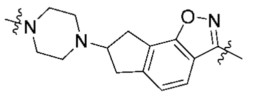 , 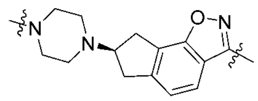 , 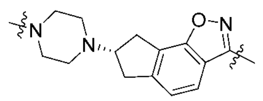 , 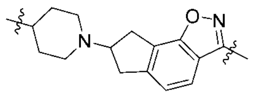 ,

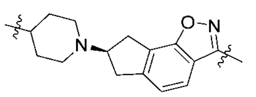 , 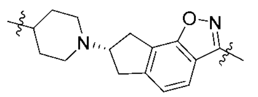 , 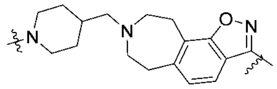 , 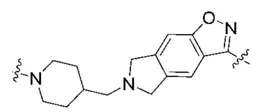 ,

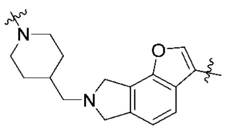 , 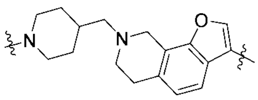 , 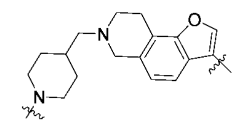 , 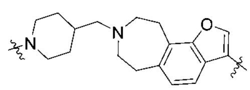 ,

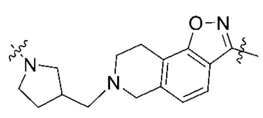 , 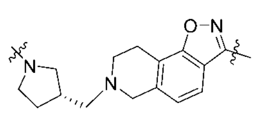 , and 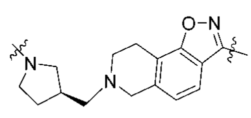 .

In some embodiments, $R^f$ is selected from the group consisting of H, fluorine, chlorine, bromine, deuterium, and $C_{1-3}$ alkyl optionally substituted with one or more substituents. In some embodiments, $R^f$ is selected from the group consisting of H, fluorine, chlorine, bromine, deuterium, and $C_{1-3}$ alkyl, wherein the $C_{1-3}$ alkyl is optionally substituted with one or more of the following groups: halogen, -OH, $-NH_2$, and -CN. In some embodiments, $R^f$ is selected from the group consisting of H, fluorine, chlorine, bromine, deuterium, and $C_{1-3}$ alkyl. In some embodiments, $R^f$ is selected from the group consisting of H, fluorine, deuterium, and methyl. In some embodiments, $R^f$ is H.

In some embodiments, $X^5$ is selected from the group consisting of CH and N. In some embodiments, $X^5$ is CH.

In some embodiments, $X^6$ is selected from the group consisting of -O-, -NH-, and -N($C_{1-3}$ alkyl)-. In some embodiments, $X^6$ is selected from the group consisting of -O- and -N($CH_3$)-. In some embodiments, $X^6$ is -O-.

In some embodiments, each $R^2$, each $R^3$, and each $R^4$ are independently selected from the group consisting of halogen, -OH, -$NH_2$, -CN, $C_{1-6}$ alkyl, $C_{1-6}$ alkoxy, and halogenated $C_{1-6}$ alkyl, wherein the -OH, -$NH_2$, -CN, $C_{1-6}$ alkyl, $C_{1-6}$ alkoxy, or halogenated $C_{1-6}$ alkyl is optionally substituted with one or more substituents. In some embodiments, each $R^2$, each $R^3$, and each $R^4$ are independently selected from the group consisting of halogen, -OH, -$NH_2$, -CN, $C_{1-4}$ alkyl, $C_{1-4}$ alkoxy, and halogenated $C_{1-4}$ alkyl.

In some embodiments, each $R^2$ is independently selected from the group consisting of halogen, -OH, -$NH_2$, -CN, $C_{1-3}$ alkyl, $C_{1-3}$ alkoxy, and halogenated $C_{1-3}$ alkyl. In some embodiments, each $R^2$ is independently selected from the group consisting of halogen, -OH, -$NH_2$, -CN, $C_{1-3}$ alkyl, and $C_{1-3}$ alkoxy. In some embodiments, each $R^2$ is independently selected from the group consisting of fluorine, chlorine, bromine, -OH, -$NH_2$, -CN, methyl, and methoxy. In some embodiments, each $R^2$ is independently selected from the group consisting of fluorine, chlorine, bromine, -CN, and methoxy. In some embodiments, each $R^2$ is independently selected from the group consisting of -CN and methoxy. In some embodiments, each $R^2$ is independently selected from the group consisting of -CN and $C_{1-6}$ alkoxy (e.g., $C_{1-6}$ alkoxy, $C_{1-4}$ alkoxy, or $C_{1-3}$ alkoxy).

In some embodiments, each $R^3$ is independently selected from the group consisting of halogen, -OH, -$NH_2$, -CN, $C_{1-3}$ alkyl, $C_{1-3}$ alkoxy, and halogenated $C_{1-3}$ alkyl. In some embodiments, each $R^3$ is independently selected from the group consisting of halogen, -OH, -$NH_2$, -CN, and $C_{1-3}$ alkyl. In some embodiments, each $R^3$ is independently selected from the group consisting of $C_{1-6}$ alkyl, $C_{1-5}$ alkyl, and $C_{1-4}$ alkyl (preferably $C_{1-3}$ alkyl). In some embodiments, each $R^3$ is independently methyl.

In some embodiments, each $R^4$ is independently selected from the group consisting of halogen, -OH, -$NH_2$, -CN, $C_{1-3}$ alkyl, $C_{1-3}$ alkoxy, and halogenated $C_{1-3}$ alkyl. In some embodiments, each $R^4$ is independently selected from the group consisting of halogen, -OH, -$NH_2$, -CN, and $C_{1-3}$ alkyl. In some embodiments, each $R^4$ is independently selected from the group consisting of fluorine, chlorine, bromine, -OH, -$NH_2$, and -CN.

In some embodiments, m is selected from the group consisting of 0, 1, 2, and 3. In some embodiments, m is selected from the group consisting of 1, 2, and 3. In some embodiments, m is 2. In some embodiments, p is selected from the group consisting of 1, 2, 3, and 4. In some embodiments, p is selected from the group consisting of 3 and 4. In some embodiments, p is 4. In some embodiments, q is selected from the group consisting of 0, 1, 2, and 3. In some embodiments, q is selected from the group consisting of 0 and 1. In some embodiments, q is 0.

In some embodiments, the structural fragment

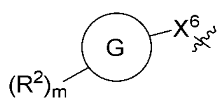 is 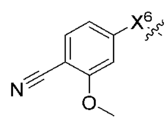.

In some embodiments, ring G is selected from the group consisting of phenyl and 5- to 6-membered heteroaryl. In some embodiments, ring G is selected from the group consisting of phenyl and 6-membered heteroaryl (6-membered heteroaryl containing 1-2 N atoms). In some embodiments, ring G is phenyl.

In some embodiments, ring E is selected from the group consisting of $C_{3-9}$ cycloalkyl and 3- to 9-membered heterocycloalkyl. In some embodiments, ring E is selected from the group consisting of $C_{4-9}$ cycloalkyl and 4- to 9-membered heterocycloalkyl. In some embodiments, ring E is selected from the group consisting of $C_{4-7}$ cycloalkyl and 4- to 7-membered heterocycloalkyl. In some embodiments, ring E is selected from $C_{4-7}$ cycloalkyl (e.g., $C_{4-6}$ cycloalkyl or $C_{4-5}$ cycloalkyl). In some embodiments, ring E is selected from the group consisting of cyclobutyl, cyclopentyl, and cyclohexyl.

In some embodiments, ring E is cyclobutyl. In some embodiments, ring E is

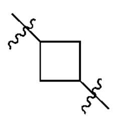.

In some embodiments, ring E is

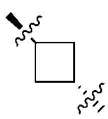.

In some embodiments, the structural moiety

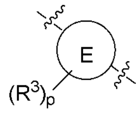 is 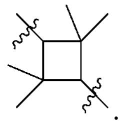.

In some embodiments, the structural moiety

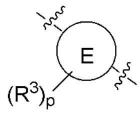 is 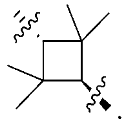.

In some embodiments, ring F is selected from the group consisting of $C_{6-10}$ aryl and 5- to 7-membered heteroaryl. In some embodiments, ring F is selected from the group consisting of phenyl and 5- to 6-membered heteroaryl. In some embodiments, ring F is selected from the group consisting of phenyl and 6-membered heteroaryl (e.g., 6-membered heteroaryl containing 1-3 or 1-2 N atoms). In some embodiments, ring F is selected from the group consisting of phenyl, pyridinyl, pyridazinyl, pyrimidinyl, and pyrazinyl. In some specific embodiments, ring F is phenyl. In some embodiments, ring F is pyridinyl. In some embodiments, ring F is pyrimidinyl. In some embodiments, ring F is pyrazinyl. In some embodiments, ring F is pyridazinyl. In some embodiments, ring F is selected from the group consisting of

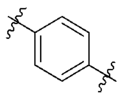, 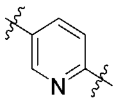, 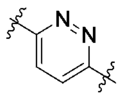, 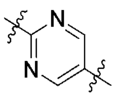, and 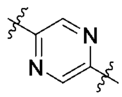.

In some embodiments, ring F is

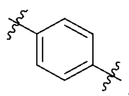.

In some embodiments, ring F is

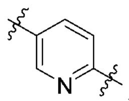.

In some embodiments, ring F is

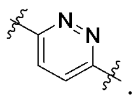.

**In** some embodiments, ring F is

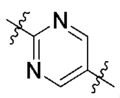.

In some embodiments, ring F is

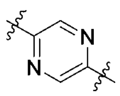.

In some embodiments,

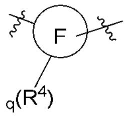

is selected from the group consisting of

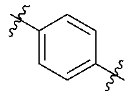, 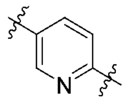, 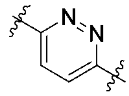,

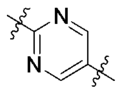, and 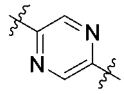.

In some embodiments, $R^t$ is selected from the group consisting of hydrogen, -OH, $C_{1-4}$ alkyl, $C_{3-6}$ cycloalkyl, and 3-to 6-membered heterocycloalkyl, wherein the $C_{1-4}$ alkyl, $C_{3-6}$ cycloalkyl, or 3- to 6-membered heterocycloalkyl is optionally substituted. In some embodiments, $R^t$ is selected from the group consisting of hydrogen, -OH, $C_{1-3}$ alkyl, $C_{3-4}$ cycloalkyl, and 3- to 4-membered heterocycloalkyl, wherein the $C_{1-3}$ alkyl, $C_{3-4}$ cycloalkyl, or 3- to 4-membered heterocycloalkyl is optionally substituted. In some embodiments, $R^t$ is selected from the group consisting of hydrogen and $C_{1-3}$ alkyl (e.g., methyl, ethyl, or propyl). In some embodiments, $R^t$ is hydrogen.

In some embodiments, the optional substitution refers to optional substitution with one or more of the following groups: halogen (e.g., fluorine, chlorine, bromine, or iodine), -CN, -OH, and $-NH_2$.

In some embodiments, the structural fragment

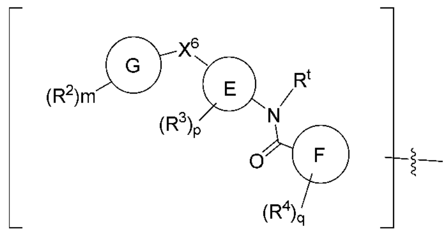

is selected from

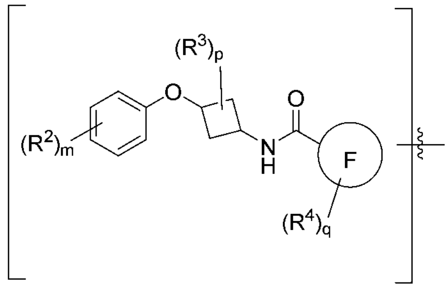

.

In some embodiments, the structural fragment

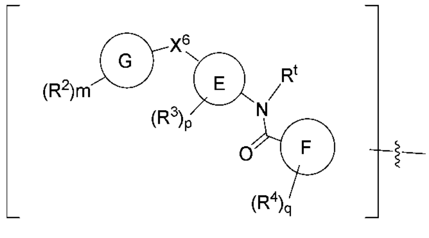

is selected from

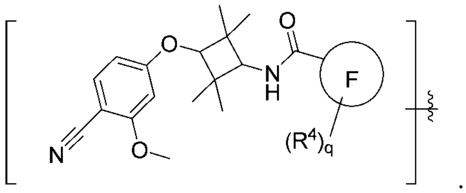

In some embodiments, the structural fragment

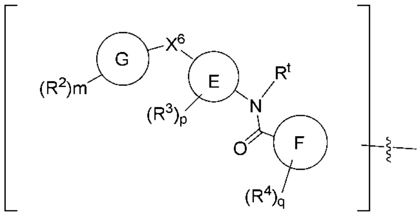

is selected from

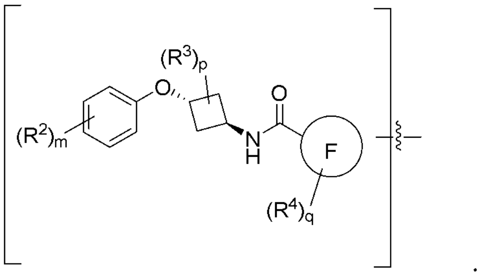

In some embodiments, the structural fragment

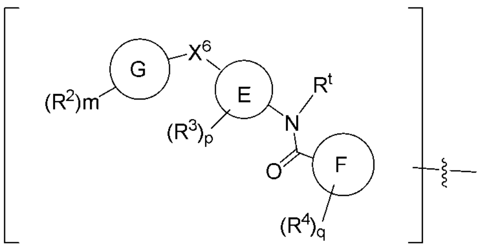

is selected from

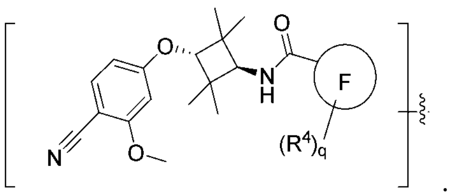

In some embodiments, the structural fragment

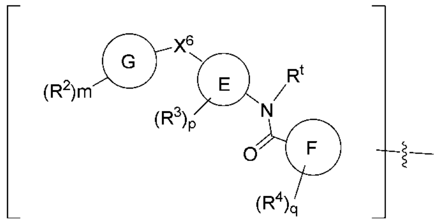

is selected from

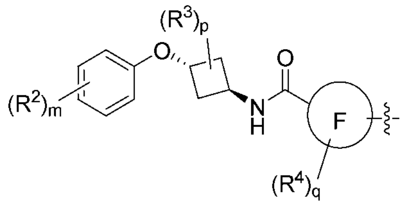

.

In some embodiments, the structural fragment

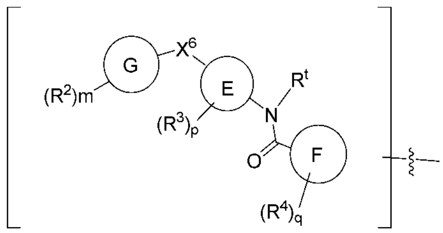

is selected from

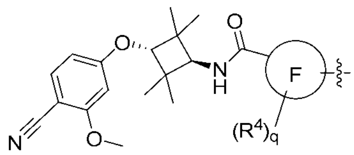

.

In some embodiments, the structural fragment

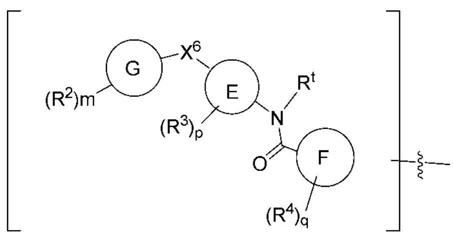

is selected from the group consisting of

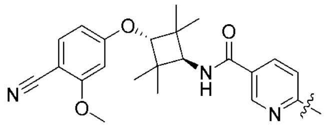

,

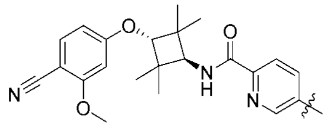

,

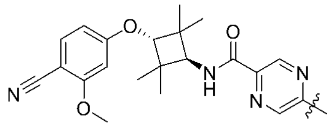

,

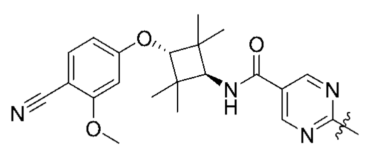, 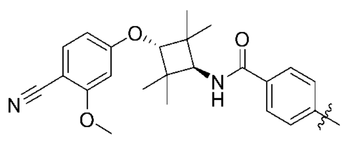, 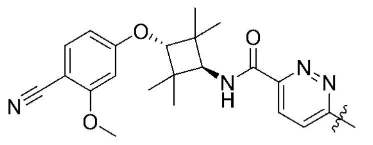,

and

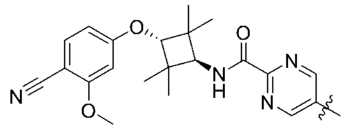.

In some embodiments, the structural fragment

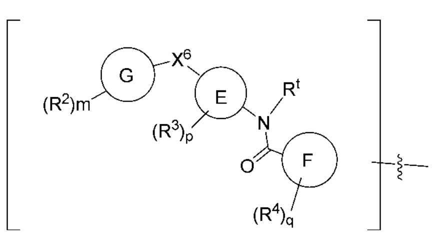, 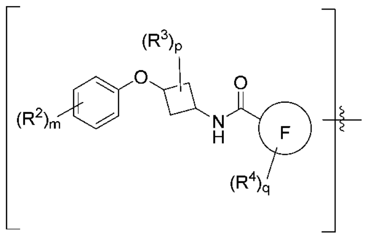, 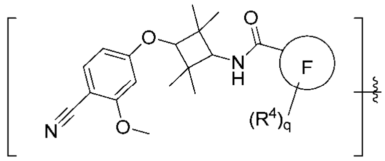,

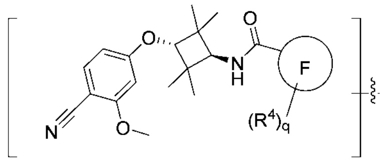, or 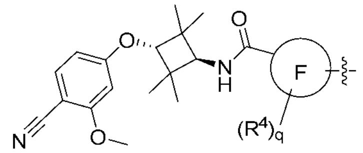 is 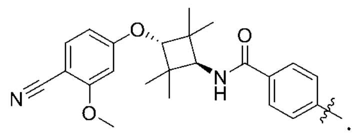.

In some embodiments, the heterocycloalkenyl, heteroaryl, heterocycloalkyl, or heteroalkylene described above comprises one or more heteroatoms or heteroatom groups independently selected from the group consisting of -O-, -NH-, -N-, -S-, C=O, -C(=O)NH-, -C(=O)O-, -S(=O)-, and $-S(=O)_2-$; in some embodiments, the heterocycloalkenyl, heteroaryl, heterocycloalkyl, or heteroalkylene described above comprises one or more heteroatoms or heteroatom groups independently selected from the group consisting of -O-, -NH-, -N-, and -S-; in some embodiments, the heterocycloalkenyl, heteroaryl, heterocycloalkyl, or heteroalkylene described above comprises one or more heteroatoms or heteroatom groups independently selected from the group consisting of -O-, -NH-, and -N-. In some embodiments, the number of the heteroatoms or heteroatom groups is independently selected from the group consisting of 1, 2, 3, 4, 5, and 6, or is selected from the group consisting of 1, 2, 3, and 4, or is selected from the group consisting of 1, 2, and 3, or is selected from the group consisting of 1 and 2.

In some embodiments, the heteroatom in the heterocycloalkenyl described above is selected from the group consisting of N, NH, O, and S. In some embodiments, the heteroatom in the heterocycloalkenyl described above is selected from the group consisting of N, O, and S. In some specific embodiments, the heteroatom in the heterocycloalkenyl described above is selected from the group consisting of N and O. In some embodiments, the number of the heteroatoms in the heterocycloalkenyl described above is selected from the group consisting of 1, 2, 3, 4, 5, and 6. In some embodiments, the number of the heteroatoms in the heterocycloalkenyl described above is selected from the group consisting of 1, 2, 3, and 4. In some embodiments, the number of the heteroatoms in the heterocycloalkenyl described above is selected from the group consisting of 1, 2, and 3. In some specific embodiments, the number of the heteroatoms in the heterocycloalkenyl described above is selected from the group consisting of 1 and 2.

It should be understood that any embodiment of the compounds of the present application as described above, and any specific substituents set forth herein with respect to particular ring A, ring B, ring C, ring E, ring F, ring G, $R^1$, $R^2$, $R^3$, $R^4$, $R^t$, L, $X^5$, and $X^6$ substituents in the compounds of the present application as described above, may be independently combined with other embodiments of the present application and/or substituents of the compounds to form embodiments of the present disclosure not specifically set forth above. Further, when a range of substituents is disclosed in specific embodiments and/or claims with respect to any particular ring A, ring B, ring C, ring E, ring F, ring G, $R^1$, $R^2$, $R^3$, $R^4$, $R^t$, L, $X^5$, and $X^6$ substituents, it should be understood that one or more substituents may be deleted from the range and the remaining range of substituents should also be considered as an embodiment of the present application.

The present application relates to a compound of formula I-1 or I-1A, a stereoisomer thereof, or a pharmaceutically acceptable salt thereof,

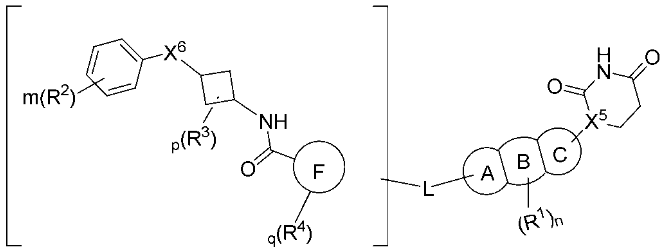

**I-1**

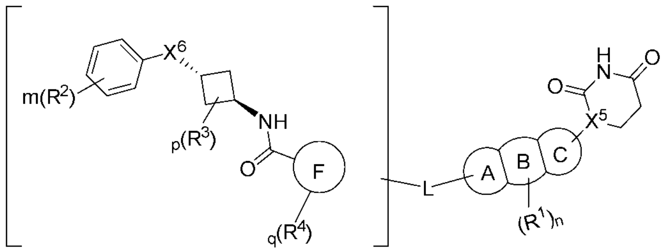

**I-1A**

wherein ring A, ring B, ring C, ring F, $R^1$, n, $R^2$, m, $R^3$, p, $R^4$, q, $X^5$, $X^6$, and L are as defined herein.

In some embodiments, the structural moiety

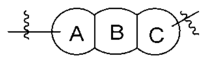

is as described herein.

The present application relates to a compound of formula I-2, formula I-2A, formula I-3, or formula I-3A, a stereoisomer thereof, or a pharmaceutically acceptable salt thereof,

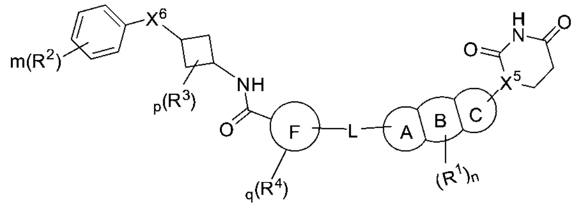

**I-2** ,

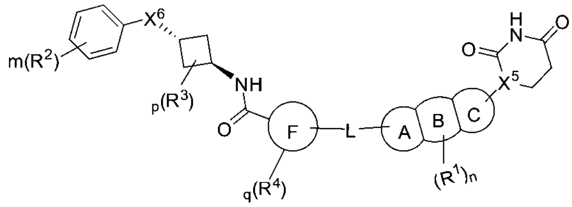

**I-2A** ,

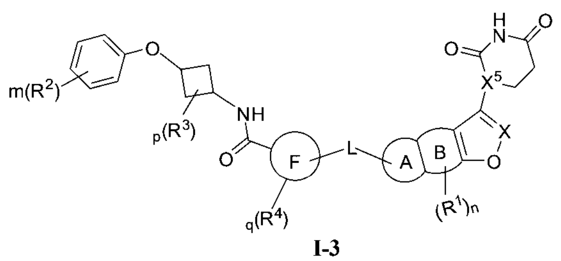

**I-3** , or

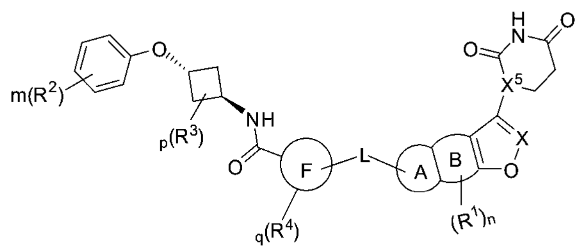

**I-3A** ,

wherein ring A, ring B, ring C, ring F, $R^1$, n, $R^2$, m, $R^3$, p, $R^4$, q, $X^5$, and L are as defined herein;
X is selected from the group consisting of CH and N.

In some embodiments, the structural fragments

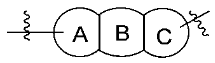 and 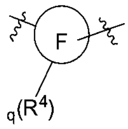

are as defined herein.

In some embodiments, optionally, the compound of the present application is not the following compound:

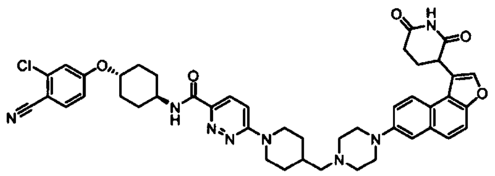,

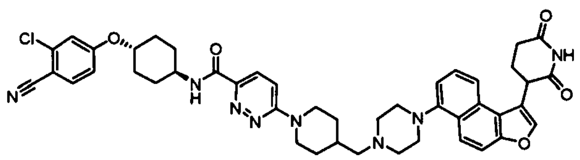,

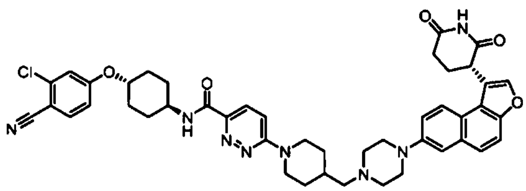,

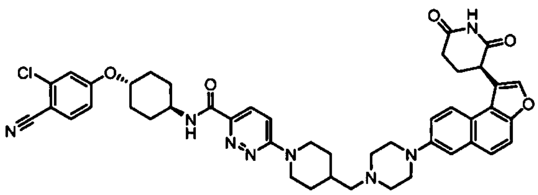,

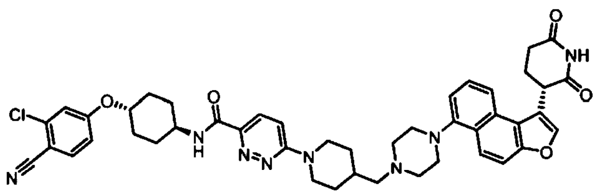, or

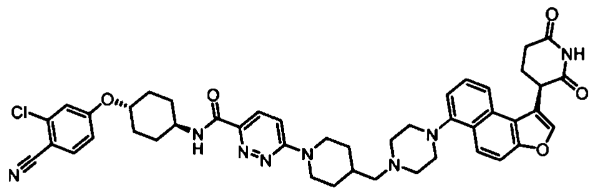.

In some embodiments, optionally, the compound of the present application is not the following compound:

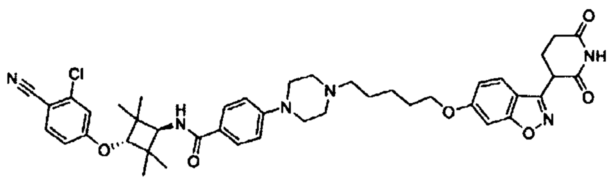

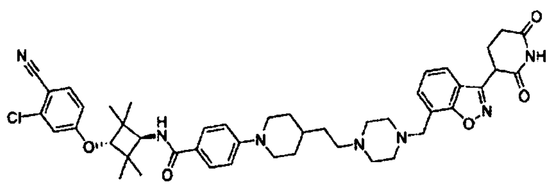

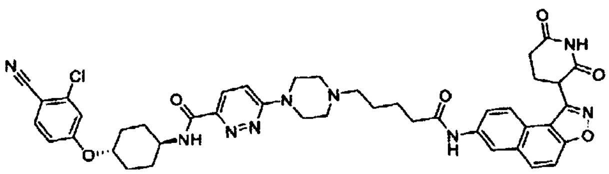

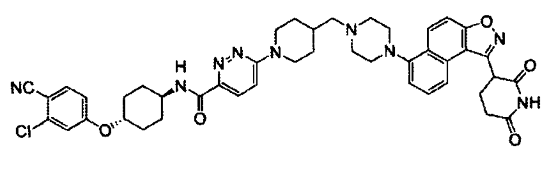

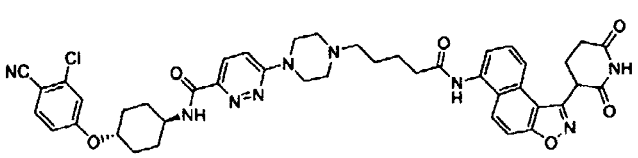

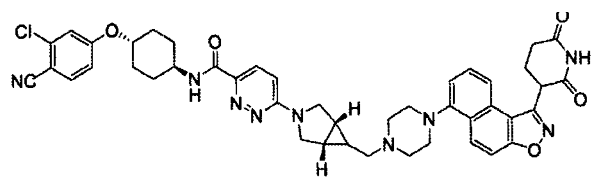

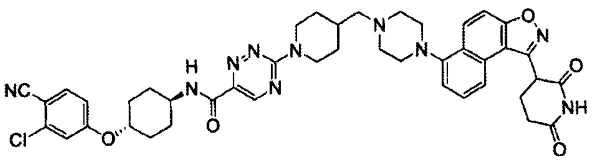 or 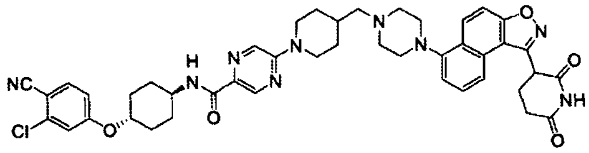 .

In some embodiments, optionally, the compound of the present application is not the following compound:

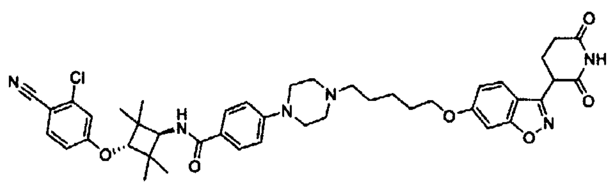 or 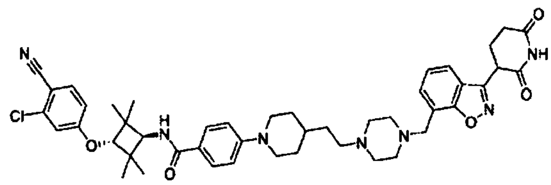 .

In some embodiments, the structural moiety

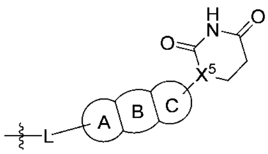 or 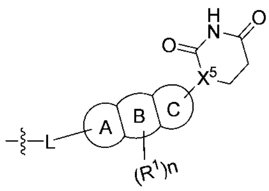

is not selected from the group consisting of

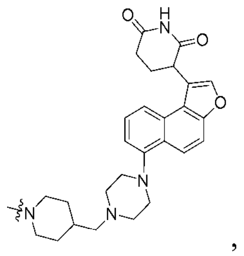 , 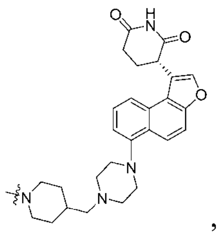 , 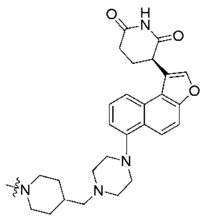 , 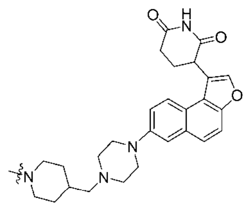 ,

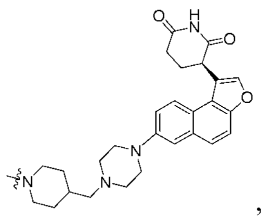 , and 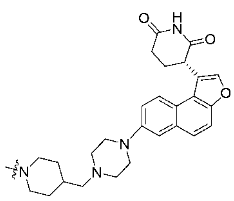 .

In some embodiments, the structural moiety

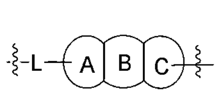 or 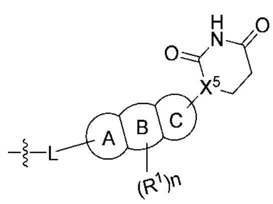

is not selected from the group consisting of

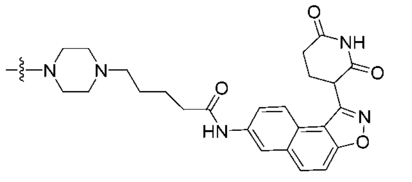,
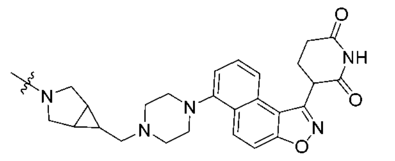,

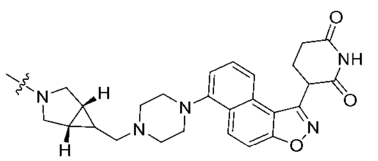,
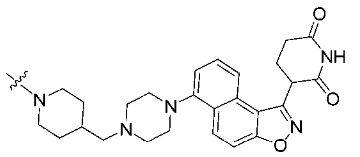,
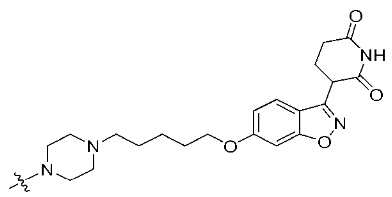,

and

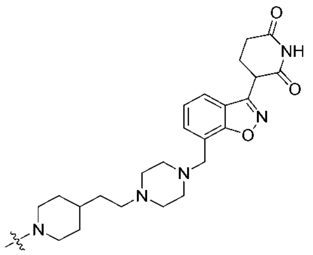

.In some embodiments, the structural moiety

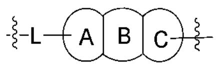

or

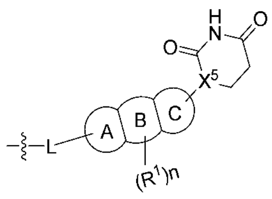

is not selected from the group consisting of

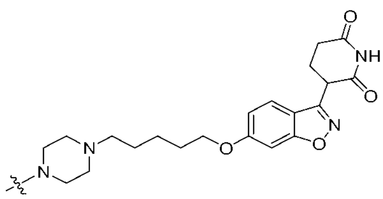

and

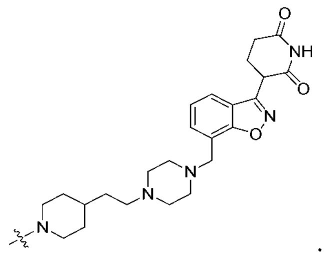

.

In some embodiments, the structural moiety

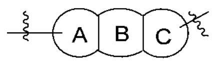 or 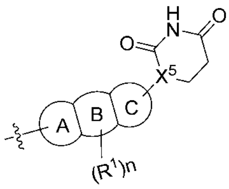

is not selected from the group consisting of

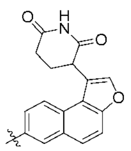, 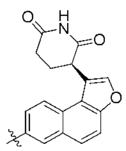, 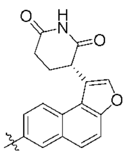, 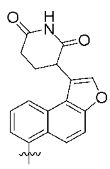, 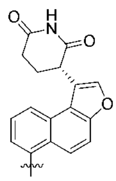, and 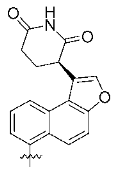.

**In** some embodiments, the structural moiety

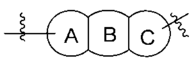 or 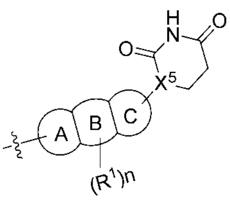

is not selected from the group consisting of

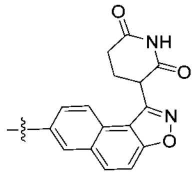, 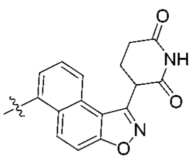, 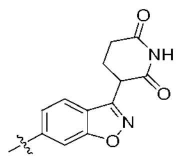, and 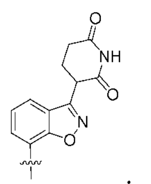.

In some embodiments, the structural moiety

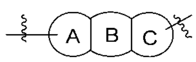 or 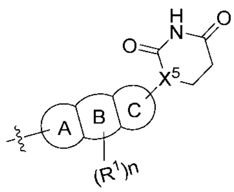

is not selected from the group consisting of

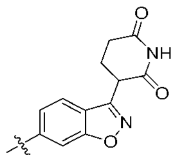 and 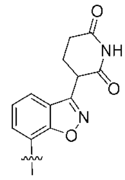 .

In another aspect, the present application relates to a compound of formula I' or I", a moiety, a stereoisomer thereof, a derivative (specifically, such as a Protac molecule), or a pharmaceutically acceptable salt thereof,

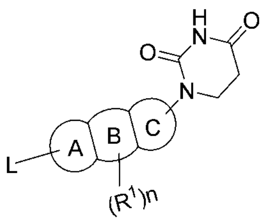

I'

or

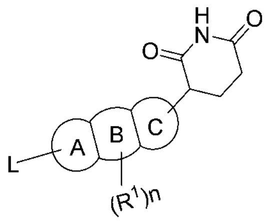

I" ,

wherein ring A is absent or selected from the group consisting of $C_{5-10}$ cycloalkenyl, 5- to 10-membered heterocycloalkenyl, phenyl, and 5- to 6-membered heteroaryl;
ring B is phenyl;
ring C is selected from the group consisting of isoxazolyl and furanyl;
L is a connecting group; optionally, L is as defined herein; optionally, $R^1$ and n are as defined herein.

In some embodiments, the present application relates to the following moiety, or a compound (including a Protac molecule) comprising the moiety, a stereoisomer thereof, or a pharmaceutically acceptable salt thereof:

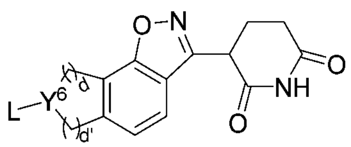

wherein L is as defined above, $Y^6$ is $C(R^5)$ or N, and $R^5$ is H, deuterium, tritium, $C_{1-3}$ alkyl, or =O (preferably H); d and d' may be identical or different and are each independently 1, 2, or 3.

In some embodiments, the present application relates to the following moiety, or a compound (including a Protac molecule) comprising the moiety, a stereoisomer thereof, or a pharmaceutically acceptable salt thereof:

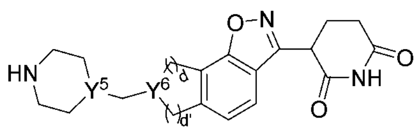

wherein L, $Y^6$, d, and d' are as defined herein, $Y^5$ is $C(R^5)$ or N, and $R^5$ is H, deuterium, tritium, $C_{1-3}$ alkyl, or =O (preferably H).

In some embodiments, the structural moiety

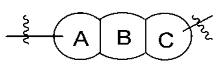

is as described herein.

In another aspect, the present application relates to a compound of formula I'-a or I"-a, a moiety, a stereoisomer thereof, a derivative (specifically, such as a Protac molecule), or a pharmaceutically acceptable salt thereof,

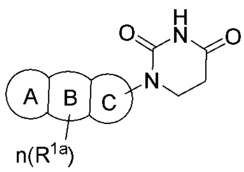 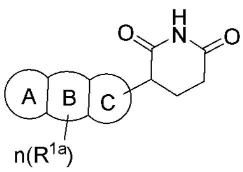

I'-a I''-a

wherein ring A is absent or selected from the group consisting of $C_{5-10}$ cycloalkenyl, 5- to 10-membered heterocycloalkenyl, phenyl, and 5- to 6-membered heteroaryl;
ring B is phenyl;
ring C is selected from the group consisting of isoxazolyl and furanyl;
each $R^{1a}$ is independently selected from the group consisting of halogen, -OH, $-NH_2$, -CN, =O, -CHO, $C_{1-4}$ alkyl, $C_{1-4}$ alkoxy, $C_{1-6}$ alkyl OC(O)-, $C_{3-12}$ cycloalkyl, and 4- to 12-membered heterocycloalkyl, wherein the $C_{1-4}$ alkyl, $C_{1-4}$ alkoxy, $C_{3-12}$ cycloalkyl, or 4- to 12-membered heterocycloalkyl is optionally substituted with one or more of the following groups: halogen, =O, -OH, $-NH_2$, -CN, CHO, COOH, $-C_{1-4}$ alkyl-OH, $C_{1-6}$ alkyl OC(O)-, and 4- to 10-membered heterocycloalkyl optionally substituted with $C_{1-6}$ alkyl COC(O)-;
n is selected from the group consisting of 0, 1, 2, and 3.

In some embodiments, ring A is absent or selected from the group consisting of $C_{5-7}$ cycloalkenyl, 5- to 9-membered heterocycloalkenyl, phenyl, and 5- to 6-membered heteroaryl.

In some embodiments, ring A is absent or selected from the group consisting of $C_{5-6}$ cycloalkenyl, 5- to 10-membered heterocycloalkenyl, phenyl, and 5- to 6-membered heteroaryl.

In some embodiments, ring A is absent or selected from the group consisting of $C_{5-6}$ cycloalkenyl, 5- to 9-membered heterocycloalkenyl, phenyl, and 5- to 6-membered heteroaryl.

In some embodiments, ring A is absent or selected from the group consisting of $C_{5-6}$ cycloalkenyl, 5- to 9-membered heterocycloalkenyl, phenyl, and 5-membered heteroaryl.

In some embodiments, ring A is absent or selected from the group consisting of $C_{5-6}$ cycloalkenyl, 5- to 9-membered heterocycloalkenyl, phenyl, pyrrolyl, pyrazolyl, furanyl, and oxazolyl.

In some embodiments, ring A is absent or selected from the group consisting of $C_5$ cycloalkenyl, $C_6$ cycloalkenyl, 5-membered, 6-membered, 7-membered, 8-membered, and 9-membered heterocycloalkenyl, phenyl, pyrrolyl, pyrazolyl, furanyl, and oxazolyl.

In some embodiments, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl.

In some specific embodiments, ring A is selected from the group consisting of $C_{5-9}$ cycloalkenyl, 5- to 9-membered heterocycloalkenyl, phenyl, and 5-membered heteroaryl.

In some specific embodiments, ring A is selected from the group consisting of $C_{5-6}$ cycloalkenyl, 5- to 9-membered heterocycloalkenyl, phenyl, and 5-membered heteroaryl.

In some specific embodiments, ring A is selected from the group consisting of $C_{5-9}$ cycloalkenyl and 5- to 9-membered heterocycloalkenyl. In some specific embodiments, ring A is selected from the group consisting of $C_{5-6}$ cycloalkenyl and 5- to 9-membered heterocycloalkenyl.

In some specific embodiments, ring A is selected from $C_{5-9}$ cycloalkenyl. In some specific embodiments, ring A is selected from $C_{5-6}$ cycloalkenyl. In some specific embodiments, ring A is selected from 5- to 9-membered heterocycloalkenyl.

In some specific embodiments, ring A is selected from the group consisting of cyclopentenyl, dihydropyrrolyl, tetrahydropyridinyl, tetrahydroazepinyl, azaspirononenyl, azaspirooctenyl, phenyl, pyrrolyl, and pyrazolyl.

In some specific embodiments, ring A is selected from the group consisting of cyclopentenyl, dihydropyrrolyl, tetrahydropyridinyl, tetrahydroazepinyl, azaspirononenyl, and azaspirooctenyl. In some specific embodiments, ring A is selected from the group consisting of cyclopentenyl, dihydropyrrolyl, tetrahydropyridinyl, tetrahydroazepinyl, and azaspirooctenyl.

In some specific embodiments, ring A is selected from cyclopentenyl. In some specific embodiments, ring A is selected from the group consisting of dihydropyrrolyl, tetrahydropyridinyl, tetrahydroazepinyl, and azaspirooctenyl. In some specific embodiments, ring A is selected from the group consisting of phenyl, pyrrolyl, and pyrazolyl.

In some embodiments, ring A is selected from the group consisting of

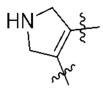, 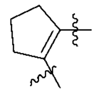, 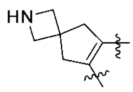, 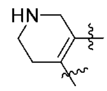,

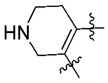, 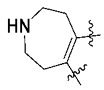, 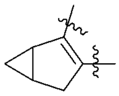, 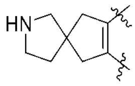, 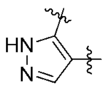, 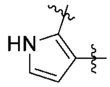, 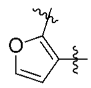, 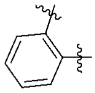, 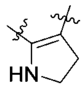, 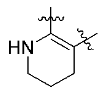,

and

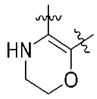.

In some specific embodiments, ring C is isoxazolyl. In some specific embodiments, ring C is furanyl.

In some embodiments, the structural moiety

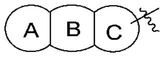

is selected from the group consisting of

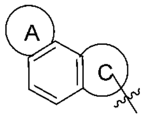

and

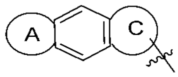.

In some embodiments, the structural fragment

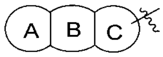

is selected from the group consisting of

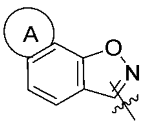,

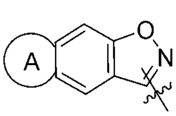, 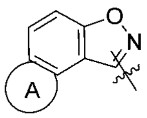, 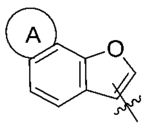, 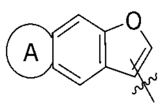, and 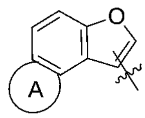.

In some embodiments, the structural fragment

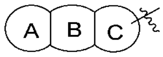

is selected from the group consisting of

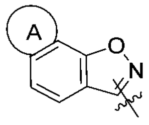, 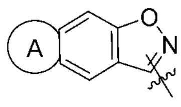,

and

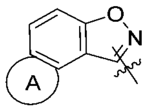;

or, the structural fragment

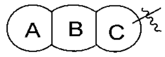

is selected from the group consisting of

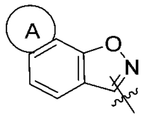

and

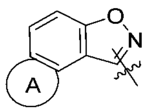;

or, the structural fragment

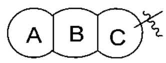

is selected from

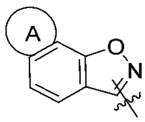.

In some embodiments, the structural fragment

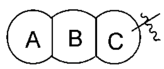

is selected from the group consisting of

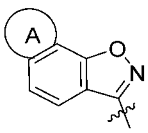, 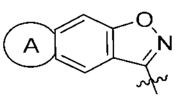, 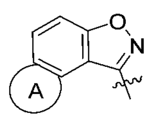, 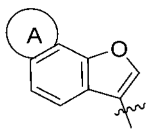, 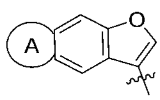, and 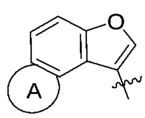.

**In** some embodiments, the structural fragment

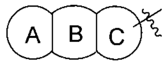

is selected from the group consisting of

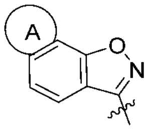 ,

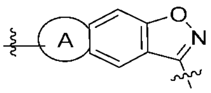 , and 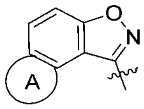 ;

or, the structural fragment

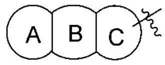

is selected from the group consisting of

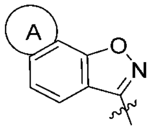 and 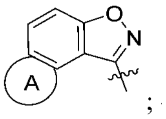 ;

or, the structural fragment

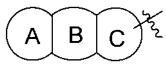 is 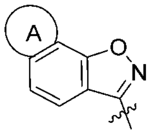 .

In some embodiments, the structural fragment

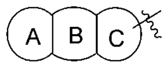

is selected from the group consisting of

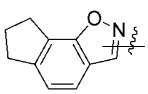 , 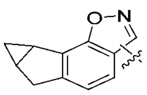 , 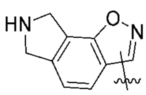 , 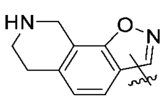 , 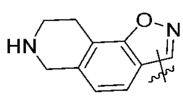 , 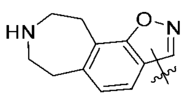 ,

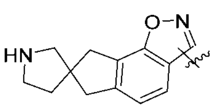 , 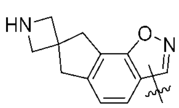 , 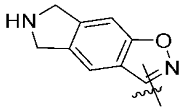 , 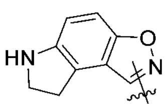 , 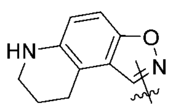 , 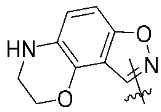 ,

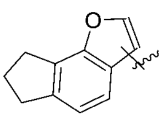 , 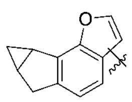 , 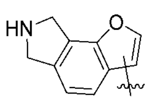 , 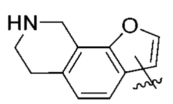 , 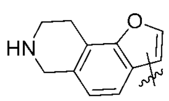 , 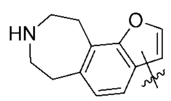 ,

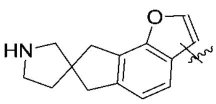, 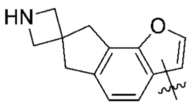, 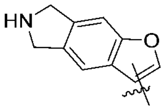, 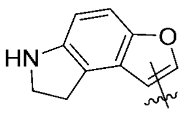, 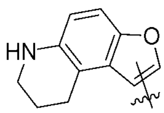, 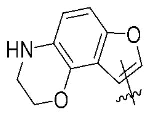,

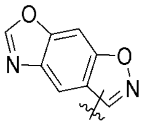, 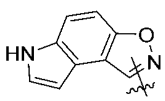, 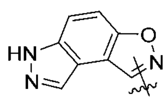, 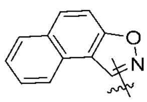, 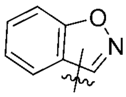, 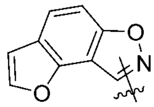, and 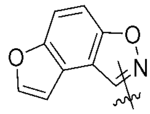.

In some specific embodiments, the structural fragment

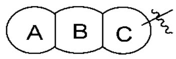

is not selected from the group consisting of

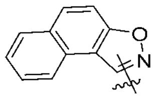 and 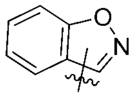.

In some embodiments, the structural fragment

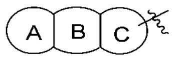

is selected from the group consisting of

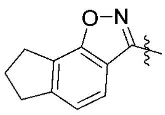, 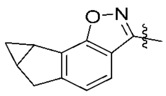, 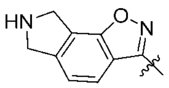, 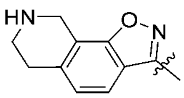, 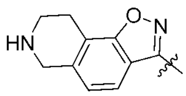, 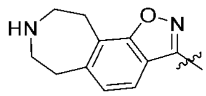,

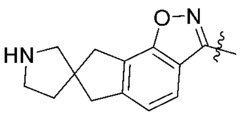, 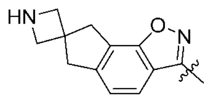, 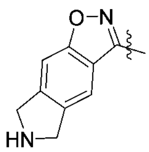, 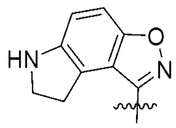, 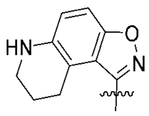, 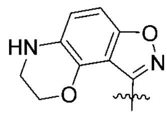,

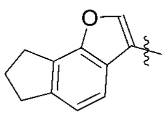, 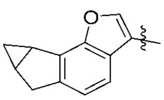, 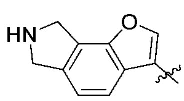, 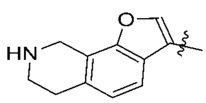, 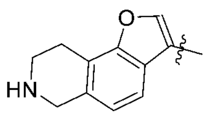, 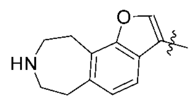,

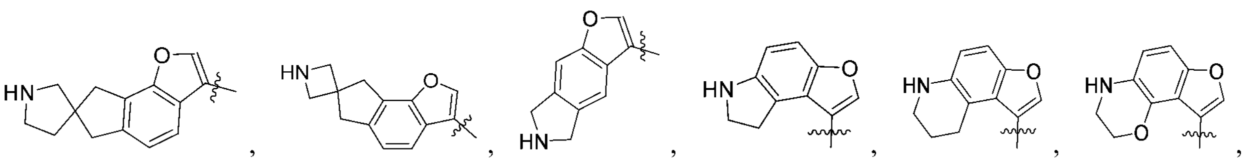 , , , , , ,

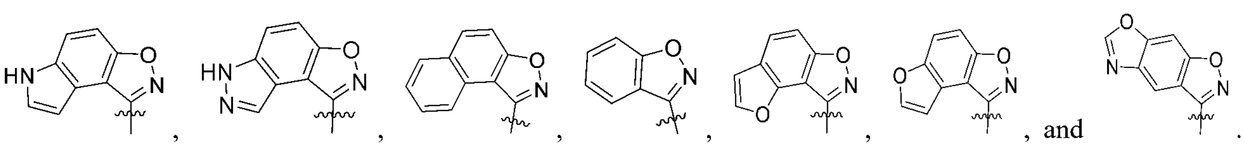 , , , , , , and .

In some embodiments, each $R^{1a}$ is independently selected from the group consisting of halogen, -OH, $-NH_2$, -CN,-CHO, $C_{1-6}$ alkyl OC(O)-, $C_{1-4}$ alkyl, $C_{1-4}$ alkoxy, $C_{3-10}$ cycloalkyl, and 4- to 10-membered heterocycloalkyl, wherein the $C_{1-4}$ alkyl, $C_{1-4}$ alkoxy, $C_{3-10}$ cycloalkyl, or 4- to 10-membered heterocycloalkyl is optionally substituted with one or more of the following groups: halogen, =O, -OH, $-NH_2$, -CN, -CHO, -COOH, $-C_{1-4}$ alkyl-OH, $C_{1-6}$ alkyl OC(O)-, and 4- to 10-membered heterocycloalkyl optionally substituted with $C_{1-6}$ alkyl COC(O)-.

In some embodiments, each $R^{1a}$ is independently selected from the group consisting of halogen, -OH, $-NH_2$, -CN, - CHO, $C_{1-4}$ alkyl OC(O)-, $C_{1-3}$ alkyl, $C_{1-3}$ alkoxy, $C_{3-9}$ cycloalkyl, and 4- to 9-membered heterocycloalkyl, wherein the $C_{1-3}$ alkyl, $C_{1-3}$ alkoxy, $C_{3-9}$ cycloalkyl, or 4- to 9-membered heterocycloalkyl is optionally substituted with one or more of the following groups: halogen, -OH, =O, $-NH_2$, -CN, -CHO, -COOH, $-C_{1-4}$ alkyl-OH, $C_{1-4}$ alkyl OC(O)-, and 4- to 9-membered heterocycloalkyl optionally substituted with $C_{1-4}$ alkyl COC(O)-.

In some embodiments, each $R^{1a}$ is independently selected from the group consisting of halogen, -OH, $-NH_2$, -CN, - CHO, $C_{1-6}$ alkyl OC(O)-, $C_{1-3}$ alkyl, $C_{1-3}$ alkoxy, $C_{3-6}$ cycloalkyl, and 4- to 6-membered heterocycloalkyl, wherein the $C_{1-3}$ alkyl, $C_{1-3}$ alkoxy, $C_{4-6}$ cycloalkyl, or 4- to 6-membered heterocycloalkyl is optionally substituted with one or more of the following groups: halogen, -OH, =O, $-NH_2$, -CN, -CHO, -COOH, $-C_{1-4}$ alkyl-OH, $C_{1-6}$ alkyl OC(O)-, and 3- to 6-membered heterocycloalkyl optionally substituted with $C_{1-6}$ alkyl COC(O)-.

In some embodiments, each $R^{1a}$ is independently selected from the group consisting of halogen, -OH, $-NH_2$, -CN, - CHO, $C_{1-4}$ alkyl OC(O)-, $C_{1-3}$ alkyl, and 4- to 6-membered heterocycloalkyl, wherein the $C_{1-3}$ alkyl or 4- to 6-membered heterocycloalkyl is optionally substituted with one or more of the following groups: -OH, =O, $-NH_2$, - CN, -CHO, -COOH, $C_{1-4}$ alkyl OC(O)-, and 4- to 6-membered heterocycloalkyl optionally substituted with $C_{1-4}$ alkyl OC(O)-.

In some embodiments, each $R^{1a}$ is independently selected from the group consisting of halogen, -OH, -CHO, $(CH_3)_3COC(O)$-, $C_{1-3}$ alkyl, cyclobutyl, and piperidinyl, wherein the $C_{1-3}$ alkyl, cyclobutyl, or piperidinyl is optionally substituted with one or more of the following groups: -OH, $(CH_3)_3COC(O)$-, and cyclobutyl optionally substituted with $(CH_3)_3COC(O)$-.

In some embodiments, each $R^{1a}$ is independently selected from the group consisting of F, -OH, -CHO, $(CH_3)_3COC(O)$-, $-CH_2OH$,

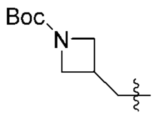, 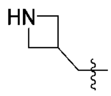, 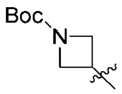, 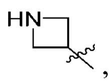, 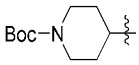, and 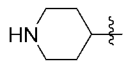.

In some embodiments, n is selected from the group consisting of 0, 1, and 2. In some embodiments, n is selected from the group consisting of 0 and 1. In some embodiments, n is 0.

The present application relates to the following compound, a moiety, a stereoisomer thereof, a derivative (specifically, such as a Protac molecule), or a pharmaceutically acceptable salt thereof,

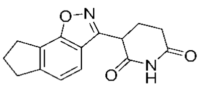, 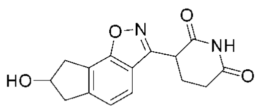, 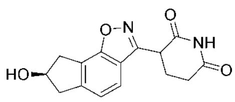, 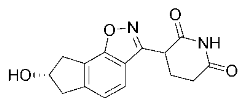,

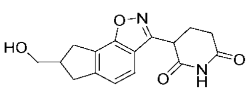, 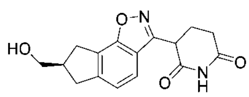, 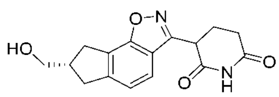, 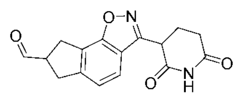,

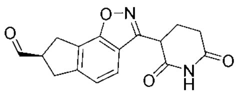, 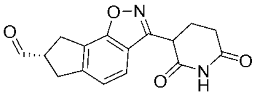, 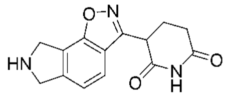, 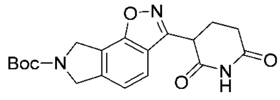,

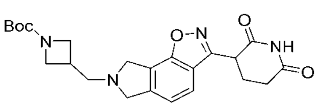, 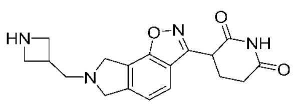, 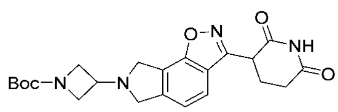,

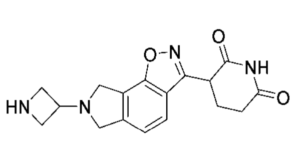, 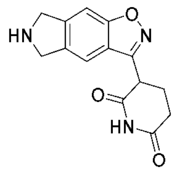, 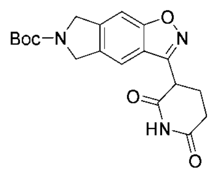, 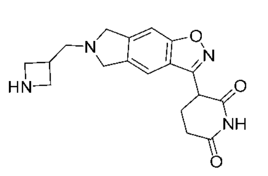,

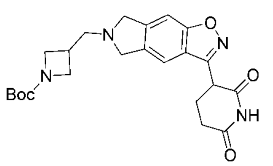, 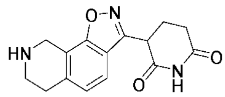, 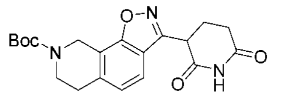, 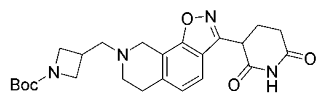,

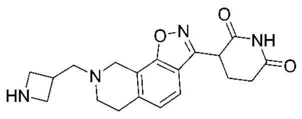, 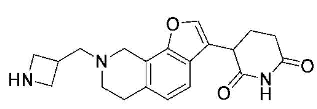, 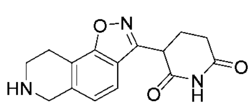, 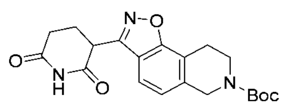,

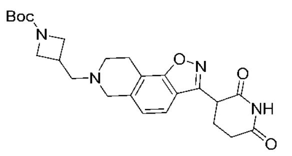, 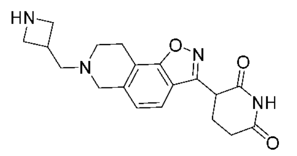, 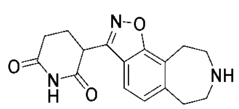, 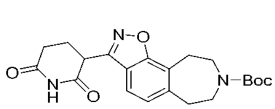,

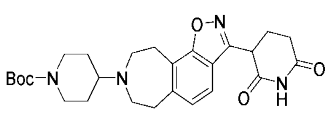, 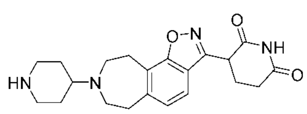, 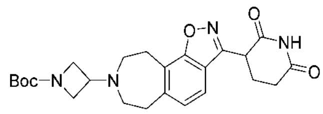,

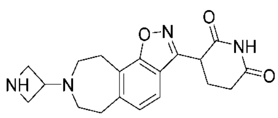, 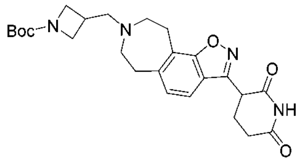, 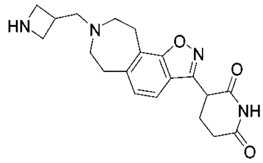, 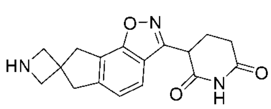,

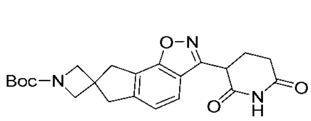, 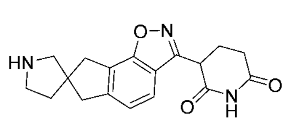, 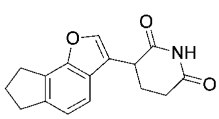, 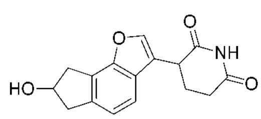,

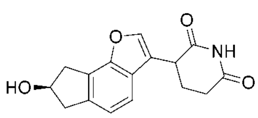, 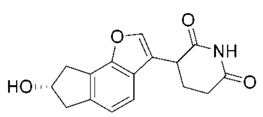, 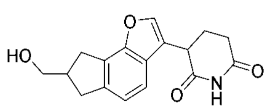, 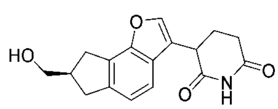,

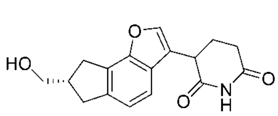, 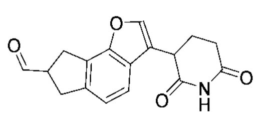, 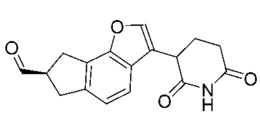, 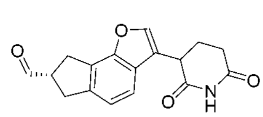,

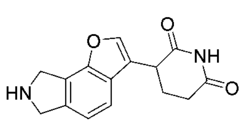, 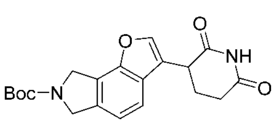, 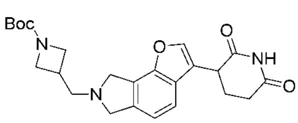, 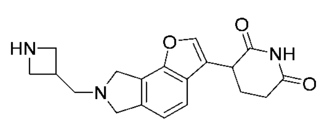,

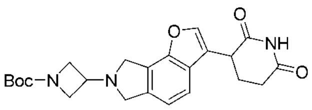, 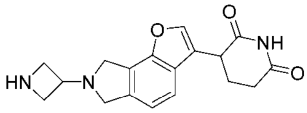, 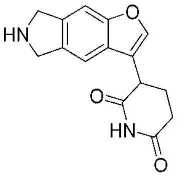, 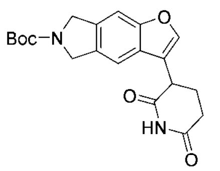,

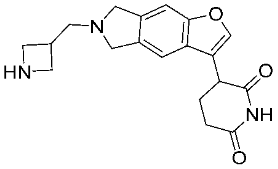, 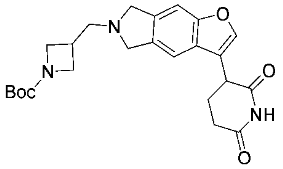, 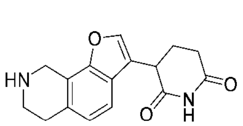, 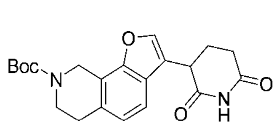,

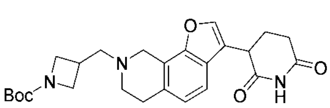, 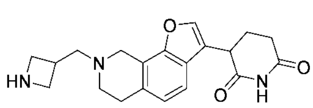, 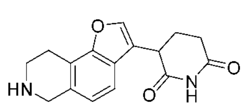, 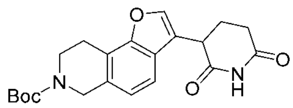,

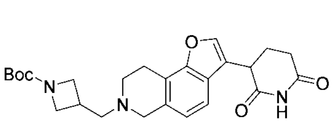, 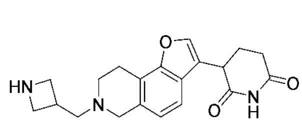, 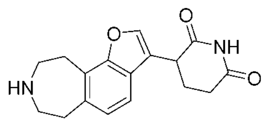, 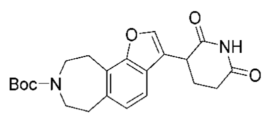,

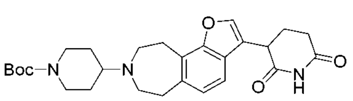, 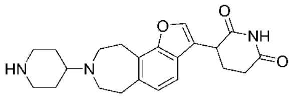, 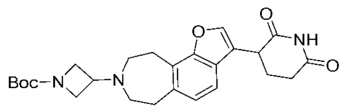,

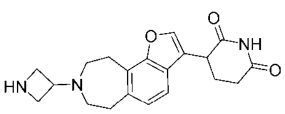, 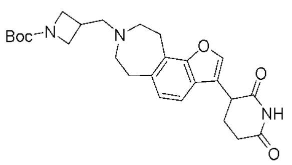, 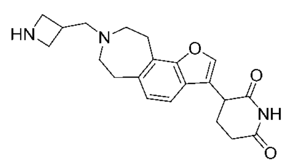, 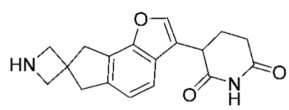,

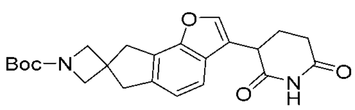, or 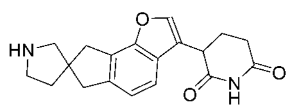,

or,

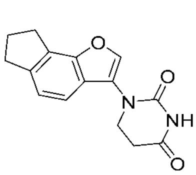, 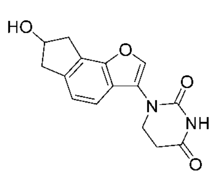, 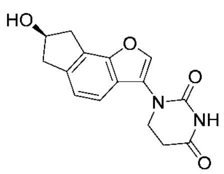, 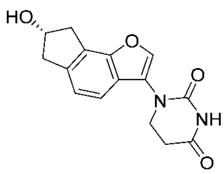, 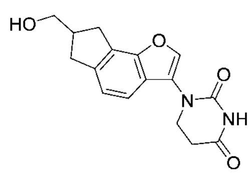,

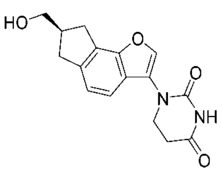, 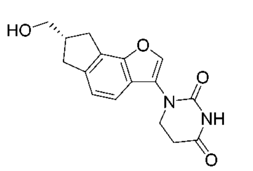, 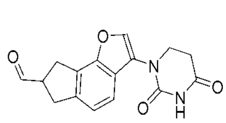, 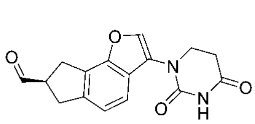,

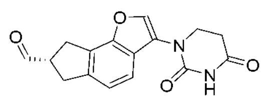 , 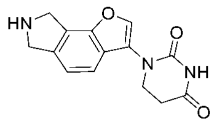 , 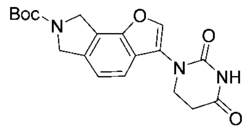 , 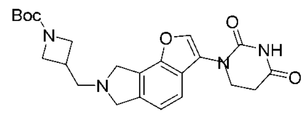 ,

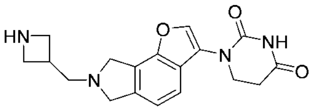 , 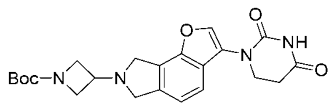 , 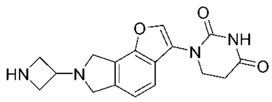 , 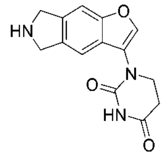 ,

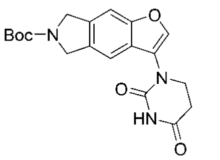 , 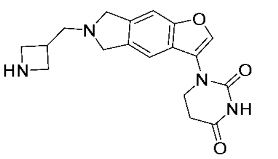 , 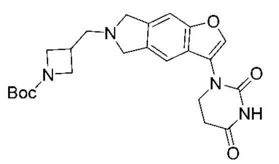 , 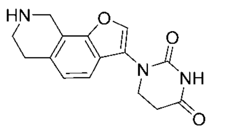 ,

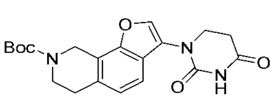 , 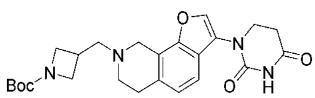 , 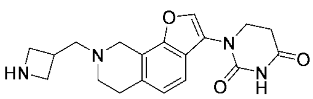 , 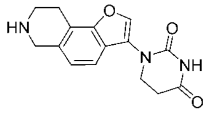 ,

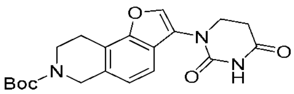 , 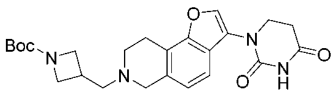 , 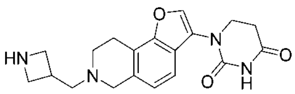 , 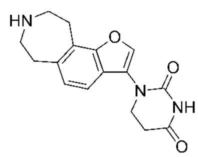 ,

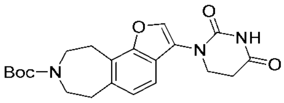 , 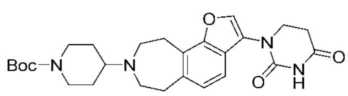 , 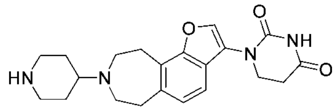 ,

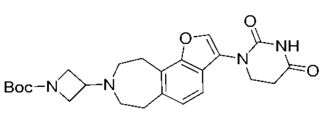 , 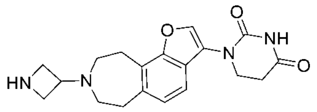 , 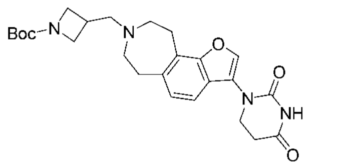 ,

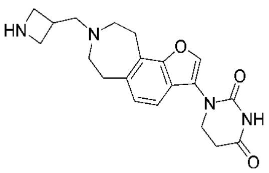 , 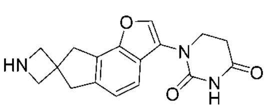 , 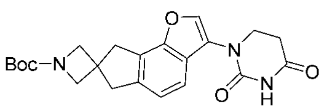 , 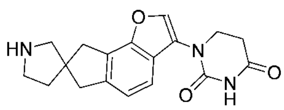 ,

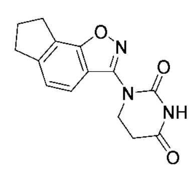 , 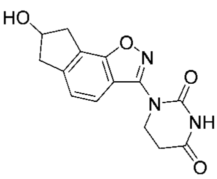 , 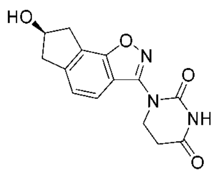 , 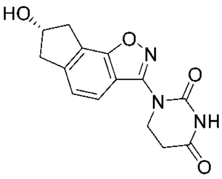 , 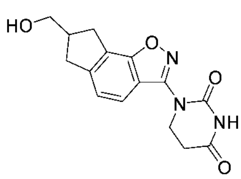 ,

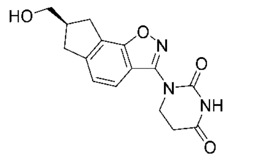, 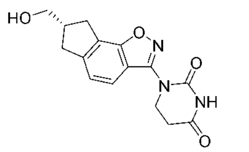, 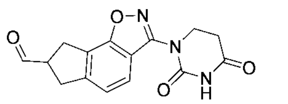, 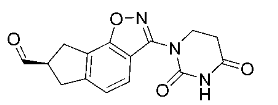,

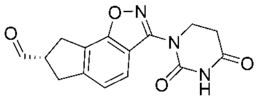, 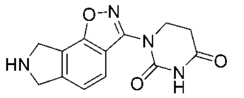, 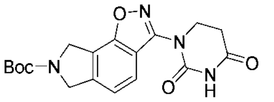, 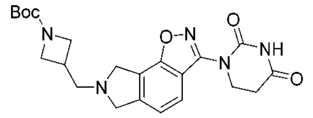,

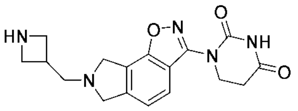, 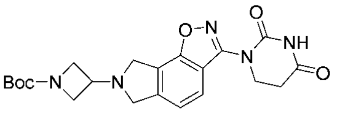, 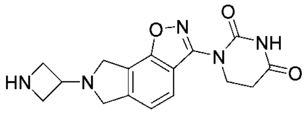, 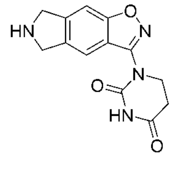,

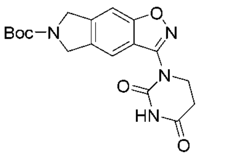, 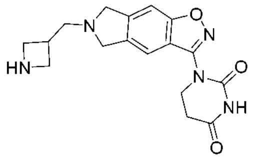, 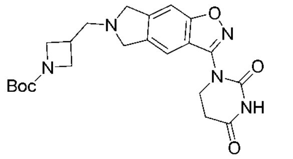, 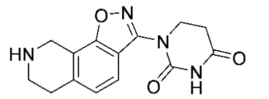,

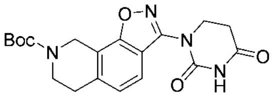, 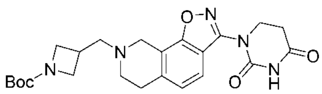, 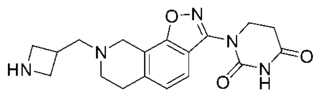, 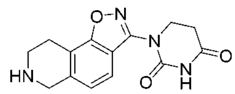,

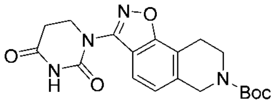, 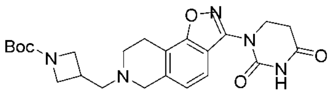, 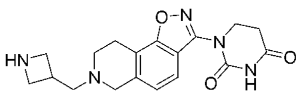, 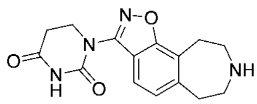,

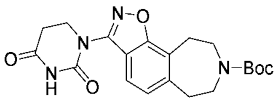, 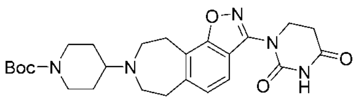, 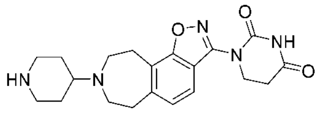,

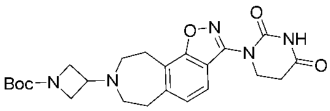, 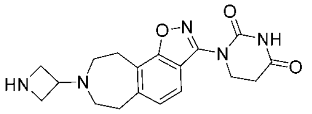, 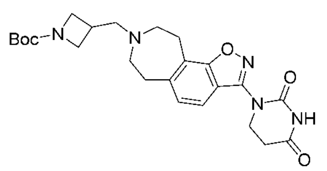,

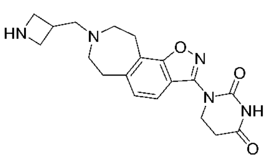, 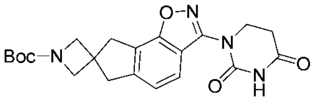, 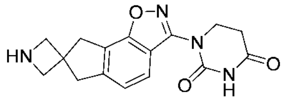, or 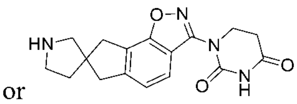.

In some embodiments, the present application relates to a compound of formula I, a stereoisomer thereof, or a pharmaceutically acceptable salt thereof, wherein ring A is $C_{5-10}$ cycloalkenyl or 5- to 15-membered heterocycloalkenyl containing 1-2 heteroatoms selected from the group consisting of N and O (e.g., $C_{5-8}$ cycloalkenyl or 5- to 8-membered heterocycloalkenyl containing 1-2 heteroatoms selected from the group consisting of N and O); ring B is phenyl; ring C is 5- to 6-membered heteroaryl containing 1-2 heteroatoms selected from the group consisting of N and O; n is 0; $X^5$ is selected

from $C(R^f)$, and $R^f$ is selected from the group consisting of H, deuterium, and $C_{1-6}$ alkyl;

L is $C_{1-10}$ alkylene, wherein 1 or more $-CH_2-$ in the $C_{1-10}$ alkylene are optionally replaced by $C_{5-8}$ cycloalkyl or 5- to 8-membered heterocycloalkyl containing 1-3 or 1-2 N atoms or O atoms;
each $R^2$ is independently -OH, $-NH_2$, -CN, $C_{1-10}$ alkyl, $C_{1-10}$ alkoxy, or halogenated $C_{1-10}$ alkyl; each $R^3$ is independently halogen, $C_{1-10}$ alkyl, or halogenated $C_{1-10}$ alkyl; q is 0, m is 0, 1, 2, or 3, and p is 0, 1, 2, 3, or 4; $X^6$ is -O- or -NH-; ring G is phenyl or 6-membered heteroaryl containing 1-2 N atoms or O atoms; ring E is $C_{3-6}$ cycloalkyl; ring F is phenyl or 6-membered heteroaryl containing 1-3 or 1-2 N atoms or O atoms; $R^t$ is hydrogen, -OH, or $C_{1-6}$ alkyl.

In some embodiments, the present application relates to the following compound, a stereoisomer thereof, or a pharmaceutically acceptable salt thereof:

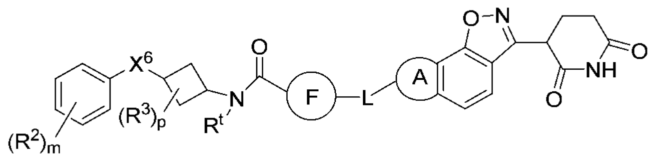

wherein each $R^2$ is independently $-NH_2$, -CN, $C_{1-6}$ alkyl, $C_{1-6}$ alkoxy, or halogenated $C_{1-6}$ alkyl (e.g., -CN, $C_{1-6}$ alkoxy, $C_{1-5}$ alkoxy, $C_{1-4}$ alkoxy, or $C_{1-3}$ alkoxy);
each $R^3$ is independently $C_{1-6}$ alkyl or halogenated $C_{1-6}$ alkyl (e.g., $C_{1-6}$ alkyl, $C_{1-5}$ alkyl, $C_{1-4}$ alkyl, or $C_{1-3}$ alkyl); m is 1, 2, or 3 (preferably 1 or 2), and p is 0, 1, 2, 3, or 4 (preferably 2, 3, or 4);
$X^6$ is -O- or -NH- (preferably -O-);
$R^t$ is hydrogen, -OH, or $C_{1-6}$ alkyl (preferably hydrogen);
ring F is $C_{6-10}$ aryl or 6- to 10-membered heteroaryl (preferably phenyl or 6-membered heteroaryl containing 1-4, 1-3, or 1-2 N atoms);
L is $C_{1-6}$ alkylene, wherein 1 or more $-CH_2-$ in the $C_{1-6}$ alkylene are optionally replaced by 5- to 7-membered heterocycloalkyl containing 1-3 or 1-2 N atoms

(e.g., 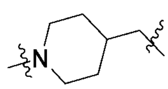, 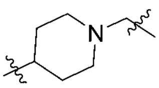, or 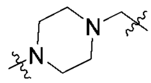,

, preferably

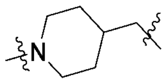 or 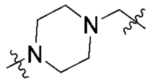);

ring A is $C_{5-9}$ cycloalkenyl or 5- to 9-membered heterocycloalkenyl containing 1-2 N atoms (preferably $C_{5-7}$ cycloalkenyl or 5- to 7-membered heterocycloalkenyl containing 1 N atom).

In some embodiments, the present application relates to the following compound, a stereoisomer thereof, or a pharmaceutically acceptable salt thereof:

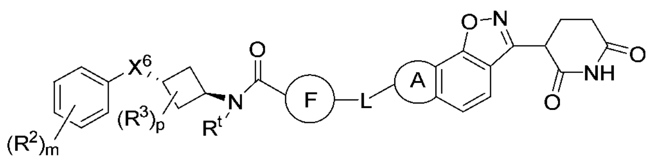

;

preferably

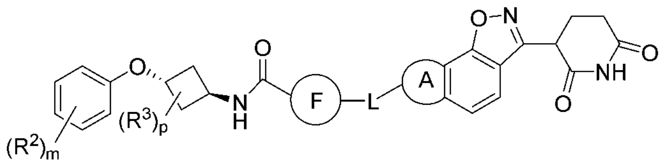

,

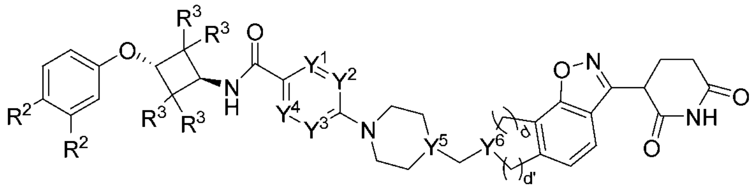

,

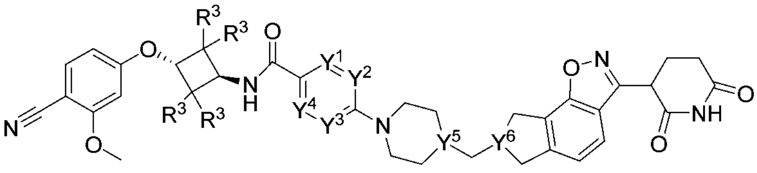

,

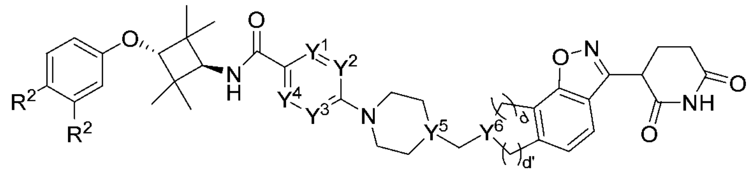

,

or

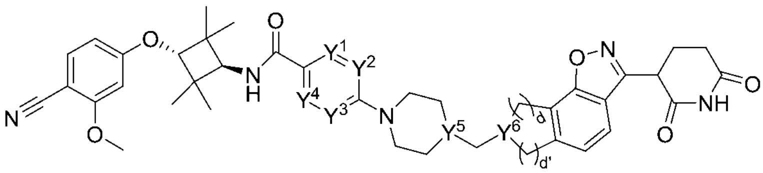

,

wherein $R^2$, $R^3$, m, p, $X^6$, $R^t$, ring F, L, and ring A are as defined above, and each $R^2$ may be identical or different, and each $R^3$ may be identical or different; $Y^1$, $Y^2$, $Y^3$, $Y^4$, $Y^5$, and $Y^6$ are each independently $C(R^5)$ or N, and $R^5$ is H, deuterium, tritium, $C_{1-3}$ alkyl, or =O (preferably H, deuterium, or tritium); d and d' may be identical or different and are each independently 1, 2, or 3. In some preferred embodiments, at most 3 of $Y^1$, $Y^2$, $Y^3$, and $Y^4$ are N, and more preferably, at most 2 of $Y^1$, $Y^2$, $Y^3$, and $Y^4$ are N.

In some embodiments, $Y^1$ is CH or N. In some embodiments, $Y^1$ is CH. In some embodiments, $Y^1$ is N.

In some embodiments, $Y^2$ is CH or N. In some embodiments, $Y^2$ is CH. In some embodiments, $Y^2$ is N.

In some embodiments, $Y^3$ is CH or N. In some embodiments, $Y^3$ is CH. In some embodiments, $Y^3$ is N.

In some embodiments, $Y^4$ is CH or N. In some embodiments, $Y^4$ is CH. In some embodiments, $Y^4$ is N.

In some embodiments, $Y^5$ is CH or N. In some embodiments, $Y^5$ is CH. In some embodiments, $Y^5$ is N.

In some embodiments, $Y^6$ is CH or N. In some embodiments, $Y^6$ is CH. In some embodiments, $Y^6$ is N.

In another aspect, the present application relates to use of the compound (e.g., formula I' or formula I", formula I'-a or formula I"-a, or a specific compound), the moiety, the isomer (e.g., stereoisomer) thereof, or the derivative thereof for preparing a Protac molecule. In another aspect, the present application relates to use of the compound (e.g., formula I' or formula I", formula I'-a or formula I"-a, or a specific compound), the moiety, the isomer (e.g., stereoisomer) thereof, or the derivative thereof for constituting part of a Protac molecule. In another aspect, the present application relates to the compound (e.g., formula I' or formula I", formula I'-a or formula I"-a, or a specific compound), the moiety, the isomer (e.g., stereoisomer) thereof, or the derivative thereof present in the form of a Protac molecule. In another aspect, the present application relates to use of the compound (e.g., formula I' or formula I", formula I'-a or formula I"-a, or a specific compound), the moiety, the isomer (e.g., stereoisomer) thereof, or the derivative thereof for degrading a protein (e.g., AR),

and for example, the compound (e.g., formula I' or formula I", formula I'-a or formula I"-a, or a specific compound), the moiety, the isomer (e.g., stereoisomer) thereof, or the derivative thereof degrades the protein in the form of a Protac molecule. In another aspect, the present application relates to use of the compound (e.g., formula I' or formula I", formula I'-a or formula I"-a, or a specific compound), the moiety, the isomer (e.g., stereoisomer) thereof, or the derivative thereof for degrading a protein in the form of a Protac molecule. The present application relates to use of the compound (e.g., formula I' or formula I", formula I'-a or formula I"-a, or a specific compound), the moiety, the isomer (e.g., stereoisomer) thereof, or the derivative thereof (e.g., as a preparation intermediate) for preparing a Protac molecule. The present application relates to use of the compound (e.g., formula I' or formula I", formula I'-a or formula I"-a, or a specific compound), the moiety, the isomer (e.g., stereoisomer) thereof, or the derivative thereof (e.g., as a preparation intermediate) for preparing a protein degrader.

The present application relates to the following compound, a stereoisomer thereof, or a pharmaceutically acceptable salt thereof:

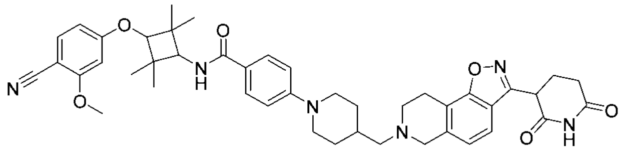
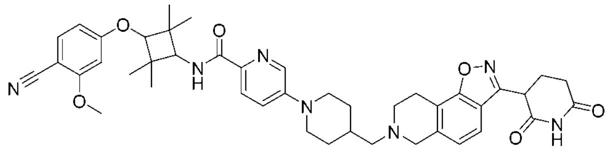

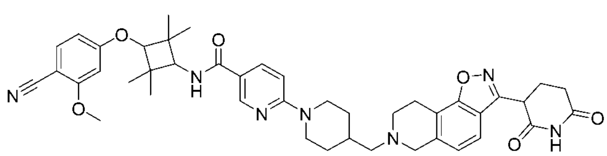
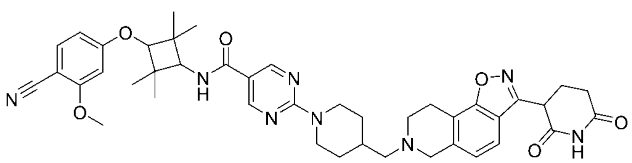

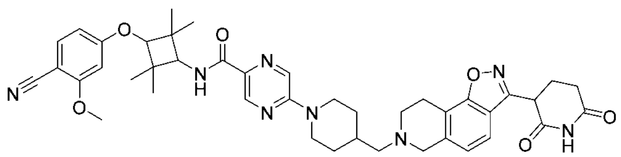
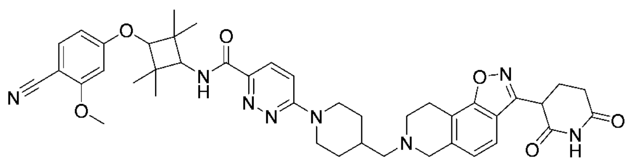

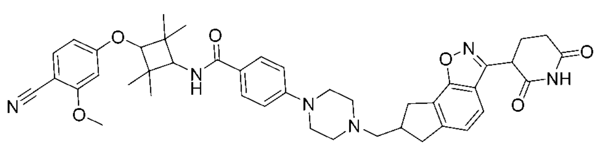
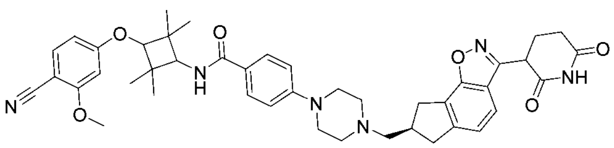

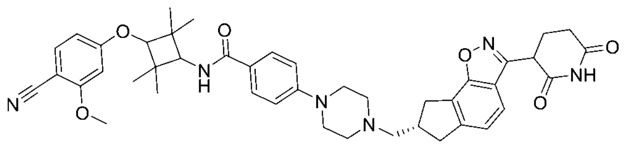
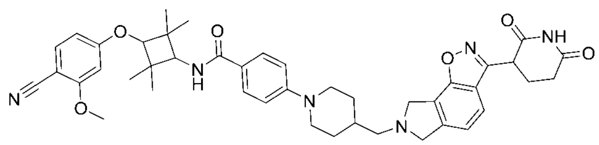

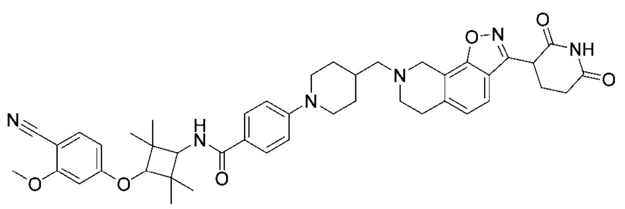
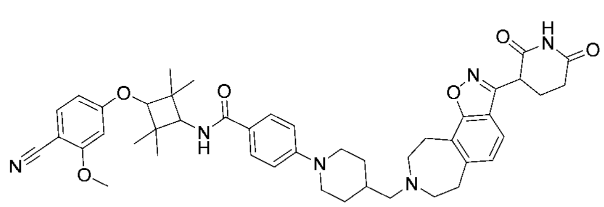

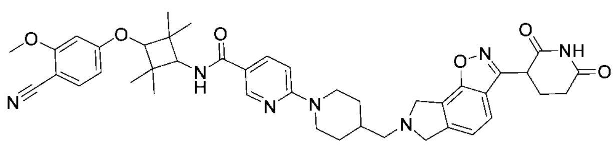
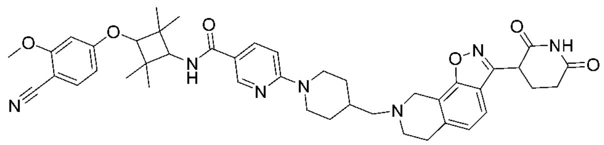

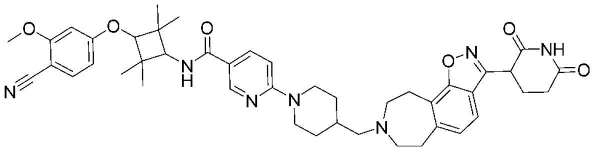

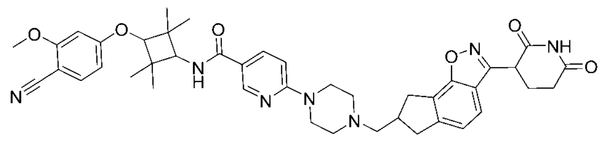

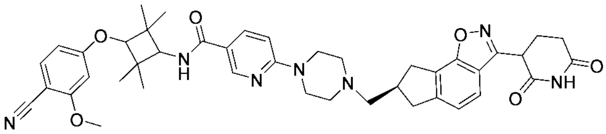

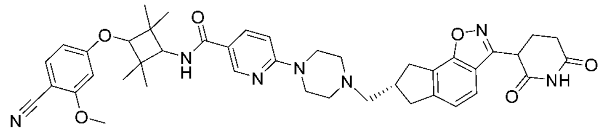

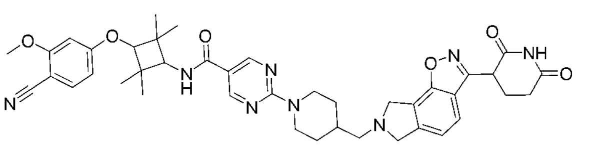

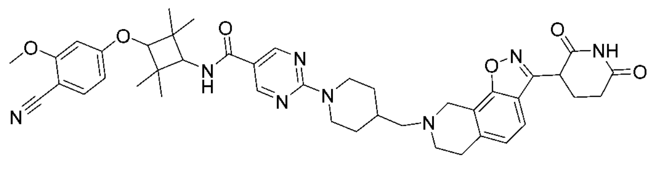

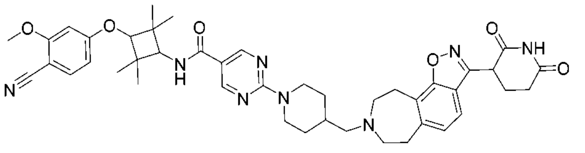

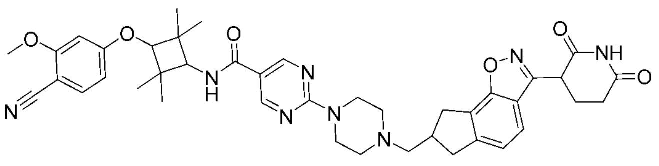

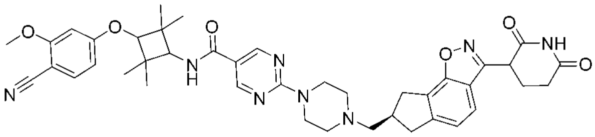

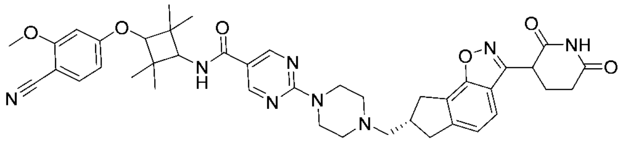

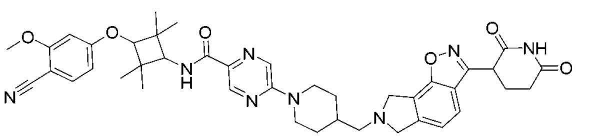

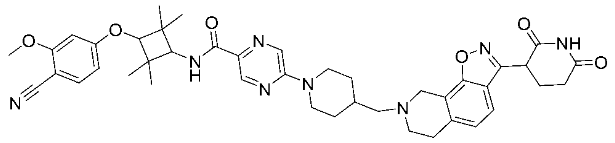

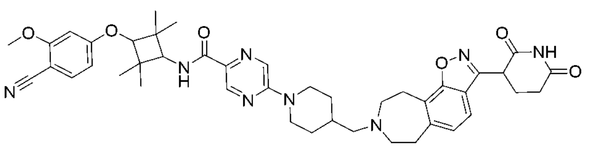

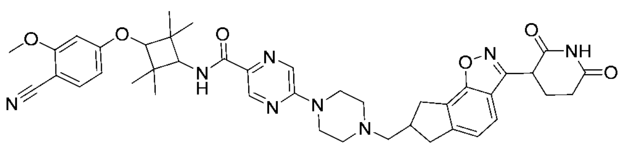

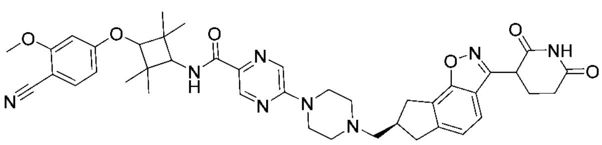

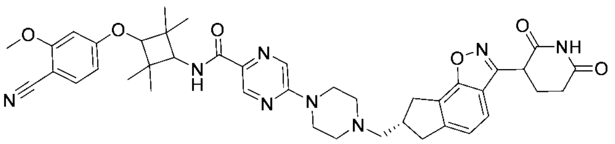

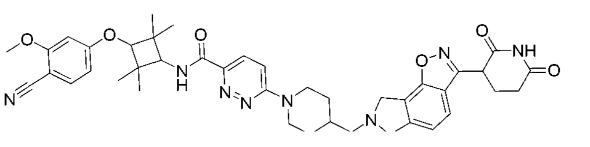

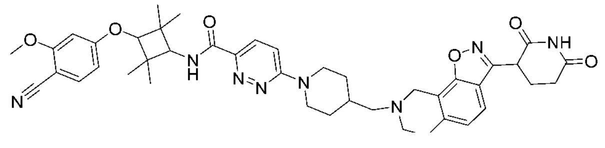

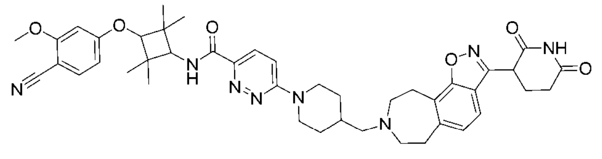

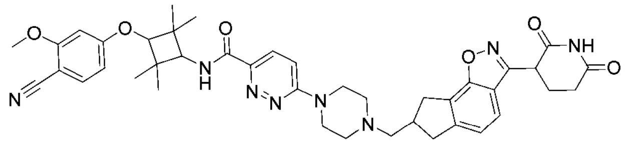

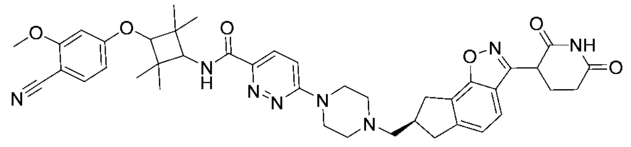

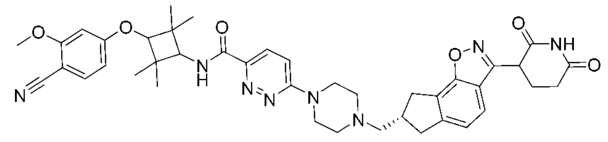

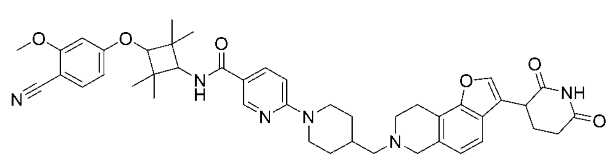

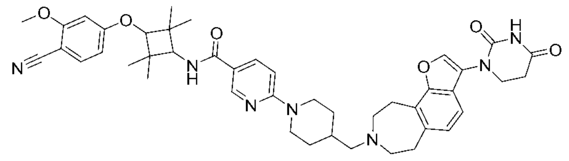

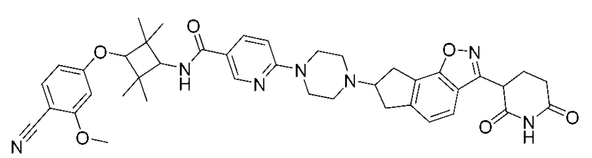

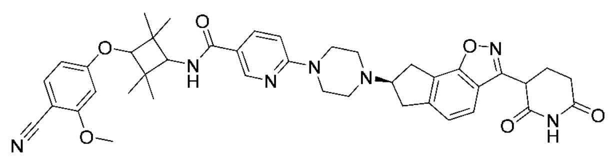

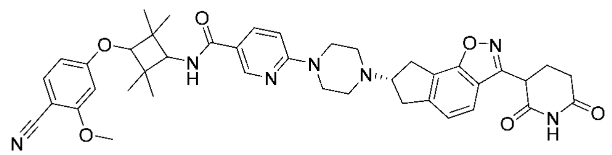

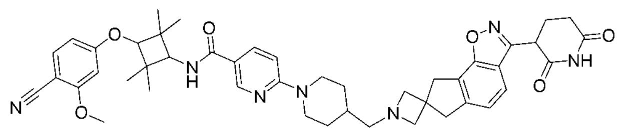

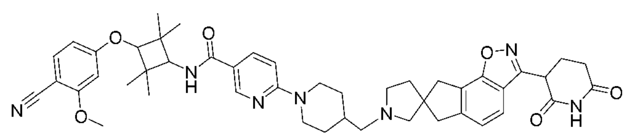

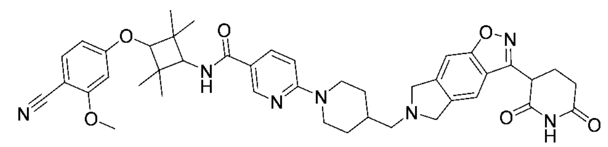

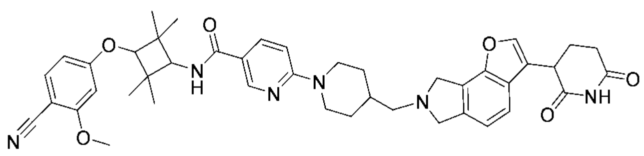

or

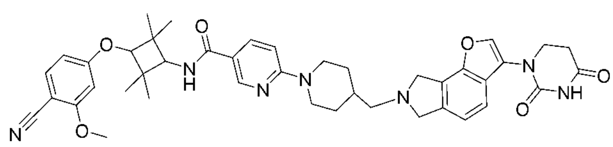

.

The present application also relates to the following compound, a stereoisomer thereof, or a pharmaceutically acceptable salt thereof:

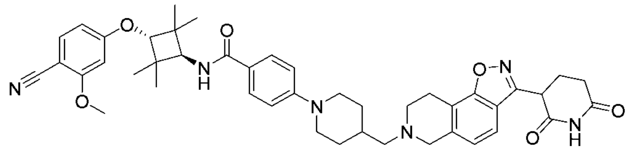

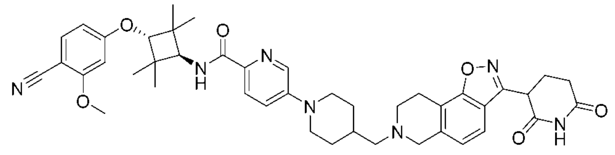

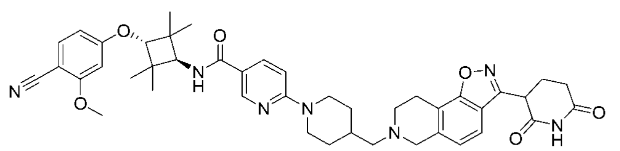

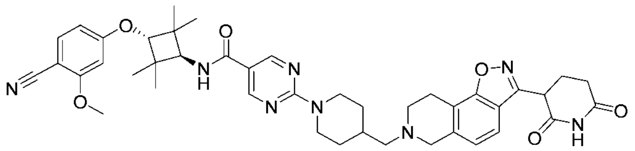

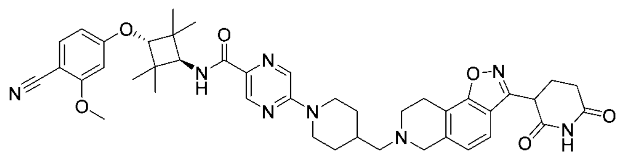

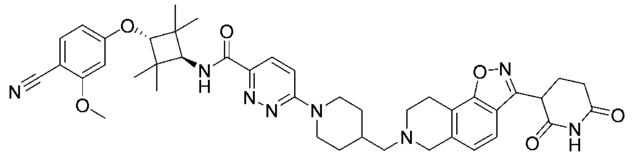

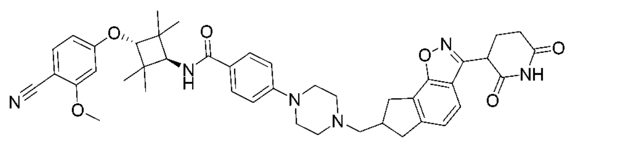

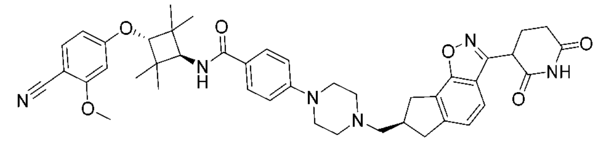

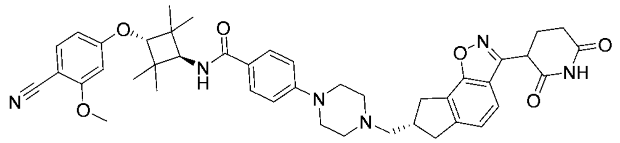

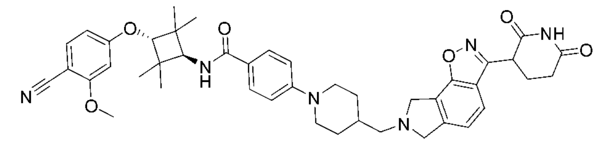

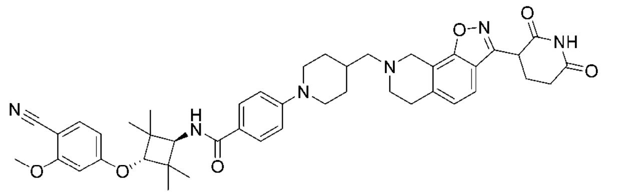

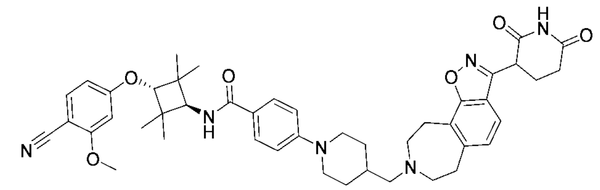

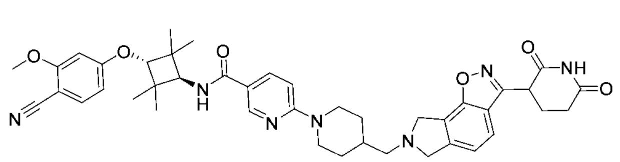

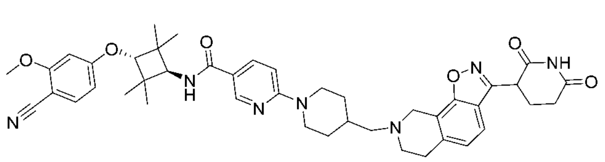

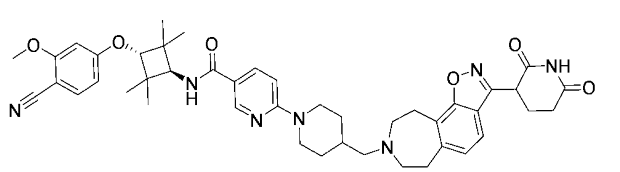

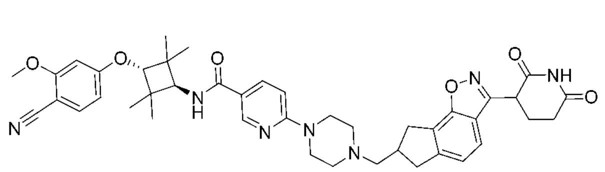

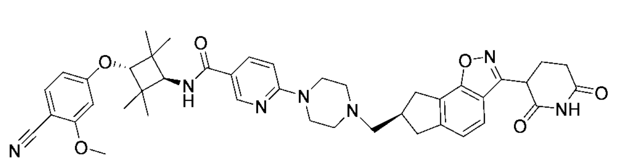

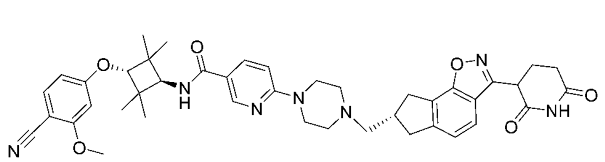

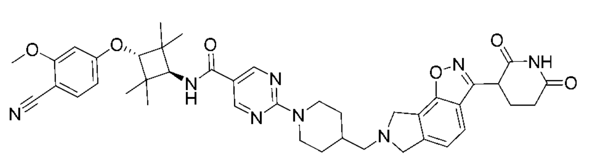
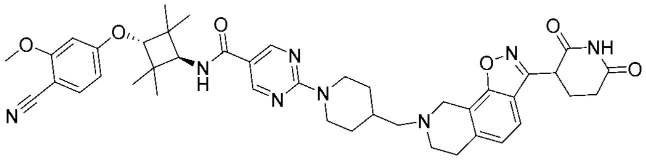

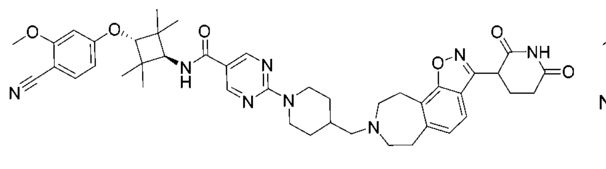
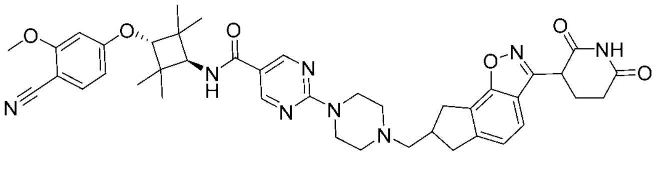

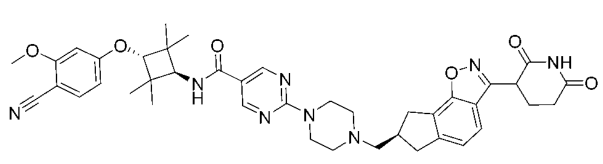
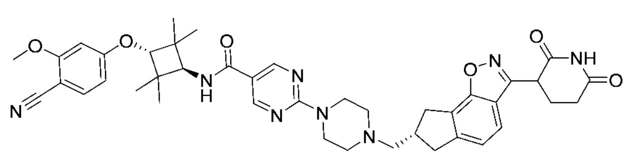

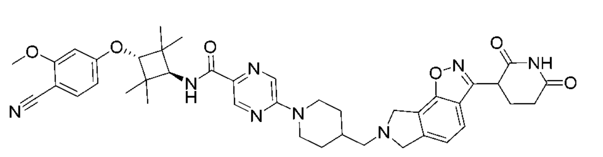
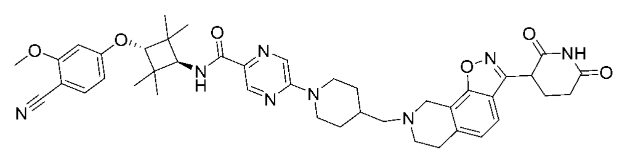

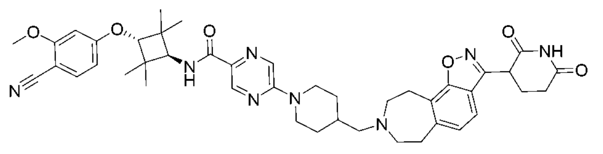
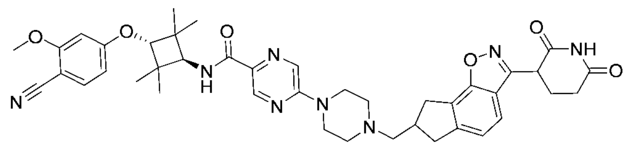

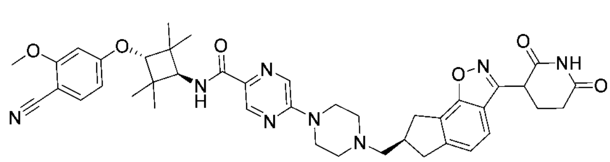
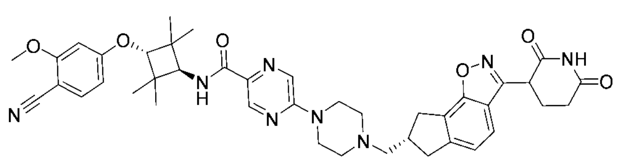

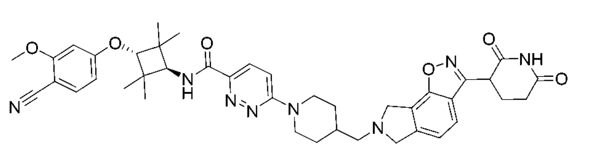
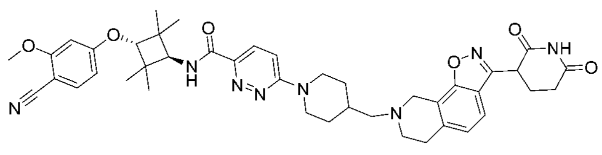

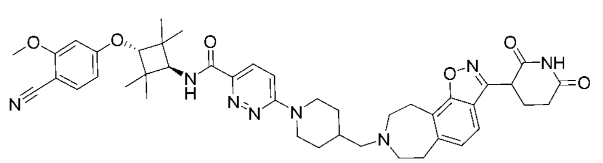
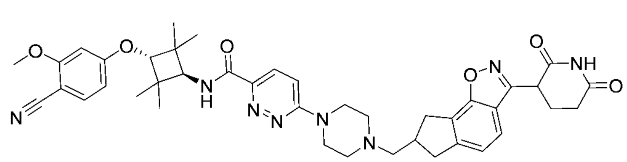

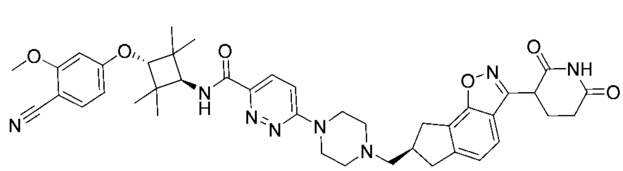
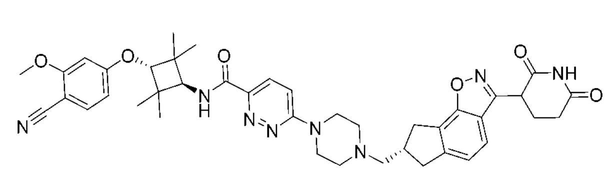

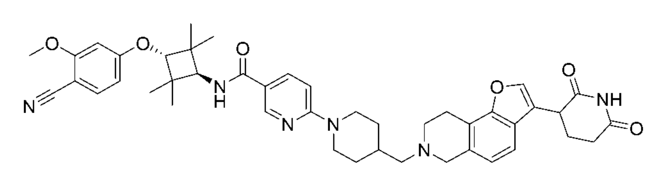

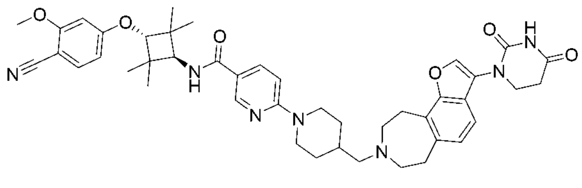

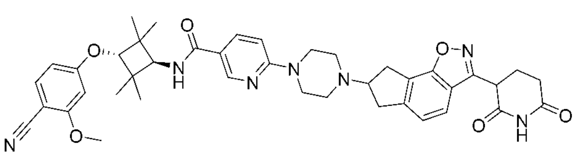

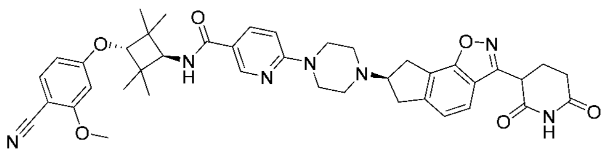

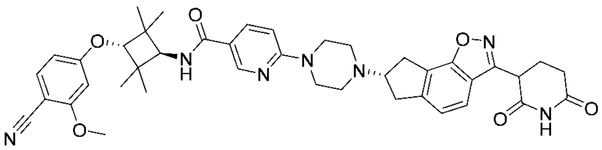

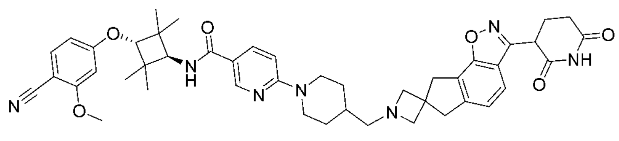

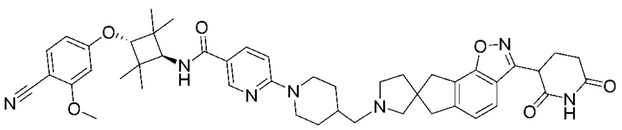

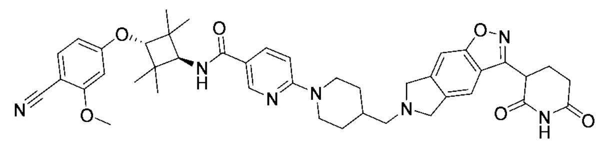

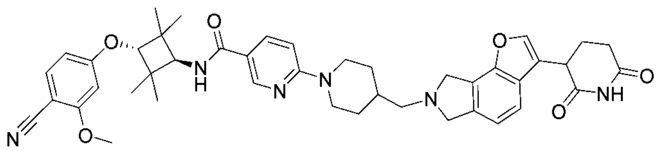

or

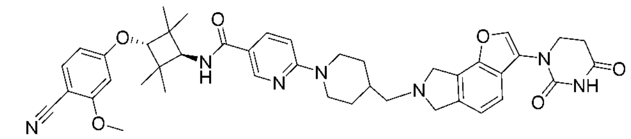

.

The present application also covers solutions obtained by any combination, deletion, or exchange of the embodiments described above.

In another aspect, the present application relates to a pharmaceutical composition, and the pharmaceutical composition comprises the compound, the stereoisomer thereof, or the pharmaceutically acceptable salt thereof of the present application described above; optionally, the pharmaceutical composition of the present application also comprises a pharmaceutically acceptable excipient.

In another aspect, the present application relates to use of the compound, the stereoisomer thereof, or the pharmaceutically acceptable salt thereof, or the pharmaceutical composition thereof described above for preparing a medicament for preventing or treating a disorder treated by degrading a target protein (e.g., androgen receptor, AR) that binds to a targeting ligand.

In another aspect, the present application relates to use of the compound, the stereoisomer thereof, or the pharmaceutically acceptable salt thereof, or the pharmaceutical composition thereof described above for preparing a medicament for preventing or treating a disorder treated by binding to cereblon protein (CRBN) *in vivo*.

In another aspect, the present application relates to use of the compound, the stereoisomer thereof, or the pharmaceutically acceptable salt thereof, or the pharmaceutical composition thereof described above for preparing a medicament for preventing or treating an AR-related disease.

The present application relates to a method for treating or preventing a disorder treated by degrading a target protein (e.g., androgen receptor, AR) that binds to a targeting ligand in a mammal, which comprises administering to a mammal (preferably a human) in need of such treatment a therapeutically effective amount of the compound, the

stereoisomer thereof, or the pharmaceutically acceptable salt thereof, or the pharmaceutical composition thereof of the present application described above.

The present application relates to a method for treating or preventing a disorder treated by binding to cereblon protein *in vivo,* which comprises administering to a mammal (preferably a human) in need of such treatment a therapeutically effective amount of the compound, the stereoisomer thereof, or the pharmaceutically acceptable salt thereof, or the pharmaceutical composition thereof of the present application described above.

In another aspect, the present application relates to a method for treating an AR-related disease in a mammal, which comprises administering to a mammal (preferably a human) in need of such treatment a therapeutically effective amount of the compound, the stereoisomer thereof, or the pharmaceutically acceptable salt thereof, or the pharmaceutical composition thereof of the present application described above.

In another aspect, the present application relates to the compound, the stereoisomer thereof, or the pharmaceutically acceptable salt thereof, or the pharmaceutical composition thereof described above for use in preventing or treating a disorder treated by degrading a target protein (e.g., androgen receptor, AR) that binds to a targeting ligand.

In another aspect, the present application relates to the compound, the stereoisomer thereof, or the pharmaceutically acceptable salt thereof, or the pharmaceutical composition thereof described above for use in preventing or treating a disorder treated by binding to cereblon protein *in vivo.*

In another aspect, the present application relates to the compound, the stereoisomer thereof, or the pharmaceutically acceptable salt thereof, or the pharmaceutical composition thereof described above for use in preventing or treating an AR-related disease.

In another aspect, the present application relates to use of the compound, the stereoisomer thereof, or the pharmaceutically acceptable salt thereof, or the pharmaceutical composition thereof described above for preventing or treating a disorder treated by degrading a target protein (e.g., androgen receptor, AR) that binds to a targeting ligand.

In another aspect, the present application relates to use of the compound, the stereoisomer thereof, or the pharmaceutically acceptable salt thereof, or the pharmaceutical composition thereof described above for preventing or treating a disorder treated by binding to cereblon protein *in vivo.*

In another aspect, the present application relates to use of the compound, the stereoisomer thereof, or the pharmaceutically acceptable salt thereof, or the pharmaceutical composition thereof described above for preventing or treating an AR-related disease.

In some specific embodiments, the AR-related disease described above is selected from the group consisting of disorders treated by degrading and/or inhibiting a protein (androgen receptor, AR) that binds to an AR target protein ligand; in some specific embodiments, the AR-related disease described above is selected from the group consisting of disorders treated by binding to cereblon protein *in vivo*; in some embodiments, the disease or disorder described above is selected from the group consisting of cancers, such as prostate cancer.

In some specific embodiments, the disorder treated by binding to cereblon protein *in vivo* and/or by degrading a target protein that binds to a targeting ligand described above is selected from the group consisting of AR-related diseases; in some specific embodiments, the AR-related disease described above is selected from the group consisting of cancers, such as prostate cancer.

"One or more" used herein refers to an integer ranging from one to ten. For example, "one or more" refers to one, two, three, four, five, six, seven, eight, nine, or ten; in some embodiments, the "one or more" is selected from the group consisting of one, two, three, four, five, and six. In some embodiments, the "one or more" is selected from the group consisting of one, two, and three. In some embodiments, the "one or more" is selected from the group consisting of one and two.

In some embodiments, the present application encompasses the variables defined above and embodiments thereof, as well as any combination thereof.

## Technical Effects

The compounds of the present application have protein degradation activity for AR or mutants thereof and have anti-proliferative activity for related cells, and for example, the compounds have degradation activity for AR in VCaP cells, and/or AR in MDA-PCA-2B cells (including L702H and/or T878A mutations thereof), AR in 22RV1 cells (including H875Y and/or ARV7 mutations thereof), AR in HEK293 cells (including L702H mutation thereof), and/or AR in LNCaP cells (including T878A mutation thereof); additionally, the compounds have anti-proliferative activity for VCaP cells, and/or LNCaP cells (including T878A mutation thereof), and/or 22RV1 cells (including H875Y and/or ARV7 mutations thereof), and/or MDA-PCA-2B cells (including L702H and/or T878A mutations thereof), and/or HEK293 cells (including L702H mutation thereof). In addition, the compounds of the present application also have good *in vitro* liver microsome stability and good *in vivo* pharmacokinetic properties (specifically, such as AUC, $t_{1/2}$, and other parameters) in mammals (e.g., mice), can inhibit the growth of tumors *in vivo,* and show good druggability.

## Definitions

Unless otherwise stated, the following terms used in the present application shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

Unless otherwise specified,

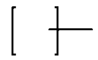

is used to indicate that a hydrogen atom at any position of a group within [ ] may be substituted with a group linked by "-".

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted; oxo is not possible on an aromatic group.

The term "optional" or "optionally" means that the subsequently described event or circumstance may, but does not necessarily, occur. The description includes instances where the event or circumstance occurs and instances where it does not. "Optionally substituted" includes unsubstituted and substituted. For example, ethyl being "optionally" substituted with halogen means that the ethyl may be unsubstituted ($CH_2CH_3$), monosubstituted (for example, $CH_2CH_2F$), polysubstituted (for example, $CHFCH_2F$ and $CH_2CHF_2$), or fully substituted ($CF_2CF_3$). It will be appreciated by those skilled in the art that for any groups comprising one or more substituents, any substitutions or substituting patterns that may not exist and/or cannot be synthesized spatially are not introduced.

$C_{m-n}$ used herein means that the moiety has an integer number of carbon atoms in the given range. For example, "$C_{1-6}$" means that the group may have 1 carbon atom, 2 carbon atoms, 3 carbon atoms, 4 carbon atoms, 5 carbon atoms, or 6 carbon atoms.

When any variable (e.g., R) occurs more than once in the constitution or structure of a compound, the definition of the variable in each case is independent. For example, if a group contains 2 R, the definition of each R is independent. When a bond is crosslinked to two atoms of a ring (including monocyclic, fused, or spiro ring), the bond may be bonded to any atom on the ring (including monocyclic, fused, or spiro ring). For example, the structural unit

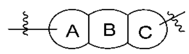

indicates that the bonds on two sides may be linked to any two different atoms on ring A, ring B, or ring C; for another example,

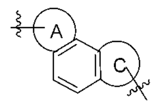

indicates that the bonds on two sides may be linked to any two different atoms on ring A, the middle benzene ring, or ring C; for further example,

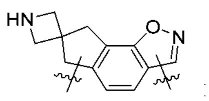

indicates that the bonds on two sides may be linked to any two different atoms of the four rings in the system.

The term "halo-" or "halogen" refers to fluorine, chlorine, bromine, and iodine. The term "hydroxyl" refers to an -OH group. The term "amino" refers to an $-NH_2$ group. The term "cyano" refers to a -CN group.

The term "alkyl" refers to hydrocarbyl with a general formula of $C_nH_{2n+1}$. The alkyl may be linear or branched. For example, the term "$C_{1-6}$ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described above.

The term "alkylene" refers to a divalent group formed by the removal of one hydrogen at any position of alkyl. For example, the term "$C_{1-6}$ alkylene" refers to alkylene containing 1 to 6 carbon atoms; the term "$C_{1-4}$ alkylene" refers to alkylene containing 1 to 4 carbon atoms, including but not limited to $-CH_2-$, $-CH_2CH_2-$, $-CH_2CH_2CH_2-$, or -

$CH_2CH_2CH_2CH_2$-.

The term "alkenylene" refers to a divalent group formed by the removal of one hydrogen at any position of alkenyl. For example, the term "$C_{2-6}$ alkenylene" refers to alkenylene containing 2 to 6 carbon atoms; the term "$C_{2-4}$ alkenylene" refers to alkenylene containing 2 to 4 carbon atoms, including but not limited to $-CH_2CH=CH-$, - $CH_2CH_2CH=CH-$, or $-CH_2CH=CHCH_2-$.

The term "alkynylene" refers to a divalent group formed by the removal of one hydrogen at any position of alkynyl. For example, the term "$C_{2-6}$ alkynylene" refers to alkynylene containing 2 to 6 carbon atoms; the term "$C_{2-4}$ alkynylene" refers to alkynylene containing 2 to 4 carbon atoms, including but not limited to $-C{\equiv}C-$, $-H_2C-C{\equiv}C-$, $-H_2C-H_2C-C{\equiv}C-$, or $-H_2C-C{\equiv}C-CH_2-$.

The term "heteroalkyl" is linear or branched alkyl consisting of a certain number of carbon atoms and at least one heteroatom; it has preferably 1 to 14 carbons, more preferably 1 to 10 carbons, even more preferably 1 to 6 carbons, and most preferably 1 to 3 carbons in the chain, and preferably has 1, 2, or 3 heteroatoms selected from the group consisting of S, O, and N. For example, $C_m$ heteroalkyl represents alkyl consisting of m carbon atoms and at least one heteroatom (e.g., 1-3 heteroatoms selected from the group consisting of S, O, and N) located between any two carbon atoms and having a heteroatom inserted in the chain. The nitrogen and sulfur atoms are optionally oxidized, and the nitrogen atom is optionally quaternized. The heteroatom or heteroatom group can be located at any interior position of the heterohydrocarbyl, including the position at which the hydrocarbyl is attached to the rest of the molecule. Exemplary heteroalkyl includes alkyl ether, secondary alkylamine and tertiary alkylamine, amide, alkyl sulfide, and the like, including alkoxy, alkylthio, and alkylamino; unless otherwise specified, $C_{1-6}$ heteroalkyl includes $C_1$, $C_2$, $C_3$, $C_4$, $C_5$, and $C_6$ heteroalkyl, e.g., $C_{1-6}$ alkoxy, $C_{1-6}$ alkylthio, or $C_{1-6}$ alkylamino.

The term "heteroalkylene" refers to a divalent group formed by the removal of one hydrogen at any position of heteroalkyl.

The term "alkoxy" refers to -O-alkyl.

The term "alkenyl" refers to linear or branched unsaturated aliphatic hydrocarbyl consisting of carbon atoms and hydrogen atoms and having at least one double bond. Non-limiting examples of the alkenyl include, but are not limited to, ethenyl, 1-propenyl, 2-propenyl, 1-butenyl, isobutenyl, 1,3-butadienyl, and the like.

The term "cycloalkenyl" refers to a non-aromatic carbocyclic ring that is not fully saturated and may exist as a monocyclic ring, a bicyclic bridged ring, or a spiro ring. Unless otherwise specified, the carbocyclic ring is usually a 4- to 16-membered ring, a 4- to 12-membered ring, a 4- to 10-membered ring, or a 4- to 8-membered ring. Non-limiting examples of the cycloalkenyl include, but are not limited to, cyclopentenyl, cyclopentadienyl, cyclohexenyl, cyclohexadienyl, cycloheptenyl, cycloheptadienyl, and the like.

The term "cycloalkyl" refers to a carbocyclic ring that is fully saturated and may exist as a monocyclic ring, a bridged ring, or a spiro ring. Unless otherwise specified, the carbocyclic ring is usually a 3- to 16-membered ring (e.g., a 3-to 10-membered ring or a 5- to 8-membered ring). Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocycloalkyl" refers to a cyclic group that is fully saturated and may exist as a monocyclic ring, a bridged ring, or a spiro ring. Unless otherwise specified, the heterocyclyl is usually a 3- to 16-membered, 3- to 11-membered, 3- to 10-membered, 3- to 7-membered, 3- to 6-membered, or 3- to 5-membered ring containing 1 to 3 heteroatoms (preferably 1 or 2 heteroatoms) independently selected from the group consisting of sulfur, oxygen, and nitrogen. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiapyranyl, morpholinyl, piperazinyl, 1,4-oxathianyl, 1,4-dioxanyl, sulfomorpholinyl, 1,3-dithianyl, and 1,4-dithianyl. Examples of 7-membered heterocycloalkyl include, but are not limited to, azepanyl, oxepanyl, and thiepanyl. Monocyclic heterocycloalkyl having 5 or 6 ring atoms is preferred.

The term "spiro ring" refers to a fully saturated or partially unsaturated polycyclic ring system in which monocyclic rings share one carbon atom (referred to as a spiro atom), including carbocyclic rings and heterocyclic rings. Unless otherwise specified, the spiro ring is 5- to 20-membered, preferably 6- to 14-membered, and more preferably 8- to 12-membered. When the spiro ring is a heterocyclic ring, one or more ring atoms in the polycyclic ring are heteroatoms (preferably 1 or 2 heteroatoms) selected from the group consisting of N, O, $S(O)_n$, and $P(O)_n$ (wherein n is 0, 1, or 2), and the remaining ring atoms are carbon atoms.

The term "spirocycloalkyl" refers to a fully saturated all-carbon polycyclic ring in which monocyclic rings share one carbon atom (referred to as a spiro atom). Unless otherwise specified, the spirocycloalkyl is 5- to 20-membered, preferably 6- to 14-membered, and more preferably 8- to 12-membered. According to the number of spiro atoms shared among the rings, the spirocycloalkyl is monospirocycloalkyl, bispirocycloalkyl, or polyspirocycloalkyl, preferably monospirocycloalkyl

or bispirocycloalkyl, and more preferably 4-membered/4-membered, 4-membered/5-membered, 4-membered/6-membered, 5-membered/5-membered, or 5-membered/6-membered monospirocycloalkyl. Non-limiting examples of the spirocycloalkyl include

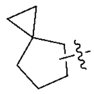, 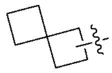, 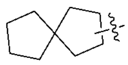,

and

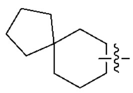.

The term "spiro-heterocycloalkyl" refers to a fully saturated polycyclic ring in which monocyclic rings share one carbon atom (referred to as a spiro atom), wherein one or more ring atoms in the polycyclic ring are heteroatoms (preferably 1 or 2 heteroatoms) selected from the group consisting of N, O, $S(O)_n$, and $P(O)_n$ (wherein n is 0, 1, or 2), and the remaining ring atoms are carbon atoms. Unless otherwise specified, the spiro-heterocycloalkyl is 5- to 20-membered, preferably 6- to 14-membered, and more preferably 6- to 10-membered. According to the number of spiro atoms shared among the rings, the spiro heterocyclic ring is a monospiro heterocyclic ring, a bispiro heterocyclic ring, or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered, or 5-membered/6-membered monospiro heterocyclic ring. Non-limiting examples of the spiro-heterocycloalkyl include

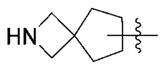, 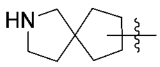, 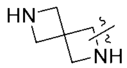, 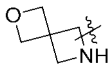, 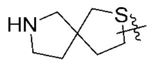, 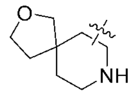,

or the like.

The term "heterocycloalkenyl" includes cycloalkenyl in which one or more (e.g., 1-5, 1-4, 1-3, or 1-2) carbon atoms are substituted with a heteroatom, specifically, such as cycloalkenyl in which at most 3 carbon atoms, in one embodiment at most 2 carbon atoms, and in another embodiment 1 carbon atom, are each independently replaced by O, S, S(O), or N, provided that at least one cycloalkenyl carbon-carbon double bond is preserved. The heterocycloalkenyl may be a cyclic group that exists as a monocyclic ring, a bridged ring, or a spiro ring and may be a 3- to 16-membered ring (for example, a 3- to 12-membered or 5- to 8-membered ring, specifically, for example, a 5-membered, 6-membered, 7-membered, 8-membered, 9-membered, 10-membered, or 11-membered ring). Examples of the heterocycloalkenyl include, but are not limited to, dihydropyrrolyl, tetrahydropyridinyl, tetrahydroazepinyl, or azaspirooctenyl.

The term "aryl" refers to an all-carbon aromatic monocyclic or fused polycyclic group having a conjugated π-electron system. For example, aryl may have 6-20 carbon atoms, 6-14 carbon atoms, or 6-12 carbon atoms. Non-limiting examples of the aryl include, but are not limited to, phenyl, naphthyl, anthryl, 1,2,3,4-tetrahydronaphthalene, and the like.

The term "heteroaryl" refers to a monocyclic or fused polycyclic system that comprises at least one (e.g., 1-5, 1-4, 1-3, or 1-2) ring atom selected from the group consisting of N, O, and S, with the remaining ring atoms being C, and that has at least one aromatic ring. Preferably, the heteroaryl has a single 4- to 8-membered ring, in particular a 5- to 8-membered ring (e.g., 5-membered, 6-membered, 7-membered, or 8-membered), or has a plurality of fused rings comprising 6 to 14 ring atoms, in particular 6 to 10 (e.g., 6, 7, 8, 9, or 10) ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

Unless otherwise specified, the term "hetero" refers to a heteroatom or a heteroatom group (i.e., a heteroatom-containing group), including atoms other than carbon (C) and hydrogen (H) and groups containing such heteroatoms, including, for example, oxygen (O), nitrogen (N), sulfur (S), phosphorus (P), silicon (Si), germanium (Ge), aluminum (Al), boron (B), -O-, -S-, =O, =S, -P(=O)-, $-P(=O)_2-$, -P(=O)O-, $-P(=O)_2O-$, -C(=O)O-, -C(=O)-, -C(=S)-, -S(=O)-, $-S(=O)_2-$, and optionally substituted -C(=O)N(H)-, -N(H)-, -C(=NH)-, $-S(=O)_2N(H)-$, or -S(=O)N(H)-. The term "derivative" refers to a new compound or a group of new compounds that are produced through one or more chemical reactions or structural evolutions. The derivative retains the basic structure of the parent compound, with changes or modifications occurring in the side chains, functional groups, or substituents only.

The substitution or substitution with a substituent in the term "substituent", "optionally substituted with one or more substituents", or "optionally substituted" includes all substituents mentioned in the text, and may be, e.g., the terms

"halogen", "deuterium", "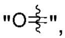", "$-NH_2$", "-NH($C_{1-4}$ alkyl)", "-N($C_{1-4}$ alkyl)$_2$", "-OH", "-$OC_{1-4}$ alkyl", "-CN", "$C_{1-4}$ alkyl", and "3- to 6-membered heterocycloalkyl", and the corresponding non-limiting or exemplary groups mentioned below, wherein some non-limiting examples of the "substituent" include sulfydryl, nitro, nitroso, cyano, azido, a sulfoxide group, a sulfone group, a sulfonamide group, carboxyl, an aldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, arylalkylene, arylalkoxy, arylalkylthio, heteroaryl, heteroaryloxy, heteroarylthio, heteroarylalkylene, heteroarylalkoxy, heteroarylalkylthio, heterocyclyl, heterocyclyloxy, heterocyclylthio, heterocyclylalkylene, heterocyclylalkoxy, heterocyclylalkylthio, acyl, acyloxy, a carbamate group, amido, ureido, an epoxy group, an ester group, oxo, and the like, wherein these substituents are optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxyl, -C(O)O-alkyl, -OC(O)-alkyl, -C(O)$NH_2$, -C(O)NH-alkyl, - C(O) N(alkyl)$_2$, -NHC(O)-alkyl, -C(O)-alkyl, -S(O)-alkyl, -S(O)$_2$-alkyl, -S(O)$_2$$NH_2$, -S(O)$_2$NH-alkyl, - S(O)$_2$N(alkyl)$_2$, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

In some embodiments herein, the substituent is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, $C_{1-12}$ alkyl, halogenated $C_{1-12}$ alkyl, 3- to 12-membered cycloalkyl, halogenated 3- to 12-membered cycloalkyl, $C_{2-12}$ alkenyl, halogenated $C_{2-12}$ alkenyl, 3- to 12-membered cycloalkenyl, halogenated 3- to 12-membered cycloalkenyl, $C_{2-12}$ alkynyl, halogenated $C_{2-12}$ alkynyl, 8- to 12-membered cycloalkynyl, halogenated 8- to 12-membered cycloalkynyl, $C_{1-12}$ heteroalkyl, halogenated $C_{1-12}$ heteroalkyl, $C_{1-12}$ alkoxy, $C_{1-12}$ alkylthio, 6- to 10-membered aryl, 6- to 10-membered aryloxy, 6- to 10-membered arylthio, 6- to 10-membered aryl $C_{1-12}$ alkylene, 6- to 10-membered aryl $C_{1-12}$ alkoxy, 6- to 10-membered aryl $C_{1-12}$ alkylthio, 5- to 10-membered heteroaryl, 5- to 10-membered heteroaryloxy, 5- to 10-membered heteroarylthio, 5- to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl $C_{1-12}$ alkylene, 3-to 12-membered heterocyclyl $C_{1-12}$ alkoxy, 3- to 12-membered heterocyclyl $C_{1-12}$ alkylthio, $C_{1-12}$ acyl, $C_{1-12}$ acyloxy, a carbamate group, $C_{1-12}$ amido, ureido, an epoxy group, a $C_{2-12}$ ester group, and oxo, wherein these substituents are optionally substituted with one or more substituents selected from the group consisting of the following substituents: oxo, hydroxy, amino, nitro, halogen, cyano, $C_{1-12}$ alkyl, $C_{2-12}$ alkenyl, $C_{2-12}$ alkynyl, $C_{1-12}$ alkoxy, halogenated $C_{1-12}$ alkoxy, $C_{1-12}$ alkylamino, di-$C_{1-12}$ alkylamino, halogenated $C_{1-12}$ alkylamino, halogenated di-$C_{1-12}$ alkylamino, carboxyl, -C(O)O-$C_{1-12}$ alkyl, -OC(O)-$C_{1-12}$ alkyl, -C(O)$NH_2$, -C(O)NH-$C_{1-12}$ alkyl, -C(O)N($C_{1-12}$ alkyl)$_2$, -NHC(O)-$C_{1-12}$ alkyl, -C(O)-$C_{1-12}$ alkyl, -S(O)-$C_{1-12}$ alkyl, -S(O)$_2$-$C_{1-12}$ alkyl, -S(O)$_2$$NH_2$, - S(O)$_2$NH-$C_{1-12}$ alkyl, -S(O)$_2$N($C_{1-12}$ alkyl)$_2$, 3- to 12-membered cycloalkyl, 3- to 12-membered cycloalkyl $C_{1-12}$ alkylene, 3- to 12-membered cycloalkyloxy, 3- to 12-membered heterocyclyl, 3- to 12-membered heterocyclyl $C_{1-12}$ alkylene, 3- to 12-membered heterocyclyloxy, 3- to 12-membered heterocycloalkyl, 3- to 12-membered heterocycloalkyl $C_{1-12}$ alkylene, 3- to 12-membered heterocycloalkyloxy, 5- to 10-membered heteroaryl, 5- to 10-membered heteroaryl $C_{1-12}$ alkylene, 5- to 10-membered heteroaryloxy, 6- to 10-membered aryl, 6- to 10-membered aryl $C_{1-12}$ alkylene, and 6- to 10-membered aryloxy.

Unless otherwise specified, the term "hetero" refers to a heteroatom or a heteroatom group (i.e., a heteroatom-containing group), including atoms other than carbon (C) and hydrogen (H) and groups containing such heteroatoms. For example, heteroatoms include, but are not limited to, oxygen (O), nitrogen (N), sulfur (S), phosphorus (P), silicon (Si), germanium (Ge), aluminum (Al), and boron (B); specific heteroatoms or heteroatom groups are, e.g., -O-, -S-, -N=, =O, =S, -P(=O)-, -P(=O)$_2$-, -P(=O)O-, -P(=O)$_2$O-, -C(=O)O-, -C(=O)-, -C(=S)-, -S(=O), -S(=O)$_2$-, and optionally substituted -C(=O) N(H)-, -N(H)-, -C(=NH)-, -S(=O)$_2$N(H)-, or -S(=O)N(H)-. Preferably, the term "hetero" means that a heteroatom or a heteroatom of a heteroatom group (i.e., a heteroatom-containing group) is selected from the group consisting of oxygen, nitrogen, and sulphur.

In the present application, one of the absolute configurations (e.g., one of

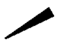 or 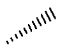

; specifically,

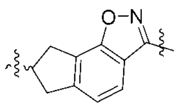

represents

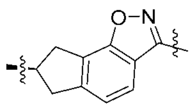
or
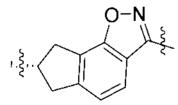

) or one of the relative configurations (e.g.,

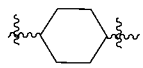

represents

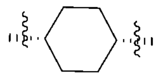
or
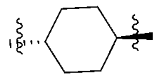

) of a stereogenic center is represented by a wavy line (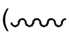). Unless otherwise specified, the compounds described herein include both E and Z geometric isomers when they contain olefinic double bonds or other centers of geometric asymmetry. Likewise, all tautomeric forms are included within the scope of the present application.

The group or structural fragment in the present application, such as $-LNK^1-Cy^1-LNK-Cy^2-LNK^2-$, $-Cy^1-Cy^2-LNK^2-$, LNK, $Cy^1$, $Cy^2$, $-Cy^1-LNK-Cy^2-$, $-Cy^1-LNK-$, or $-LNK-Cy^2-$, and specific options thereof, optionally can be linked to the left-side group and the right-side group of the group or fragment respectively in the general formula in a left-to-right reading order. For example, in the case that L is selected from $-Cy^1-LNK-$, when $Cy^1$ is selected from

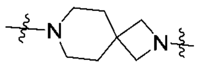,

according to a left-to-right reading order, the left side of $Cy^1$ is linked to the fragment

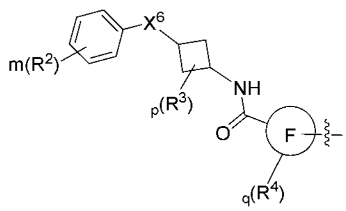

on the corresponding left side in the general formula, and the right side is linked to the fragment

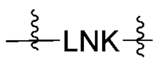

on the right side, resulting in a fragment

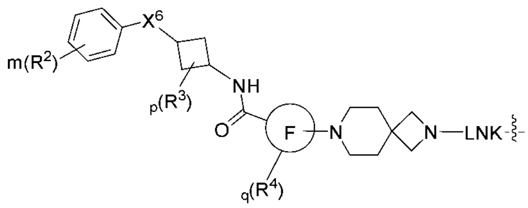.

Optionally, the group or structural fragment in the present application, such as $-LNK^1-Cy^1-LNK-Cy^2-LNK^2-$, $-Cy^1-Cy^2-LNK^2-$, LNK, $Cy^1$, $Cy^2$, $-Cy^1-LNK-Cy^2-$, $-Cy^1-LNK-$, or $-LNK-Cy^2-$, and specific options thereof, can be linked to the left-side group and the right-side group of the group or fragment respectively in the general formula in a right-to-left reading order. For example, in the case that L is selected from $-Cy^1-LNK-$, when $Cy^1$ is selected from

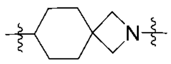

,

according to a right-to-left reading order, the right side of $Cy^1$ is linked to the fragment

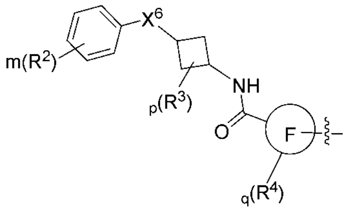

on the corresponding left side in the general formula, and the left side is linked to the fragment

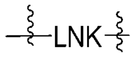

on the right side in the general formula, resulting in a fragment

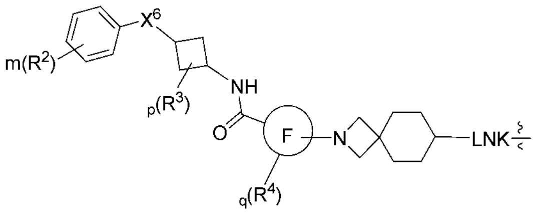

.

The other groups are as described above.

The term "treat", "treating", or "treatment" means administering the compound or formulation of the present application to ameliorate or eliminate a disease or one or more symptoms associated with the disease, and includes: (i) inhibiting the disease or disease state, i.e., arresting its progression; (ii) relieving the disease or disease state, i.e., causing regression of the disease or disease state.

The term "prevent", "preventing", or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The term "therapeutically effective amount" refers to an amount of the compound of the present application for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organic entity.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, and water.

The word "comprise" and variations thereof such as "comprises" or "comprising" will be understood in an open, non-exclusive sense, i.e., "including but not limited to".

Unless otherwise specified clearly in the context, singular terms herein encompass plural referents, and vice versa. Similarly, unless otherwise specified clearly in the context, the word "or" herein is intended to include "and". Unless otherwise stated, all numbers expressing the amounts of ingredients, measurements, or reaction conditions used herein are to be understood as being modified in all instances by the term "about". The term "about" when connected to a percentage may mean, for example, ±1%, preferably, ±0.5%, and more preferably, ±0.1%.

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogens. A valence tautomer includes the interconversion via recombination of some bonding electrons. Specifically, any of the compounds of the present disclosure in which, for example, pyrazolyl is independent or as part of heterocyclyl, may exist in the form of any tautomer or a mixture of two tautomers in any amount, i.e.,

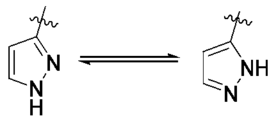

or

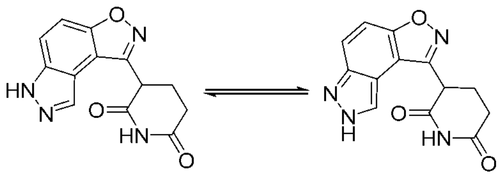

.

The present disclosure includes all possible tautomers of the compounds of the present disclosure, either as a single tautomer or any mixture of the tautomers in any ratio.

The present application also includes isotopically labeled compounds of the present application, which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine, and chlorine, such as $^{2}$H, $^{3}$H, $^{11}$C, $^{13}$C, $^{14}$C, $^{13}$N, $^{15}$N, $^{15}$O, $^{17}$O, $^{18}$O, 31p, $^{32}$P, $^{35}$S, $^{18}$F, $^{123}$I, $^{125}$I, and $^{36}$Cl.

Certain isotopically labeled compounds of the present application (e.g., those labeled with $^{3}$H and $^{14}$C) can be used to analyze compounds and/or substrate tissue distribution. Tritiated (i.e., $^{3}$H) and carbon-14 (i.e., $^{14}$C) isotopes are particularly preferred for their ease of preparation and detectability. Positron emitting isotopes, such as $^{15}$O, $^{13}$N, $^{11}$C, and $^{18}$F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., $^{2}$H) may provide certain therapeutic advantages (e.g., increased *in vivo* half-life or reduced dose) resulting from greater metabolic stability and hence may be preferred in some circumstances in which deuterium substitution may be partial or complete, wherein partial deuterium substitution refers to substitution of at least one hydrogen with at least one deuterium.

The compound of the present application may be asymmetrical, for example, having one or more stereoisomers. Unless otherwise stated, all stereoisomers include, for example, enantiomers and diastereoisomers. The compound of the present application containing asymmetrical carbon atoms may be separated in an optically pure form or in a racemic form. The optically pure form can be separated from a racemic mixture or can be synthesized using a chiral raw material or a chiral reagent.

The pharmaceutical composition of the present application may be prepared by combining the compound of the present application with a suitable pharmaceutically acceptable excipient, and may be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present application may be manufactured using methods well known in the art, such as conventional mixing, dissolving, granulating, dragee-making, levigating, emulsifying, and lyophilizing. In some embodiments, the pharmaceutical composition is in an oral form. For oral administration, the pharmaceutical composition can be formulated by mixing the active compounds with pharmaceutically acceptable excipients well known in

the art. These excipients enable the compounds of the present application to be formulated into tablets, pills, pastilles, dragees, capsules, liquids, gels, slurries, suspensions, and the like for oral administration to a patient. A solid oral composition can be prepared by conventional mixing, filling, or tableting. For example, the solid oral composition can be obtained by the following method: mixing the active compound with solid excipients, optionally grinding the resulting mixture, adding additional suitable excipients if desired, and then processing the mixture into granules to obtain the core parts of tablets or dragees. Suitable excipients include, but are not limited to: binders, diluents, disintegrants, lubricants, glidants, sweeteners, flavoring agents, or the like.

The pharmaceutical composition may also be suitable for parenteral administration, such as sterile solutions, suspensions, or lyophilized products in suitable unit dosage forms.

In all of the administration methods of the compound of general formula I described herein, the daily dose administered is from 0.01 mg/kg of body weight to 200 mg/kg of body weight, given in individual or separated doses. The compounds of the present application can be prepared using a variety of synthetic methods well known to those skilled in the art, including the specific embodiments listed below, embodiments formed by combining them with other chemical synthetic methods, and equivalent substitutions well known to those skilled in the art. The preferred embodiments include, but are not limited to, the examples of the present application.

The chemical reactions in the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protective groups for reactive functional groups (e.g., amino in the present application). For example, reference may be made to Greene's Protective Groups in Organic Synthesis (4th Ed.) Hoboken, New Jersey: John Wiley & Sons, Inc.

In some embodiments, when L is $-Cy^1$-LNK-, the compound of formula I-2 of the present application can be prepared by one skilled in the art of organic synthesis using the following intermediates or salts thereof by the following routes:

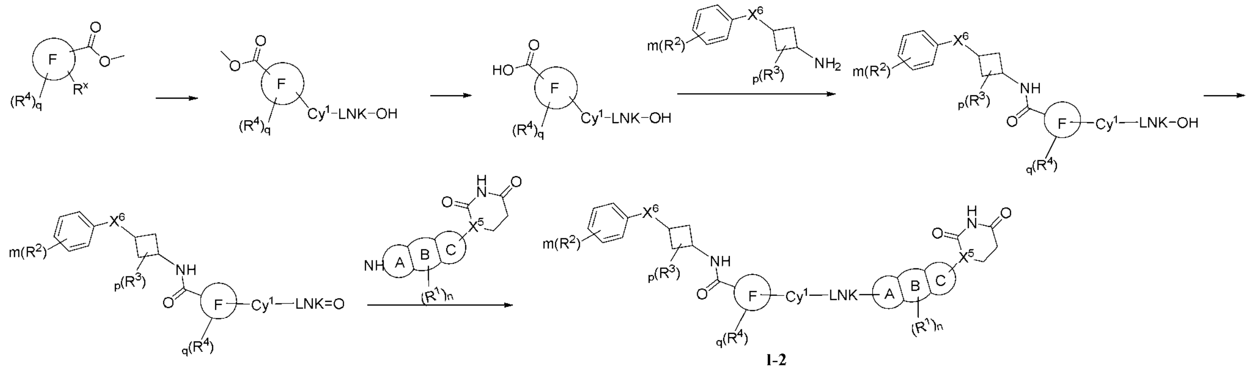

I-2

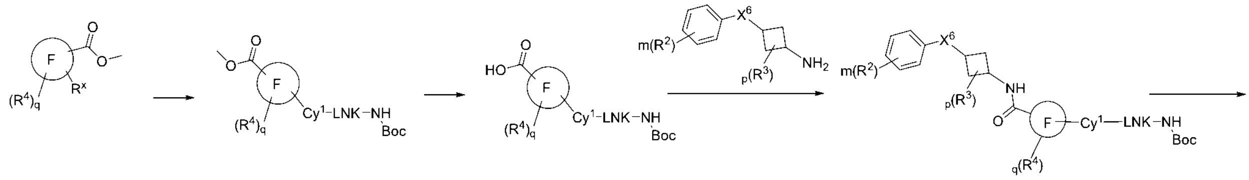

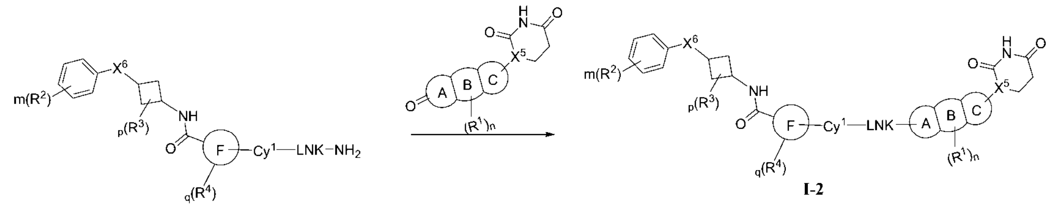

I-2

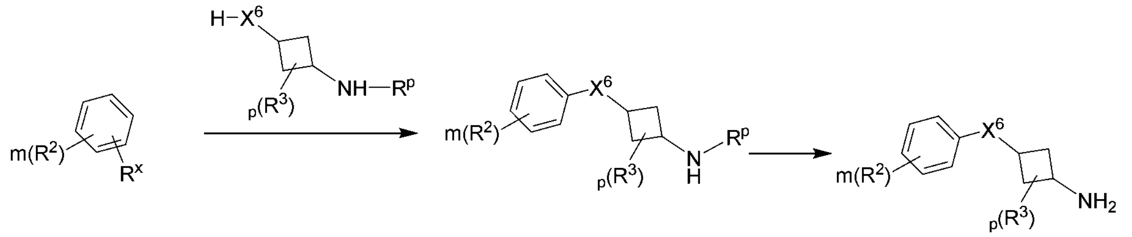

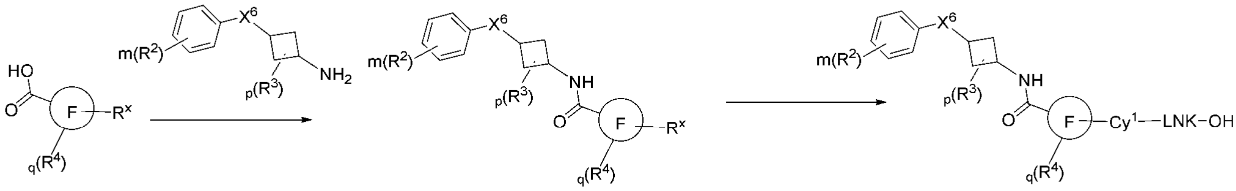

wherein ring A, ring B, ring C, ring F, $R^1$, n, $R^2$, m, $R^3$, p, $R^4$, q, $X^5$, $X^6$, $Cy^1$, and LNK are as defined herein;
$R^x$ is selected from a leaving group, such as halogen (fluorine, chlorine, bromine, or iodine);
$R^p$ is selected from a protecting group, such as Boc.

The following abbreviations are used in the present application:
DMSO represents dimethyl sulfoxide; DMF represents N,N-dimethylformamide; MeOH represents methanol; IBX represents 2-iodoxybenzoic acid; DIPEA represents N,N'-diisopropylethylamine; HATU represents O-(7-azabenzotriazol-1-yl)-N,N,N',N'-tetramethyluronium hexafluorophosphate; DCE represents dichloroethane; iPrOH represents isopropanol; Boc represents tert-butoxycarbonyl; $DC_{50}$ represents the drug concentration at which the degradation rate reaches 50%; Dmax represents the maximum degradation rate.

For clarity, the present disclosure is further described with the following examples, which are, however, not intended to limit the scope of the present application. All reagents used in the present application are commercially available and can be used without further purification.

**DETAILED DESCRIPTION**

Example 1: Synthesis of Compound 1

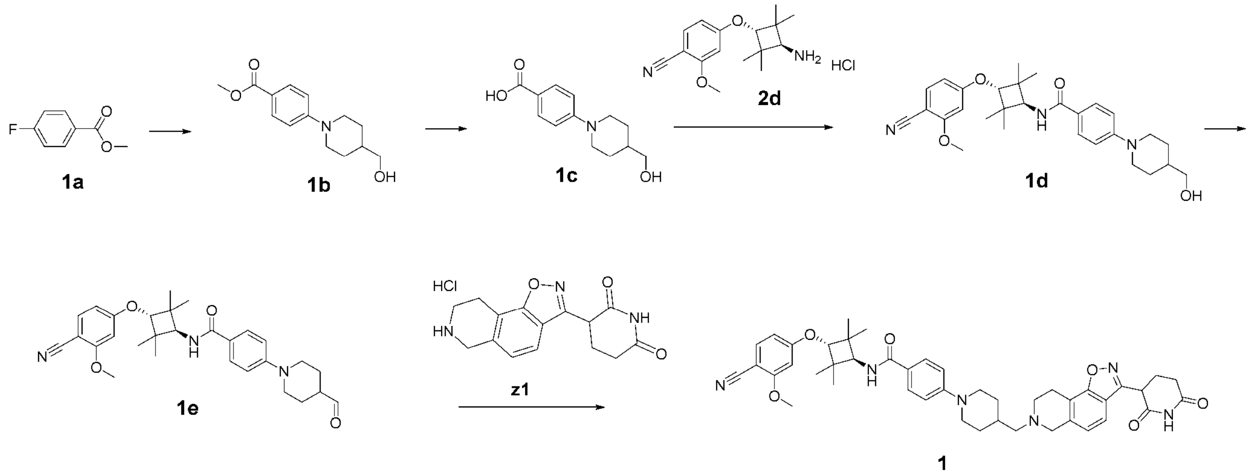


1a 1b 1c 1d 1e 1

Step 1: Preparation of intermediate 1b

Intermediate 1a (10 g), 4-piperidinemethanol (8.97 g), DMSO (100 mL), and DIPEA (16.77 g) were added to a single-necked flask in sequence, and the mixture was heated to 90 °C and reacted. After the reaction was completed, the reaction solution was cooled to room temperature, and ethyl acetate and water were added to the system for extraction. The organic phase was separated, and the aqueous phase was then extracted with ethyl acetate. The organic phases were combined, washed with saturated brine, dried over anhydrous sodium sulfate, filtered, concentrated by evaporation under reduced pressure to remove the solvent, and purified by silica gel column chromatography to give intermediate **1b** (10.03 g). MS(ESI, $[M+H]^+$) *m/z: 250.2*

Step 2: Preparation of intermediate **1c**

Intermediate **1b** (2.7 g), MeOH (40 mL), water (5 mL), and sodium hydroxide (1.24 g) were added to a single-necked flask in sequence, and the mixture was heated to 50 °C and reacted. After the reaction was completed, the reaction solution was cooled to room temperature, and 3 N diluted hydrochloric acid was added to the system to adjust the pH to weak acidity. The solvent was removed by evaporation under reduced pressure, and methanol was added to the concentrate to dissolve the product. The mixture was filtered, and the filtrate was collected and concentrated by evaporation under reduced pressure to remove the solvent to give intermediate 1c (1.82 g). MS(ESI, $[M-H]^-$) *m/z:* 234.1

Step 3: Preparation of intermediate **1d**

Intermediate **1c** (235 mg), intermediate **2d** (175 mg), DMF (5 mL), DIPEA (0.562 mL), and HATU (321 mg) were added to a single-necked flask in sequence, and the mixture was reacted at room temperature. After the reaction was completed, ethyl acetate and water were added to the system for extraction. The organic phase was separated, and the aqueous phase was extracted with ethyl acetate. The organic phases were combined, washed with saturated brine, dried over anhydrous sodium sulfate, filtered, concentrated by evaporation under reduced pressure to remove the solvent, and purified by silica gel column chromatography to give intermediate **1d** (0.22 g). MS(ESI, $[M+H]^+$) *m/z:* 492.1

Step 4: Preparation of intermediate **1e**

Intermediate **1d** (220 mg), DMSO (2 mL), and IBX (405 mg) were added to a single-necked flask in sequence, and the mixture was reacted at room temperature. After the reaction was completed, ethyl acetate and a saturated aqueous sodium bicarbonate solution were added to the system for extraction. The organic phase was separated, and the aqueous phase was then extracted with ethyl acetate. The organic phases were combined, washed with saturated brine, dried over anhydrous sodium sulfate, filtered, and concentrated by evaporation under reduced pressure to remove the solvent to give intermediate **1e** (190 mg).

Step 5: Preparation of compound 1

Intermediate 1e (200 mg), intermediate **z1** (131 mg) (with reference to Example **19** of WO2023088406 for the synthetic route), DCE/iPrOH = 5/1 (5 mL), sodium acetate (35.4 mg), and sodium triacetoxyborohydride (268 mg) were added to a single-necked flask in sequence, and the mixture was reacted at room temperature. After the reaction was completed, the mixture was concentrated by evaporation under reduced pressure to remove the solvent and purified by silica gel column chromatography to give compound 1 (128 mg).

MS(ESI, $[M+H]^+$) m/z: 759.6. $^1H$ NMR (500 MHz, DMSO-$d_6$) $\delta$ 11.08 (s, 1H), 7.74 (d, *J* = 8.5 Hz, 2H), 7.64 (d, *J* = 8.6 Hz, 1H), 7.58 (d, *J* = 8.1 Hz, 1H), 7.48 (d, *J*= *9.2* Hz, 1H), 7.11 (d, *J*= 8.3 Hz, 1H), 6.96 (d, *J*= 8.6 Hz, 2H), 6.64 (d, *J*= 2.2 Hz, 1H), 6.54 (dd, J= 8.6, 2.2 Hz, 1H), 4.56 (dd, *J*= 11.9, 5.0 Hz, 1H), 4.27 (s, 1H), 4.05 (d, *J*= *9.1* Hz, 1H), 3.91 (s, 5H), 3.71 (s, 2H), 3.00 (t, *J* = 5.6 Hz, 2H), 2.85 - 2.72 (m, 5H), 2.61 (dt, *J* = 17.3, 4.3 Hz, 1H), 2.49 - 2.44 (m, 1H), 2.40 (d, *J*=7.1 Hz, 2H), 2.19 (dq, J= 13.8, 4.8 Hz, 1H), 1.95 - 1.79 (m, 3H), 1.22 (s, 8H), 1.15 (s, 6H).

**Example** 2: Synthesis of Compound 2

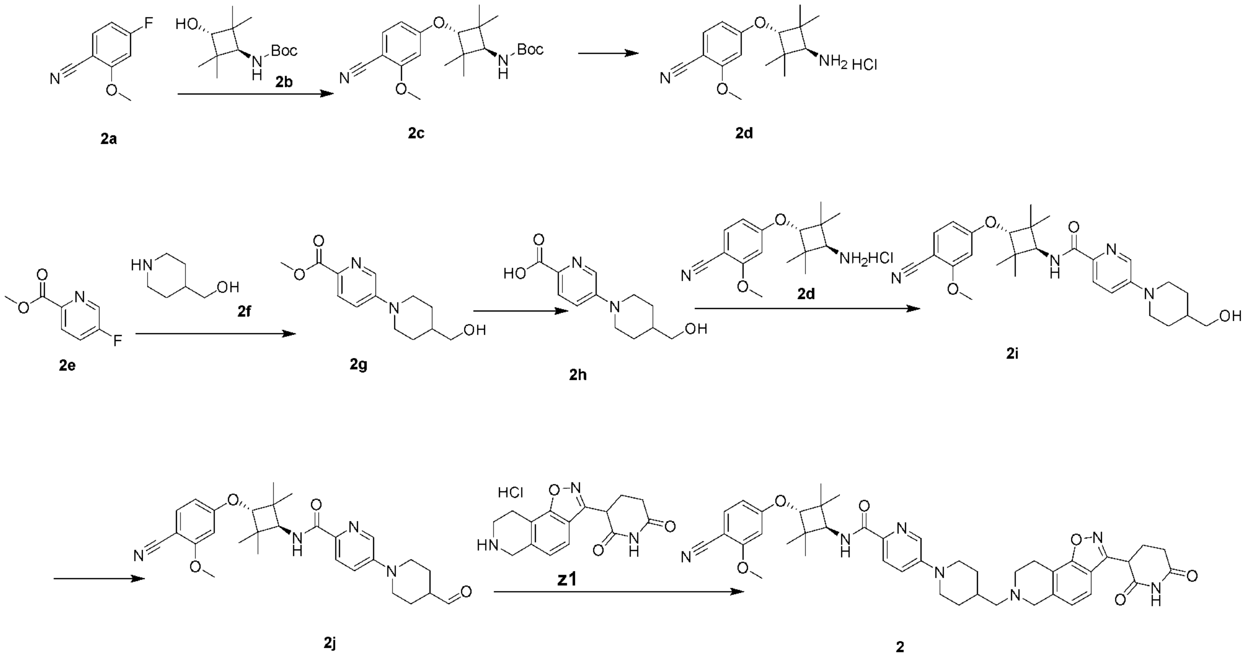


Step 1: Preparation of intermediate 2c

Intermediate 2a (6 g) and DMF (30 mL) were added to a reaction flask in sequence, sodium hydride (1.479 g) was added in an ice bath, and the mixture was stirred in the ice bath for 20 min. Intermediate 2b (4.47 g) was added, and the mixture was reacted at room temperature. After the reaction was completed, the reaction solution was slowly poured into ice water (300 mL) to quench the reaction, and the mixture was filtered to give a filter cake. The filter cake was dried to give intermediate 2c (8.9 g). MS(ESI, $[M+H]^+$) *m/z:* 275.42. $^1H$ NMR (500 MHz, DMSO-$d_6$) $\delta$ 7.62 (d, *J* = 8.6 Hz, 1H), 6.65 (d, *J* = 9.5 Hz, 1H), 6.60 (d, *J* = 2.2 Hz, 1H), 6.50 (dd, *J* = 8.7, 2.3 Hz, 1H), 4.12 (s, 1H), 3.93 (s, 1H), 3.89 (s, 3H), 1.40 (s, 9H), 1.13 (s, 6H), 1.06 (s, 6H).

Step 2: Preparation of intermediate **2d**

Intermediate **2c** (8.9 g) and a solution of hydrochloric acid in dioxane (59.4 mL) were added to a single-necked flask in sequence, and the mixture was reacted at room temperature. After the reaction was completed, the mixture was concentrated by evaporation under reduced pressure to remove the solvent and dried in vacuum to give intermediate **2d** (8.2 g). MS(ESI, $[M+H]^+$) *m/z:* 275.21. $^1H$ NMR (500 MHz, DMSO-$d_6$) $\delta$ 8.47 (d, *J* = 6.3 Hz, 3H), 7.64 (d, *J* = 8.6 Hz, 1H), 6.63 (d, J= 2.3 Hz, 1H), 6.53 (dd, J= 8.7, 2.3 Hz, 1H), 4.31 (s, 1H), 3.90 (s, 3H), 3.06 (q, J= 6.0 Hz, 1H), 1.32 (s, 6H), 1.11 (s, 6H).

Step 3: Preparation of intermediate **2g**

Intermediate **2e** (2 g), intermediate **2f** (1.782 g), DMF (20 mL), and potassium carbonate (5.35 g) were added to a single-necked flask in sequence, and the mixture was heated to 110 °C and reacted. After the reaction was completed, the reaction solution was cooled to room temperature, and ethyl acetate and water were added to the system for extraction. The organic phase was separated, and the aqueous phase was then extracted with ethyl acetate. The organic phases were combined, washed with saturated brine, dried over anhydrous sodium sulfate, filtered, concentrated by evaporation under reduced pressure to remove the solvent, and purified by silica gel column chromatography to give intermediate **2g** (0.72 g). MS(ESI, $[M+H]^+$) *m/z:* 251.0. $^1H$ NMR (500 MHz, DMSO-*d*$_6$) $\delta$ 8.36 (d, *J* = 3.0 Hz, 1H), 7.84 (d, *J* = 8.9 Hz, 1H), 7.31 (dd, *J*= *8.9,* 3.1 Hz, 1H), 4.49 (t, *J* = 5.3 Hz, 1H), 4.02 - 3.95 (m, 2H), 3.79 (s, 3H), 3.30 - 3.24 (m, 2H), 2.86 (td, *J*= 12.7, 2.8 Hz, 2H), 1.81 - 1.71 (m, 2H), 1.69 - 1.56 (m, 1H), 1.25 - 1.12 (m, 2H).

Step 4: Preparation of intermediate **2h**

Referring to the preparation process of intermediate **1c** in Step 2 of Example 1, intermediate 2h (0.86 g) was prepared by the reaction with intermediate 1b being replaced by intermediate 2g.

MS(ESI, $[M+H]^+$) m/z: 237.23. $^1H$ NMR (500 MHz, DMSO-*d*$_6$) $\delta$ 8.30 (d, *J*= 3.0 Hz, 1H), 8.03 (d, *J*= 9.1 Hz, 1H), 7.75 (dd, *J* = 9.2, 2.9 Hz, 1H), 4.06 (dt, *J* = 13.4, 3.4 Hz, 2H), 3.28 (d, *J*= 6.2 Hz, 2H), 3.00 (td, *J*= 12.6, 2.6 Hz, 2H), 1.83 - 1.73 (m, 2H), 1.68 (tq, *J* = 11.4, 3.5 Hz, 1H), 1.21 (qd, *J*= 12.5, 4.1 Hz, 2H).

Step 5: Preparation of intermediate 2i

Referring to the preparation process of intermediate **1d** in Step 3 of Example 1, intermediate 2i (0.25 g) was prepared by the reaction with intermediate **1c** being replaced by intermediate 2h.

MS(ESI, $[M+H]^+$) m/z: 493.44. $^1H$ NMR (500 MHz, DMSO-*d*$_6$) $\delta$ 8.33 (d, *J* = 2.9 Hz, 1H), 8.09 (d, *J*= *9.1* Hz, 1H), 7.82 (d, *J*= 8.8 Hz, 1H), 7.64 (d, *J* = 8.6 Hz, 1H), 7.41 (dd, *J* = 8.9, 2.9 Hz, 1H), 6.67 (d, *J* = 2.2 Hz, 1H), 6.57 (dd, *J*= 8.7, 2.2 Hz, 1H), 4.49 (t, *J* = 5.3 Hz, 1H), 4.37 (s, 1H), 3.99 - 3.93 (m, 3H), 3.91 (s, 3H), 3.28 (t, *J* = 5.7 Hz, 2H), 2.85 (td, *J* = 12.6, 2.7 Hz, 2H), 2.69 (s, 1H), 1.74 (dd, *J*= 13.7, 3.6 Hz, 2H), 1.62 (dtd, *J*= 14.1, 7.7, 3.4 Hz, 1H), 1.23 *(d, J*= 8.7 Hz, 1H), 1.20 (s, 6H), 1.15 (s, 6H).

Step 6: Preparation of intermediate 2j

Referring to the preparation process of intermediate 1e in Step 4 of Example 1, intermediate 2j (210 mg) was prepared by the reaction with intermediate **1d** being replaced by intermediate 2i.

MS(ESI, $[M+H]^+$) m/z: 491.25

Step 7: Preparation of compound 2

Referring to the preparation process of compound 1 in Step 5 of Example 1, compound 2 (160 mg) was prepared by the reaction with intermediate 1e being replaced by intermediate 2j.

MS(ESI, $[M+H]^+$) *m/z:* 760.01. $^1H$ NMR (500 MHz, DMSO-*d*$_6$) $\delta$ 11.08 (s, 1H), 8.34 (d, *J*= 3.0 Hz, 1H), 8.09 (d, *J* = 9.1 Hz, 1H), 7.83 (d, *J* = 8.8 Hz, 1H), 7.64 (d, *J* = 8.6 Hz, 1H), 7.58 (d, *J* = 8.2 Hz, 1H), 7.42 (dd, *J*= 9.0, 2.9 Hz, 1H), 7.11 (d, *J* = 8.3 Hz, 1H), 6.67 (d, *J* = 2.2 Hz, 1H), 6.57 (dd, *J* = 8.6, 2.2 Hz, 1H), 4.56 (dd, *J* = 11.9, 5.0 Hz, 1H), 4.37 (s, 1H), 3.95 (dd, *J*= 10.7, 5.0 Hz, 3H), 3.91 (s, 3H), 3.71 (s, 2H), 3.00 (t, *J* = 5.7 Hz, 2H), 2.94 - 2.85 (m, 2H), 2.82 - 2.73 (m, 3H), 2.61 (dt, *J* = 17.2, 4.2 Hz, 1H), 2.47 (dd, *J* = 12.1, 4.4 Hz, 1H), 2.40 (d, *J* = 7.1 Hz, 2H), 2.19 (dq, *J* = 13.4, 4.6 Hz, 1H), 1.94 (d, *J* = 7.0 Hz, 1H), 1.84 (d, *J* = 13.0 Hz, 2H), 1.20 (s, 8H), 1.15 (s, 6H).

**Example** 3: Synthesis of Compound 3

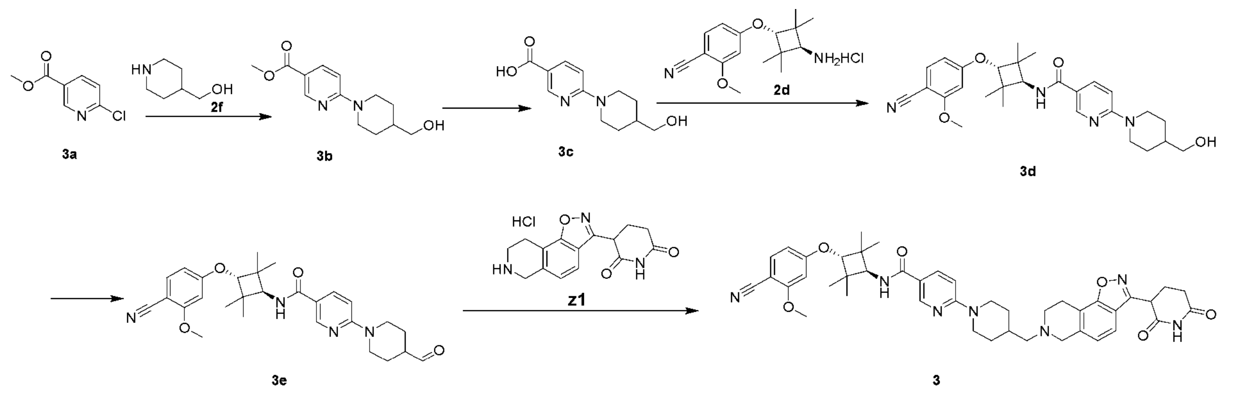


3a 3b 3c 3d 3e 3

Step 1: Preparation of intermediate 3b

Referring to the preparation of intermediate 2g in Step 3 of Example 2, intermediate 3b (2.5 g) was prepared with intermediate 2e being replaced by intermediate 3a.

MS(ESI, $[M+H]^+$) *m/z:* 251.31. $^1H$ NMR (500 MHz, DMSO-$d_6$) $\delta$ 8.62 (d, *J* = 2.3 Hz, 1H), 7.90 (dd, *J*= *9.0,* 2.5 Hz, 1H), 6.85 (d, *J*= *9.1* Hz, 1H), 4.51 - 4.40 (m, 3H), 3.78 (s, 3H), 3.27 (t, *J* = 5.7 Hz, 2H), 2.90 (td, *J*= 12.8, 2.6 Hz, 2H), 1.77 - 1.62 (m, 3H), 1.09 (tdd, *J*= 12.7, 11.2, 4.3 Hz, 2H).

Step 2: Preparation of intermediate 3c

Referring to the preparation process of intermediate **1c** in Step 2 of Example 1, intermediate 3c (0.86 g) was prepared by the reaction with intermediate 1b being replaced by intermediate 3b.

MS(ESI, $[M+H]^+$) *m/z:* 237.31. $^1H$ NMR (500 MHz, DMSO-$d_6$) $\delta$ 8.41 (d, *J* = 2.3 Hz, 1H), 8.11 (dd, *J*= 9.6, 2.3 Hz, 1H), 7.31 (d, *J*= 9.6 Hz, 1H), 4.44 (d, *J* = 13.4 Hz, 2H), 3.28 (d, *J*= 5.9 Hz, 2H), 3.24 - 3.17 (m, 2H), 1.86 - 1.71 (m, 3H), 1.30 - 1.16 (m, 2H).

Step 3: Preparation of intermediate 3d

Referring to the preparation process of intermediate **1d** in Step 3 of Example 1, intermediate 3d (0.25 g) was prepared by the reaction with intermediate **1c** being replaced by intermediate 3c.

MS(ESI, $[M+H]^+$) m/z: 493.45. $^1H$ NMR (500 MHz, DMSO-$d_6$) $\delta$ 8.61 (d, *J* = 2.5 Hz, 1H), 7.93 (dd, *J*= *9.0,* 2.6 Hz, 1H), 7.64 (d, *J*= 8.6 Hz, 1H), 7.58 (d, *J*= *9.3* Hz, 1H), 6.85 (d, *J*= *9.0* Hz, 1H), 6.64 (d, *J*= 2.2 Hz, 1H), 6.54 (dd, *J*= 8.6, 2.3 Hz, 1H), 4.50 - 4.39 (m, 3H), 4.25 (s, 1H), 4.05 (d, *J*= *9.2* Hz, 1H), 3.91 (s, 3H), 3.27 (t, *J* = 5.7 Hz, 2H), 2.87 (td, *J*= 12.8, 2.6 Hz, 2H), 1.77 - 1.62 (m, 3H), 1.22 (s, 6H), 1.14 (s, 6H), 1.12 - 1.07 (m, 2H).

Step 4: Preparation of intermediate 3e

Referring to the preparation process of intermediate 1e in Step 4 of Example 1, intermediate 3e (210 mg) was prepared by the reaction with intermediate **1d** being replaced by intermediate 3d. MS(ESI, $[M+H]^+$) *m/z:* 491.27.

Step 5: Preparation of compound 3

Referring to the preparation process of compound 1 in Step 5 of Example 1, compound 3 (150 mg) was prepared by the reaction with intermediate **1e** being replaced by intermediate 3e. MS(ESI, $[M+H]^+$) m/z: 759.90. $^1H$ NMR (500 MHz, DMSO-$d_6$) $\delta$ 11.08 (s, 1H), 8.62 (d, *J*= 2.5 Hz, 1H), 7.93 (dd, *J*= 9.0, 2.5 Hz, 1H), 7.64 (d, *J*= 8.5 Hz, 1H), 7.58 (d, *J* = 8.6 Hz, 2H), 7.11 (d, *J* = 8.3 Hz, 1H), 6.85 (d, *J* = 9.1 Hz, 1H), 6.64 (d, *J*= 2.2 Hz, 1H), 6.54 (dd, *J* = 8.7, 2.2 Hz, 1H), 4.56 (dd, *J*= 11.9, 5.0 Hz, 1H), 4.42 (d, *J*= 13.0 Hz, 2H), 4.25 (s, 1H), 4.05 (d, *J*= 9.1 Hz, 1H), 3.91 (s, 3H), 3.71 (s, 2H), 3.00 (t, *J* = 5.8 Hz, 2H), 2.93 (td, *J* = 13.1, 2.6 Hz, 2H), 2.82 - 2.72 (m, 3H), 2.61 (dt, *J*= 17.3, 4.3 Hz, 1H), 2.47 (dd, *J* = 12.2, 4.4 Hz, 1H), 2.38 (d, *J* = 7.1 Hz, 2H), 2.19 (dq, *J* = 13.6, 4.8 Hz, 1H), 1.99 (t, *J*= *3.8* Hz, 1H), 1.83 (d, *J*= 12.8 Hz, 2H), 1.22 (s, 6H), 1.14 (s, 8H).

**Example** 4: Synthesis of Compound 4

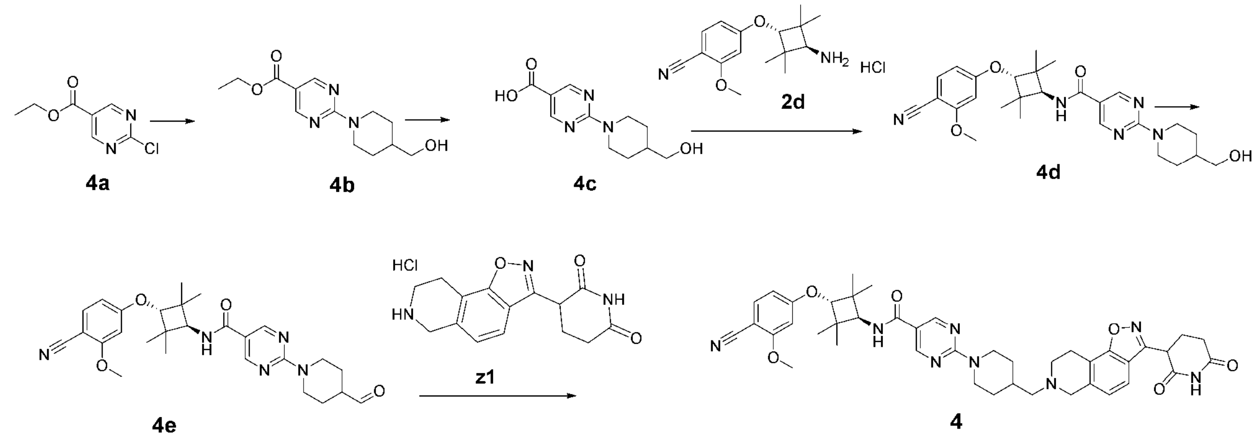

4a 4b 4c 2d 4d

4e z1 4

Step 1: Preparation of intermediate 4b

Referring to the preparation of intermediate 2g in Step 3 of Example 2, intermediate 4b (6.8 g) was prepared with intermediate 2e being replaced by intermediate 4a.

MS(ESI, $[M+H]^+$) m/z: 266.2.

Step 2: Preparation of intermediate 4c

Referring to the preparation process of intermediate **1c** in Step 2 of Example 1, intermediate 4c (1.54 g) was prepared by the reaction with intermediate 1b being replaced by intermediate 4b. MS(ESI, $[M-H]^-$) *m/z:* 236.1.

Step 3: Preparation of intermediate 4d

Referring to the preparation process of intermediate **1d** in Step 3 of Example 1, intermediate 4d (0.26 g) was prepared by the reaction with intermediate **1c** being replaced by intermediate **4c.** MS(ESI, $[M+H]^+$) *m/z:* 494.1.

Step 4: Preparation of intermediate **4e**

Referring to the preparation process of intermediate 1e in Step 4 of Example 1, intermediate 4e (224 mg) was prepared by the reaction with intermediate **1d** being replaced by intermediate **4d.**

Step 5: Preparation of compound **4**

Referring to the preparation process of compound 1 in Step 5 of Example 1, compound 4 (142 mg) was prepared by the reaction with intermediate 1e being replaced by intermediate **4e.**

MS(ESI, $[M+H]^+$) *m/z:* 760.9. $^1$H NMR (500 MHz, DMSO-$d_6$) $\delta$ 11.07 (s, 1H), 8.75 (s, 2H), 7.70 (d, *J*= 9.2 Hz, 1H), 7.64 (d, *J* = 8.6 Hz, 1H), 7.58 (d, *J* = 8.2 Hz, 1H), 7.11 (d, *J* = 8.3 Hz, 1H), 6.64 (d, *J*= 2.3 Hz, 1H), 6.54 (dd, *J* = 8.6, 2.2 Hz, 1H), 4.75 (d, *J* = 13.0 Hz, 2H), 4.56 (dd, *J* = 11.9, 5.0 Hz, 1H), 4.23 (s, 1H), 4.03 (d, *J* = 9.2 Hz, 1H), 3.91 (s, 3H), 3.71 (s, 2H), 2.99 (d, *J* = 11.1 Hz, 4H), 2.77 (tt, *J* = 11.8, 5.9 Hz, 3H), 2.61 (dt, *J* = 17.3, 4.3 Hz, 1H), 2.53 (s, 1H), 2.49 - 2.43 (m, 1H), 2.39 (d, *J*= 7.2 Hz, 2H), 2.19 (dq, *J*= 13.5, 4.9 Hz, 1H), 1.85 (d, *J*= 12.1 Hz, 2H), 1.22 (s, 6H), 1.13 (s, 8H).

**Example 5:** Synthesis of Compound **5**

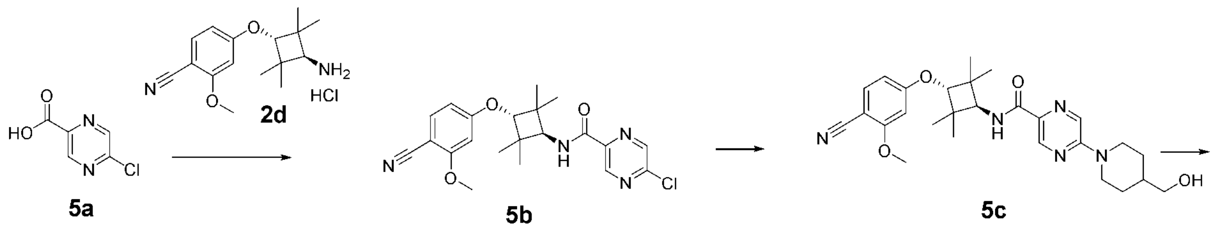

5a 2d 5b 5c

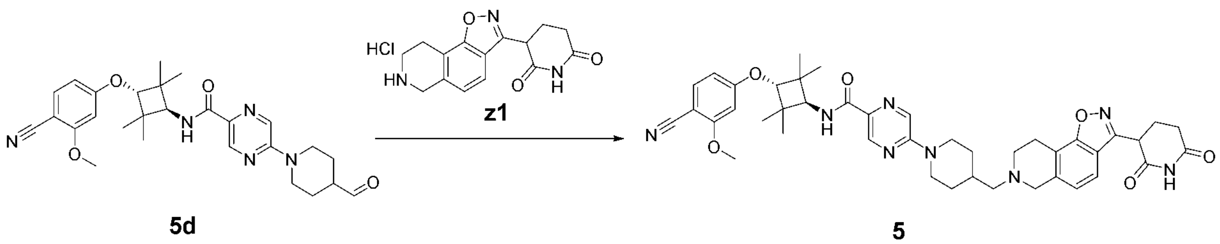

5d z1 5

Step 1: Preparation of intermediate **5b**

Referring to the preparation process of intermediate **1d** in Step 3 of Example 1, intermediate **5b** (0.56 g) was prepared by the reaction with intermediate **1c** being replaced by intermediate **5a.** MS(ESI, $[M+H]^+$) *m/z:* 415.4.

Step 2: Preparation of intermediate **5c**

Intermediate **5b** (0.56 g), intermediate 2f (0.3 g), acetonitrile (20 mL), and potassium carbonate (0.55 g) were added to a single-necked flask in sequence, and the mixture was warmed to 80 °C and reacted. After the reaction was completed, ethyl acetate and water were added to the system for extraction. The organic phase was separated, and the aqueous phase was then extracted with ethyl acetate. The organic phases were combined, washed with saturated brine, dried over anhydrous sodium sulfate, filtered, concentrated by evaporation under reduced pressure to remove the solvent, and purified by silica gel column chromatography to give intermediate 5c (0.48 g). MS(ESI, $[M+H]^+$) *m/z:* 494.1.

Step 3: Preparation of intermediate **5d**

Referring to the preparation process of intermediate 1e in Step 4 of Example 1, intermediate **5d** (425 mg) was prepared by the reaction with intermediate **1d** being replaced by intermediate **5c.**

Step 4: Preparation of compound **5**

Referring to the preparation process of compound 1 in Step 5 of Example 1, compound **5** (98 mg) was prepared by the reaction with intermediate 1e being replaced by intermediate **5d.**

MS(ESI, $[M+H]^+$) *m/z:* 761.0. $^1$H NMR (500 MHz, DMSO-$d_6$) $\delta$ 11.08 (s, 1H), 8.61 (d, *J* = 1.2 Hz, 1H), 8.33 (d, *J* = 1.5 Hz, 1H), 7.81 (d, *J= 9.1* Hz, 1H), 7.64 (d, *J* = 8.6 Hz, 1H), 7.58 (d, *J*= 8.2 Hz, 1H), 7.11 (d, *J* = 8.2 Hz, 1H), 6.66 (d, *J= 2.3* Hz, 1H), 6.56 (dd, *J* = 8.7, 2.3 Hz, 1H), 4.56 (dd, *J* = 11.9, 5.0 Hz, 1H), 4.49 (d, *J* = 13.2 Hz, 2H), 4.37 (s, 1H), 3.98 - 3.89 (m, 4H), 3.72 (s, 2H), 3.08 - 2.97 (m, 4H), 2.76 (dt, *J* = 16.9, 5.5 Hz, 3H), 2.61 (dt, *J* = 17.3, 4.4 Hz, 1H), 2.47 (dd, J= 12.2, 4.3 Hz, 1H), 2.40 (d, *J* = 7.1 Hz, 2H), 2.24 - 2.15 (m, 1H), 2.08 - 1.97 (m, 1H), 1.86 (d, *J* = 12.6 Hz, 2H), 1.17 (d, *J* = 26.2 Hz, 14H).

**Example 6:** Synthesis of Compound **6**

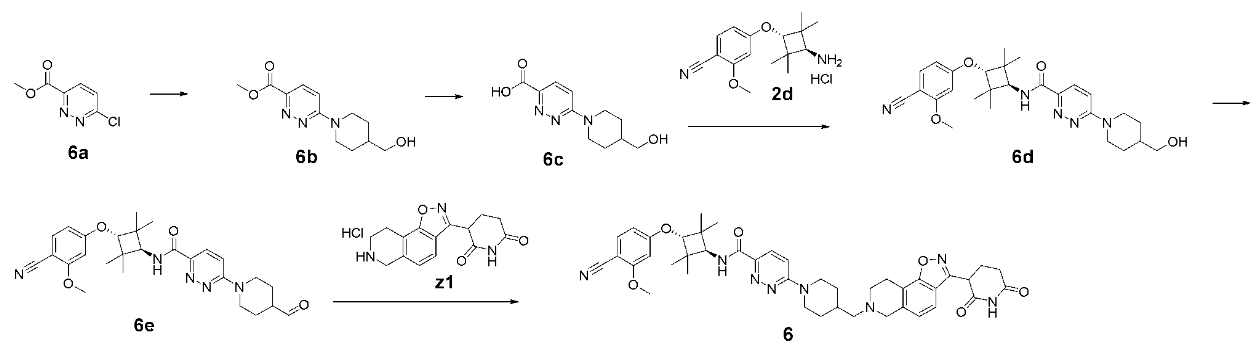


6a 6b 6c 6d 6e 6

Step 1: Preparation of intermediate **6b**

Referring to the preparation process of intermediate **1b** in Step 1 of Example 1, intermediate **6b** (6.8 g) was prepared by the reaction with intermediate 1a being replaced by intermediate 6a. MS(ESI, $[M+H]^+$) *m/z:* 252.0.

Step 2: Preparation of intermediate **6c**

Referring to the preparation process of intermediate **1c** in Step 2 of Example 1, intermediate **6c** (2.62 g) was prepared by the reaction with intermediate 1b being replaced by intermediate **6b.** MS(ESI, $[M-H]^-$) *m/z:* 236.1.

Step 3: Preparation of intermediate **6d**

Referring to the preparation process of intermediate **1d** in Step 3 of Example 1, intermediate **6d** (0.17 g) was prepared by the reaction with intermediate **1c** being replaced by intermediate **6c.**

MS(ESI, $[M+H]^+$) m/z: 494.1.

Step 4: Preparation of intermediate **6e**

Referring to the preparation process of intermediate 1e in Step 4 of Example 1, intermediate 6e (162 mg) was prepared by the reaction with intermediate **1d** being replaced by intermediate **6d.**

Step 5: Preparation of compound **6**

Referring to the preparation process of compound 1 in Step 5 of Example 1, compound 6 (78 mg) was prepared by the reaction with intermediate **1e** being replaced by intermediate **6e.**

MS(ESI, $[M+H]^+$) m/z: 761.6. $^1$H NMR (500 MHz, DMSO-$d_6$) $\delta$ 11.08 (s, 1H), 8.24 (d, *J*= *9.2* Hz, 1H), 7.82 (d, *J* = 9.5 Hz, 1H), 7.64 *(d, J*= 8.6 Hz, 1H), 7.58 (d, J= 8.1 Hz, 1H), 7.37 (d, J= 9.7 Hz, 1H), 7.11 *(d, J*= 8.3 Hz, 1H), 6.67 (d, *J*= 2.2 Hz, 1H), 6.57 (dd, *J* = 8.6, 2.2 Hz, 1H), 4.60 - 4.47 (m, 3H), 4.40 (s, 1H), 4.00 (d, *J* = 9.2 Hz, 1H), 3.91 (s, 3H), 3.72 (s, 2H), 3.11 - 2.97 (m, 4H), 2.77 (dt, *J* = 17.3, 5.5 Hz, 3H), 2.61 (dt, *J* = 17.2, 4.2 Hz, 1H), 2.54 (d, *J*= *4.6* Hz, 1H), 2.47 (dd, *J*= 12.1, 4.2 Hz, 1H), 2.40 (d, *J* = 7.2 Hz, 2H), 2.19 (dq, *J* = 8.6, 4.5 Hz, 1H), 1.87 (d, *J* = 12.1 Hz, 2H), 1.20 (d, *J* = 34.9 Hz, 14H).

**Example 7:** Synthesis of Compound **7**

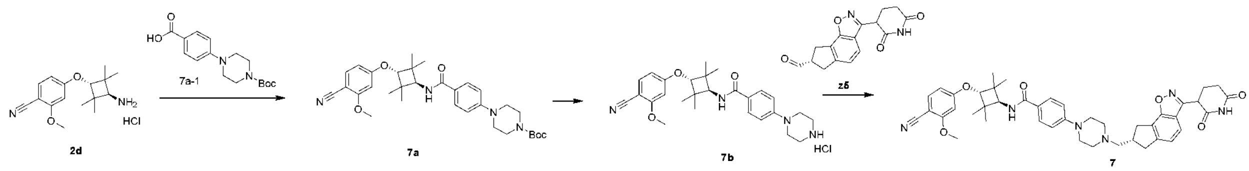


2d 7a 7b 7

Step 1: Preparation of intermediate **7a**

Intermediate **2d** (0.20 g), **7a-1** (0.19 g), DIPEA (0.33 g), and DMF (5 mL) were mixed, and HATU (0.29 g) was added. The mixture was reacted at room temperature until the reaction was completed. The reaction solution was poured

into water, and the mixture was extracted with ethyl acetate and purified by silica gel column chromatography to give intermediate **7a** (0.28 g).

MS (ESI) *m/z* $[M+H]^+$: 563.31.

Step 2: Preparation of intermediate **7b**

Intermediate **7a** (0.28 g), DCM (10 mL), and a 4 M solution of hydrochloric acid in dioxane (5 mL) were mixed, and the mixture was reacted at room temperature until the reaction was completed. The mixture was concentrated to give intermediate **7b** (0.19 g). MS (ESI) *m/z* $[M+H]^+$: 463.25.

Step 3: Preparation of compound **7**

Intermediate **7b** (0.11 g), intermediate **z5** (0.07 g) (with reference to intermediate **26f** of WO2024037616 for the synthetic route), sodium acetate (0.04 g), and DCE/IPA (v:v = 5:1) (5 mL) were mixed, and sodium triacetoxyborohydride (0.10 g) was then added. The mixture was reacted at room temperature until the reaction was completed. The reaction solution was concentrated and purified by silica gel column chromatography to give compound **7** (0.06 g).

MS (ESI) *m/z* $[M+H]^+$: 745.43. $^1H$ NMR (500 MHz, DMSO) δ 11.08 (s, 1H), 7.76 (d, *J* = 8.6 Hz, 2H), 7.63 (dd, *J*= 15.6, 8.3 Hz, 2H), 7.52 (d, *J*= 9.2 Hz, 1H), 7.28 (d, *J*= 8.1 Hz, 1H), 6.99 (d, *J* = 8.9 Hz, 2H), 6.64 (d, *J*= 2.2 Hz, 1H), 6.54 (dd, *J*= 8.7, 2.2 Hz, 1H), 4.57 (ddd, *J*= 11.9, 5.0, 1.8 Hz, 1H), 4.27 (s, 1H), 4.06 (d, *J*= *9.2* Hz, 1H), 3.91 (s, 3H), 3.35 (s, 1H), 3.30 - 3.17 (m, 6H), 2.93 (d, *J* = 13.0 Hz, 2H), 2.86 (dd, *J* = 16.0, 5.2 Hz, 1H), 2.77 (ddd, *J* = 17.2, 11.9, 5.4 Hz, 1H), 2.66 - 2.54 (m, 5H), 2.41 (d, *J*= *7.3* Hz, 2H), 2.23 - 2.16 (m, 1H), 1.23 (s, 6H), 1.15 (s, 6H).

With reference to the synthetic method for intermediate 7b, the following intermediates were synthesized:

| **Intermediate No.** | **Starting material structure** | **Intermediate structure** | MS (ESI) m/z data |
|---|---|---|---|
| **7c** | 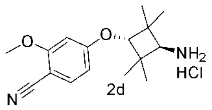 2d 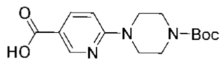 | 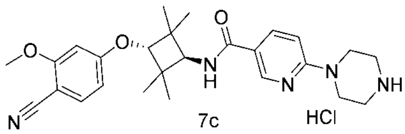 7c | MS (ESI) m/z $[M+H]^+$:464.5 |
| **7d** | 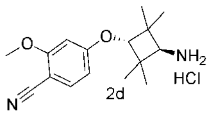 2d 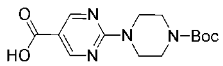 | 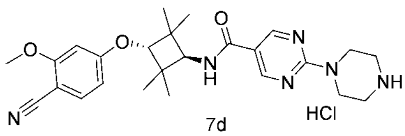 7d | MS (ESI) m/z $[M+H]^+$:465.3 |
| **7e** | 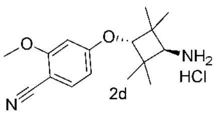 2d 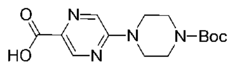 | 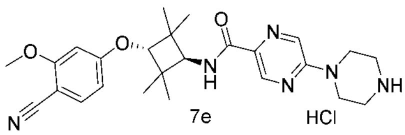 7e | MS (ESI) m/z $[M+H]^+$:465.3 |
| **7f** | 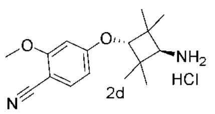 2d 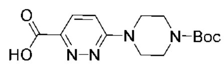 | 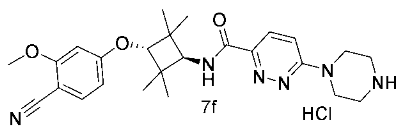 7f | MS (ESI) m/z $[M+H]^+$:465.5 |

**Example 8:** Synthesis of Compound **8**

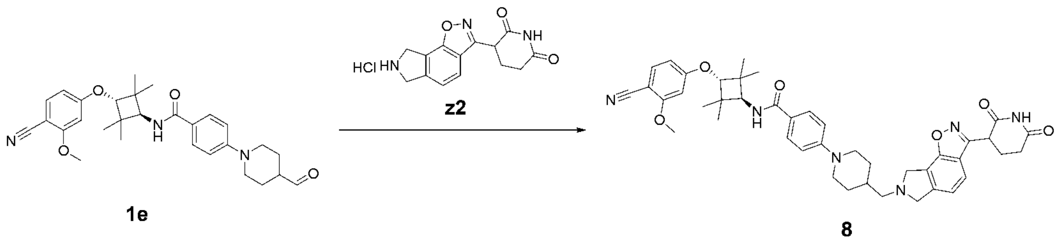


1e

8

Intermediate 1e (0.1 g), intermediate **z2** (0.08 g) (with reference to Example 1 of WO2024037616 for the synthetic route), sodium acetate (0.04 g), 1,2-dichloroethane (10 mL), and isopropanol (2 mL) were added to a reaction flask in sequence, and the mixture was reacted with stirring at room temperature for 10 min. Sodium triacetoxyborohydride (0.1 g) was then added, and the mixture was reacted at room temperature for 1 h. After the reaction was completed, the reaction was quenched with a saturated sodium bicarbonate solution, and the reaction solution was extracted with dichloromethane, washed with saturated brine, concentrated, and then purified by silica gel column chromatography to give compound 8 (0.10 g). MS(ESI, $[M+H]^+$) m/z: 745.41. $^1H$ NMR (500 MHz, DMSO) δ 11.09 (s, 1H), 7.73 (dd, *J*= 14.9, 8.3 Hz, 3H), 7.64 (d, *J* = 8.6 Hz, 1H), 7.49 (d, *J* = 9.2 Hz, 1H), 7.31 (d, *J* = 8.2 Hz, 1H), 6.97 (d, *J* = 9.0 Hz, 2H), 6.64 (d, *J* = 2.2 Hz, 1H), 6.54 (dd, *J*= 8.7, 2.2 Hz, 1H), 4.60 (dd, *J* = 11.9, 5.0 Hz, 1H), 4.27 (s, 1H), 4.16 (s, 2H), 4.09 - 4.03 (m, 3H), 3.91 (s, 5H), 2.80 (dddd, *J* = 29.4, 17.1, 12.2, 3.9 Hz, 3H), 2.69 - 2.58 (m, 3H), 2.54 (d, *J* = 4.5 Hz, 1H), 2.20 (dq, *J* = 13.6, 4.8 Hz, 1H), 1.93 - 1.85 (m, 2H), 1.81 (d, *J* = 3.8 Hz, 1H), 1.31 - 1.24 (m, 2H), 1.23 (s, 6H), 1.15 (s, 6H).

**Example 9:** Synthesis of Compound **9**

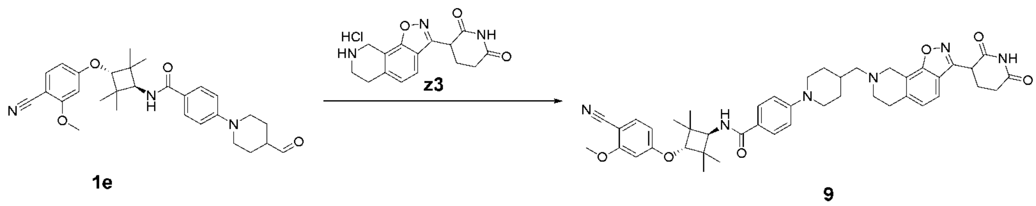


1e

9

Referring to the method in Example **8,** compound **9** (0.08 g) was synthesized with intermediate **z2** being replaced by intermediate **z3** (with reference to Example 3 of WO2024037616 for the synthetic route). MS(ESI, $[M+H]^+$) m/z: 759.39. $^1H$ NMR (500 MHz, DMSO) δ 11.07 (s, 1H), 7.74 (d, *J*= 8.6 Hz, 2H), 7.64 (d, *J*= 8.6 Hz, 1H), 7.59 (d, *J*= 8.1 Hz, 1H), 7.48 (d, *J*= *9.2* Hz, 1H), 7.15 (d, *J*= 8.2 Hz, 1H), 7.00 - 6.93 (m, 2H), 6.64 (d, *J*= 2.3 Hz, 1H), 6.54 (dd, *J*= 8.7, 2.2 Hz, 1H), 4.56 (dd, *J*= 11.9, 5.0 Hz, 1H), 4.27 (s, 1H), 4.05 (d, *J*= *9.2* Hz, 1H), 3.91 (s, 3H), 3.86 (d, *J*= 12.9 Hz, 2H), 3.81 (s, 2H), 2.96 (t, *J*= 5.7 Hz, 2H), 2.89 - 2.72 (m, 5H), 2.60 (dt, *J*= 17.3, 4.2 Hz, 1H), 2.48 (d, *J*= 4.3 Hz, 1H), 2.44 (d, *J*= *6.9* Hz, 2H), 2.19 (dt, *J*= 13.2, 4.8 Hz, 1H), 1.99 - 1.80 (m, 3H), 1.22 (s, 8H), 1.14 (s, 6H).

**Example 10:** Synthesis of Compound **10**

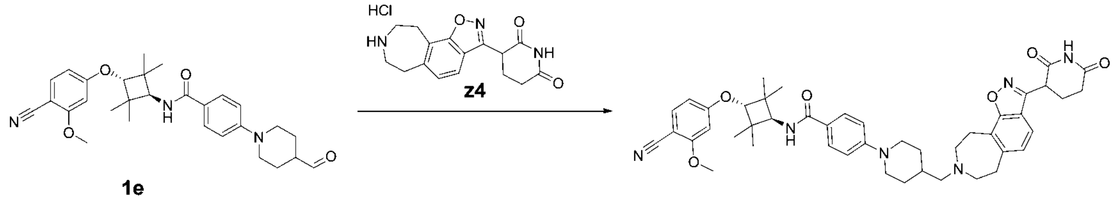


1e

10

Referring to the method in Example **8,** compound **10** (0.07 g) was synthesized with intermediate **z2** being replaced by intermediate **z4** (with reference to Example 5 of WO2024037616 for the synthetic route). MS(ESI, $[M+H]^+$) m/z: 773.42. $^1H$ NMR (500 MHz, DMSO) δ 11.07 (s, 1H), 7.74 (d, *J* = 8.8 Hz, 2H), 7.65 (d, *J* = 8.6 Hz, 1H), 7.54 (d, *J* = 7.9 Hz, 1H), 7.49 (d, *J*= *9.2* Hz, 1H), 7.18 (d, *J*= 8.1 Hz, 1H), 7.01 - 6.94 (m, 2H), 6.64 (d, *J*= 2.2 Hz, 1H), 6.54 (dd, *J*= 8.6, 2.2 Hz, 1H), 4.54 (dd, *J* = 11.9, 5.0 Hz, 1H), 4.27 (s, 1H), 4.05 (d, *J* = 9.2 Hz, 1H), 3.91 (s, 5H), 3.20 - 3.13 (m, 2H), 3.09 - 3.01 (m, 2H), 2.85

- 2.73 (m, 3H), 2.62 (ddt, *J* = 21.6, 17.5, 5.1 Hz, 5H), 2.47 (dd, *J* = 12.3, 4.4 Hz, 1H), 2.34 (d, *J* = 6.8 Hz, 2H), 2.18 (dq, *J* = 13.6, 4.1 Hz, 1H), 1.83 (t, *J* = 14.6 Hz, 3H), 1.23 (s, 8H), 1.15 (s, 6H).

The following compounds were synthesized with reference to the synthetic method for compounds **7** and **8:**

| Compound No. | Starting material | Compound structure | Data analysis |
|---|---|---|---|
| **11** | **3e,z2** | 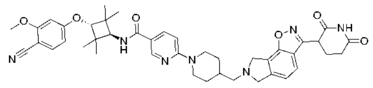 11 | MS (ESI) m/z $[M+H]^+$:746.39<br>$^1H$ NMR (500 MHz, DMSO) δ 11.09 (s, 1H), 8.62 (d, J = 2.5 Hz, 1H), 7.94 (dd, J = 9.0, 2.5 Hz, 1H), 7.71 (d, J = 8.0 Hz, 1H), 7.64 (d, J = 8.6 Hz, 1H), 7.59 (d, J = 9.2 Hz, 1H), 7.30 (d, J = 8.1 Hz, 1H), 6.87 (d, J = 9.1 Hz, 1H), 6.64 (d, J = 2.2 Hz, 1H), 6.54 (dd, J = 8.7, 2.2 Hz, 1H), 4.60 (dd, J = 11.9, 5.0 Hz, 1H), 4.44 (d, J = 13.0 Hz, 2H), 4.25 (s, 1H), 4.15 (s, 2H), 4.05 (d, J = 9.0 Hz, 3H), 3.91 (s, 3H), 3.00 - 2.91 (m, 2H), 2.77 (ddd, J = 17.1, 12.0, 5.3 Hz, 1H), 2.68 - 2.58 (m, 3H), 2.54 (d, J = 4.6 Hz, 1H), 2.20 (dq, J = 13.5, 3.8 Hz, 1H), 1.88 (d, J = 13.0 Hz, 3H), 1.23 (s, 6H), 1.14 (s, 8H). |
| **12** | **3e,z3** | 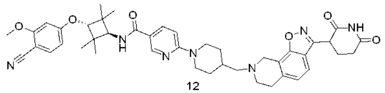 12 | MS (ESI) m/z $[M+H]^+$:760.4<br>$^1H$ NMR (500 MHz, DMSO) δ 11.08 (s, 1H), 8.62 (d, J = 2.5 Hz, 1H), 7.93 (dd, J = 9.0, 2.5 Hz, 1H), 7.64 (d, J = 8.6 Hz, 1H), 7.59 (dd, J = 8.8, 6.9 Hz, 2H), 7.15 (d, J = 8.2 Hz, 1H), 6.85 (d, J = 9.1 Hz, 1H), 6.64 (d, J = 2.2 Hz, 1H), 6.54 (dd, J = 8.7, 2.2 Hz, 1H), 4.56 (dd, J = 11.9, 5.0 Hz, 1H), 4.42 (d, J = 13.1 Hz, 2H), 4.25 (s, 1H), 4.05 (d, J = 9.2 Hz, 1H), 3.91 (s, 3H), 3.81 (s, 2H), 3.17 (d, J = 5.3 Hz, 1H), 2.95 (dt, J = 14.0, 8.1 Hz, 4H), 2.76 (p, J = 5.7 Hz, 3H), 2.65 - 2.57 (m, 1H), 2.48 (d, J = 4.2 Hz, 1H), 2.43 (d, J = 7.2 Hz, 2H), 2.22 - 2.16 (m, 1H), 1.83 (d, J = 12.6 Hz, 2H), 1.22 (s, 6H), 1.14 (s, 8H). |
| **13** | **3e,z4** | 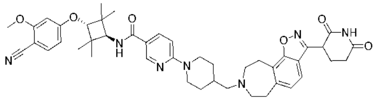 13 | MS (ESI) m/z $[M+H]^+$:774.3<br>$^1H$ NMR (500 MHz, DMSO) δ 11.07 (s, 1H), 8.63 (d, J = 2.5 Hz, 1H), 7.94 (dd, J = 9.0, 2.5 Hz, 1H), 7.65 (d, J = 8.6 Hz, 1H), 7.58 (d, J = 9.3 Hz, 1H), 7.54 (d, J = 8.0 Hz, 1H), 7.18 (d, J = 8.1 Hz, 1H), 6.86 (d, J = 9.1 Hz, 1H), 6.64 (d, J = 2.2 Hz, 1H), 6.54 (dd, J = 8.6, 2.2 Hz, 1H), 4.54 (dd, J = 11.9, 5.0 Hz, 1H), 4.43 (d, J = 13.0 Hz, 2H), 4.26 (s, 1H), 4.07 - 4.04 (m, 1H), 3.91 (s, 3H), 3.16 (d, J = 4.8 Hz, 2H), 3.05 (dd, J = 6.6, 3.4 Hz, 2H), 2.98 - 2.89 (m, 2H), 2.77 (ddd, J = 17.2, 11.9, 5.3 Hz, 1H), 2.63 (ddt, J = 21.4, 17.6, 4.8 Hz, 5H), 2.47 (dd, J = 12.3, 4.4 Hz, 1H), 2.33 (d, J = 6.8 Hz, 2H), 2.18 (dt, J = 13.2, 4.4 Hz, 1H), 1.85 (t, J = 15.7 Hz, 3H), 1.23 (s, 6H), 1.14 (s, 8H). |
| **14** | **7c,z5** | 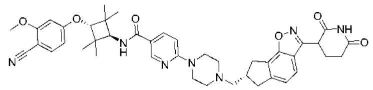 14 | MS (ESI) m/z $[M+H]^+$:746.2<br>$^1H$ NMR (500 MHz, DMSO) δ 11.08 (s, 1H), 8.64 (d, J = 2.5 Hz, 1H), 7.97 (dd, J = 9.0, 2.5 Hz, 1H), 7.67 - 7.59 (m, 3H), 7.28 (d, J = 8.1 Hz, 1H), 6.88 (d, J = 9.0 Hz, 1H), 6.64 (d, J = 2.2 Hz, 1H), 6.54 (dd, J = 8.6, 2.2 Hz, 1H), 4.57 (ddd, J = 11.9, 5.0, 1.9 Hz, 1H), 4.25 (s, 1H), 4.06 (d, J = 9.2 Hz, 1H), 3.91 (s, 3H), 3.64 (t, J = 5.0 Hz, 4H), 3.34 (s, 1H), 3.31 - 3.14 (m, 3H), 2.93 (d, J = 13.4 Hz, 2H), 2.86 (dd, J = 16.1, 5.1 Hz, 1H), 2.77 (ddd, J = 17.2, 11.8, 5.3 Hz, 1H), 2.67 - 2.58 (m, 1H), 2.52 (s, 2H), 2.47 (dd, J = 12.1, 4.3 Hz, 1H), 2.40 (d, J = 7.5 Hz, 2H), 2.19 (dq, J = 8.6, 4.4 Hz, 1H), 1.23 (s, 6H), 1.14 (s, 6H). |
| **15** | **4e,z2** | 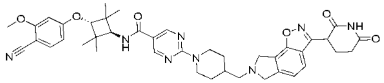 15 | MS (ESI) m/z $[M+H]^+$:747.39 |

(continued)

| Compound No. | Starting material | Compound structure | Data analysis |
|---|---|---|---|
| **16** | **4e,z3** | 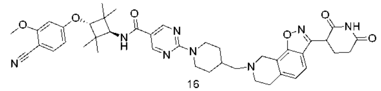 16 | MS (ESI) m/z [M+H]$^+$:761.43 |
| **17** | **4e,z4** | 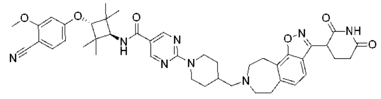 17 | MS (ESI) m/z [M+H]$^+$:775.41 |
| **18** | **7d,z5** | 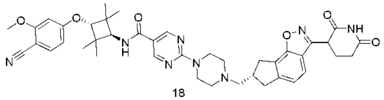 18 | MS (ESI) m/z [M+H]$^+$:747.37 |
| **19** | **5d,z2** | 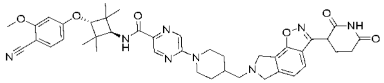 19 | MS (ESI) m/z [M+H]$^+$:747.46 |
| **20** | **5d,z3** | 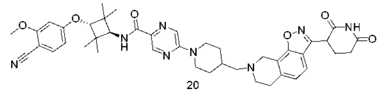 20 | MS (ESI) m/z [M+H]$^+$:761.45 |
| **21** | **5d,z4** | 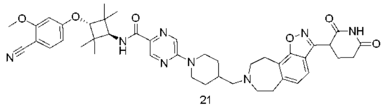 21 | MS (ESI) m/z [M+H]$^+$:775.3 |
| **22** | **7e,z5** | 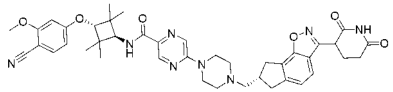 22 | MS (ESI) m/z [M+H]$^+$:747.3 |
| **23** | **6e,z2** | 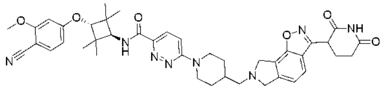 23 | MS (ESI) m/z [M+H]$^+$:747.3 |

(continued)

| Compound No. | Starting material | Compound structure | Data analysis |
|---|---|---|---|
| **24** | **6e,z3** | 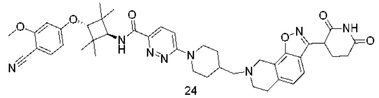 24 | MS (ESI) m/z $[M+H]^+$:761.4 |
| **25** | **6e,z4** | 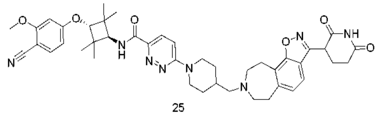 25 | MS (ESI) m/z $[M+H]^+$:775.3 |
| **26** | **7f,z5** | 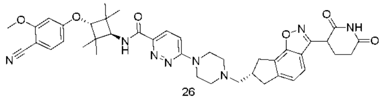 26 | MS (ESI) m/z $[M+H]^+$:747.3 |

**Example 27:** Synthesis of Compound **27**

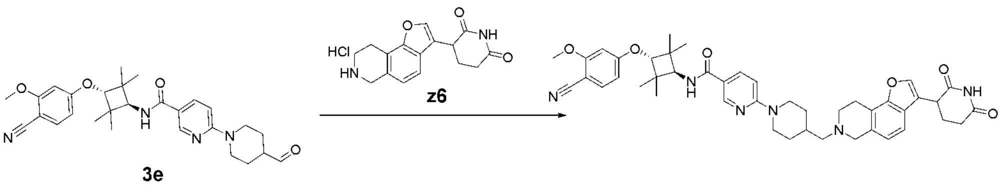

3e z6 27

Referring to the method in Example **8,** compound **27** (0.15 g) was synthesized with intermediate 1e being replaced by intermediate **3e** and intermediate **z2** being replaced by intermediate **z6** (with reference to Example 12 of WO2024037616 for the synthetic route).

MS(ESI, $[M+H]^+$) m/z: 759.33. $^1$H NMR (500 MHz, DMSO) $\delta$ 10.87 (s, 1H), 8.62 (d, *J* = 2.5 Hz, 1H), 7.93 (dd, *J* = 9.0, 2.5 Hz, 1H), 7.84 (s, 1H), 7.64 (d, *J*= 8.6 Hz, 1H), 7.58 (d, *J*= 9.3 Hz, 1H), 7.33 (d, *J* = 8.0 Hz, 1H), 6.96 (d, *J= 8.1* Hz, 1H), 6.85 (d, *J*= 9.0 Hz, 1H), 6.64 (d, *J* = 2.2 Hz, 1H), 6.54 (dd, *J* = 8.6, 2.2 Hz, 1H), 4.42 (d, *J*= 13.2 Hz, 2H), 4.25 (s, 1H), 4.10 (dd, *J* = 11.8, 4.9 Hz, 1H), 4.05 (d, *J* = 9.2 Hz, 1H), 3.91 (s, 3H), 3.65 (s, 2H), 3.05 - 2.87 (m, 4H), 2.74 (tt, *J* = 12.0, 5.8 Hz, 3H), 2.57 (dt, *J* = 17.3, 4.2 Hz, 1H), 2.41 - 2.23 (m, 3H), 2.11 (dq, *J* = 13.7, 4.8 Hz, 1H), 1.83 (d, *J*= 12.8 Hz, 2H), 1.18 (d, *J* = 41.6 Hz, 14H).

**Example 28:** Synthesis of Compound **28**

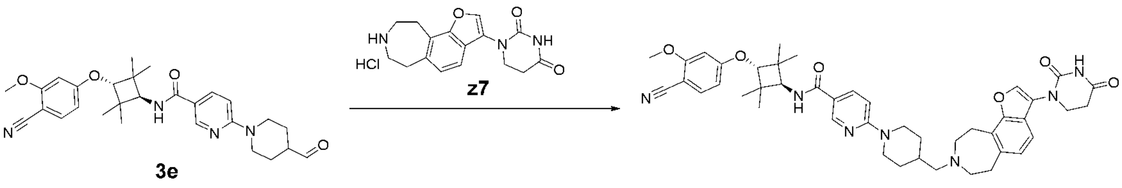

3e z7 28

Referring to the method in Example **8,** compound **28** (0.11 g) was synthesized with intermediate **1e** being replaced by intermediate **3e** and intermediate **z2** being replaced by intermediate **z7** (with reference to Example 75 of WO2024037616 for the synthetic route).

MS(ESI, $[M+H]^+$) m/z: 774.43. $^1$H NMR (500 MHz, DMSO) $\delta$ 10.50 (s, 1H), 8.63 (d, *J*= 2.5 Hz, 1H), 8.05 (s, 1H), 7.94 (dd, J= 9.0, 2.5 Hz, 1H), 7.64 (d, J= 8.6 Hz, 1H), 7.58 (d, *J* = 9.3 Hz, 1H), 7.29 (d, *J* = 7.8 Hz, 1H), 7.06 (d, J = 8.0 Hz, 1H), 6.86 (d, *J= 9.0* Hz, 1H), 6.64 (d, *J= 2.2* Hz, 1H), 6.54 (dd, J= 8.6, 2.2 Hz, 1H), 4.43 (d, *J*= 13.0 Hz, 2H), 4.25 (s, 1H), 4.05 (d, *J* = 9.5 Hz, 1H), 3.91 (s, 3H), 3.82 (t, *J* = 6.7 Hz, 2H), 3.12 (d, *J* = 6.1 Hz, 2H), 3.03 - 2.88 (m, 4H), 2.77 (t, *J*= 6.6 Hz, 2H), 2.69 - 2.56 (m, 4H), 2.32 (d, *J* = 6.6 Hz, 2H), 1.85 (t, *J*= 14.8 Hz, 3H), 1.23 (s, 6H), 1.14 (s, 6H), 1.13 - 1.05 (m, 2H).

Compounds 29-36 were prepared by referring to the synthetic procedures in Example 3 or those for compound 14 or those disclosed in the prior art:

| Compound | Data analysis |
|---|---|
| 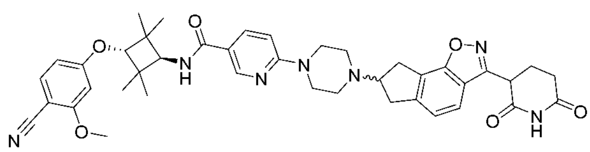 30<br>Prepared by using intermediate 7c and referring to the preparation process in Example 54 of WO2023088406 | MS (ESI) m/z $[M+H]^+$: 732.4 |

(continued)

| Compound | Data analysis |
|---|---|
| 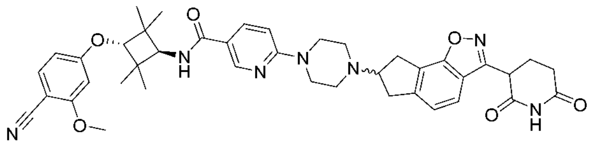 31 Reference is made to the preparation process in Example 55 of WO2023088406 | MS (ESI) m/z $[M+H]^+$: 732.3 |
| 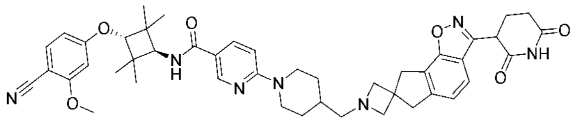 32 | MS (ESI) m/z $[M+H]^+$: 786.3 |
| 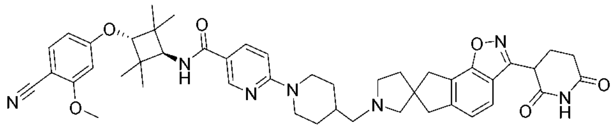 33 | MS (ESI) m/z $[M+H]^+$: 800.2 |
| 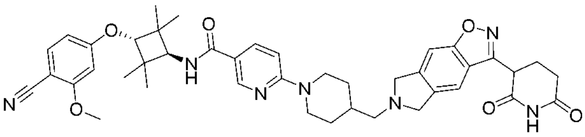 34 | MS (ESI) m/z $[M+H]^+$: 746.4 |
| 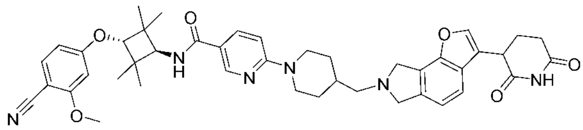 35 | MS (ESI) m/z $[M+H]^+$: 745.5 |
| 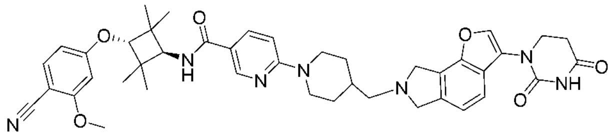 36 | MS (ESI) m/z $[M+H]^+$: 746.5 |

**Test Example 1: Effect of Compounds on AR Degradation in Cells**

1.1 Assay on degradation activity for AR in VCaP cells

The detection was carried out using the HTRF HUMAN ANDROGEN RECEPTOR DETECTION KIT (cisbio, 64ANDRPEG), 4× Supplemented Lysis buffer was prepared using the Blocking reagent stock solution (100×) and Lysis buffer stock solution (4×), and 1× Supplemented Lysis buffer was prepared using the 4× Supplemented Lysis buffer and $ddH_2O$. Premixed antibody solutions were prepared using Detection buffer, Androgen Receptor Eu Cryptate antibody, and Androgen Receptor d2 antibody.

VCaP cells in a good growth state were taken, washed with PBS, and digested with pancreatin, and a phenol red-free DMEM complete medium containing 1% CSS-FBS was added to terminate the digestion. The cells were collected into a centrifuge tube, adjusted to a cell density of $5 \times 10^5$ cells/mL, and seeded in a 96-well plate (100 μL/well). The cells were cultured overnight in a cell incubator. Compounds were added using a nanoliter pipettor and subjected to a 5-fold gradient dilution such that the final concentrations of the compounds were 100 nM-0.032 nM. 2 duplicate wells were set, and meanwhile, a control was set. After the cells were cultured in the cell incubator for another 24 h, the medium was pipetted out, the 1× Supplemented Lysis buffer was added at 40 μL/well to lyse the cells. After the cells were oscillated for 40 min at room temperature, 16 μL of the cell lysis buffer was added to a 384-well plate, the Premixed antibody solutions were added at 4 μL/well, and the mixture was left to stand at 25 °C for 2 h. Fluorescence values at 665 nm/620 nm were measured using an Envision microplate reader, the four-parameter analysis was performed, the dose-response curves were fit, and $DC_{50}$ and Dmax were calculated. The results are shown in Table 1.

Table 1. Degradation activity of compounds for AR in VCaP cells

| Example No. | $DC_{50}$ (nM) | Dmax | Example No. | $DC_{50}$ (nM) | Dmax |
|---|---|---|---|---|---|
| 1 | <5 | >75% | 8 | <5 | >75% |
| 3 | <5 | >75% | 10 | <5 | >75% |
| 4 | <5 | >75% | 11 | <5 | >75% |
| 5 | <5 | >75% | 13 | <5 | >75% |
| 6 | <5 | >75% | | | |

The test results show that the compounds of the present application (e.g., the example compounds) have degradation activity for AR in VCaP cells.

1.2 Assay on degradation activity for AR in LNCaP (T878A) cells

The detection was carried out using the HTRF HUMAN ANDROGEN RECEPTOR DETECTION KIT (cisbio, 64ANDRPEG), 4× Supplemented Lysis buffer was prepared using the Blocking reagent stock solution (100×) and Lysis buffer stock solution (4×), and 1× Supplemented Lysis buffer was prepared using the 4× Supplemented Lysis buffer and $ddH_2O$. Premixed antibody solutions were prepared using Detection buffer, Androgen Receptor Eu Cryptate antibody, and Androgen Receptor d2 antibody.

LNCaP cells in a good growth state were taken, washed with PBS, and digested with pancreatin, and a phenol red-free 1640 complete medium containing 2% CSS-FBS was added to terminate the digestion. The cells were collected into a centrifuge tube, adjusted to a cell density of $1 \times 10^6$ cells/mL, and seeded in a 96-well plate (100 μL/well). The cells were cultured overnight in a cell incubator. Compounds were added using a nanoliter pipettor and subjected to a 2-fold gradient dilution such that the final concentrations of the compounds were 500 nM-0.488 nM. 2 duplicate wells were set, and meanwhile, a control was set. After the cells were cultured in the cell incubator for another 24 h, the medium was pipetted out, the 1× Supplemented Lysis buffer was added at 40 μL/well to lyse the cells. After the cells were oscillated for 40 min at room temperature, 16 μL of the cell lysis buffer was added to a 384-well plate, the Premixed antibody solutions were added at 4 μL/well, and the mixture was left to stand at 25 °C for 2 h. Fluorescence values at 665 nm/620 nm were measured using an Envision microplate reader, the four-parameter analysis was performed, the dose-response curves were fit, and $DC_{50}$ and Dmax were calculated. The test results are shown in Table 2.

Table 2. Degradation activity of compounds for AR in LNCaP (T878A) cells

| Example No. | Dmax | Example No. | Dmax |
|---|---|---|---|
| 1 | >90% | 4 | >90% |
| 2 | >90% | 5 | >90% |
| 3 | >90% | 6 | >90% |

The test results show that the compounds of the present application (e.g., the example compounds) have degradation activity for AR in LNCaP cells.

1.3 Assay on degradation activity for AR in MDA-PCA-2B (L702H\T878A) cells

The detection was carried out using the HTRF HUMAN ANDROGEN RECEPTOR DETECTION KIT (cisbio, 64ANDRPEG), 4× Supplemented Lysis buffer was prepared using the Blocking reagent stock solution (100×) and Lysis buffer stock solution (4×), and 1× Supplemented Lysis buffer was prepared using the 4× Supplemented Lysis buffer and $ddH_2O$. Premixed antibody solutions were prepared using Detection buffer, Androgen Receptor Eu Cryptate antibody, and Androgen Receptor d2 antibody.

MDA-PCA-2B cells in a good growth state were taken, washed with PBS, and digested with pancreatin, and an MDA-PCA-2B complete medium was added to terminate the digestion. The cells were collected into a centrifuge tube, adjusted to a cell density of $1 \times 10^6$ cells/mL, and seeded in a 96-well plate (100 μL/well). The cells were cultured overnight in a cell incubator. Compounds were added using a nanoliter pipettor and subjected to a 5-fold gradient dilution such that the final concentrations of the compounds were 5000 nM-1.6 nM. 2 duplicate wells were set, and meanwhile, a control was set. After the cells were cultured in the cell incubator for another 24 h, the medium was pipetted out, the 1× Supplemented Lysis buffer was added at 40 μL/well to lyse the cells. After the cells were oscillated for 40 min at room temperature, 16 μL of

the cell lysis buffer was added to a 384-well plate, the Premixed antibody solutions were added at 4 μL/well, and the mixture was left to stand at 25 °C for 2 h. Fluorescence values at 665 nm/620 nm were measured using an Envision microplate reader, the four-parameter analysis was performed, the dose-response curves were fit, and $DC_{50}$ and Dmax were calculated. The results are shown in Table 3.

Table 3

| Example No. | $DC_{50}$ (nM) | Dmax | Example No. | $DC_{50}$ (nM) | Dmax |
|---|---|---|---|---|---|
| 1 | <500 | >70% | 9 | <500 | >70% |
| 3 | <500 | >70% | 10 | <500 | >70% |
| 4 | <500 | >70% | 11 | <500 | >70% |
| 5 | <500 | >70% | 12 | <500 | >70% |
| 6 | <500 | >70% | 13 | <500 | >70% |
| 8 | <500 | >70% | | | |

The test results show that the compounds of the present application (e.g., the example compounds) have degradation activity for AR in MDA-PCA-2B cells.

Test Example 2: **Inhibitory Activity for *In Vitro* Cell Proliferation**

2.1 Assay on inhibitory activity for VCaP cell proliferation

VCaP cells in a good growth state were taken, washed with PBS, and digested with pancreatin, and a phenol red-free DMEM complete medium containing 5% CSS-FBS was added to terminate the digestion. The cells were collected into a centrifuge tube, adjusted to a cell density of $1 \times 10^5$ cells/mL, and seeded in a 96-well plate (100 μL/well). Meanwhile, compounds were added using a nanoliter pipettor and subjected to a 2-fold gradient dilution such that the final concentrations of the compounds were 1000 nM-0.061 nM and 5000 nM-0.305 nM. 2 duplicate wells were set, and meanwhile, a control was set. After the cells were cultured in the cell incubator for another 168 h, the detection reagent CCK-8 (manufacturer: Dojindo, 10 μL/well) was added. After 7.0 h of incubation in the cell incubator, absorbance values at 450 nm were measured using a PerkinElmer Envision microplate reader, the four-parameter analysis was performed, the dose-response curves were fit, and $IC_{50}$ was calculated. The results are shown in Table 4.

Table 4. Inhibitory activity of compounds for LNCaP cell proliferation

| Example No. | $IC_{50}$ (μM) | Example No. | $IC_{50}$ (μM) |
|---|---|---|---|
| 1 | <2 | 10 | <2 |
| 2 | <2 | 11 | <2 |
| 3 | <2 | 13 | <2 |
| 4 | <2 | 14 | <2 |
| 5 | <2 | 28 | <2 |
| 7 | <2 | | |

The test results show that the compounds of the present application (e.g., the example compounds) have inhibitory activity for the proliferation of VCaP cells.

2.2 Assay on inhibitory activity for LNCaP (T878A) cell proliferation

LNCaP cells in a good growth state were taken, washed with PBS, and digested with pancreatin, and a phenol red-free 1640 complete medium containing 2% CSS-FBS was added to terminate the digestion. The cells were collected into a centrifuge tube, adjusted to a cell density of $1 \times 10^5$ cells/mL, and seeded in a 96-well plate (100 μL/well). Meanwhile, compounds were added using a nanoliter pipettor and subjected to a 2-fold gradient dilution such that the final concentrations of the compounds were 1000 nM-0.061 nM and 5000 nM-0.305 nM. 2 duplicate wells were set, and meanwhile, a control was set. After the cells were cultured in the cell incubator for another 168 h, the detection reagent

CCK-8 (manufacturer: Dojindo, 10 μL/well) was added. After 2.0 h of incubation in the cell incubator, absorbance values at 450 nm were measured using a PerkinElmer Envision microplate reader, the four-parameter analysis was performed, the dose-response curves were fit, and $IC_{50}$ was calculated. The test results are shown in Table 5.

Table 5. Inhibitory activity of compounds for LNCaP (T878A) cell proliferation

| Example No. | $IC_{50}$ (nM) | Example No. | $IC_{50}$ (nM) |
|---|---|---|---|
| 1 | <50 | 4 | <50 |
| 2 | <50 | 5 | <50 |
| 3 | <50 | 6 | <50 |

The test results show that the compounds of the present application (e.g., the example compounds) have inhibitory activity for the proliferation of LNCaP cells.

2.3 Assay on inhibitory activity for 22RV1 (H875Y/ARV7) cell proliferation

22RV1 cells in a good growth state were taken, washed with PBS, and digested with pancreatin, and a 1640 complete medium containing 5% FBS was added to terminate the digestion. The cells were collected into a centrifuge tube, adjusted to a cell density of $5 \times 10^4$ cells/mL, and seeded in a 96-well plate (100 μL/well). Meanwhile, compounds were added using a nanoliter pipettor and subjected to a 3-fold gradient dilution such that the final concentrations of the compounds were 5000 nM-2.286 nM. 2 duplicate wells were set, and meanwhile, a control was set. After the cells were cultured in the cell incubator for another 168 h, the assay reagent CellTiter-Glo (manufacturer: Promega, 50 μL/well) was added. After 10 min of incubation at room temperature, the mixture was transferred to a white plate at 100 μL/well. Luminescence values were measured using a PerkinElmer Envision multi-functional microplate reader, the four-parameter analysis was performed, the dose-response curves were fit, and $IC_{50}$ was calculated. The test results are shown in Table 6.

Table 6. Inhibitory activity of compounds for 22RV1 (H875Y/ARV7) cell proliferation

| Example No. | $IC_{50}$ (nM) | Example No. | $IC_{50}$ (nM) |
|---|---|---|---|
| 1 | <500 | 4 | <500 |
| 3 | <500 | 5 | <500 |

The test results show that the compounds of the present application (e.g., the example compounds) have inhibitory activity for the proliferation of 22RV1 cells.

2.4 Assay on inhibitory activity for MDA-PCA-2B (L702H\T878A) cell proliferation

MDA-PCA-2B cells in a good growth state were taken, washed with PBS, and digested with pancreatin, and an MDA-PCA-2B complete medium was added to terminate the digestion. The cells were collected into a centrifuge tube, adjusted to a cell density of $1 \times 10^5$ cells/mL, and seeded in a 96-well plate (100 μL/well). Meanwhile, compounds were added using a nanoliter pipettor and subjected to a 3-fold gradient dilution such that the final concentrations of the compounds were 5000 nM-2.286 nM. 2 duplicate wells were set, and meanwhile, a control was set. After the cells were cultured in the cell incubator for another 168 h, the detection reagent CCK-8 (manufacturer: Dojindo, 10 μL/well) was added. After 5.0 h of incubation in the cell incubator, absorbance values at 450 nm were measured using a PerkinElmer Envision microplate reader, the four-parameter analysis was performed, the dose-response curves were fit, and $IC_{50}$ was calculated. The test results are shown in Table 7.

Table 7. Inhibitory activity of compounds for MDA-PCA-2B (L702H\T878A) cell proliferation

| Example No. | $IC_{50}$ (nM) | Example No. | $IC_{50}$ (nM) |
|---|---|---|---|
| 1 | <500 | 4 | <500 |
| 3 | <500 | 5 | <500 |

The test results show that the compounds of the present application (e.g., the example compounds) have

inhibitory activity for the proliferation of MDA-PCA-2B cells.

**Test Example 3: *In Vitro* Liver Microsome Stability**

Liver microsome incubation samples were prepared by mixing a PBS buffer (pH 7.4), a liver microsome solution (0.5 mg/mL), a test compound, and an NADPH + $MgCl_2$ solution, followed by incubation at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted with a 1:1 (v:v) mixed solution of acetonitrile and water, and then assayed by LC/MS/MS. The results are shown in Tables 8 and 9.

Table 8

| Example No. | HLM (human) Remaining amount at 60 min % | Example No. | HLM (human) Remaining amount at 60 min % |
|---|---|---|---|
| 1 | >70% | 8 | >70% |
| 2 | >70% | 9 | >70% |
| 3 | >70% | 10 | >70% |
| 4 | >70% | 11 | >70% |
| 5 | >70% | 12 | >70% |

Table 9

| Example No. | MLM (mouse) Remaining amount at 60 min % | Example No. | MLM (mouse) Remaining amount at 60 min % |
|---|---|---|---|
| 1 | >70% | 6 | >70% |
| 2 | >70% | 8 | >70% |
| 3 | >70% | 10 | >70% |
| 4 | >70% | 11 | >70% |
| 5 | >70% | 13 | >70% |

The test results show that the compounds of the present application have *in vitro* liver microsome (species: human, dog, rat, or mouse) stability.

**Test Example 4: Mouse Pharmacokinetics**

ICR mice weighing 18-22 g were randomized into groups of 9 after 3-5 days of acclimatization. The mice in the intragastric administration group were intragastrically given the test compound solution at a dose of 10 mg/kg (20 mg/kg or 30 mg/kg), and the mice in the intravenous injection administration group were intravenously injected with the test compound solution at a dose of 1 mg/kg.

Blood collection time points for the intragastric administration group were 15 min, 0.5 h, 1 h, 2 h, 4 h, 6 h, 8 h, 10 h, 24 h, and 48 h after administration. Blood collection time points for the intravenous injection administration group were 0.083 min, 15 min, 0.5 h, 1 h, 2 h, 4 h, 6 h, 8 h, 10 h, 24 h, and 48 h after administration. Blood was collected from the orbit to prepare plasma samples to be tested.

30 μL of each of the plasma samples to be tested and a standard curve sample were taken, and an acetonitrile solution containing an internal standard was added. Supernatants were obtained by protein precipitation, diluted with a 1:1 (v:v) mixed solution of acetonitrile and water, and then assayed by LC/MS/MS. Data were fitted using a non-compartmental model. The test results are shown in Table 10.

Table 10. Mouse pharmacokinetic test results of compounds

| Example No. | Mouse i.v. (0-48 h), 1 mpk | | | | Mouse i.g. (0-48 h), 10 mpk | | | |
|---|---|---|---|---|---|---|---|---|
| | AUC (ng*h/mL) | $t_{1/2}$ (h) | CL (L/hr/kg) | $V_{ss}$ (L/kg) | AUC (ng*h/mL) | $t_{1/2}$ (h) | $C_{max}$ (ng/mL) | F |
| 1 | 8377 | 13.9 | 0.107 | 2.13 | 26795 | 24.9 | 786 | 31.99% |
| 4 | 7729 | 10.5 | 0.118 | 2.14 | 38962 | 11.7 | 1364 | 55.36% |
| 5 | 4345 | 9.91 | 0.21 | 3.79 | 22066 | 9.72 | 1013 | 50.78% |
| 8 | 20235 | 18 | 0.042 | 1.1 | 77641 | 15 | 2813 | 37.00% |
| 10 | 5405 | 13 | 0.17 | 3.1 | 16245 | 11 | 620 | 30.00% |
| 13 | 6813 | 9.7 | 0.14 | 2 | 27985 | 8.3 | 1392 | 41.00% |

The test results show that the compounds of the present application have good *in vivo* pharmacokinetic properties (e.g., AUC, $C_{max}$, $t_{1/2}$, F, and other parameters), for example, AUC > 15000 ng × h/mL, $C_{max}$ > 600 ng/mL, and $t_{1/2}$ > 8 h for intragastric administration (10 mpk).

**Test Example 5: Evaluation of Drug Effect of DPTC-AR on VCap Human Prostate Cancer B-NDG Mouse Subcutaneous Xenograft Tumor Model**

SPF-grade male B-NDG mice (source: Biocytogen) were inoculated subcutaneously at the right armpit with $5 \times 10^6$ VCap human prostate cancer cells (inoculated by mixing with Matrigel at a ratio of 1:1). When the mean tumor volume reached about 250 $mm^3$, the animals were grouped. The day of grouping was defined as day 0, and intragastric administration was performed once a day (1-10 mpk) from day 0. Meanwhile, the vehicle (DMSO + PEG40) was administered as a control group. The tumor volume was measured twice to thrice every week. Meanwhile, the mice were weighed, and the data were recorded. The general behavior of the mice was observed and recorded every day. After the experiment was completed, the tumors were removed, weighed, and photographed.

The detection index and the calculation formula are as follows:

$$\text{Tumor volume TV (mm}^3\text{)} = 1/2 \times (a \times b^2),$$

where a represents the long diameter of the tumor, and b represents the short diameter of the tumor.

$$\text{Relative tumor volume RTV} = TV_t/TV_0,$$

where $TV_0$ is the tumor volume on day 0, and $TV_t$ is the tumor volume at each measurement.

$$\text{Relative tumor proliferation rate T/C (\%)} = (T_{RTV}/C_{RTV}) \times 100\%,$$

where $T_{RTV}$ represents RTV of the treatment group, and $C_{RTV}$ represents RTV of the vehicle control group.

$$\text{Tumor growth inhibition rate TGI (\%)} = (1 - TW/TW0) \times 100\%,$$

where TW represents the tumor weight of the treatment group, and TW0 represents the tumor weight of the vehicle control group.

Weight change rate WCR (%) = $(Wtt - Wt_0)/Wt_0 \times 100\%$, where $Wt_0$ represents the mouse body weight on day 0, and Wtt represents the mouse body weight at each measurement.

The test results show that the example compounds of the present application have a good *in vivo* tumor inhibitory effect (e.g., TV, RTV, T/C (%), TGI (%), WCR (%), and other parameters). For example, at an administration dose of 3 mpk, on day 23, the relative tumor proliferation rate (T/C) was <40%, and the tumor growth inhibition rate TGI (%) was >66%.

## Claims

**1.** A compound of formula I, a stereoisomer thereof, or a pharmaceutically acceptable salt thereof,

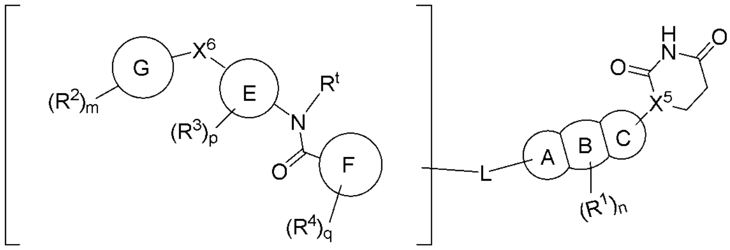

I

wherein

ring A is absent or selected from the group consisting of $C_{5-15}$ cycloalkenyl, 5- to 15-membered heterocycloalkenyl, phenyl, and 5- to 6-membered heteroaryl;
ring B is selected from the group consisting of phenyl and 5- to 6-membered heteroaryl;
ring C is selected from 5- to 6-membered heteroaryl;
each $R^1$ is independently selected from the group consisting of halogen, -OH, $-NH_2$, -CN, $C_{1-10}$ alkyl, $C_{1-10}$ alkoxy, and halogenated $C_{1-10}$ alkyl, wherein the -OH, $-NH_2$, $C_{1-10}$ alkyl, $C_{1-10}$ alkoxy, or halogenated $C_{1-10}$ alkyl is optionally substituted with one or more substituents;
n is selected from the group consisting of 0, 1, 2, and 3;
L is selected from a connecting group;
$X^5$ is selected from the group consisting of $C(R^f)$ and N;
$R^f$ is selected from the group consisting of H, halogen, deuterium, and $C_{1-6}$ alkyl optionally substituted with one or more substituents;
$X^6$ is selected from the group consisting of -O-, -NH-, and $-N(C_{1-6}$ alkyl)-, wherein the -NH- or $-N(C_{1-6}$ alkyl)- is optionally substituted with one or more substituents;
each $R^2$, each $R^3$, and each $R^4$ are independently selected from the group consisting of halogen, -OH, $-NH_2$, -CN, $C_{1-10}$ alkyl, $C_{1-10}$ alkoxy, and halogenated $C_{1-10}$ alkyl, wherein the -OH, $-NH_2$, $C_{1-10}$ alkyl, $C_{1-10}$ alkoxy, or halogenated $C_{1-10}$ alkyl is optionally substituted with one or more substituents;
m, p, and q are each independently selected from the group consisting of 0, 1, 2, 3, 4, 5, and 6;
ring G is selected from the group consisting of $C_{6-10}$ aryl and 5- to 10-membered heteroaryl;
ring E is selected from the group consisting of $C_{3-10}$ cycloalkyl and 3- to 10-membered heterocycloalkyl;
ring F is selected from the group consisting of $C_{6-10}$ aryl and 5- to 10-membered heteroaryl;
$R^t$ is selected from the group consisting of hydrogen, -OH, $C_{1-6}$ alkyl, $C_{3-10}$ cycloalkyl, and 3- to 10-membered heterocycloalkyl, wherein the $C_{1-6}$ alkyl, $C_{3-10}$ cycloalkyl, or 3- to 10-membered heterocycloalkyl is optionally substituted with one or more substituents.

**2.** The compound, the stereoisomer thereof, or the pharmaceutically acceptable salt thereof according to claim 1, wherein ring A is absent or selected from the group consisting of $C_{5-10}$ cycloalkenyl, 5- to 10-membered heterocycloalkenyl, phenyl, and 5- to 6-membered heteroaryl;

or, ring A is absent or selected from the group consisting of $C_{5-9}$ cycloalkenyl, 5- to 9-membered heterocycloalkenyl, phenyl, and 5- to 6-membered heteroaryl;
or, ring A is absent or selected from the group consisting of $C_{5-7}$ cycloalkenyl, 5- to 9-membered heterocycloalkenyl, phenyl, and 5- to 6-membered heteroaryl;
or, ring A is absent or selected from the group consisting of $C_{5-6}$ cycloalkenyl, 5- to 10-membered heterocycloalkenyl, phenyl, and 5- to 6-membered heteroaryl;
or, ring A is absent or selected from the group consisting of $C_{5-6}$ cycloalkenyl, 5- to 9-membered heterocycloalkenyl, phenyl, and 5- to 6-membered heteroaryl;
or, ring A is absent or selected from the group consisting of $C_{5-6}$ cycloalkenyl, 5- to 9-membered heterocycloalkenyl, phenyl, and 5-membered heteroaryl;
or, ring A is absent or selected from the group consisting of $C_{5-6}$ cycloalkenyl, 5- to 9-membered heterocycloalkenyl, phenyl, pyrrolyl, pyrazolyl, furanyl, and oxazolyl;
or, ring A is absent or selected from the group consisting of $C_5$ cycloalkenyl, $C_6$ cycloalkenyl, 5-membered heterocycloalkenyl, 6-membered heterocycloalkenyl, 7-membered heterocycloalkenyl, 8-membered heterocycloalkenyl, 9-membered heterocycloalkenyl, phenyl, pyrrolyl, pyrazolyl, furanyl, and oxazolyl;
or, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl,

pyrazolyl, furanyl, oxazolyl, and dihydrooxazinyl; and/or,
ring B is selected from the group consisting of phenyl and 6-membered heteroaryl;
or, ring B is selected from phenyl; and/or,
ring C is selected from 5-membered heteroaryl;
or, ring C is selected from the group consisting of isoxazolyl and furanyl.

**3.** The compound, the stereoisomer thereof, or the pharmaceutically acceptable salt thereof according to claim 1 or 2, wherein the structural fragment

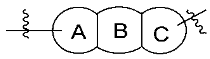

is selected from the group consisting of

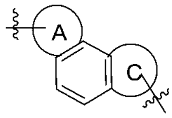

and

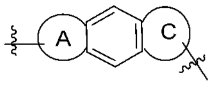;

or, the structural fragment

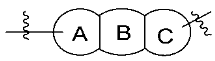

is selected from the group consisting of

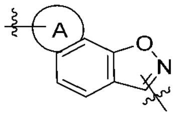,

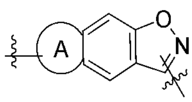, 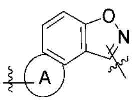, 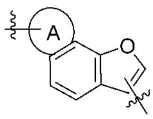, 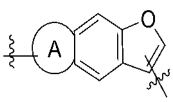, and 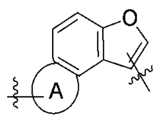;

or, the structural fragment

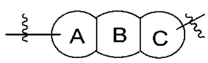

is selected from the group consisting of

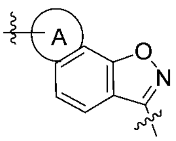,

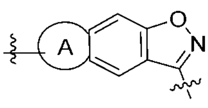, 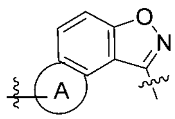, 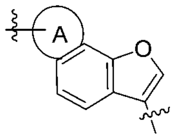, 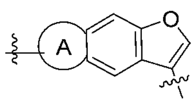, and 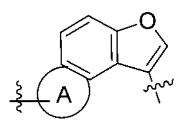;

or, the structural fragment

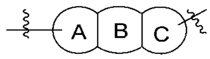

is selected from the group consisting of

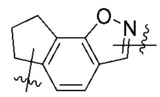,

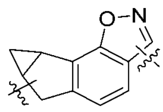, 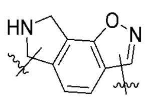, 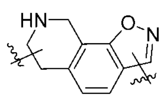, 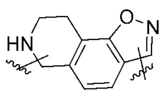, 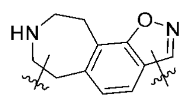, 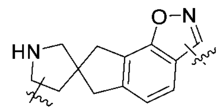,

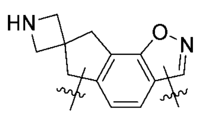, 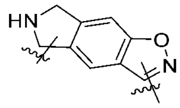, 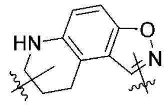, 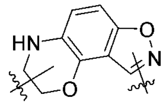, 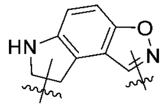, 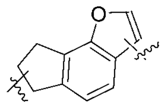,

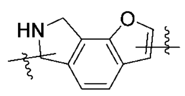, 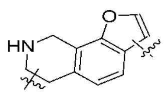, 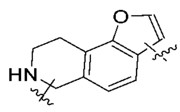, 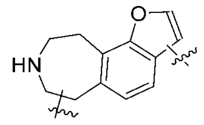, 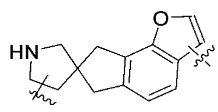, 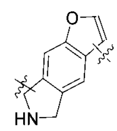,

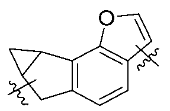, 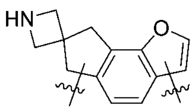, 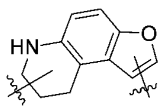, 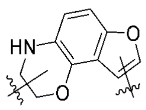, 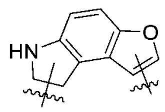, 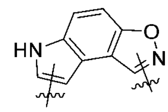,

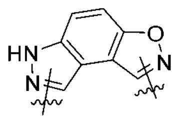, 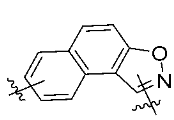, 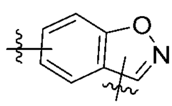, 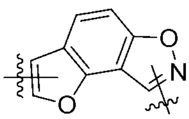, 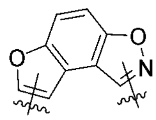, and 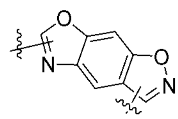;

or, the structural fragment

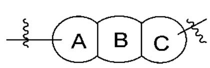

is selected from the group consisting of

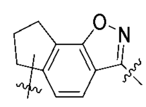,

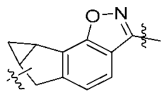, 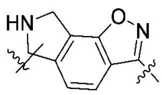, 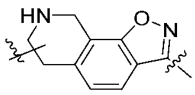, 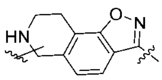, 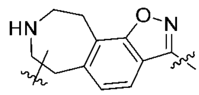,

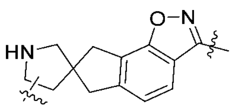, 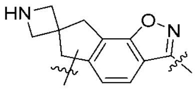, 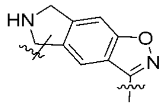, 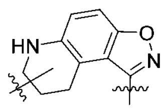, 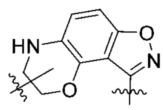, 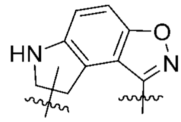,

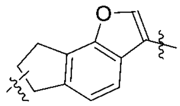, 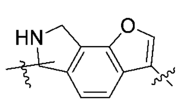, 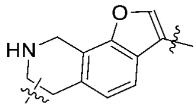, 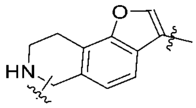, 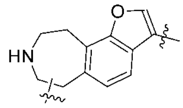,

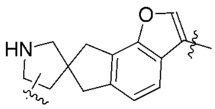, 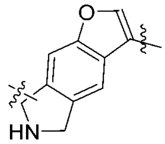, 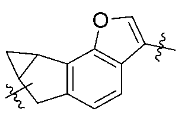, 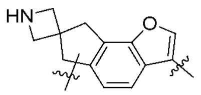, 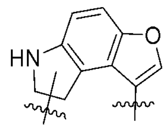, 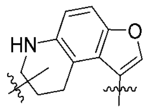,

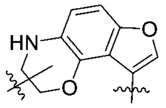, 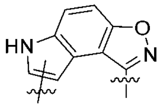, 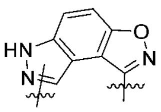, 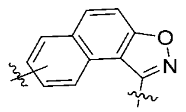, 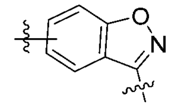, 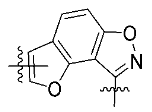,

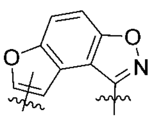, and 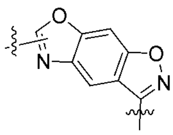;

or, the structural fragment

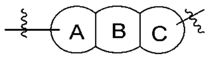

is selected from the group consisting of

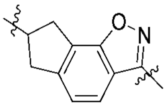,

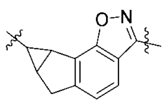, 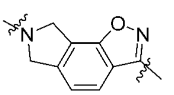, 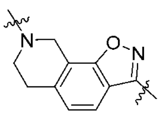, 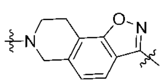, 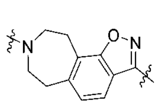, 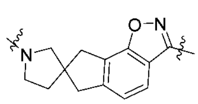,

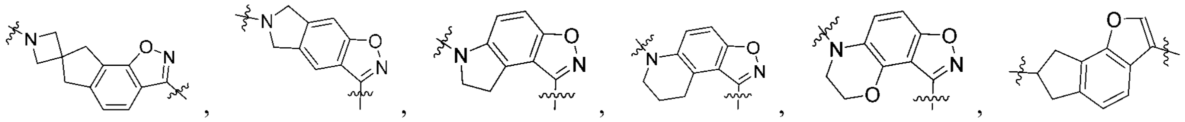, , , , , ,

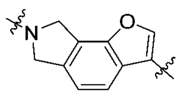, 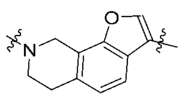, 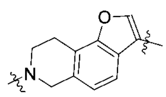, 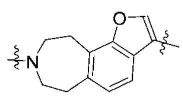, 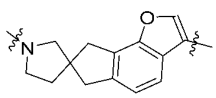, 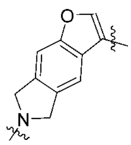,

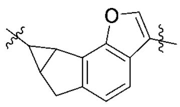, 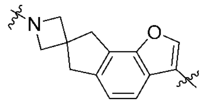, 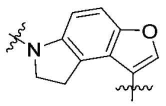, 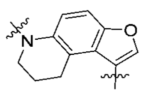, 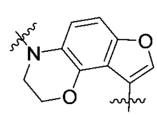, 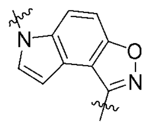,

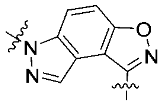, 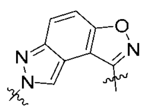, 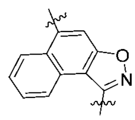, 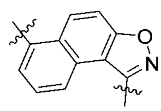, 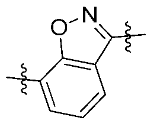, 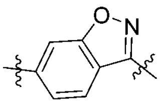, 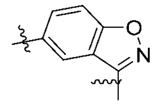,

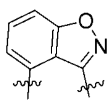, 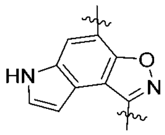, 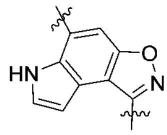, 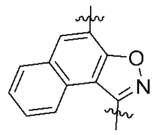, 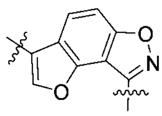, 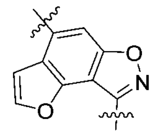, 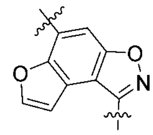,

and

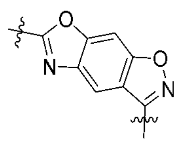.

4. The compound, the stereoisomer thereof, or the pharmaceutically acceptable salt thereof according to any one of claims 1-3, wherein each $R^1$ is independently selected from the group consisting of halogen, -OH, $-NH_2$, -CN, $C_{1-6}$ alkyl, $C_{1-6}$ alkoxy, and halogenated $C_{1-6}$ alkyl, wherein the -OH, $-NH_2$, $C_{1-6}$ alkyl, $C_{1-6}$ alkoxy, or halogenated $C_{1-6}$ alkyl is optionally substituted with one or more substituents;

or, each $R^1$ is independently selected from the group consisting of halogen, -OH, $-NH_2$, -CN, $C_{1-4}$ alkyl, $C_{1-4}$ alkoxy, and halogenated $C_{1-4}$ alkyl;
or, each $R^1$ is independently selected from the group consisting of halogen, -OH, $-NH_2$, -CN, $C_{1-3}$ alkyl, $C_{1-3}$ alkoxy, and halogenated $C_{1-3}$ alkyl;
or, each $R^1$ is independently selected from the group consisting of fluorine, chlorine, bromine, -OH, $-NH_2$, and -CN; or, each $R^1$ is independently selected from the group consisting of fluorine, chlorine, and bromine;
or, each $R^1$ is independently selected from fluorine; and/or,
n is selected from the group consisting of 0, 1, and 2;
or, n is selected from the group consisting of 0 and 1.

5. The compound, the stereoisomer thereof, or the pharmaceutically acceptable salt thereof according to any one of claims 1-4, wherein L is selected from the group consisting of the following groups optionally substituted with one or more substituents: $C_{1-50}$ alkylene, $C_{2-50}$ alkenylene, and $C_{2-50}$ alkynylene, wherein 1 or more $-CH_2-$ of the $C_{1-50}$ alkylene, $C_{2-50}$ alkenylene, or $C_{2-50}$ alkynylene are optionally replaced by -O-, $C_{3-15}$ cycloalkyl, 3- to 15-membered heterocycloalkyl, 4- to 15-membered heterocycloalkenyl, $C_{6-15}$ aryl, 5- to 15-membered heteroaryl, -NH-, $-N(C_{1-6}$ alkyl)-, or -S-;

or, L is selected from the group consisting of the following groups optionally substituted with one or more substituents: $C_{1-30}$ alkylene, $C_{2-30}$ alkenylene, and $C_{2-30}$ alkynylene, wherein 1 or more $-CH_2-$ of the $C_{1-30}$ alkylene, $C_{2-30}$ alkenylene, or $C_{2-30}$ alkynylene are optionally replaced by -O-, $C_{3-12}$ cycloalkyl, 3- to 12-membered heterocycloalkyl, 4- to 12-membered heterocycloalkenyl, $C_{6-12}$ aryl, 5- to 12-membered heteroaryl, -NH-, $-N(C_{1-6}$ alkyl)-, or -S-;

or, L is selected from the group consisting of the following groups optionally substituted with one or more substituents: $C_{1-20}$ alkylene, $C_{2-20}$ alkenylene, and $C_{2-20}$ alkynylene, wherein 1 or more $-CH_2-$ of the $C_{1-20}$ alkylene, $C_{2-20}$ alkenylene, or $C_{2-20}$ alkynylene are optionally replaced by -O-, $C_{3-10}$ cycloalkyl, 3- to 11-membered heterocycloalkyl, 4- to 10-membered heterocycloalkenyl, $C_{6-10}$ aryl, 5- to 10-membered heteroaryl, -NH-, -N($C_{1-6}$ alkyl)-, or -S-;

or, L is selected from the group consisting of the following groups optionally substituted with one or more substituents: $C_{1-15}$ alkylene, $C_{2-15}$ alkenylene, and $C_{2-15}$ alkynylene, wherein 1 or more $-CH_2-$ of the $C_{1-15}$ alkylene, $C_{2-15}$ alkenylene, or $C_{2-15}$ alkynylene are optionally replaced by -O-, $C_{3-9}$ cycloalkyl, 3- to 11-membered heterocycloalkyl, 4- to 8-membered heterocycloalkenyl, $C_{6-8}$ aryl, 5- to 8-membered heteroaryl, -NH-, -N($C_{1-4}$ alkyl)-, or -S-;

or, L is selected from the group consisting of the following groups optionally substituted with one or more substituents: $C_{1-10}$ alkylene, $C_{2-10}$ alkenylene, and $C_{2-10}$ alkynylene, wherein 1 or more $-CH_2-$ of the $C_{1-10}$ alkylene, $C_{2-10}$ alkenylene, or $C_{2-10}$ alkynylene are optionally replaced by -O-, $C_{3-9}$ cycloalkyl, 3- to 11-membered heterocycloalkyl, 4- to 6-membered heterocycloalkenyl, $C_6$ aryl, 5- to 6-membered heteroaryl, -NH-, -N($C_{1-3}$ alkyl)-, or -S-;

or, L is selected from the group consisting of the following groups optionally substituted with one or more substituents: $C_{1-6}$ alkylene, $C_{2-6}$ alkenylene, and $C_{2-6}$ alkynylene, wherein 1 or more $-CH_2-$ of the $C_{1-6}$ alkylene, $C_{2-6}$ alkenylene, or $C_{2-6}$ alkynylene are optionally replaced by -O-, $C_{3-9}$ cycloalkyl, 3- to 11-membered heterocycloalkyl, 4- to 6-membered heterocycloalkenyl, $C_6$ aryl, 5- to 6-membered heteroaryl, -NH-, -N($C_{1-3}$ alkyl)-, or -S-;

or, L is selected from the group consisting of the following groups optionally substituted with one or more substituents: $C_{1-4}$ alkylene, $C_{2-4}$ alkenylene, and $C_{2-4}$ alkynylene, wherein 1 or more (e.g., 1 or 2, 1 or 3, etc.) $-CH_2-$ of the $C_{1-4}$ alkylene, $C_{2-4}$ alkenylene, or $C_{2-4}$ alkynylene are optionally replaced by -O-, $C_{4-6}$ cycloalkyl, 4- to 6-membered heterocycloalkyl, 4- to 6-membered heterocycloalkenyl, $C_6$ aryl, 5- to 6-membered heteroaryl, -NH-, -N($C_{1-3}$ alkyl)-, or -S-; or, L is selected from $C_{1-6}$ alkylene, wherein 1 or more $-CH_2-$ of the $C_{1-6}$ alkylene are optionally replaced by a group selected from the group consisting of -O-, $C_{3-10}$ cycloalkyl, 4- to 11-membered heterocycloalkyl, 4- to 10-membered heterocycloalkenyl, -NH-, -N($C_{1-3}$ alkyl)-, and -S-, and the $C_{1-6}$ alkylene is optionally substituted with one or more substituents;

or, L is selected from $C_{1-6}$ alkylene, wherein 1 or more $-CH_2-$ of the $C_{1-6}$ alkylene are optionally replaced by a group selected from the group consisting of -O-, $C_{3-10}$ cycloalkyl, 4- to 11-membered heterocycloalkyl, 5- to 6-membered heterocycloalkenyl, -NH-, -N($C_{1-3}$ alkyl)-, and -S-, and the $C_{1-6}$ alkylene is optionally substituted with one or more substituents.

**6.** The compound, the stereoisomer thereof, or the pharmaceutically acceptable salt thereof according to any one of claims 1-4, wherein L is selected from $-LNK^1-Cy^1-LNK-Cy^2-LNK^2-Cy^3-$, wherein

$Cy^1$, $Cy^2$, or $Cy^3$ is each independently selected from the group consisting of a bond and the following groups optionally substituted with one or more $R^a$: $C_{3-12}$ cycloalkyl, 4- to 12-membered heterocycloalkyl, and 4- to 12-membered heterocycloalkenyl;

LNK, $LNK^1$, and $LNK^2$ are each independently selected from the group consisting of a bond, NH, O, S, and the following groups optionally substituted with one or more $R^b$: $C_{1-12}$ alkylene, $C_{2-12}$ alkenylene, $C_{2-12}$ alkynylene, and $C_{1-12}$ heteroalkylene;

each $R^a$ and each $R^b$ are independently selected from the group consisting of halogen, =O, -OH, $-NH_2$, -CN, $C_{1-6}$ alkyl, $C_{1-6}$ alkoxy, halogenated $C_{1-6}$ alkyl, $C_{1-6}$ alkylamino, di-$C_{1-6}$ alkylamino, $C_{3-12}$ cycloalkyl, and 4- to 12-membered heterocycloalkyl.

**7.** The compound, the stereoisomer thereof, or the pharmaceutically acceptable salt thereof according to claim 6, wherein LNK, $LNK^1$, and $LNK^2$ are each independently selected from the group consisting of a bond, NH, O, S, and the following groups optionally substituted with one or more $R^b$: $C_{1-10}$ alkylene, $C_{2-10}$ alkenylene, $C_{2-10}$ alkynylene, and $C_{1-10}$ heteroalkylene;

or, LNK, $LNK^1$, and $LNK^2$ are each independently selected from the group consisting of a bond, NH, O, S, and the following groups optionally substituted with one or more $R^b$: $C_{1-6}$ alkylene, $C_{2-6}$ alkenylene, $C_{2-6}$ alkynylene, and $C_{1-6}$ heteroalkylene;

or, LNK, $LNK^1$, and $LNK^2$ are each independently selected from the group consisting of a bond, NH, O, S, and the following groups optionally substituted with one or more $R^b$: $C_{1-4}$ alkylene, $C_{2-4}$ alkenylene, $C_{2-4}$ alkynylene, and $C_{1-4}$ heteroalkylene;

or, LNK, $LNK^1$, and $LNK^2$ are each independently selected from the group consisting of a bond, NH, O, S, and the

following groups optionally substituted with one or more $R^b$: $C_{1-6}$ alkylene and $C_{1-6}$ heteroalkylene;
or, LNK, $LNK^1$, and $LNK^2$ are each independently selected from the group consisting of a bond, NH, O, S, and the following groups optionally substituted with one or more $R^b$: $C_{1-4}$ alkylene and $C_{1-4}$ heteroalkylene;
or, LNK, $LNK^1$, and $LNK^2$ are each independently selected from the group consisting of a bond, NH, O, and the following groups optionally substituted with one or more $R^b$: $C_{1-3}$ alkylene and $C_{1-3}$ heteroalkylene;
or, LNK, $LNK^1$, and $LNK^2$ are each independently selected from the group consisting of a bond, NH, O, and the following groups optionally substituted with one or more $R^b$: $C_{1-2}$ alkylene and $C_{1-2}$ heteroalkylene;
or, LNK, $LNK^1$, and $LNK^2$ are each independently selected from the group consisting of a bond, NH, O, $-NHCH_2-$, $-CH_2NHCH_2-$, $-CH_2-$, $-CH_2CH_2-$, -C(O)-, and $-C(O)CH_2-$; and/or
$Cy^1$, $Cy^2$, or $Cy^3$ is each independently selected from the group consisting of a bond and the following groups optionally substituted with one or more $R^a$: $C_{3-11}$ cycloalkyl, 4- to 12-membered heterocycloalkyl, and 4- to 11-membered heterocycloalkenyl;
or, $Cy^1$, $Cy^2$, or $Cy^3$ is each independently selected from the group consisting of a bond and the following groups optionally substituted with one or more $R^a$: $C_{4-10}$ cycloalkyl, 4- to 11-membered heterocycloalkyl, and 5- to 6-membered heterocycloalkenyl;
or, $Cy^1$, $Cy^2$, or $Cy^3$ is each independently selected from the group consisting of a bond and the following groups optionally substituted with one or more $R^a$: $C_{4-9}$ cycloalkyl, 4- to 11-membered heterocycloalkyl, and 5- to 6-membered heterocycloalkenyl;
or, $Cy^1$, $Cy^2$, or $Cy^3$ is each independently selected from the group consisting of a bond and the following groups optionally substituted with one or more $R^a$: $C_{4-6}$ cycloalkyl, $C_9$ cycloalkyl, 4- to 11-membered heterocycloalkyl, and 6-membered heterocycloalkenyl;
or, $Cy^1$, $Cy^2$, or $Cy^3$ is each independently selected from the group consisting of a bond and the following groups optionally substituted with one or more $R^a$: cyclobutyl, cyclopentyl, cyclohexyl, spirononanyl, azetidinyl, pyrrolidinyl, piperidinyl, tetrahydropyridinyl, piperazinyl, monoazaspiroheptyl, monoazaspirononanyl, diazaspirononanyl, monoazaspirodecyl, diazaspirodecyl, monoazaspiroundecanyl, diazaspiroundecanyl, octahydrocyclopentapyrrolyl, diazabicyclooctyl, and monoazabicyclononanyl.

**8.** The compound, the stereoisomer thereof, or the pharmaceutically acceptable salt thereof according to claim 6 or 7, wherein each $R^a$ and each $R^b$ are independently selected from the group consisting of halogen, =O, -OH, $-NH_2$, - CN, $C_{1-6}$ alkyl, $C_{1-6}$ alkoxy, halogenated $C_{1-6}$ alkyl, $C_{1-6}$ alkylamino, di-$C_{1-6}$ alkylamino, $C_{3-10}$ cycloalkyl, and 4- to 10-membered heterocycloalkyl;

or, each $R^a$ and each $R^b$ are independently selected from the group consisting of halogen, =O, OH, $NH_2$, CN, $C_{1-6}$ alkyl, $C_{1-6}$ alkoxy, halogenated $C_{1-6}$ alkyl, $C_{1-6}$ alkylamino, and di-$C_{1-6}$ alkylamino;
or, each $R^a$ and each $R^b$ are independently selected from the group consisting of halogen, =O, -OH, $-NH_2$, -CN, $C_{1-4}$ alkyl, $C_{1-4}$ alkoxy, halogenated $C_{1-4}$ alkyl, $C_{1-4}$ alkylamino, and di-$C_{1-4}$ alkylamino;
or, each $R^a$ and each $R^b$ are independently selected from the group consisting of halogen, =O, -OH, $-NH_2$, -CN, and $C_{1-3}$ alkyl;
or, each $R^a$ and each $R^b$ are independently selected from the group consisting of halogen, =O, -OH, $-NH_2$, and -CN; or, each $R^a$ and each $R^b$ are independently selected from =O.

**9.** The compound, the stereoisomer thereof, or the pharmaceutically acceptable salt thereof according to any one of claims 1-8, wherein L is selected from the group consisting of a bond, $-NHCH_2-$, $-CH_2NHCH_2-$, $-CH_2-$, $-C(O)CH_2-$,

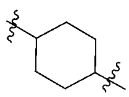, 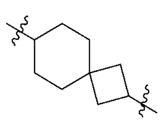, 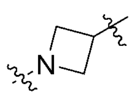, 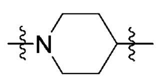, 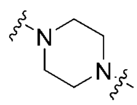, 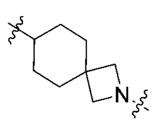, 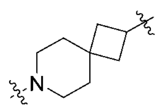,

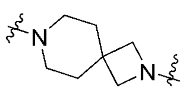, 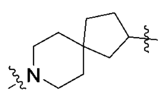, 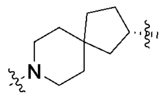, 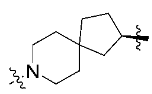, 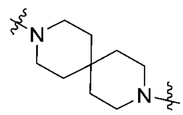, 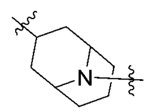,

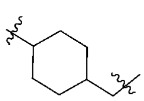, 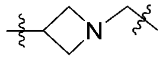, 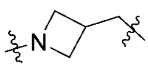, 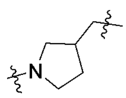, 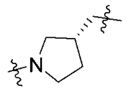, 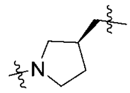, 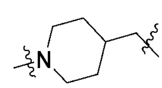,

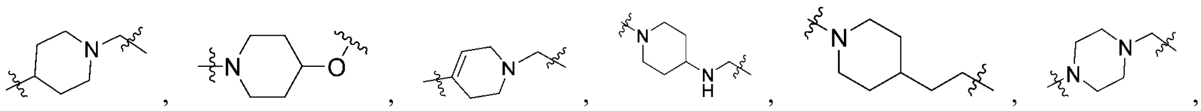
, , , , , ,

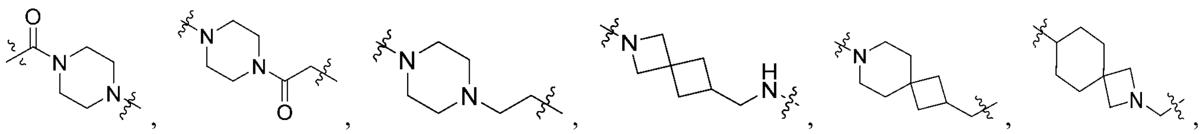
, , , , , ,

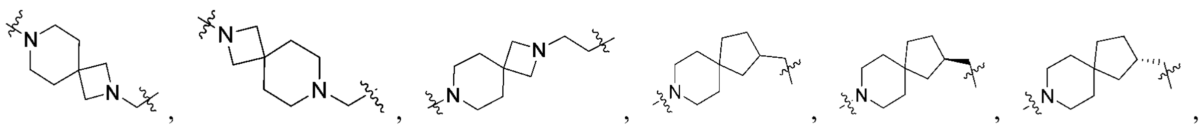
, , , , , ,

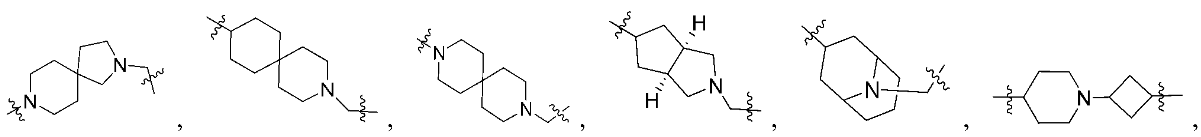
, , , , , ,

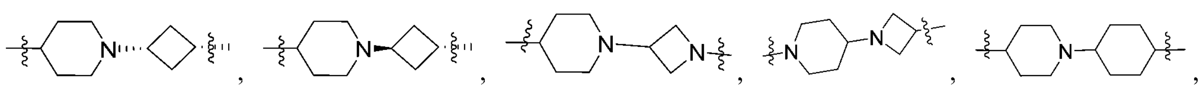
, , , , ,

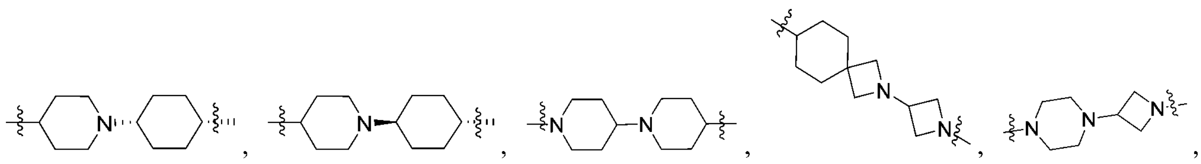
, , , , ,

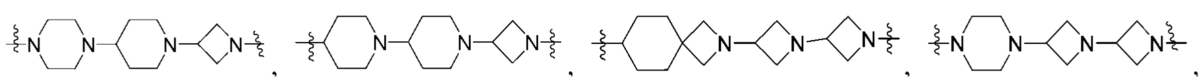
, , , ,

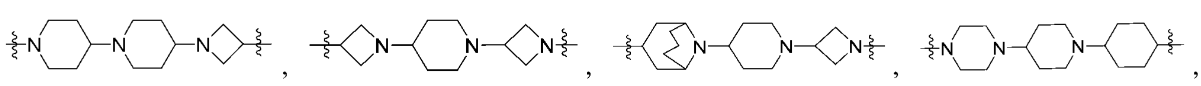
, , , ,

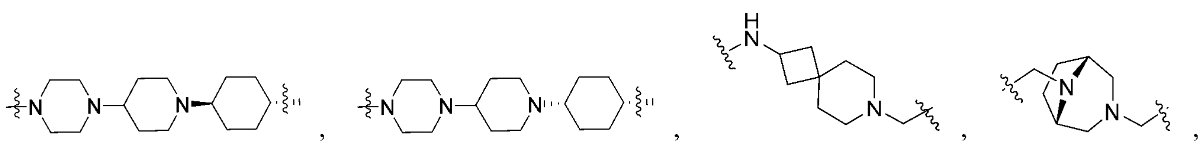
, , , ,

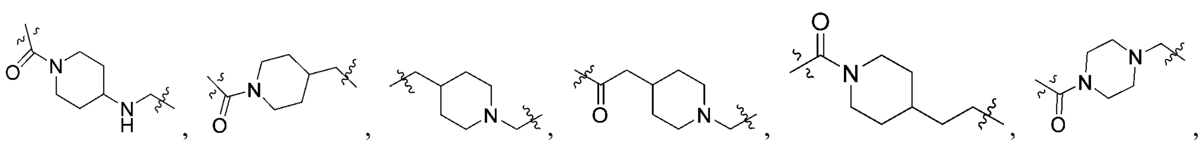
, , , , , ,

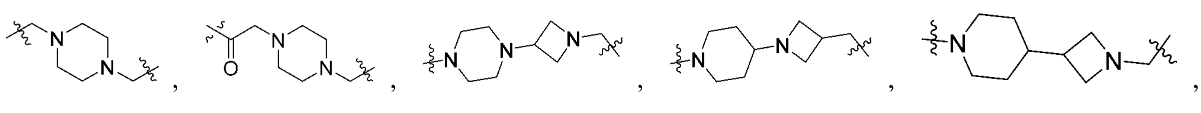
, , , , ,

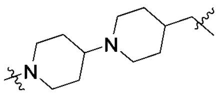, 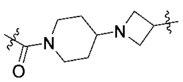, 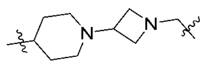, 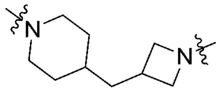, 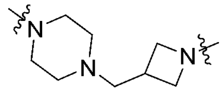,

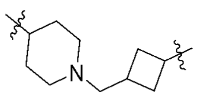, 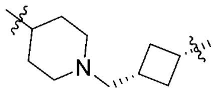, 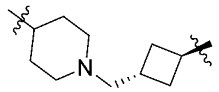, 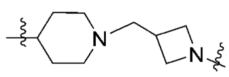, 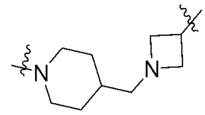,

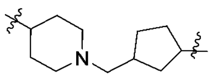, 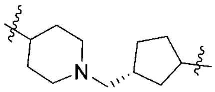, 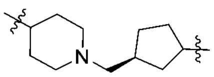, 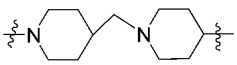, 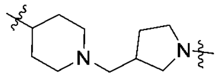,

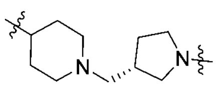, 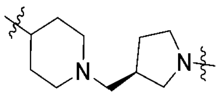, 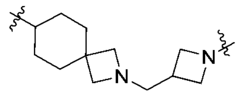, 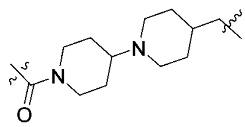,

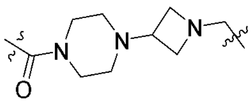, 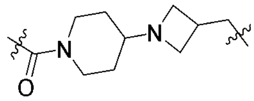, 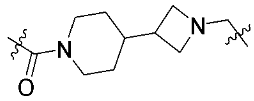, and 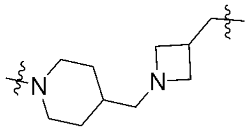.

**10.** The compound, the stereoisomer thereof, or the pharmaceutically acceptable salt thereof according to any one of claims 1-9, wherein $R^f$ is selected from the group consisting of H, fluorine, chlorine, bromine, deuterium, and $C_{1-3}$ alkyl optionally substituted with one or more substituents;

or, $R^f$ is selected from the group consisting of H, fluorine, chlorine, bromine, deuterium, and $C_{1-3}$ alkyl, wherein the $C_{1-3}$ alkyl is optionally substituted with one or more of the following groups: halogen, -OH, $-NH_2$, and -CN;
or, $R^f$ is selected from the group consisting of H, fluorine, chlorine, bromine, deuterium, and $C_{1-3}$ alkyl;
or, $R^f$ is selected from the group consisting of H, fluorine, deuterium, and methyl; and/or $X^5$ is selected from the group consisting of CH and N; and/or
$X^6$ is selected from the group consisting of -O-, -NH-, and $-N(C_{1-3}$ alkyl)-; or, $X^6$ is selected from the group consisting of -O- and $-N(CH_3)-$; or, $X^6$ is selected from -O-.

**11.** The compound, the stereoisomer thereof, or the pharmaceutically acceptable salt thereof according to any one of claims 1-10, wherein each $R^2$, each $R^3$, and each $R^4$ are independently selected from the group consisting of halogen, -OH, $-NH_2$, -CN, $C_{1-6}$ alkyl, $C_{1-6}$ alkoxy, and halogenated $C_{1-6}$ alkyl, wherein the -OH, $-NH_2$, -CN, $C_{1-6}$ alkyl, $C_{1-6}$ alkoxy, or halogenated $C_{1-6}$ alkyl is optionally substituted with one or more substituents;

or, each $R^2$, each $R^3$, and each $R^4$ are independently selected from the group consisting of halogen, -OH, $-NH_2$, -CN, $C_{1-4}$ alkyl, $C_{1-4}$ alkoxy, and halogenated $C_{1-4}$ alkyl;
or, each $R^2$ is independently selected from the group consisting of halogen, -OH, $-NH_2$, -CN, $C_{1-3}$ alkyl, $C_{1-3}$ alkoxy, and halogenated $C_{1-3}$ alkyl;
or, each $R^2$ is independently selected from the group consisting of halogen, -OH, $-NH_2$, -CN, $C_{1-3}$ alkyl, and $C_{1-3}$ alkoxy;
or, each $R^2$ is independently selected from the group consisting of fluorine, chlorine, bromine, -OH, $-NH_2$, -CN, methyl, and methoxy;
or, each $R^2$ is independently selected from the group consisting of fluorine, chlorine, bromine, -CN, and methoxy;
or, each $R^2$ is independently selected from the group consisting of -CN and methoxy; and/or
each $R^3$ is independently selected from the group consisting of halogen, -OH, $-NH_2$, -CN, $C_{1-3}$ alkyl, $C_{1-3}$ alkoxy, and halogenated $C_{1-3}$ alkyl;
or, each $R^3$ is independently selected from the group consisting of halogen, -OH, $-NH_2$, -CN, and $C_{1-3}$ alkyl;
or, each $R^3$ is independently selected from $C_{1-3}$ alkyl;
or, each $R^3$ is independently selected from methyl; and/or

each $R^4$ is independently selected from the group consisting of halogen, -OH, $-NH_2$, -CN, $C_{1-3}$ alkyl, $C_{1-3}$ alkoxy, and halogenated $C_{1-3}$ alkyl;
or, each $R^4$ is independently selected from the group consisting of halogen, -OH, $-NH_2$, -CN, and $C_{1-3}$ alkyl;
or, each $R^4$ is independently selected from the group consisting of fluorine, chlorine, bromine, -OH, $-NH_2$, and -CN.

**12.** The compound, the stereoisomer thereof, or the pharmaceutically acceptable salt thereof according to any one of claims 1-11, wherein m is selected from the group consisting of 0, 1, 2, and 3; or, m is selected from the group consisting of 1, 2, and 3; or, m is selected from 2; and/or

p is selected from the group consisting of 1, 2, 3, and 4; or, p is selected from the group consisting of 3 and 4; or, p is selected from 4; and/or
q is selected from the group consisting of 0, 1, 2, and 3; or, q is selected from the group consisting of 0 and 1; or, q is selected from 0.

**13.** The compound, the stereoisomer thereof, or the pharmaceutically acceptable salt thereof according to any one of claims 1-12, wherein ring G is selected from the group consisting of phenyl and 5- to 6-membered heteroaryl; or, ring G is selected from the group consisting of phenyl and 6-membered heteroaryl; or, ring G is phenyl.

**14.** The compound, the stereoisomer thereof, or the pharmaceutically acceptable salt thereof according to any one of claims 1-13, wherein ring E is selected from the group consisting of $C_{3-9}$ cycloalkyl and 3- to 9-membered heterocycloalkyl; or, ring E is selected from the group consisting of $C_{4-9}$ cycloalkyl and 4- to 9-membered heterocycloalkyl; or, ring E is selected from the group consisting of $C_{4-7}$ cycloalkyl and 4- to 7-membered heterocycloalkyl; or, ring E is selected from $C_{4-7}$ cycloalkyl;

or, ring E is selected from the group consisting of cyclobutyl, cyclopentyl, and cyclohexyl;
or, ring E is cyclobutyl; or, ring E is

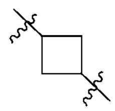,

or ring E is

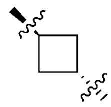.

**15.** The compound, the stereoisomer thereof, or the pharmaceutically acceptable salt thereof according to any one of claims 1-14, wherein ring F is selected from the group consisting of $C_{6-10}$ aryl and 5- to 7-membered heteroaryl; or, ring F is selected from the group consisting of phenyl and 5- to 6-membered heteroaryl; or, ring F is selected from the group consisting of phenyl and 6-membered heteroaryl; or, ring F is selected from the group consisting of phenyl, pyridinyl, pyridazinyl, pyrimidinyl, and pyrazinyl; or, ring F is selected from phenyl; or, ring F is selected from the group consisting of

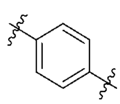, 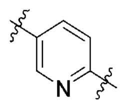, 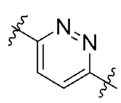, 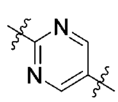, and 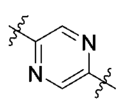.

**16.** The compound, the stereoisomer thereof, or the pharmaceutically acceptable salt thereof according to any one of claims 1-15, wherein $R^t$ is selected from the group consisting of hydrogen, -OH, $C_{1-4}$ alkyl, $C_{3-6}$ cycloalkyl, and 3-to 6-membered heterocycloalkyl, wherein the $C_{1-4}$ alkyl, $C_{3-6}$ cycloalkyl, or 3- to 6-membered heterocycloalkyl is optionally substituted; or, $R^t$ is selected from the group consisting of hydrogen, -OH, $C_{1-3}$ alkyl, $C_{3-4}$ cycloalkyl, and 3- to 4-membered heterocycloalkyl, wherein the $C_{1-3}$ alkyl, $C_{3-4}$ cycloalkyl, or 3-to 4-membered heterocycloalkyl is optionally substituted; or, $R^t$ is selected from the group consisting of hydrogen and $C_{1-3}$ alkyl (e.g., methyl, ethyl, or propyl); or, $R^t$

is selected from hydrogen.

**17.** The compound, the stereoisomer thereof, or the pharmaceutically acceptable salt thereof according to any one of claims 1-16, wherein the structural fragment

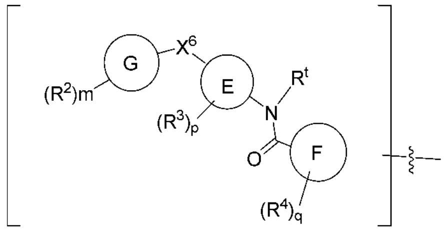

is selected from

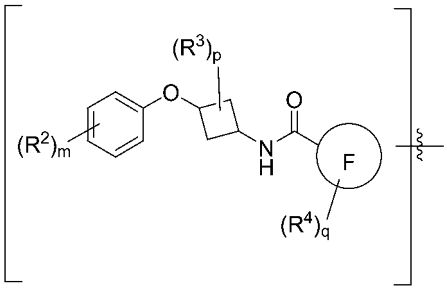

;

or, the structural fragment

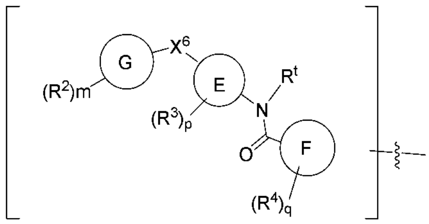

is selected from

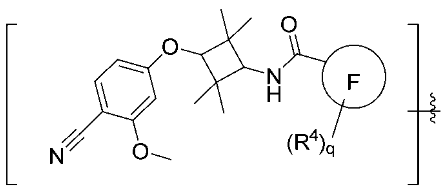

; or, the structural fragment

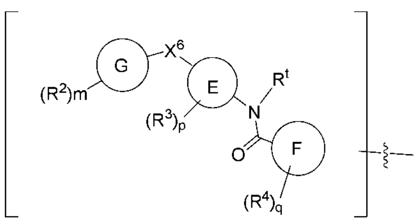

is selected from

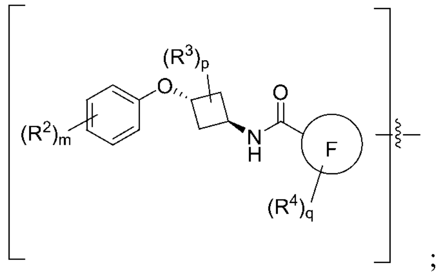

or, the structural fragment

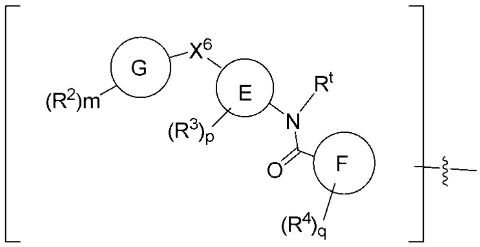

is selected from

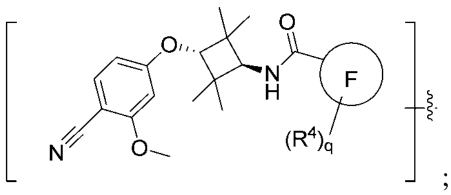

or, the structural fragment

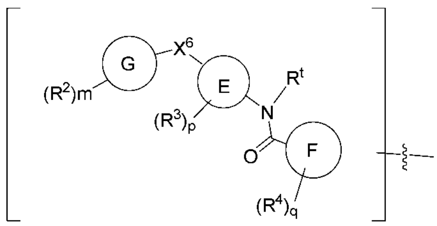

is selected from

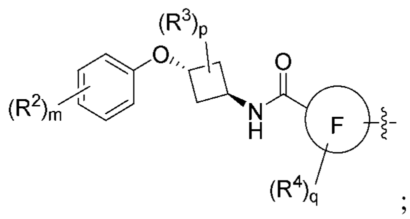

or, the structural fragment

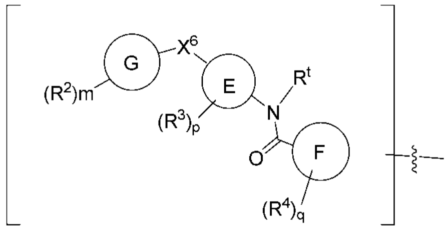

is selected from

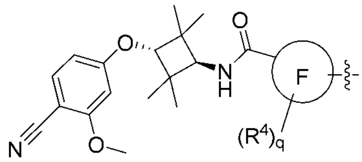

;

or, the structural fragment

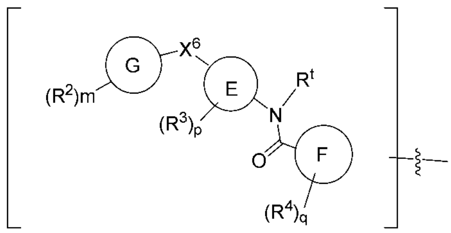

is selected from the group consisting of

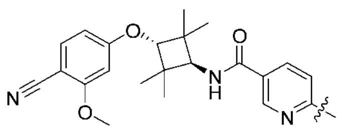

,

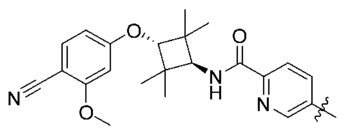

,

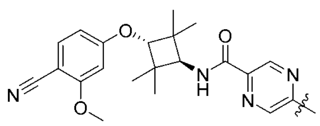

,

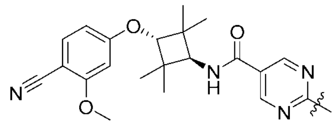

,

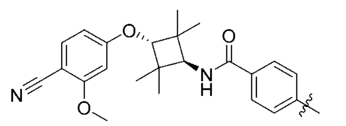

,

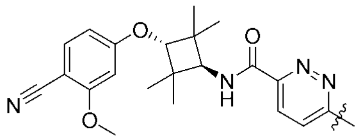

, and

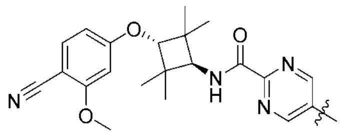

.

**18.** The compound, the stereoisomer thereof, or the pharmaceutically acceptable salt thereof according to any one of claims 1-17, wherein the compound, the stereoisomer thereof, or the pharmaceutically acceptable salt thereof is a compound of formula I-1 or I-1A, formula I-2, formula I-2A, formula I-3, or formula I-3A, a stereoisomer thereof, or a pharmaceutically acceptable salt thereof,

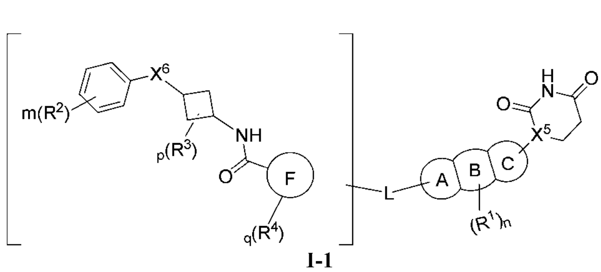

I-1

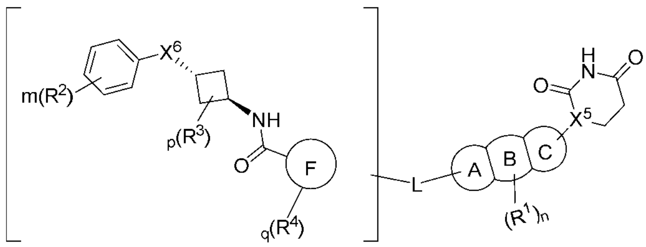

I-1A

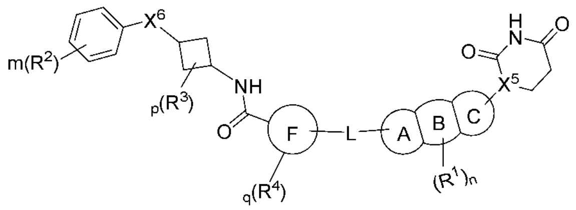

I-2

or

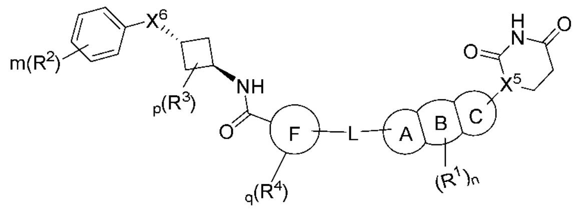

I-2A

,

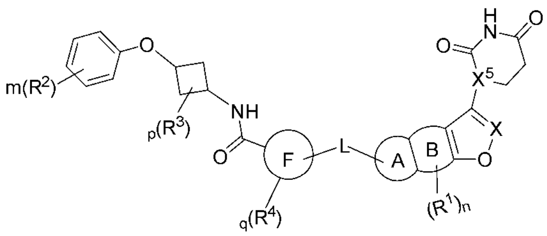

I-3

, or

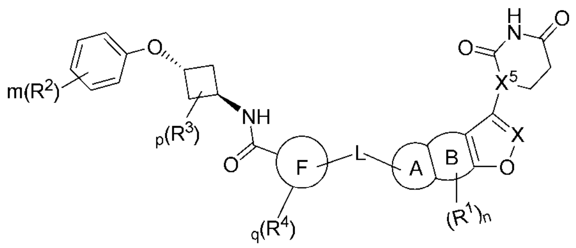

I-3A

,

X is selected from the group consisting of CH and N.

**19.** The compound, the stereoisomer thereof, or the pharmaceutically acceptable salt thereof according to any one of claims 1-18, wherein ring A is $C_{5-10}$ cycloalkenyl or 5- to 15-membered heterocycloalkenyl containing 1-2 heteroatoms selected from the group consisting of N and O; ring B is phenyl; ring C is 5- to 6-membered heteroaryl containing 1-2 heteroatoms selected from the group consisting of N and O; n is 0; $X^5$ is selected from $C(R^f)$, and $R^f$ is selected from the group consisting of H, deuterium, and $C_{1-6}$ alkyl;

L is $C_{1-10}$ alkylene, wherein 1 or more $-CH_2-$ in the $C_{1-10}$ alkylene are optionally replaced by $C_{5-8}$ cycloalkyl or 5- to 8-membered heterocycloalkyl containing 1-3 or 1-2 N atoms or O atoms;
each $R^2$ is independently -OH, $-NH_2$, -CN, $C_{1-10}$ alkyl, $C_{1-10}$ alkoxy, or halogenated $C_{1-10}$ alkyl; each $R^3$ is independently halogen, $C_{1-10}$ alkyl, or halogenated $C_{1-10}$ alkyl; q is 0, m is 0, 1, 2, or 3, and p is 0, 1, 2, 3, or 4; $X^6$ is -O- or -NH-; ring G is phenyl or 6-membered heteroaryl containing 1-2 N atoms or O atoms; ring E is $C_{3-6}$ cycloalkyl; ring F is phenyl or 6-membered heteroaryl containing 1-3 or 1-2 N atoms or O atoms; $R^t$ is hydrogen, -OH, or $C_{1-6}$ alkyl.

**20.** A compound of formula I' or formula I", a moiety, a stereoisomer thereof, a derivative, or a pharmaceutically acceptable salt thereof,

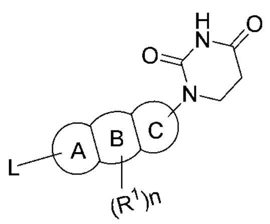

I'

or

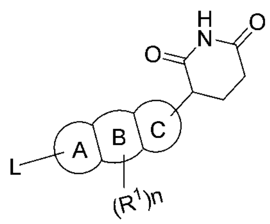

I"

,

wherein ring A is absent or selected from the group consisting of $C_{5-10}$ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
L is selected from a connecting group.

**21.** A compound of formula I'-a or I"-a, a moiety, a stereoisomer thereof, a derivative, or a pharmaceutically acceptable salt thereof,

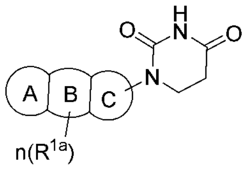

I'-a

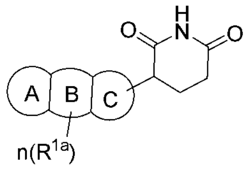

I"-a

wherein ring A is absent or selected from the group consisting of $C_{5-10}$ cycloalkenyl, 5- to 10-membered heterocycloalkenyl, phenyl, and 5- to 6-membered heteroaryl;
ring B is selected from phenyl;
ring C is selected from the group consisting of isoxazolyl and furanyl;
each $R^{1a}$ is independently selected from the group consisting of halogen, -OH, $-NH_2$, -CN, =O, -CHO, $C_{1-4}$ alkyl, $C_{1-4}$ alkoxy, $C_{1-6}$ alkyl OC(O)-, $C_{3-12}$ cycloalkyl, and 4- to 12-membered heterocycloalkyl, wherein the $C_{1-4}$ alkyl, $C_{1-4}$ alkoxy, $C_{3-12}$ cycloalkyl, or 4- to 12-membered heterocycloalkyl is optionally substituted with one or more of the following groups: halogen, =O, -OH, $-NH_2$, -CN, CHO, COOH, $-C_{1-4}$ alkyl-OH, $C_{1-6}$ alkyl OC(O)-, and 4- to 10-membered heterocycloalkyl optionally substituted with $C_{1-6}$ alkyl COC(O)-;
n is selected from the group consisting of 0, 1, 2, and 3.

**22.** The compound, the stereoisomer thereof, or the pharmaceutically acceptable salt thereof according to any one of claims 1-19, wherein the compound is:

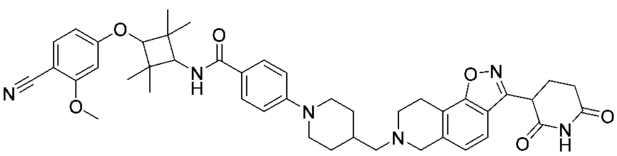
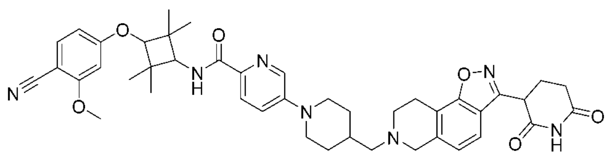
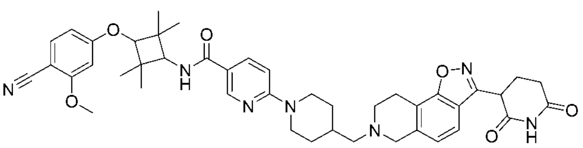
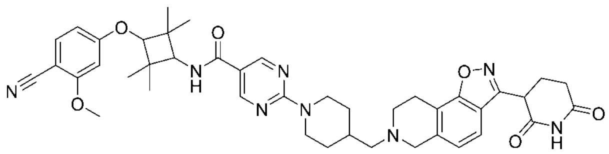
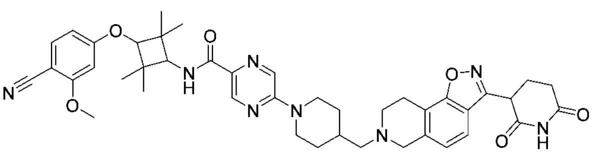
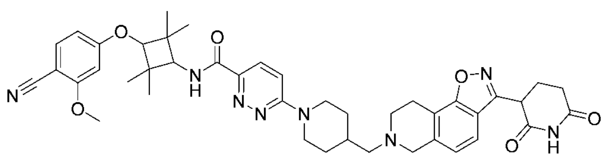
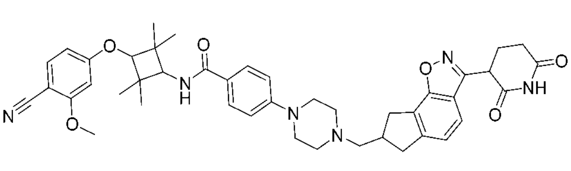
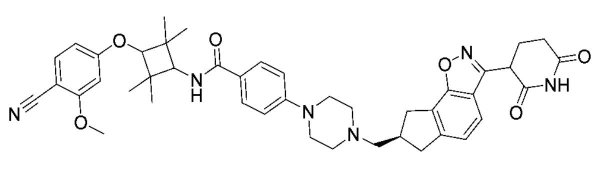

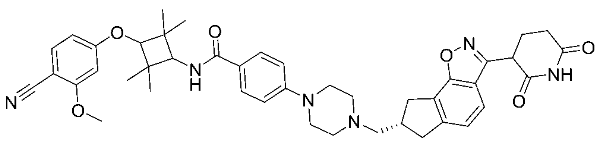
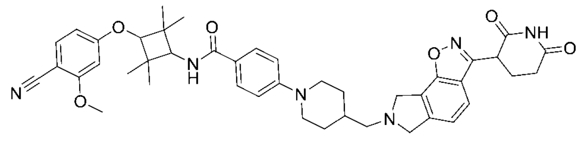
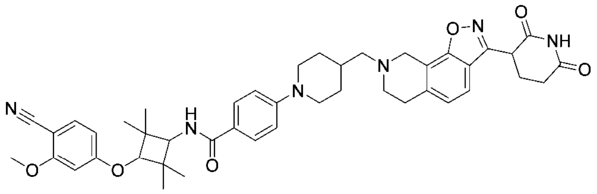
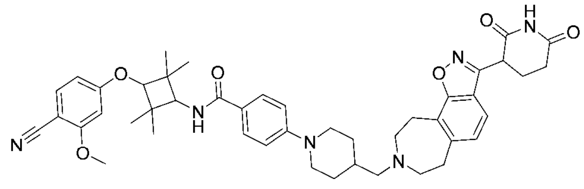
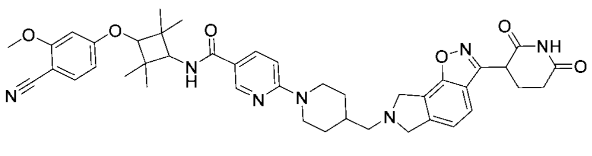
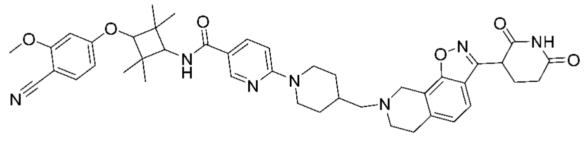
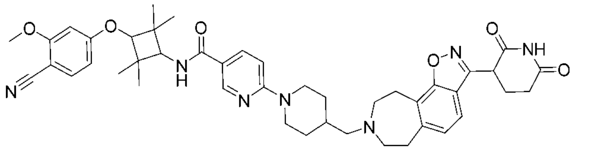
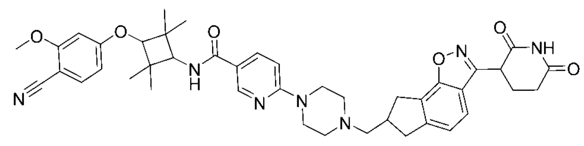
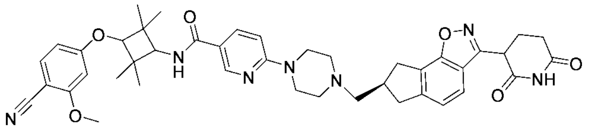
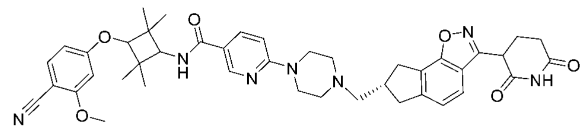
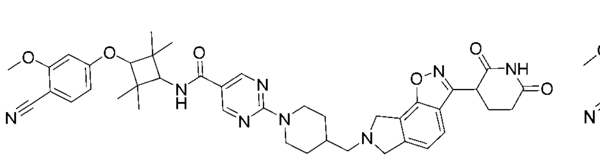
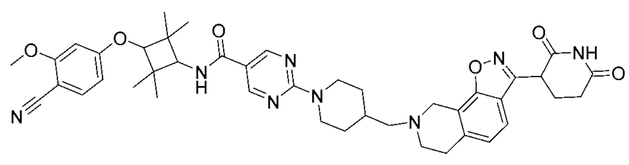
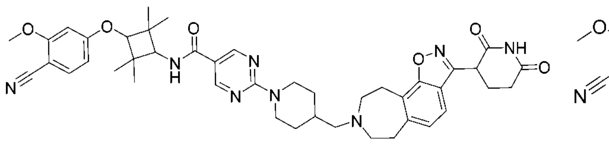
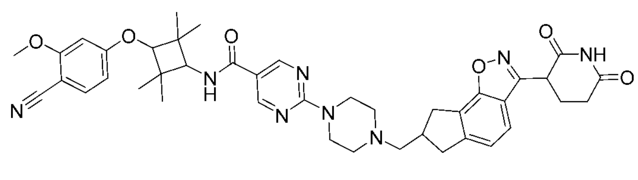
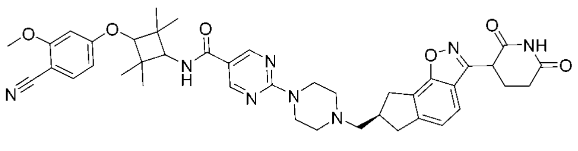
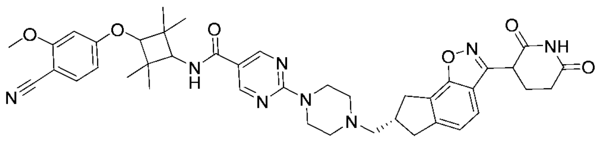
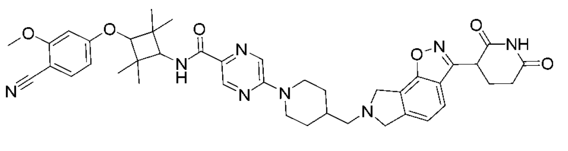
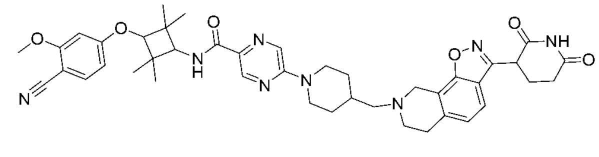

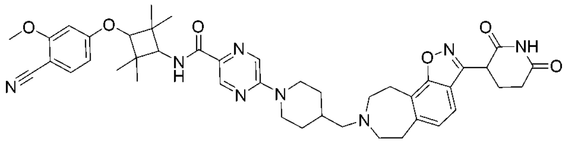
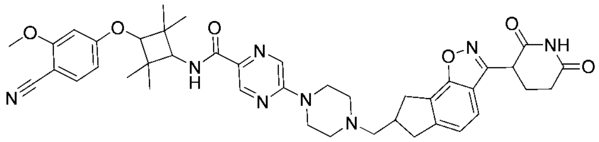

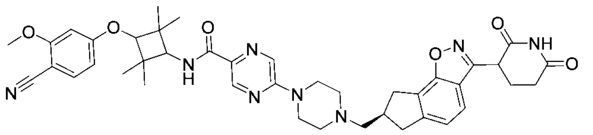
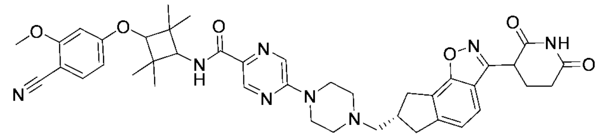

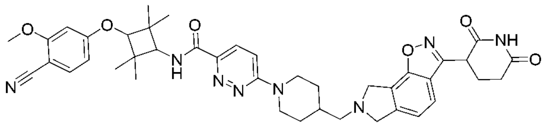
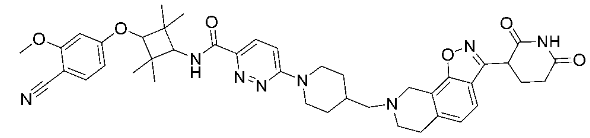

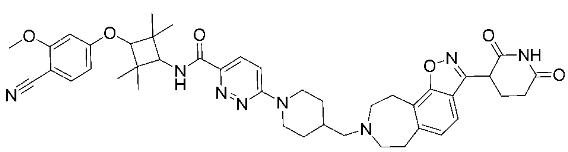
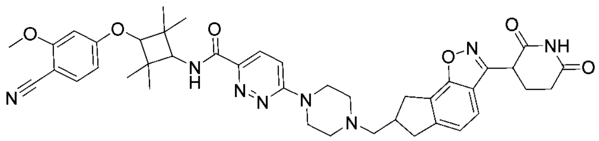

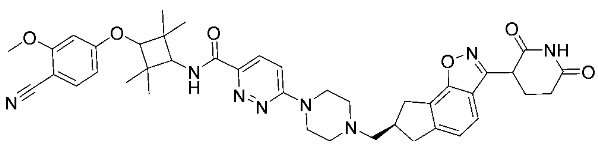
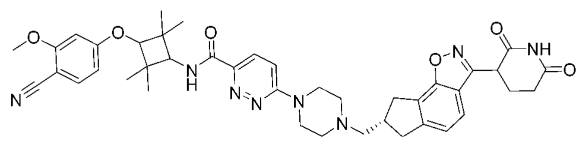

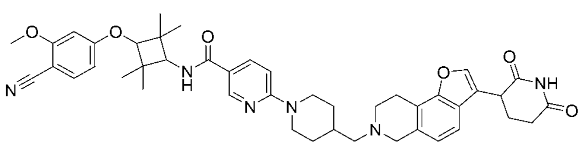
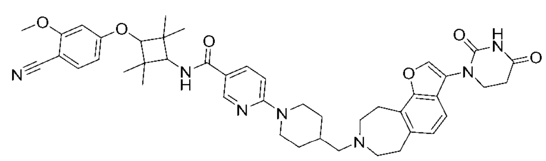

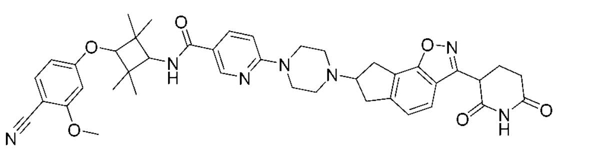
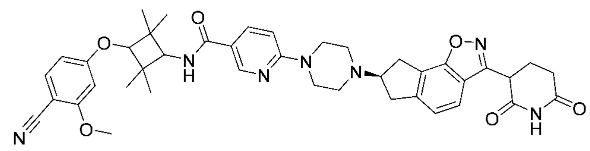

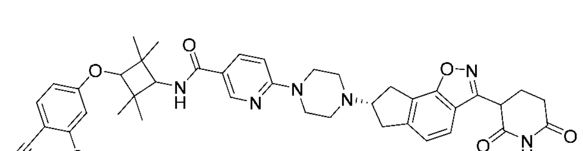
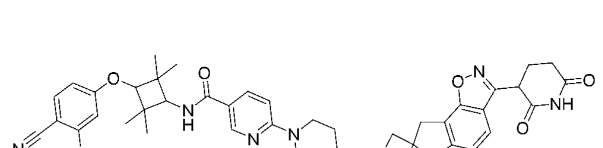

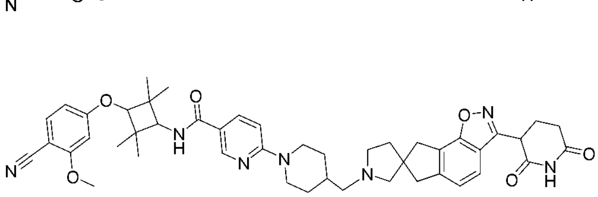
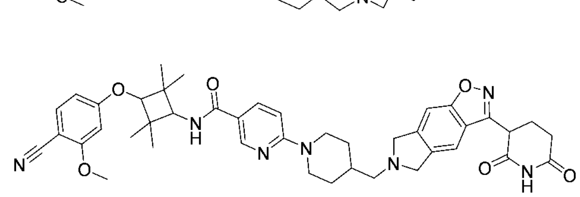

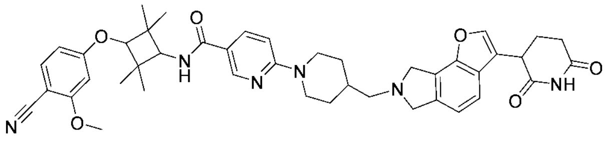

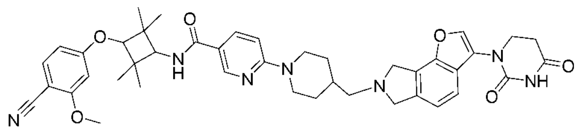

;

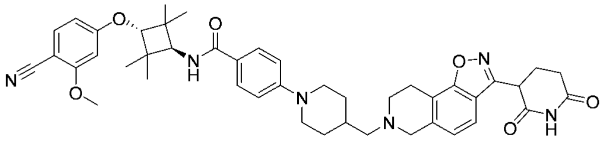

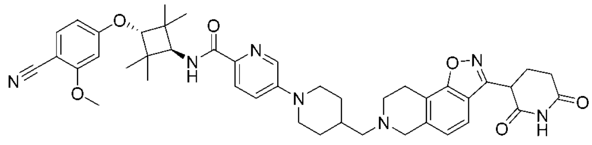

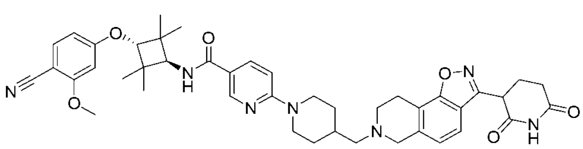

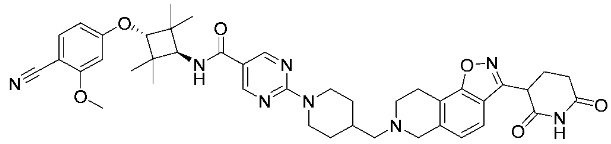

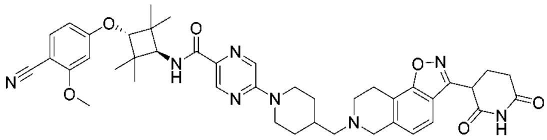

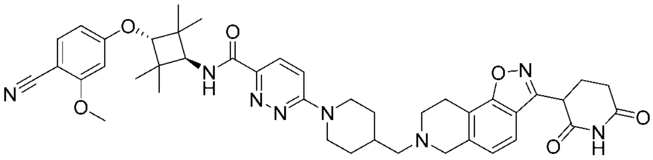

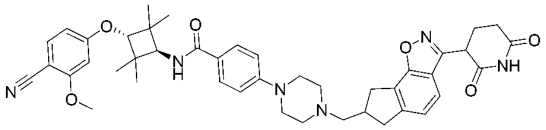

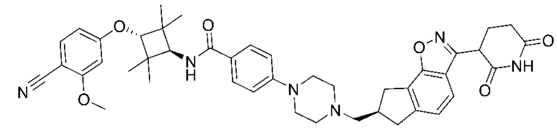

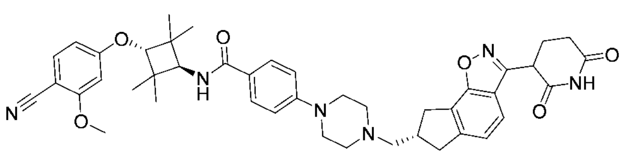

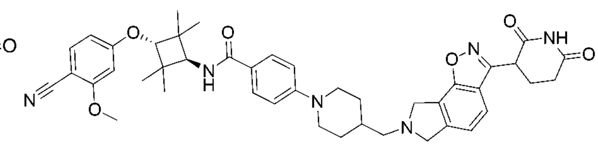

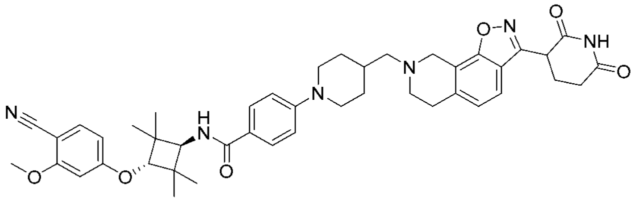

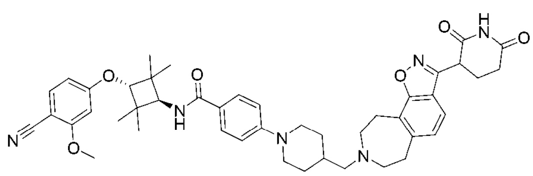

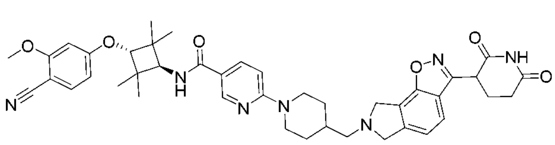

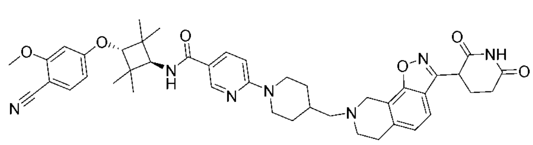

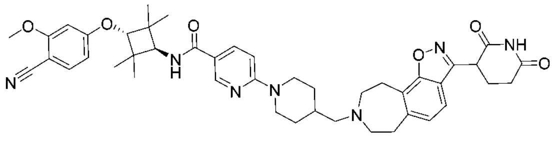

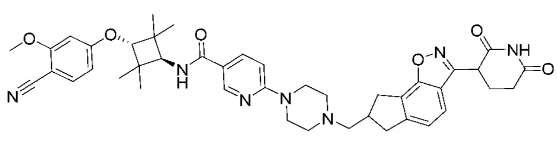

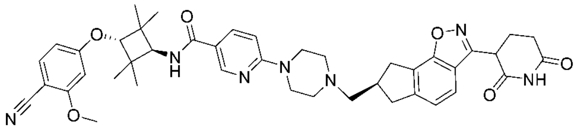
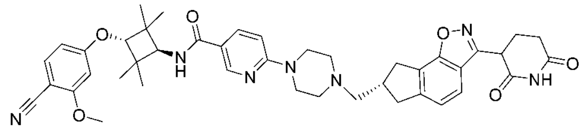

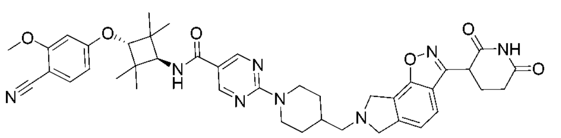
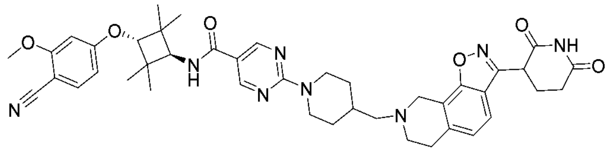

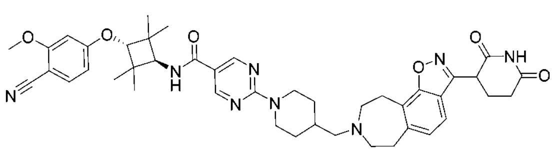
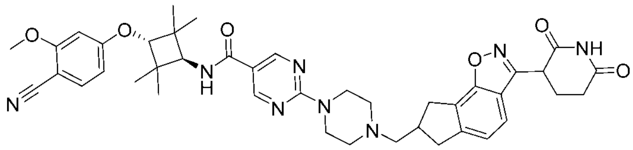

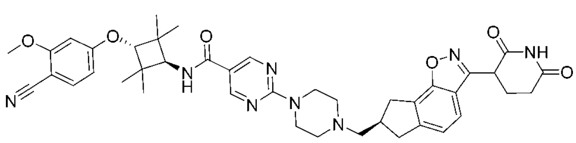
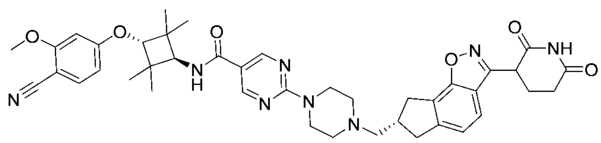

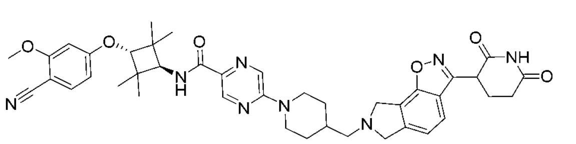
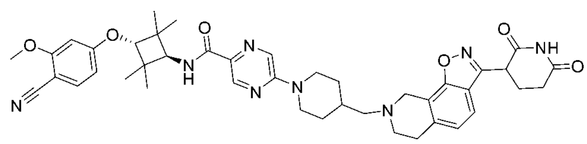

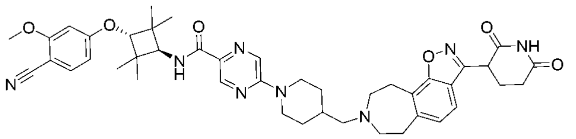
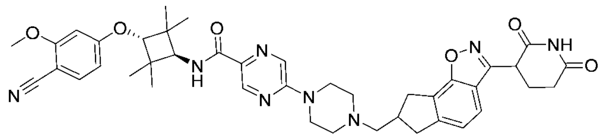

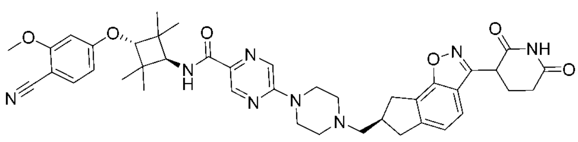
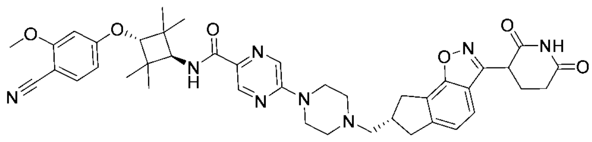

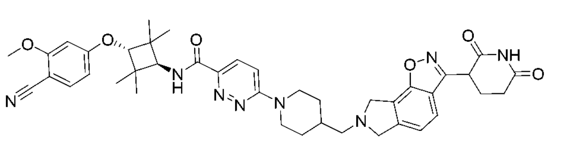
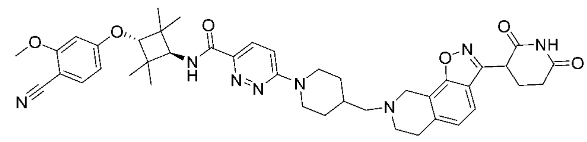

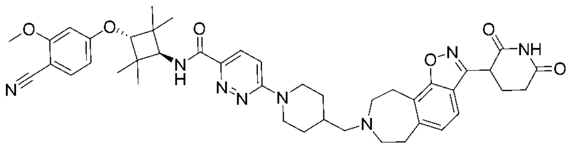
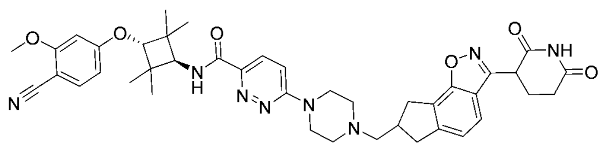

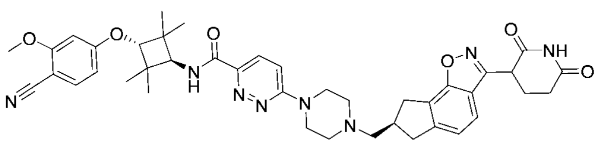
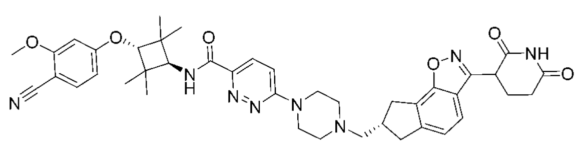

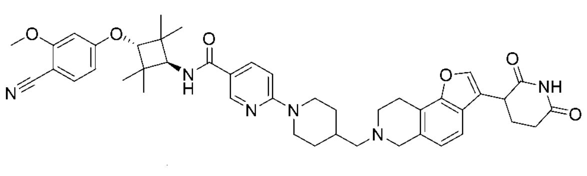

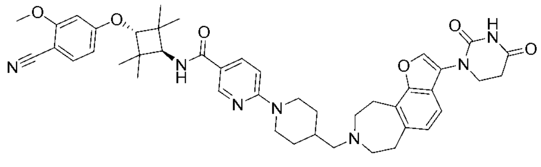

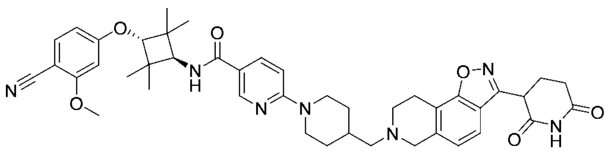

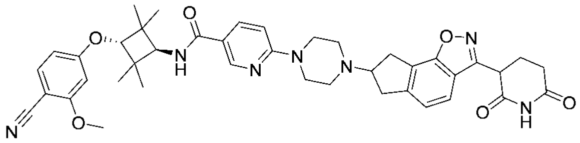

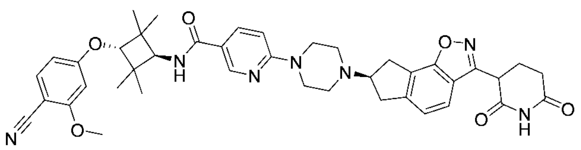

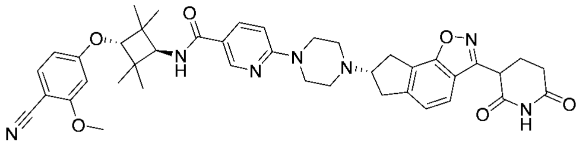

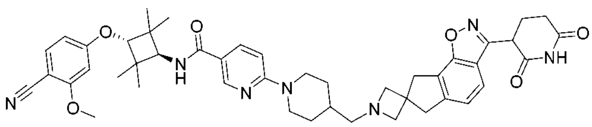

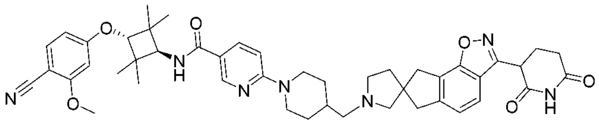

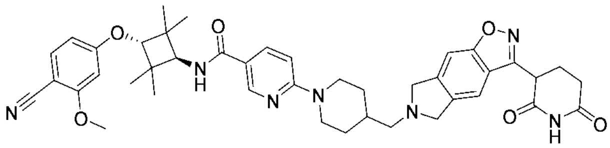

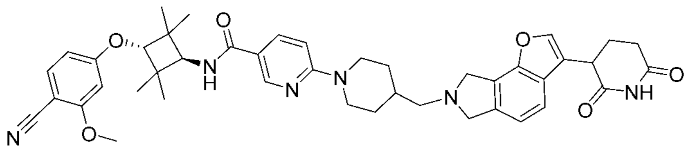

or

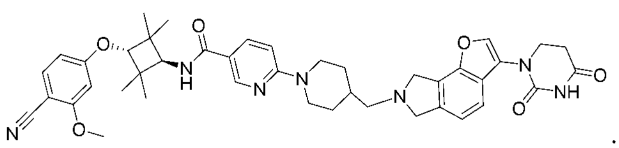

**23.** A pharmaceutical composition, comprising the compound, the stereoisomer thereof, or the pharmaceutically acceptable salt thereof according to any one of claims 1-19 and 22, or the compound, the moiety, the stereoisomer thereof, the derivative, or the pharmaceutically acceptable salt thereof according to claim 20 or 21.

**24.** Use of the compound, the stereoisomer thereof, or the pharmaceutically acceptable salt thereof according to any one of claims 1-19 and 22, or the compound, the moiety, the stereoisomer thereof, the derivative, or the pharmaceutically acceptable salt thereof according to claim 20 or **21,** or the pharmaceutical composition according to claim 23 for preparing a medicament for preventing or treating a disorder treated by degrading a target protein that binds to a targeting ligand.

**25.** Use of the compound, the stereoisomer thereof, or the pharmaceutically acceptable salt thereof according to any one of claims 1-19 and 22, or the compound, the moiety, the stereoisomer thereof, the derivative, or the pharmaceutically acceptable salt thereof according to claim 20 or **21,** or the pharmaceutical composition according to claim 23 for preparing a medicament for preventing or treating a disorder treated by binding to cereblon protein *in vivo.*

**26.** The use according to claim 24 or 25, wherein the disease or disorder is selected from cancer.

**INTERNATIONAL SEARCH REPORT**

International application No.
**PCT/CN2024/113870**

**A. CLASSIFICATION OF SUBJECT MATTER**

C07D498/04(2006.01)i; C07D493/00(2006.01)i; C07D491/00(2006.01)i; A61K31/553(2006.01)i; A61K31/423(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNABS, VEN, CNKI, STN (REGISTRY, CAPLUS): 正大天晴, 雌激素受体, 靶向蛋白降解, 异恶唑, 呋喃, 三环, androgen receptor, PROTAC, +isoxazole+, +tricyclic+, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2024037616 A1 (CHIA TAI TIANQING PHARMACEUTICAL GROUP CO., LTD.) 22 February 2024 (2024-02-22)<br>claims 1-20 | 1-26 |
| X | WO 2022262782 A1 (MEDSHINE DISCOVERY INC.) 22 December 2022 (2022-12-22)<br>claim 11, description, page 1, paragraph 3, page 11, paragraph 2, embodiments 2-3, and reference examples 8-9, 12, 14, 17-19, and 22 | 1-26 |
| X | WO 2023088406 A1 (CHIA TAI TIANQING PHARMACEUTICAL GROUP CO., LTD.) 25 May 2023 (2023-05-25)<br>description, pages 53 and 61, and claims 25-43 | 20-21, 23-26 |
| X | WO 2020048546 A1 (MEDSHINE DISCOVERY INC.) 12 March 2020 (2020-03-12)<br>description, pages 2-13 and 20-90 | 20-21, 24-26 |
| X | WO 2020048548 A1 (CHIA TAI TIANQING PHARMACEUTICAL GROUP CO., LTD. et al.) 12 March 2020 (2020-03-12)<br>description, pages 2-24 and 122-126 | 20-21, 24-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 November 2024** | **14 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/113870**

**C. DOCUMENTS CONSIDERED TO BE RELEVANT**

| Category* | Citation of document, with indication, where appropriate, of the relevant passages | Relevant to claim No. |
|---|---|---|
| X | WO 2022194221 A1 (MEDSHINE DISCOVERY INC.) 22 September 2022 (2022-09-22) claims 1-11 | 1-15, 24-26 |
| A | CN 114641337 A (THE REGENTS OF THE UNIVERSITY OF MICHIGAN) 17 June 2022 (2022-06-17) entire document | 1-26 |

Form PCT/ISA/210 (second sheet) (July 2022)

**INTERNATIONAL SEARCH REPORT**
**Information on patent family members**

International application No.
**PCT/CN2024/113870**

| Patent document cited in search report | Publication date (day/month/year) | Patent family member(s) | Publication date (day/month/year) |
|---|---|---|---|
| WO 2024037616 A1 | 22 February 2024 | TW 202409025 A | 01 March 2024 |
| WO 2022262782 A1 | 22 December 2022 | None | |
| WO 2023088406 A1 | 25 May 2023 | KR 20240107324 A | 09 July 2024 |
| | | IL 312763 A | 01 July 2024 |
| | | MX 2024005702 A | 26 June 2024 |
| | | AU 2022393875 A1 | 13 June 2024 |
| | | TW 202325300 A | 01 July 2023 |
| | | EP 4434992 A1 | 25 September 2024 |
| | | CA 3238118 A1 | 25 May 2023 |
| WO 2020048546 A1 | 12 March 2020 | US 2021317109 A1 | 14 October 2021 |
| | | EP 3848363 A1 | 14 July 2021 |
| | | EP 3848363 A4 | 10 August 2022 |
| | | EP 3848363 B1 | 19 July 2023 |
| | | KR 20210056397 A | 18 May 2021 |
| | | JP 2021535184 A | 16 December 2021 |
| | | JP 7098825 B2 | 11 July 2022 |
| WO 2020048548 A1 | 12 March 2020 | CA 3111649 A1 | 12 March 2020 |
| | | AU 2019334065 A1 | 15 April 2021 |
| | | IL 281296 A | 29 April 2021 |
| | | IL 281296 B1 | 01 April 2024 |
| | | IL 281296 B2 | 01 August 2024 |
| | | EP 3848367 A1 | 14 July 2021 |
| | | EP 3848367 A4 | 08 June 2022 |
| | | BR 112021004269 A2 | 25 May 2021 |
| | | US 2022119376 A1 | 21 April 2022 |
| | | KR 20210057767 A | 21 May 2021 |
| | | JP 2021536480 A | 27 December 2021 |
| | | JP 7422139 B2 | 25 January 2024 |
| | | SG 11202102271 WA | 29 April 2021 |
| WO 2022194221 A1 | 22 September 2022 | BR 112023018839 A2 | 26 December 2023 |
| | | CA 3212104 A1 | 22 September 2022 |
| | | JP 2024511342 A | 13 March 2024 |
| | | ZA 202309588 B | 26 June 2024 |
| | | KR 20230154953 A | 09 November 2023 |
| | | US 2024190851 A1 | 13 June 2024 |
| | | EP 4310083 A1 | 24 January 2024 |
| | | TW 202241404 A | 01 November 2022 |
| | | TWI 807697 B | 01 July 2023 |
| | | AU 2022240688 A1 | 02 November 2023 |
| | | AU 2022240688 B2 | 11 July 2024 |
| CN 114641337 A | 17 June 2022 | CA 3151824 A1 | 04 March 2021 |
| | | WO 2021041664 A1 | 04 March 2021 |
| | | AU 2020336381 A1 | 03 March 2022 |
| | | EP 4021580 A1 | 06 July 2022 |
| | | US 2022388978 A1 | 08 December 2022 |
| | | JP 2022545735 A | 28 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311073391
- CN 202410544791
- CN 202411125992
- WO 2023088406 A
- WO 2024037616 A


### Non-patent literature cited in the description


- Greene's Protective Groups in Organic Synthesis. John Wiley & Sons, Inc